(19) Europäisches Patentamt
European Patent Office
Office européen des brevets

(11) EP 3 858 846 A1

(12) EUROPEAN PATENT APPLICATION
published in accordance with Art. 153(4) EPC

(43) Date of publication:
**04.08.2021 Bulletin 2021/31**

(21) Application number: **19867901.1**

(22) Date of filing: **30.09.2019**

(51) Int Cl.:
***C07H 21/00*** (2006.01) ***A61K 48/00*** (2006.01)
***A61P 35/00*** (2006.01)

(86) International application number:
**PCT/CN2019/109563**

(87) International publication number:
**WO 2020/064017 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2018 CN 201811163404**
**30.09.2018 CN 201811163405**
**30.09.2018 CN 201811163406**
**30.09.2018 CN 201811163407**
**30.09.2018 CN 201811161984**
**30.09.2018 CN 201811161985**
**30.09.2018 CN 201811161967**
**30.09.2018 CN 201811161910**
**30.09.2018 CN 201811162073**

(71) Applicant: **Bai Yao Zhi Da (Beijing) Nanobio Technology Co., Ltd.**
**Beijing 100080 (CN)**

(72) Inventors:
- **WANG, Liyuan**
  **Beijing 100080 (CN)**
- **WANG, Meng**
  **Beijing 100080 (CN)**

(74) Representative: **De Clercq & Partners**
**Edgard Gevaertdreef 10a**
**9830 Sint-Martens-Latem (BE)**

# (54) NUCLEIC ACID NANOCARRIER MEDICAMENT, PREPARATION METHOD THEREFOR, PHARMACEUTICAL COMPOSITION THEREOF, AND USE THEREOF

(57) Provided are a nucleic acid nanocarrier drug, a preparation method thereof, a pharmaceutical composition and an application thereof. The drug includes nucleic acid nanoparticle and a drug, the drug is loaded on the nucleic acid nanoparticle, and the drug includes one or more of tacrine, epirubicin, methotrexate, pirarubicin, daunorubicin, pentafluorouracil, 10-hydroxycamptothecin, aspirin and gemcitabine; and the nucleic acid nanoparticle includes a nucleic acid domain, the nucleic acid domain includes a sequence a, a sequence b and a sequence c, the sequence a includes a sequence al or a sequence in which at least one base is inserted, deleted or substituted in the sequence al, the sequence b includes a sequence b1 or a sequence in which at least one base is inserted, deleted or substituted in the sequence b1, and the sequence c includes a sequence c1 or a sequence in which at least one base is inserted, deleted or substituted in the sequence c1. The nucleic acid nanocarrier drug is a stably delivered targeting drug formed because the nucleic acid domain has better targeting properties after being modified by means of a target head.

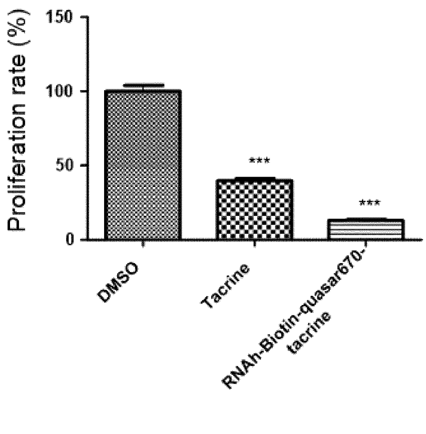


Fig.14

EP 3 858 846 A1

SEQUENCE LISTING

<110> BAI YAO ZHI DA (Beijing) NanoBio Technology Co., Ltd

<120> Nucleic acid nanocarrier drug and preparation method thereof, pharmaceutical composition and application thereof

<130> PN114616BYZD

<141> 2019-09-30

<150> 201811163404.7
<151> 2018-09-30

<150> 201811163405.1
<151> 2018-09-30

<150> 201811163406.6
<151> 2018-09-30

<150> 201811163407.0
<151> 2018-09-30

<150> 201811161984.6
<151> 2018-09-30

<150> 201811161985.0
<151> 2018-09-30

<150> 201811161967.2
<151> 2018-09-30

<150> 201811161910.2
<151> 2018-09-30

<150> 201811162073.5
<151> 2018-09-30

<160> 174

<170> SIPOSequenceListing 1.0

<210> 1
<211> 10
<212> DNA/RNA
<213> Artificial Sequence

<220>
<221> misc_feature
<222> (1)..(10)
<223> a1 sequence

<400> 1
ccagcguucc 10

<210> 2
<211> 10
<212> DNA/RNA
<213> Artificial Sequence

<220>

<221> mRNA
<222> (1)..(10)
<223> a1 sequence

<400> 2
ccagcgttcc 10

<210> 3
<211> 9
<212> DNA/RNA
<213> Artificial Sequence

<220>
<221> misc_feature
<222> (1)..(9)
<223> b1 sequence

<400> 3
gguucgccg 9

<210> 4
<211> 9
<212> DNA/RNA
<213> Artificial Sequence

<220>
<221> misc_feature
<222> (1)..(9)
<223> b1 sequence

<400> 4
ggttcgccg 9

<210> 5
<211> 12
<212> DNA/RNA
<213> Artificial Sequence

<220>
<221> misc_feature
<222> (1)..(12)
<223> c1 sequence

<400> 5
cggccauagc gg 12

<210> 6
<211> 12
<212> DNA/RNA
<213> Artificial Sequence

<220>
<221> misc_feature
<222> (1)..(12)
<223> c1 sequence

<400> 6
cggccatagc gg 12

<210> 7
<211> 10
<212> DNA/RNA
<213> Artificial Sequence

<220>
<221> misc_feature
<222> (1)..(10)
<223> a sequence

<400> 7
ggagcguugg 10

<210> 8
<211> 9
<212> DNA/RNA
<213> Artificial Sequence

<220>
<221> misc_feature
<222> (1)..(9)
<223> b sequence

<400> 8
ccuucgccg 9

<210> 9
<211> 12
<212> DNA/RNA
<213> Artificial Sequence

<220>
<221> misc_feature
<222> (1)..(12)
<223> c sequence

<400> 9
cggccauagc cc 12

<210> 10
<211> 10
<212> DNA/RNA
<213> Artificial Sequence

<220>
<221> misc_feature
<222> (1)..(10)
<223> a sequence

<400> 10
gcagcguucg 10

<210> 11
<211> 9
<212> DNA/RNA
<213> Artificial Sequence

<220>

<221> misc_feature
<222> (1)..(9)
<223> b sequence

<400> 11
cguucgccg 9

<210> 12
<211> 12
<212> DNA/RNA
<213> Artificial Sequence

<220>
<221> misc_feature
<222> (1)..(12)
<223> c sequence

<400> 12
cggccauagc gc 12

<210> 13
<211> 10
<212> DNA/RNA
<213> Artificial Sequence

<220>
<221> misc_feature
<222> (1)..(10)
<223> a sequence

<400> 13
cgagcguugc 10

<210> 14
<211> 9
<212> DNA/RNA
<213> Artificial Sequence

<220>
<221> misc_feature
<222> (1)..(9)
<223> b sequence

<400> 14
gcuucgccg 9

<210> 15
<211> 12
<212> DNA/RNA
<213> Artificial Sequence

<220>
<221> misc_feature
<222> (1)..(12)
<223> c sequence

<400> 15
cggccauagc cg 12

<210> 16
<211> 10
<212> DNA/RNA
<213> Artificial Sequence

<220>
<221> misc_feature
<222> (1)..(10)
<223> a sequence

<400> 16
ggagcguugg 10

<210> 17
<211> 9
<212> DNA/RNA
<213> Artificial Sequence

<220>
<221> misc_feature
<222> (1)..(9)
<223> b sequence

<400> 17
ccuucgggg 9

<210> 18
<211> 12
<212> DNA/RNA
<213> Artificial Sequence

<220>
<221> misc_feature
<222> (1)..(12)
<223> c sequence

<400> 18
cccccauagc cc 12

<210> 19
<211> 10
<212> DNA/RNA
<213> Artificial Sequence

<220>
<221> misc_feature
<222> (1)..(10)
<223> a sequence

<400> 19
gcagcguucg 10

<210> 20
<211> 9
<212> DNA/RNA
<213> Artificial Sequence

<220>

<221> misc_feature
<222> (1)..(9)
<223> b sequence

<400> 20
cguucggcg 9

<210> 21
<211> 12
<212> DNA/RNA
<213> Artificial Sequence

<220>
<221> misc_feature
<222> (1)..(12)
<223> c sequence

<400> 21
cgcccauagc gc 12

<210> 22
<211> 10
<212> DNA/RNA
<213> Artificial Sequence

<220>
<221> misc_feature
<222> (1)..(10)
<223> a sequence

<400> 22
gcagcguucg 10

<210> 23
<211> 9
<212> DNA/RNA
<213> Artificial Sequence

<220>
<221> misc_feature
<222> (1)..(9)
<223> b sequence

<400> 23
cguucggcc 9

<210> 24
<211> 12
<212> DNA/RNA
<213> Artificial Sequence

<220>
<221> misc_feature
<222> (1)..(12)
<223> c sequence

<400> 24
ggcccauagc gc 12

<210> 25
<211> 10
<212> DNA/RNA
<213> Artificial Sequence

<220>
<221> misc_feature
<222> (1)..(10)
<223> a sequence

<400> 25
cgagcguugc 10

<210> 26
<211> 9
<212> DNA/RNA
<213> Artificial Sequence

<220>
<221> misc_feature
<222> (1)..(9)
<223> b sequence

<400> 26
gcuucggcg 9

<210> 27
<211> 12
<212> DNA/RNA
<213> Artificial Sequence

<220>
<221> misc_feature
<222> (1)..(12)
<223> c sequence

<400> 27
cgcccauagc cg 12

<210> 28
<211> 10
<212> DNA/RNA
<213> Artificial Sequence

<220>
<221> misc_feature
<222> (1)..(10)
<223> a sequence

<400> 28
ggagcgttgg 10

<210> 29
<211> 9
<212> DNA/RNA
<213> Artificial Sequence

<220>

<221> misc_feature
<222> (1)..(9)
<223> b sequence

<400> 29
ccttcgccg 9

<210> 30
<211> 12
<212> DNA/RNA
<213> Artificial Sequence

<220>
<221> misc_feature
<222> (1)..(12)
<223> c sequence

<400> 30
cggccatagc cc 12

<210> 31
<211> 10
<212> DNA/RNA
<213> Artificial Sequence

<220>
<221> misc_feature
<222> (1)..(10)
<223> a sequence

<400> 31
gcagcgttcg 10

<210> 32
<211> 9
<212> DNA/RNA
<213> Artificial Sequence

<220>
<221> misc_feature
<222> (1)..(9)
<223> b sequence

<400> 32
cgttcgccg 9

<210> 33
<211> 12
<212> DNA/RNA
<213> Artificial Sequence

<220>
<221> misc_feature
<222> (1)..(12)
<223> c sequence

<400> 33
cggccatagc gc 12

<210> 34
<211> 10
<212> DNA/RNA
<213> Artificial Sequence

<220>
<221> misc_feature
<222> (1)..(10)
<223> a sequence

<400> 34
cgagcgttgc 10

<210> 35
<211> 9
<212> DNA/RNA
<213> Artificial Sequence

<220>
<221> misc_feature
<222> (1)..(9)
<223> b sequence

<400> 35
gcttcgccg 9

<210> 36
<211> 12
<212> DNA/RNA
<213> Artificial Sequence

<220>
<221> misc_feature
<222> (1)..(12)
<223> c sequence

<400> 36
cggccatagc cg 12

<210> 37
<211> 10
<212> DNA/RNA
<213> Artificial Sequence

<220>
<221> misc_feature
<222> (1)..(10)
<223> a sequence

<400> 37
ggagcgttgg 10

<210> 38
<211> 9
<212> DNA/RNA
<213> Artificial Sequence

<220>

<221> misc_feature
<222> (1)..(9)
<223> b sequence

<400> 38
ccttcgggg 9

<210> 39
<211> 12
<212> DNA/RNA
<213> Artificial Sequence

<220>
<221> misc_feature
<222> (1)..(12)
<223> c sequence

<400> 39
cccccatagc cc 12

<210> 40
<211> 10
<212> DNA/RNA
<213> Artificial Sequence

<220>
<221> misc_feature
<222> (1)..(10)
<223> a sequence

<400> 40
gcagcgttcg 10

<210> 41
<211> 9
<212> DNA/RNA
<213> Artificial Sequence

<220>
<221> misc_feature
<222> (1)..(9)
<223> b sequence

<400> 41
cgttcggcg 9

<210> 42
<211> 12
<212> DNA/RNA
<213> Artificial Sequence

<220>
<221> misc_feature
<222> (1)..(12)
<223> c sequence

<400> 42
cgcccatagc gc 12

<210> 43
<211> 10
<212> DNA/RNA
<213> Artificial Sequence

<220>
<221> misc_feature
<222> (1)..(10)
<223> a sequence

<400> 43
gcagcgttcg 10

<210> 44
<211> 9
<212> DNA/RNA
<213> Artificial Sequence

<220>
<221> misc_feature
<222> (1)..(9)
<223> b sequence

<400> 44
cgttcggcc 9

<210> 45
<211> 12
<212> DNA/RNA
<213> Artificial Sequence

<220>
<221> misc_feature
<222> (1)..(12)
<223> c sequence

<400> 45
ggcccatagc gc 12

<210> 46
<211> 10
<212> DNA/RNA
<213> Artificial Sequence

<220>
<221> misc_feature
<222> (1)..(10)
<223> a sequence

<400> 46
cgagcgttgc 10

<210> 47
<211> 9
<212> DNA/RNA
<213> Artificial Sequence

<220>

<221> misc_feature
<222> (1)..(9)
<223> b sequence

<400> 47
gcttcggcg 9

<210> 48
<211> 12
<212> DNA/RNA
<213> Artificial Sequence

<220>
<221> misc_feature
<222> (1)..(12)
<223> c sequence

<400> 48
cgcccatagc cg 12

<210> 49
<211> 77
<212> DNA/RNA
<213> Artificial Sequence

<220>
<221> misc_feature
<222> (1)..(77)
<223> a sequence

<220>
<221> misc_feature
<222> (1)..(77)
<223> m is u or t

<400> 49
cgcgcgaaaa aacgcgcgaa aaaacgcgcg cccaccagcg mmccgggcgc gcgaaaaaac 60
gcgcgaaaaa acgcgcg 77

<210> 50
<211> 75
<212> DNA/RNA
<213> Artificial Sequence

<220>
<221> misc_feature
<222> (1)..(75)
<223> b sequence

<220>
<221> misc_feature
<222> (1)..(75)
<223> m is u or t

<400> 50
cgcgcgmmmm mmcgcgcgmm mmmmcgcgcg cccggmmcgc cgccagccgc cmmmmmmgcc 60
gccmmmmmmg ccgcc 75

<210> 51
<211> 78
<212> DNA/RNA
<213> Artificial Sequence

<220>
<221> misc_feature
<222> (1)..(78)
<223> c sequence

<220>
<221> misc_feature
<222> (1)..(78)
<223> m is u or t

<400> 51
ggcggcaaaa aaggcggcaa aaaaggcggc aggcggcama gcggmgggcg cgcgmmmmmm 60
cgcgcgmmmm mmcgcgcg 78

<210> 52
<211> 29
<212> RNA
<213> Artificial Sequence

<220>
<221> misc_feature
<222> (1)..(29)
<223> a strand

<220>
<221> modified_base
<222> (1)..(1)
<223> 5'Biotin

<400> 52
cgcgcgccca ccagcguucc gggcgccgc 29

<210> 53
<211> 27
<212> RNA
<213> Artificial Sequence

<220>
<221> misc_feature
<222> (1)..(27)
<223> b strand

<220>
<221> modified_base
<222> (1)..(1)
<223> 5'Biotin

<400> 53
gcggcgcccg guucgccgcc aggcggc 27

<210> 54

<211> 31
<212> RNA
<213> Artificial Sequence

<220>
<221> misc_feature
<222> (1)..(31)
<223> c strand

<220>
<221> modified_base
<222> (1)..(1)
<223> 5'CY3

<400> 54
gccgccaggc ggccauagcg gugggcgcgc g 31

<210> 55
<211> 10
<212> RNA
<213> Artificial Sequence

<220>
<221> misc_feature
<222> (1)..(10)
<223> a strand

<400> 55
ggagcguugg 10

<210> 56
<211> 9
<212> RNA
<213> Artificial Sequence

<220>
<221> misc_feature
<222> (1)..(9)
<223> b strand

<400> 56
ccuucgccg 9

<210> 57
<211> 12
<212> RNA
<213> Artificial Sequence

<220>
<221> misc_feature
<222> (1)..(12)
<223> c strand

<400> 57
cggccauagc cc 12

<210> 58
<211> 10

<212> RNA
<213> Artificial Sequence

<220>
<221> misc_feature
<222> (1)..(10)
<223> a strand

<400> 58
gcagcguucg 10

<210> 59
<211> 9
<212> RNA
<213> Artificial Sequence

<220>
<221> misc_feature
<222> (1)..(9)
<223> b strand

<400> 59
cguucgccg 9

<210> 60
<211> 12
<212> RNA
<213> Artificial Sequence

<220>
<221> misc_feature
<222> (1)..(12)
<223> c strand

<400> 60
cggccauagc gc 12

<210> 61
<211> 10
<212> RNA
<213> Artificial Sequence

<220>
<221> misc_feature
<222> (1)..(10)
<223> a strand

<400> 61
cgagcguugc 10

<210> 62
<211> 9
<212> RNA
<213> Artificial Sequence

<220>
<221> misc_feature
<222> (1)..(9)
<223> b strand

<400> 62
gcuucgccg 9

<210> 63
<211> 12
<212> RNA
<213> Artificial Sequence

<220>
<221> misc_feature
<222> (1)..(12)
<223> c strand

<400> 63
cggccauagc cg 12

<210> 64
<211> 10
<212> RNA
<213> Artificial Sequence

<220>
<221> misc_feature
<222> (1)..(10)
<223> a strand

<400> 64
ggagcguugg 10

<210> 65
<211> 9
<212> RNA
<213> Artificial Sequence

<220>
<221> misc_feature
<222> (1)..(9)
<223> b strand

<400> 65
ccuucgggg 9

<210> 66
<211> 12
<212> RNA
<213> Artificial Sequence

<220>
<221> misc_feature
<222> (1)..(12)
<223> c strand

<400> 66
cccccauagc cc 12

<210> 67
<211> 10

<212> RNA
<213> Artificial Sequence

<220>
<221> misc_feature
<222> (1)..(10)
<223> a strand

<400> 67
gcagcguucg 10

<210> 68
<211> 9
<212> RNA
<213> Artificial Sequence

<220>
<221> misc_feature
<222> (1)..(9)
<223> b strand

<400> 68
cguucggcg 9

<210> 69
<211> 12
<212> RNA
<213> Artificial Sequence

<220>
<221> misc_feature
<222> (1)..(12)
<223> c strand

<400> 69
cgcccauagc gc 12

<210> 70
<211> 10
<212> RNA
<213> Artificial Sequence

<220>
<221> misc_feature
<222> (1)..(10)
<223> a strand

<400> 70
gcagcguucg 10

<210> 71
<211> 9
<212> RNA
<213> Artificial Sequence

<220>
<221> misc_feature
<222> (1)..(9)
<223> b strand

<400> 71
cguucggcc 9

<210> 72
<211> 12
<212> RNA
<213> Artificial Sequence

<220>
<221> misc_feature
<222> (1)..(12)
<223> c strand

<400> 72
ggcccauagc gc 12

<210> 73
<211> 10
<212> RNA
<213> Artificial Sequence

<220>
<221> misc_feature
<222> (1)..(10)
<223> a strand

<400> 73
cgagcguugc 10

<210> 74
<211> 9
<212> RNA
<213> Artificial Sequence

<220>
<221> misc_feature
<222> (1)..(9)
<223> b strand

<400> 74
gcuucggcg 9

<210> 75
<211> 12
<212> RNA
<213> Artificial Sequence

<220>
<221> misc_feature
<222> (1)..(12)
<223> c strand

<400> 75
cgcccauagc cg 12

<210> 76
<211> 29

<212> RNA
<213> Artificial Sequence

<220>
<221> misc_feature
<222> (1)..(29)
<223> a strand

<400> 76
cgcgcgccca ggagcguugg cgggcggcg 29

<210> 77
<211> 27
<212> RNA
<213> Artificial Sequence

<220>
<221> misc_feature
<222> (1)..(27)
<223> b strand

<400> 77
cgccgcccgc cuucgccgcc agccgcc 27

<210> 78
<211> 31
<212> RNA
<213> Artificial Sequence

<220>
<221> misc_feature
<222> (1)..(31)
<223> c strand

<400> 78
ggcggcaggc ggccauagcc cugggcgcgc g 31

<210> 79
<211> 29
<212> RNA
<213> Artificial Sequence

<220>
<221> misc_feature
<222> (1)..(29)
<223> a strand

<400> 79
cgcgcgccca gcagcguucg cgggcggcg 29

<210> 80
<211> 27
<212> RNA
<213> Artificial Sequence

<220>
<221> misc_feature
<222> (1)..(27)
<223> b strand

<400> 80
cgccgcccgc guucgccgcc agccgcc 27

<210> 81
<211> 31
<212> RNA
<213> Artificial Sequence

<220>
<221> misc_feature
<222> (1)..(31)
<223> c strand

<400> 81
ggcggcaggc ggccauagcg cugggcgcgc g 31

<210> 82
<211> 29
<212> RNA
<213> Artificial Sequence

<220>
<221> misc_feature
<222> (1)..(29)
<223> a strand

<400> 82
cgcgcgccca cgagcguugc ggggcggcg 29

<210> 83
<211> 27
<212> RNA
<213> Artificial Sequence

<220>
<221> misc_feature
<222> (1)..(27)
<223> b strand

<400> 83
cgccgccccg cuucgccgcc agccgcc 27

<210> 84
<211> 31
<212> RNA
<213> Artificial Sequence

<220>
<221> misc_feature
<222> (1)..(31)
<223> c strand

<400> 84
ggcggcaggc ggccauagcc gugggcgcgc g 31

<210> 85
<211> 29

<212> RNA
<213> Artificial Sequence

<220>
<221> misc_feature
<222> (1)..(29)
<223> a strand

<400> 85
cgcgcgccca ggagcguugg cccgcggcg 29

<210> 86
<211> 27
<212> RNA
<213> Artificial Sequence

<220>
<221> misc_feature
<222> (1)..(27)
<223> b strand

<400> 86
cgccgcgggc cuucggggcc agccgcc 27

<210> 87
<211> 31
<212> RNA
<213> Artificial Sequence

<220>
<221> misc_feature
<222> (1)..(31)
<223> c strand

<400> 87
ggcggcaggc ccccauagcc cugggcgcgc g 31

<210> 88
<211> 29
<212> RNA
<213> Artificial Sequence

<220>
<221> misc_feature
<222> (1)..(29)
<223> a strand

<400> 88
cgcgcgccca gcagcguucg ccccgccgc 29

<210> 89
<211> 27
<212> RNA
<213> Artificial Sequence

<220>
<221> misc_feature
<222> (1)..(27)
<223> b strand

<400> 89
gcggcggggc guucggcggc aggcggc 27

<210> 90
<211> 31
<212> RNA
<213> Artificial Sequence

<220>
<221> misc_feature
<222> (1)..(31)
<223> c strand

<400> 90
gccgccagcc gcccauagcg cugggcgcgc g 31

<210> 91
<211> 29
<212> RNA
<213> Artificial Sequence

<220>
<221> misc_feature
<222> (1)..(29)
<223> a strand

<400> 91
cgcgcgccca gcagcguucg gggcgccgc 29

<210> 92
<211> 28
<212> RNA
<213> Artificial Sequence

<220>
<221> misc_feature
<222> (1)..(28)
<223> b strand

<400> 92
gcggcgcccc guucggccgg caggcggc 28

<210> 93
<211> 32
<212> RNA
<213> Artificial Sequence

<220>
<221> misc_feature
<222> (1)..(32)
<223> c strand

<400> 93
gccgccagcc ggcccauagc gcugggcgcg cg 32

<210> 94
<211> 40

<212> DNA/RNA
<213> Artificial Sequence

<220>
<221> misc_feature
<222> (1)..(40)
<223> a strand

<400> 94
cgcgcgcgag cguugcaaug acagauaagg aaccugcutt 40

<210> 95
<211> 36
<212> RNA
<213> Artificial Sequence

<220>
<221> misc_feature
<222> (1)..(36)
<223> b strand

<400> 95
ggcagguucc uuaucuguca aagcuucggc ggcagc 36

<210> 96
<211> 23
<212> RNA
<213> Artificial Sequence

<220>
<221> misc_feature
<222> (1)..(23)
<223> c strand

<400> 96
gcagccgccc auagccgcgc gcg 23

<210> 97
<211> 39
<212> DNA
<213> Artificial Sequence

<220>
<221> misc_feature
<222> (1)..(39)
<223> EGFRapt

<400> 97
gccttagtaa cgtgctttga tgtcgattcg acaggaggc 39

<210> 98
<211> 41
<212> DNA
<213> Artificial Sequence

<220>
<221> misc_feature
<222> (1)..(41)
<223> PSMAapt

<400>  98
gggccgaaaa agacctgact tctatactaa gtctacgtcc c                          41

<210>  99
<211>  68
<212>  DNA
<213>  Artificial Sequence

<220>
<221>  misc_feature
<222>  (1)..(68)
<223>  a strand

<400>  99
cgcgcgccca ggagcgttgg cgggcggcgg ccttagtaac gtgctttgat gtcgattcga      60
caggaggc                                                                68

<210>  100
<211>  27
<212>  DNA
<213>  Artificial Sequence

<220>
<221>  misc_feature
<222>  (1)..(27)
<223>  b strand

<400>  100
cgccgcccgc cttcgccgcc agccgcc                                           27

<210>  101
<211>  31
<212>  DNA
<213>  Artificial Sequence

<220>
<221>  misc_feature
<222>  (1)..(31)
<223>  c strand

<400>  101
ggcggcaggc ggccatagcc ctgggcgcgc g                                      31

<210>  102
<211>  68
<212>  DNA
<213>  Artificial Sequence

<220>
<221>  misc_feature
<222>  (1)..(68)
<223>  a strand

<400>  102
cgcgcgccca gcagcgttcg cgggcggcgg ccttagtaac gtgctttgat gtcgattcga      60
caggaggc                                                                68

<210> 103
<211> 27
<212> DNA
<213> Artificial Sequence

<220>
<221> misc_feature
<222> (1)..(27)
<223> b strand

<400> 103
cgccgcccgc gttcgccgcc agccgcc 27

<210> 104
<211> 31
<212> DNA
<213> Artificial Sequence

<220>
<221> misc_feature
<222> (1)..(31)
<223> c strand

<400> 104
ggcggcaggc ggccatagcg ctgggcgcgc g 31

<210> 105
<211> 68
<212> DNA
<213> Artificial Sequence

<220>
<221> misc_feature
<222> (1)..(68)
<223> a strand

<400> 105
cgcgcgccca cgagcgttgc ggggcggcgg ccttagtaac gtgctttgat gtcgattcga 60
caggaggc 68

<210> 106
<211> 27
<212> DNA
<213> Artificial Sequence

<220>
<221> misc_feature
<222> (1)..(27)
<223> b strand

<400> 106
cgccgccccg cttcgccgcc agccgcc 27

<210> 107
<211> 31
<212> DNA
<213> Artificial Sequence

<220>

<221> misc_feature
<222> (1)..(31)
<223> c strand

<400> 107
ggcggcaggc ggccatagcc gtgggcgcgc g 31

<210> 108
<211> 71
<212> DNA
<213> Artificial Sequence

<220>
<221> misc_feature
<222> (1)..(71)
<223> a strand

<400> 108
cgcgcgccca ggagcgttgg cccgcggcgt gggccgaaaa agacctgact tctatactaa 60
gtctacgtcc c 71

<210> 109
<211> 27
<212> DNA
<213> Artificial Sequence

<220>
<221> misc_feature
<222> (1)..(27)
<223> b strand

<400> 109
cgccgcgggc cttcggggcc agccgcc 27

<210> 110
<211> 31
<212> DNA
<213> Artificial Sequence

<220>
<221> misc_feature
<222> (1)..(31)
<223> c strand

<400> 110
ggcggcaggc ccccatagcc ctgggcgcgc g 31

<210> 111
<211> 71
<212> DNA
<213> Artificial Sequence

<220>
<221> misc_feature
<222> (1)..(71)
<223> a strand

<400> 111

cgcgcgccca gcagcgttcg ccccgccgct gggccgaaaa agacctgact tctatactaa 60
gtctacgtcc c 71

<210> 112
<211> 27
<212> DNA
<213> Artificial Sequence

<220>
<221> misc_feature
<222> (1)..(27)
<223> b strand

<400> 112
gcggcggggc gttcggcggc aggcggc 27

<210> 113
<211> 31
<212> DNA
<213> Artificial Sequence

<220>
<221> misc_feature
<222> (1)..(31)
<223> c strand

<400> 113
gccgccagcc gcccatagcg ctgggcgcgc g 31

<210> 114
<211> 29
<212> DNA
<213> Artificial Sequence

<220>
<221> misc_feature
<222> (1)..(29)
<223> a strand

<400> 114
cgcgcgccca gcagcgttcg ggcgccgc 29

<210> 115
<211> 28
<212> DNA
<213> Artificial Sequence

<220>
<221> misc_feature
<222> (1)..(20)
<223> b strand

<400> 115
gcggcgcccc gttcggccgg caggcggc 28

<210> 116
<211> 32
<212> DNA
<213> Artificial Sequence

<220>
<221>  misc_feature
<222>  (1)..(32)
<223>  c strand

<400>  116
gccgccagcc ggcccatagc gctgggcgcg cg    32

<210>  117
<211>  29
<212>  DNA
<213>  Artificial Sequence

<220>
<221>  misc_feature
<222>  (1)..(29)
<223>  a strand

<400>  117
cgcgcgccca cgagcgttgc gggcgccgc    29

<210>  118
<211>  27
<212>  DNA
<213>  Artificial Sequence

<220>
<221>  misc_feature
<222>  (1)..(27)
<223>  b strand

<400>  118
gcggcgcccg cttcggcggc aggcggc    27

<210>  119
<211>  31
<212>  DNA
<213>  Artificial Sequence

<220>
<221>  misc_feature
<222>  (1)..(31)
<223>  c strand

<400>  119
gccgccagcc gcccatagcc gtgggcgcgc g    31

<210>  120
<211>  37
<212>  RNA
<213>  Artificial Sequence

<220>
<221>  misc_feature
<222>  (1)..(37)
<223>  a strand

<400> 120
gcggcgagcg gcgaggagcg uuggggccgg aggccgg 37

<210> 121
<211> 31
<212> RNA
<213> Artificial Sequence

<220>
<221> misc_feature
<222> (1)..(37)
<223> b strand

<400> 121
ccggccuccg gccccuucgg ggccagccgc c 31

<210> 122
<211> 35
<212> RNA
<213> Artificial Sequence

<220>
<221> misc_feature
<222> (1)..(35)
<223> c strand

<400> 122
ggcggcaggc ccccauagcc cucgccgcuc gccgc 35

<210> 123
<211> 37
<212> RNA
<213> Artificial Sequence

<220>
<221> misc_feature
<222> (1)..(37)
<223> a strand

<400> 123
gcggcgagcg gcgagcagcg uucgggccgg aggccgg 37

<210> 124
<211> 31
<212> RNA
<213> Artificial Sequence

<220>
<221> misc_feature
<222> (1)..(31)
<223> b strand

<400> 124
ccggccuccg gcccguucgc cgccagccgc c 31

<210> 125
<211> 35
<212> RNA
<213> Artificial Sequence

<220>
<221> misc_feature
<222> (1)..(35)
<223> c strand

<400> 125
ggcggcaggc ggccauagcg cucgccgcuc gccgc 35

<210> 126
<211> 37
<212> RNA
<213> Artificial Sequence

<220>
<221> misc_feature
<222> (1)..(37)
<223> a strand

<400> 126
gcggcgagcg gcgaggagcg uuggggccgg aggccgg 37

<210> 127
<211> 31
<212> RNA
<213> Artificial Sequence

<220>
<221> misc_feature
<222> (1)..(31)
<223> b strand

<400> 127
ccggccuccg gccccuucgc cgccagccgc c 31

<210> 128
<211> 35
<212> RNA
<213> Artificial Sequence

<220>
<221> misc_feature
<222> (1)..(35)
<223> c strand

<400> 128
ggcggcaggc ggccauagcc cucgccgcuc gccgc 35

<210> 129
<211> 37
<212> RNA
<213> Artificial Sequence

<220>
<221> misc_feature
<222> (1)..(37)
<223> a strand

<400> 129
gcggcgagcg gcgagcagcg uucgggccgg aggccgg 37

<210> 130
<211> 31
<212> RNA
<213> Artificial Sequence

<220>
<221> misc_feature
<222> (1)..(31)
<223> b strand

<400> 130
ccggccuccg gcccguucgg cgccagccgc c 31

<210> 131
<211> 35
<212> RNA
<213> Artificial Sequence

<220>
<221> misc_feature
<222> (1)..(35)
<223> c strand

<400> 131
ggcggcaggc gcccauagcg cucgccgcuc gccgc 35

<210> 132
<211> 37
<212> RNA
<213> Artificial Sequence

<220>
<221> misc_feature
<222> (1)..(37)
<223> a strand

<400> 132
gcggcgagcg gcgagcagcg uucgggccgg aggccgg 37

<210> 133
<211> 31
<212> RNA
<213> Artificial Sequence

<220>
<221> misc_feature
<222> (1)..(31)
<223> b strand

<400> 133
ccggccuccg gcccguucgg ccccagccgc c 31

<210> 134
<211> 35
<212> RNA
<213> Artificial Sequence

<220>
<221> misc_feature
<222> (1)..(35)
<223> c strand

<400> 134
ggcggcaggg gcccauagcg cucgccgcuc gccgc 35

<210> 135
<211> 37
<212> RNA
<213> Artificial Sequence

<220>
<221> misc_feature
<222> (1)..(37)
<223> a strand

<400> 135
gcggcgagcg gcgacgagcg uugcggccgg aggccgg 37

<210> 136
<211> 31
<212> RNA
<213> Artificial Sequence

<220>
<221> misc_feature
<222> (1)..(31)
<223> b strand

<400> 136
ccggccuccg gccgcuucgc cgccagccgc c 31

<210> 137
<211> 35
<212> RNA
<213> Artificial Sequence

<220>
<221> misc_feature
<222> (1)..(35)
<223> c strand

<400> 137
ggcggcaggc ggccauagcc gucgccgcuc gccgc 35

<210> 138
<211> 37
<212> RNA
<213> Artificial Sequence

<220>
<221> misc_feature
<222> (1)..(37)
<223> a strand

<400> 138
gcggcgagcg gcgacgagcg uugcggccgg aggccgg 37

<210> 139
<211> 31
<212> RNA
<213> Artificial Sequence

<220>
<221> misc_feature
<222> (1)..(31)
<223> b strand

<400> 139
ccggccuccg gccgcuucgg cgccagccgc c 31

<210> 140
<211> 35
<212> RNA
<213> Artificial Sequence

<220>
<221> misc_feature
<222> (1)..(35)
<223> c strand

<400> 140
ggcggcaggc gcccauagcc gucgccgcuc gccgc 35

<210> 141
<211> 37
<212> DNA
<213> Artificial Sequence

<220>
<221> misc_feature
<222> (1)..(37)
<223> a strand

<400> 141
gcggcgagcg gcgaggagcg ttggggccgg aggccgg 37

<210> 142
<211> 31
<212> DNA
<213> Artificial Sequence

<220>
<221> misc_feature
<222> (1)..(31)
<223> b strand

<400> 142
ccggcctccg gccccttcgg ggccagccgc c 31

<210> 143
<211> 35
<212> DNA
<213> Artificial Sequence

<220>
<221> misc_feature
<222> (1)..(35)
<223> c strand

<400> 143
ggcggcaggc ccccatagcc ctcgccgctc gccgc 35

<210> 144
<211> 37
<212> DNA
<213> Artificial Sequence

<220>
<221> misc_feature
<222> (1)..(37)
<223> a strand

<400> 144
gcggcgagcg gcgagcagcg ttcgggccgg aggccgg 37

<210> 145
<211> 31
<212> DNA
<213> Artificial Sequence

<220>
<221> misc_feature
<222> (1)..(31)
<223> b strand

<400> 145
ccggcctccg gcccgttcgc cgccagccgc c 31

<210> 146
<211> 35
<212> DNA
<213> Artificial Sequence

<220>
<221> misc_feature
<222> (1)..(35)
<223> c strand

<400> 146
ggcggcaggc ggccatagcg ctcgccgctc gccgc 35

<210> 147
<211> 37
<212> DNA
<213> Artificial Sequence

<220>
<221> misc_feature
<222> (1)..(37)
<223> a strand

<400> 147
gcggcgagcg gcgaggagcg ttggggccgg aggccgg 37

<210> 148
<211> 31
<212> DNA
<213> Artificial Sequence

<220>
<221> misc_feature
<222> (1)..(31)
<223> b strand

<400> 148
ccggcctccg gccccttcgc cgccagccgc c 31

<210> 149
<211> 35
<212> DNA
<213> Artificial Sequence

<220>
<221> misc_feature
<222> (1)..(35)
<223> c strand

<400> 149
ggcggcaggc ggccatagcc ctcgccgctc gccgc 35

<210> 150
<211> 37
<212> DNA
<213> Artificial Sequence

<220>
<221> misc_feature
<222> (1)..(37)
<223> a strand

<400> 150
gcggcgagcg gcgagcagcg ttcgggccgg aggccgg 37

<210> 151
<211> 31
<212> DNA
<213> Artificial Sequence

<220>
<221> misc_feature
<222> (1)..(31)
<223> b strand

<400> 151
ccggcctccg gcccgttcgg cgccagccgc c 31

<210> 152
<211> 35
<212> DNA
<213> Artificial Sequence

<220>
<221> misc_feature
<222> (1)..(35)
<223> c strand

<400> 152
ggcggcaggc gcccatagcg ctcgccgctc gccgc 35

<210> 153
<211> 37
<212> DNA
<213> Artificial Sequence

<220>
<221> misc_feature
<222> (1)..(37)
<223> a strand

<400> 153
gcggcgagcg gcgagcagcg ttcgggccgg aggccgg 37

<210> 154
<211> 31
<212> DNA
<213> Artificial Sequence

<220>
<221> misc_feature
<222> (1)..(31)
<223> b strand

<400> 154
ccggcctccg gcccgttcgg ccccagccgc c 31

<210> 155
<211> 35
<212> DNA
<213> Artificial Sequence

<220>
<221> misc_feature
<222> (1)..(35)
<223> c strand

<400> 155
ggcggcaggg gcccatagcg ctcgccgctc gccgc 35

<210> 156
<211> 37
<212> DNA
<213> Artificial Sequence

<220>
<221> misc_feature
<222> (1)..(37)
<223> a strand

<400>  156
gcggcgagcg gcgacgagcg ttgcggccgg aggccgg                                37

<210>  157
<211>  31
<212>  DNA
<213>  Artificial Sequence

<220>
<221>  misc_feature
<222>  (1)..(31)
<223>  b strand

<400>  157
ccggcctccg gccgcttcgc cgccagccgc c                                31

<210>  158
<211>  35
<212>  DNA
<213>  Artificial Sequence

<220>
<221>  misc_feature
<222>  (1)..(35)
<223>  c strand

<400>  158
ggcggcaggc ggccatagcc gtcgccgctc gccgc                                35

<210>  159
<211>  37
<212>  DNA
<213>  Artificial Sequence

<220>
<221>  misc_feature
<222>  (1)..(37)
<223>  a strand

<400>  159
gcggcgagcg gcgacgagcg ttgcggccgg aggccgg                                37

<210>  160
<211>  31
<212>  DNA
<213>  Artificial Sequence

<220>
<221>  misc_feature
<222>  (1)..(31)
<223>  b strand

<400>  160
ccggcctccg gccgcttcgg cgccagccgc c                                31

<210>  161
<211>  35
<212>  DNA
<213>  Artificial Sequence

<220>
<221> misc_feature
<222> (1)..(35)
<223> c strand

<400> 161
ggcggcaggc gcccatagcc gtcgccgctc gccgc 35

<210> 162
<211> 14
<212> DNA/RNA
<213> Artificial Sequence

<220>
<221> misc_feature
<222> (1)..(14)
<223> first elongating fragment

<400> 162
gcggcgagcg gcga 14

<210> 163
<211> 14
<212> DNA/RNA
<213> Artificial Sequence

<220>
<221> misc_feature
<222> (1)..(14)
<223> first elongating fragment

<400> 163
ucgccgcucg ccgc 14

<210> 164
<211> 13
<212> DNA/RNA
<213> Artificial Sequence

<220>
<221> misc_feature
<222> (1)..(13)
<223> first elongating fragment

<400> 164
ggccggaggc cgg 13

<210> 165
<211> 13
<212> DNA/RNA
<213> Artificial Sequence

<220>
<221> misc_feature
<222> (1)..(13)
<223> first elongating fragment

<400> 165
ccggccuccg gcc 13

<210> 166
<211> 9
<212> DNA/RNA
<213> Artificial Sequence

<220>
<221> misc_feature
<222> (1)..(9)
<223> first elongating fragment

<400> 166
ccagccgcc 9

<210> 167
<211> 9
<212> DNA/RNA
<213> Artificial Sequence

<220>
<221> misc_feature
<222> (1)..(9)
<223> first elongating fragment

<400> 167
ggcggcagg 9

<210> 168
<211> 14
<212> DNA/RNA
<213> Artificial Sequence

<220>
<221> misc_feature
<222> (1)..(14)
<223> first elongating fragment

<400> 168
gcggcgagcg gcga 14

<210> 169
<211> 14
<212> DNA/RNA
<213> Artificial Sequence

<220>
<221> misc_feature
<222> (1)..(14)
<223> first elongating fragment

<400> 169
tcgccgctcg ccgc 14

<210> 170
<211> 13
<212> DNA/RNA
<213> Artificial Sequence

<220>
<221> misc_feature
<222> (1)..(13)
<223> first elongating fragment

<400> 170
ggccggaggc cgg 13

<210> 171
<211> 13
<212> DNA/RNA
<213> Artificial Sequence

<220>
<221> misc_feature
<222> (1)..(13)
<223> first elongating fragment

<400> 171
ccggcctccg gcc 13

<210> 172
<211> 65
<212> DNA
<213> Artificial Sequence

<220>
<221> misc_feature
<222> (1)..(65)
<223> a strand

<400> 172
cgcgcgccca cgagcgttcc gggcgcgcct tagtaacgtg ctttgatgtc gattcgacag 60
gaggc 65

<210> 173
<211> 26
<212> DNA
<213> Artificial Sequence

<220>
<221> misc_feature
<222> (1)..(26)
<223> b strand

<400> 173
gcgcccggtt cgccgccagc cgccgc 26

<210> 174
<211> 33
<212> DNA
<213> Artificial Sequence

<220>
<221> misc_feature
<222> (1)..(33)
<223> c strand

<400> 174
gcggcggcag gcggccatag ccgtgggcgc gcg 33

## Description

### Technical Field

The present application relates to the field of medicines, and in particular to a nucleic acid nanocarrier drug, a preparation method thereof, a pharmaceutical composition and an application thereof.

### Background

In order to alleviate side effects caused by poor targeting of active ingredients of a drug, a drug delivery carrier is generated at the right moment, and a function thereof is to carry the active ingredients of the drug, and deliver the active ingredients into blood or tissue cells so as to treat diseases. There are already a variety of methods to achieve the targeted delivery of the different drugs. It may be achieved with instruments or devices, such as a gene gun, and an electroporator. These methods do not need to use a gene carrier, but the transfection efficiency is generally very low, the operation is complicated, and the damage to a tissue is relatively large. It is also mediated by viral carriers, such as an adenovirus, and a lentivirus. Although the viral carriers have the higher transfection activity in vitro, the immunogenicity and disadvantages thereof which may easily cause mutations bring huge safety risks to in vivo delivery. There are also non-viral carriers, especially a biodegradable polymer material, to achieve the targeted delivery of the drug. A main advantage of the non-viral carriers is that the immunogenicity and many inflammatory reactions brought by the viral carriers may be greatly reduced under a condition of guaranteeing the expected transfection activity.

In the above multiple targeted delivery modes, more studies are focused on the field of the non-viral carriers at present, and it is generally a plurality of the following carrier designs: (a) a cationic liposome; and (b) a polycationic gene carrier. At present, the more studies focus on modification of the polycationic gene carrier and the cationic liposome, so that it is suitable for the targeted delivery of the genetic substances. The cationic liposome has the higher transfection activity in vivo and in vitro. However, because normal distribution thereof in vivo is affected by positive charge on a surface, at the same time, the cationic liposome may cause the immunogenicity and the inflammatory responses in animal experiments. The development of the polycationic gene carrier is relatively mature at present, but it is difficult to guarantee that a targeting group is on a surface of a structure in a structural design, and there is an own design contradiction between the toxicity and the transfection activity, at the same time, connection thereof is difficult to achieve non-toxic degradation in vivo.

Therefore, how to improve the delivery reliability of an existing small molecular drug is one of the difficulties in solving a limited clinical application of the existing drug.

### Summary

A main purpose of the present application is to provide a nucleic acid nanocarrier drug, a preparation method thereof, a pharmaceutical composition and an application thereof, as to improve the delivery reliability of a drug.

In order to achieve the above purpose, according to one aspect of the present application, a nucleic acid nanocarrier drug is provided, and includes a nucleic acid nanoparticle and a drug loaded on the nucleic acid nanoparticle, and the drug includes one or more of tacrine, epirubicin, methotrexate, pirarubicin, daunorubicin, pentafluorouracil, 10-hydroxycamptothecin, aspirin and gemcitabine; the nucleic acid nanoparticle includes a nucleic acid domain, the nucleic acid domain includes a sequence a, a sequence b and a sequence c, the sequence a includes a sequence a1 or a sequence obtained by insertion, deletion or substitution of at least one base in the sequence a1, the sequence b comprises a sequence b1 or a sequence obtained by insertion, deletion or substitution of at least one base in the sequence b1, and the sequence c comprises a sequence c1 or a sequence obtained by insertion, deletion or substitution of at least one base in the sequence c1,, herein, the sequence a1 is SEQ ID NO:1: 5'-CCAGCGUUCC-3'or SEQ ID NO:2: 5'-CCAGCGTTCC-3'; the sequence b1 is SEQ ID NO:3: 5'-GGUUCGCCG-3'or SEQ ID NO:4: 5'-GGTTCGCCG-3'; and the sequence c1 is SEQ ID NO:5: 5'-CGGCCAUAGCGG-3'or SEQ ID NO:6: 5'-CGGCCATAGCGG-3'.

Further, when the sequence a1 is the SEQ ID NO:1, the sequence b1 is the SEQ ID NO:3, and the sequence c1 is the SEQ ID NO:5, at least one sequence of the sequence a, the sequence b and the sequence c includes a sequence obtained by insertion, deletion or substitution of at least one base in which at least one base is inserted, deleted or substituted within thereof.

Further, the insertion, deletion or substitution of at least one base is generated:

(1) on 1, 2, 4 or 5-th base starting from a 5 '-end of the sequence shown in the SEQ ID NO:1 or the SEQ ID NO:2; and/or

(2) between 8-th and 10-th bases starting from the 5 '-end of the sequence shown in the SEQ ID NO:1 or the SEQ ID NO:2; and/or

(3) between 1-th and 3-th bases starting from a 5 '-end of the sequence shown in the SEQ ID NO:3 or the SEQ ID NO:4; and/or

(4) between 6-th and 9-th bases starting from the 5 '-end of the sequence shown in the SEQ ID NO:3 or the SEQ ID NO:4; and/or

(5) between 1-th and 4-th bases starting from a 5 '-end of the sequence shown in the SEQ ID NO:5 or the SEQ ID NO:6; and/or

(6) between 9-th and 12-th bases starting from the 5 '-end of the sequence shown in the SEQ ID NO:5 or the SEQ ID NO:6.

Further, the sequence a, the sequence b and the sequence c are self-assembled into a structure shown in Formula (1):

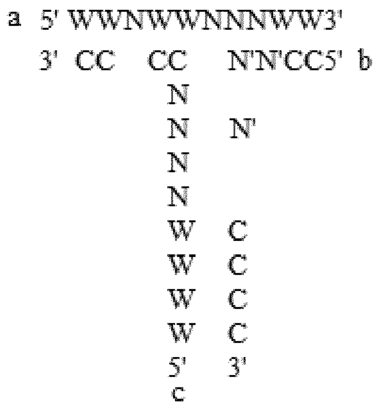


Formula (1),

Herein, W-C represents a Watson-Crick pairing, N and N' represent a non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

Further, the sequence a, the sequence b and the sequence c are any one of the following groups: (1) sequence a: 5'-GGAGCGUUGG-3', sequence b: 5'-CCUUCGCCG-3', sequence c: 5'-CGGCCAUAGCCC-3'; (2) sequence a: 5'-GCAGCGUUCG-3', sequence b: 5'-CGUUCGCCG-3', sequence c: 5'-CGGCCAUAGCGC-3'; (3) sequence a: 5'-CGAGCGUUGC-3', sequence b: 5'-GCUUCGCCG-3', sequence c: 5'-CGGCCAUAGCCG-3'; (4) sequence a: 5'-GGAGCGUUGG-3', sequence b: 5'-CCUUCGGGG-3', sequence c: 5'-CCCCCAUAGCCC-3'; (5) sequence a: 5'-GCAGCGUUCG-3', sequence b: 5'-CGUUCGGCG-3', sequence c: 5'-CGCCCAUAGCGC-3'; (6) sequence a: 5'-GCAGCGUUCG-3', sequence b: 5'-CGUUCGGCC-3', sequence c: 5'-GGCCCAUAGCGC-3'; (7) sequence a: 5'-CGAGCGUUGC-3', sequence b: 5'-GCUUCGGCG-3', sequence c: 5'-CGCCCAUAGCCG-3'; (8) sequence a: 5'-GGAGCGTTGG-3', sequence b: 5'-CCTTCGCCG-3', sequence c: 5'-CGGCCATAGCCC-3'; (9) sequence a: 5'-GCAGCGTTCG-3', sequence b: 5'-CGTTCGCCG-3', sequence c: 5'-CGGCCATAGCGC-3'; (10) sequence a: 5'-CGAGCGTTGC-3', sequence b: 5'-GCTTCGCCG-3', sequence c: 5'-CGGCCATAGCCG-3'; (11) sequence a: 5'-GGAGCGTTGG-3', sequence b: 5'-CCTTCGGGG-3', sequence c: 5'-CCCCCATAGCCC-3'; (12) sequence a: 5'-GCAGCGTTCG-3', sequence b: 5'-CGTTCGGCG-3', sequence c: 5'-CGCCCATAGCGC-3'; (13) sequence a: 5'-GCAGCGTTCG-3', sequence b: 5'-CGTTCGGCC-3', sequence c: 5'-GGCCCATAGCGC-3'; and (14) sequence a: 5'-CGAGCGTTGC-3', sequence b: 5'-GCTTCGGCG-3', sequence c: 5'-CGCCCATAGCCG-3'.

Further, the nucleic acid domain further includes a first extension fragment, the first extension fragment is an extension fragment of the Watson-Crick pairing, and the first extension fragment is positioned at the 5'-end and/or the 3'-end of any one sequence of the sequence a, the sequence b or the sequence c; preferably, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; and (9): b-strand 3'-end: 5'-CCA-3', c-strand 5'-end:

5'-TGG-3'.

Further, the nucleic acid domain further includes a second extension fragment, the second extension fragment is positioned at the 5'-end and/or the 3'-end of any one sequence of the sequence a, the sequence b, or the sequence c, and the second extension fragment is an extension fragment of the Watson-Crick pairing; preferably, the second extension fragment is an extension sequence of a CG base pair; and more preferably, the second extension fragment is an extension sequence of 1-10 CG base pairs.

Further, the nucleic acid domain further includes at least one group of the following second extension fragments: first group: a-strand 5'-end: 5'-CGCGCG-3', c-strand 3'-end: 5'-CGCGCG-3'; second group: a-strand 3'-end: 5'-CGCCGC-3', b-strand 5'-end: 5'-GCGGCG-3'; and third group: b-strand 3'-end: 5'-GGCGGC-3', c-strand 5'-end: 5'-GCCGCC-3'.

Further, the second extension fragment is an extension sequence containing both CG base pair and AT/AU base pair, and preferably the second extension fragment is an extension sequence of 2-50 base pairs.

Further, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Further, a base, a ribose and a phosphate in the sequence a, the sequence b and the sequence c have at least one modifiable site, and any one of the modifiable sites is modified by any one of the following modification adapters: -F, a methyl, an amino, a disulfide, a carbonyl, a carboxyl, a sulfhydryl and a formyl; and preferably, the base C or U in the sequence a, the sequence b and the sequence c has 2'-F modification.

Further, the drug is loaded on the nucleic acid nanoparticle in modes of physical linkage and/or covalent linkage, and a molar ratio between the drug and the nucleic acid nanoparticle is 2-300:1, preferably 10-50:1, and more preferably 15-25:1.

Further, the nucleic acid nanoparticle further include a bioactive substance, the bioactive substance is linked with the nucleic acid domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin,a phenol, a lecithin, and a small molecular drug, the small molecular drug is a small molecular drug except the tacrine, the epirubicin, the methotrexate, the pirarubicin, the daunorubicin, the pentafluorouracil, the 10-hydroxycamptothecin, the aspirin and the gemcitabine.

Further, a relative molecular weight of the nucleic acid domain is marked as $N_1$, and a total relative molecular weight of the drug and the bioactive substance is marked as $N_2$, $N,/N_2 \geq 1:1$.

Further, the bioactive substance is one or more of the target head, the fluorescein and the miRNA, herein the target head is positioned on any one sequence of the sequences a, b and c, preferably the 5'-end or the 3'-end of any one sequence of the sequences a, b and c, or inserted between GC bonds of the nucleic acid domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c, and preferably, the target head is a folic acid or a biotin, the fluorescein is any one or more of FAM, CY5 and CY3, and the anti-miRNA is anti-miR-21.

Further, the small molecular drug is a drug containing any one or more of the following groups: an amino group, a hydroxyl group, a carboxyl group, a mercapto group, a benzene ring group and an acetamido group.

Further, the protein is one or more of SOD, survivin, hTERT, EGFR and PSMA; the vitamin is L-Vc and/or esterified Vc; and the phenol is a tea polyphenol and/or a grape polyphenol.

Further, a particle size of the nucleic acid nanoparticle is 1-100 nm, preferably 5-50 nm; more preferably 10-30 nm; and further preferably 10-15 nm.

According to another aspect of the present application, a preparation method for a nucleic acid nanocarrier drug is provided, and the method includes the following steps: the above nucleic acid nanoparticle is provided; and the drug is loaded on the nucleic acid nanoparticle in a physical linkage mode and/or a covalent linkage mode, to obtain the nucleic acid nanocarrier drug.

Further, the step of loading the drug in the physical linkage mode includes the drug, the nucleic acid nanoparticle and a first solvent are mixed and stirred, to obtain a premixed system; and the premixed system is precipitated, to obtain the nucleic acid nanocarrier drug; preferably, the first solvent is selected from one or more of DCM, DCC, DMAP, Py, DMSO, PBS and glacial acetic acid; preferably, the step of precipitating the premixed system, to obtain the nucleic acid nanocarrier drug includes the premixed system is precipitated, to obtain a precipitation; and the precipitation is washed to remove impurities, as to obtain the nucleic acid nanocarrier drug; more preferably, the premixed system is mixed with absolute ethyl alcohol, and precipitated at a temperature condition lower than 10 DEG C, to obtain the precipitation, namely the nucleic acid nanocarrier drug; and more preferably, the precipitation is obtained by precipitating at a temperature condition of 0-5 DEG C. More preferably, the precipitation is washed to remove the impurities with 6-12 times of the absolute ethyl alcohol in volume, as to obtain the nucleic acid nanocarrier drug.

Further, the step of loading the drug in the covalent linkage mode includes drug solution is prepared; the drug solution reacts with the G-exocyclic amino of the nucleic acid nanoparticle under a mediating effect of the formaldehyde, to obtain a reaction system; and the reaction system is purified, to obtain the nucleic acid nanocarrier drug; preferably, the reaction step includes the drug solution is mixed with paraformaldehyde solution and the nucleic acid nanoparticle, and it is reacted in a dark condition, to obtain the reaction system; herein the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

Further, the preparation method further includes a step of preparing the nucleic acid nanoparticle, the step includes a single strand corresponding to the above nucleic acid domain is self-assembled, to obtain the nucleic acid domain; preferably, after the nucleic acid domain is obtained, the preparation method further includes the above bioactive substance is loaded on the nucleic acid domain in the modes of physical linkage and/or covalent linkage, to obtain the nucleic acid nanoparticle.

Further, in a process of loading the bioactive substance in the covalent linkage mode, the loading is performed through solvent covalent linkage, linker covalent linkage or click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or PEG; preferably, the click-linkage is that alkynyl or azide modification is simultaneously performed on a bioactive substance precursor and the nucleic acid domain, and then they are linked through the click-linkage.

Further, when the bioactive substance is linked with the nucleic acid domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid domain is selected from a G-exocyclic amino, a 2'-hydroxyl, an A-amino or a 2'-hydroxyl.

According to a third aspect of the present application, a pharmaceutical composition is further provided, and the pharmaceutical composition includes any one of the above nucleic acid nanocarrier drugs.

According to a fourth aspect of the present application, an application of any one of the above nucleic acid nanocarrier drugs in preparing a drug for treating an Alzheimer's disease, a tumor, an autoimmune disease or a heart disease is further provided.

Further, the tumor is one or more of the followings: pancreatic cancer, ovarian cancer, breast cancer, bladder cancer, cervical cancer, liver cancer, biliary tract cancer, nasopharyngeal cancer, testicular cancer, lymphoma, mesothelioma, head and neck cancer, gastric cancer, leukemia, colon cancer, rectal cancer, chorionic epithelioma, malignant hydatidiform mole, skin cancer, lung cancer, ureteral cancer, renal pelvis cancer, chorionic epithelioma, bone tumor, leukemia meningeal spinal cord infiltration, Wilms tumor, soft tissue sarcoma and medullary thyroid carcinoma; the autoimmune disease is refractory psoriasis, systemic lupus erythematosus, mandatory spondylitis or dermatomyositis.

Further, the leukemia is acute leukemia, more preferably the acute leukemia is acute lymphocytic leukemia or myeloid leukemia.

Further, the lung cancer includes bronchial lung cancer or non-small cell lung cancer.

Further, the liver cancer includes primary hepatocellular carcinoma or metastatic liver cancer.

According to a fifth aspect of the present application, a method for preventing and/or treating an Alzheimer's disease, a tumor, an autoimmune disease or a heart disease is further provided, and the method includes any one of the above nucleic acid nanocarrier drugs or pharmaceutical compositions is provided; a corresponding effective dose of the above nucleic acid nanocarrier drug or pharmaceutical composition is administered to a patient.

The nucleic acid nanocarrier drug provided by the present application includes the nucleic acid nanoparticle and the drug, and the drug is located on the nucleic acid nanoparticle in the modes of the physical linkage and/or the covalent linkage. The nucleic acid nanoparticle, through including the three sequences provided by the present application or the variant sequences thereof, not only may be self-assembled to form the nucleic acid domain, but also may be served as a carrier to link the drug at the arbitrary 5'-end and/or 3'-end of the three strands, or enables the drug to be stably inserted between the strands of the nucleic acid domain. The present application is capable of, through loading the drug on the nucleic acid nanoparticle, using internal hydrophobicity, external hydrophilicity and a base stacking effect of the nucleic acid nanoparticle to have a "coating effect" on the drug, and the drug is not dissolved within a certain period of time through the coating effect or the covalent linkage, so the delivery stability is improved. In addition, when the nucleic acid domain is modified by the target head, it may have the better targeting property, and may deliver the drug stably, the reliability is very high; at the same time, it may reduce a chance of the drug in contact with non-target cells or tissues, toxic side effects are reduced.

## Brief Description of the Drawings

Drawings of the description for constituting a part of the present application are used to provide further understanding of the disclosure, exemplary embodiments of the disclosure and descriptions thereof are used to explain the present application, and do not constitute improper limitation to the present application. In the drawings:

Fig. 1 shows an electrophoresis detection result of RNA nanoparticles formed by self-assembly in Embodiment 1 of the present application.

Fig. 2 shows an electrophoresis detection result of DNA nanoparticles formed by self-assembly in Embodiment 1 of the present application.

Fig. 3 shows a 2% agarose gel electrophoresis detection result of 7 groups of short-sequence RNA nanoparticles formed by self-assembly in Embodiment 2 of the present application.

Fig. 4 shows a 4% agarose gel electrophoresis detection result of 7 groups of short-sequence RNA nanoparticles formed by self-assembly in Embodiment 2 of the present application.

Fig. 5 shows a 2% agarose gel electrophoresis detection result of 7 groups of conventional sequence RNA nanoparticles formed by self-assembly in Embodiment 3 of the present application.

Fig. 6 shows a 4% agarose gel electrophoresis detection result of 7 groups of conventional sequence RNA nanoparticles formed by self-assembly in Embodiment 3 of the present application.

Fig. 7 shows a 2% agarose gel electrophoresis detection result of 7 groups of conventional sequence DNA nanoparticles formed by self-assembly in Embodiment 4 of the present application.

Fig. 8 shows a 4% agarose gel electrophoresis detection result of 7 groups of conventional sequence DNA nanoparticles formed by self-assembly in Embodiment 4 of the present application.

Fig. 9 shows a transmission electron microscope picture of conventional sequence DNA nanoparticles D-7 formed by self-assembly in Embodiment 4 of the present application.

Fig. 10 shows a standard curve of a tacrine absorbance in a loading rate detection process in Embodiment 5 of the present application.

Fig. 11 shows a microscopic observation result of binding and internalization of RNAh-Biotin-quasar670 nanoparticles and RNAh-Biotin-quasar670-tacrine nanoparticles with SH-SY5Y cells in Embodiment 6 of the present application.

Fig. 12 shows an electrophoresis detection result of the RNAh-Biotin-quasar670-tacrine nanoparticles, after being incubated in serum for different times, under a Coomassie Blue program in Embodiment 7 of the present application.

Fig. 13 shows an electrophoresis detection result of the RNAh-Biotin-quasar670-tacrine nanoparticles, after being incubated in serum for different times, under a Stain Free Gel program in Embodiment 7 of the present application.

Fig. 14 shows a detection result of the small molecular drug tacrine and the RNAh-Biotin-quasar670-tacrine nanoparticles for inhibiting proliferation of the SH-SY5Y cells in Embodiment 8 of the present application.

Fig. 15 shows a detection result of a fluorescence-targeted carrier Bio-Cy5-RNAh for inhibiting the proliferation of the SH-SY5Y cells in Embodiment 8 of the present application.

Fig. 16 shows non-denaturing PAGE gel electrophoresis detection results of 7 groups of extension fragment deformation + core short-sequence RNA self-assembly products in Embodiment 9 of the disclosure.

Fig. 17 shows a solubility curve of RNA nanoparticles R-15 in Embodiment 9 of the disclosure.

Fig. 18 shows a solubility curve of RNA nanoparticles R-16 in Embodiment 9 of the disclosure.

Fig. 19 shows a solubility curve of RNA nanoparticles R-17 in Embodiment 9 of the disclosure.

Fig. 20 shows a solubility curve of RNA nanoparticles R-18 in Embodiment 9 of the disclosure.

Fig. 21 shows a solubility curve of RNA nanoparticles R-19 in Embodiment 9 of the disclosure.

Fig. 22 shows a solubility curve of RNA nanoparticles R-20 in Embodiment 9 of the disclosure.

Fig. 23 shows a solubility curve of RNA nanoparticles R-21 in Embodiment 9 of the disclosure.

Fig. 24 shows non-denaturing PAGE gel electrophoresis detection results of 7 groups of extension fragment deformation + core short-sequence DNA self-assembly products in Embodiment 10 of the disclosure.

Fig. 25 shows a solubility curve of DNA nanoparticles D-8 in Embodiment 10 of the disclosure.

Fig. 26 shows a solubility curve of DNA nanoparticles D-9 in Embodiment 10 of the disclosure.

Fig. 27 shows a solubility curve of DNA nanoparticles D-10 in Embodiment 10 of the disclosure.

Fig. 28 shows a solubility curve of DNA nanoparticles D-11 in Embodiment 10 of the disclosure.

Fig. 29 shows a solubility curve of DNA nanoparticles D-12 in Embodiment 10 of the disclosure.

Fig. 30 shows a solubility curve of DNA nanoparticles D-13 in Embodiment 10 of the disclosure.

Fig. 31 shows a solubility curve of DNA nanoparticles D-14 in Embodiment 10 of the disclosure.

Fig. 32 shows an electrophoresis detection result of RNA nanoparticles R-15 after being incubated in serum for different times in Embodiment 11 of the disclosure.

Fig. 33 shows an electrophoresis detection result of RNA nanoparticles R-16 after being incubated in serum for different times in Embodiment 11 of the disclosure.

Fig. 34 shows an electrophoresis detection result of RNA nanoparticles R-17 after being incubated in serum for different times in Embodiment 11 of the disclosure.

Fig. 35 shows an electrophoresis detection result of RNA nanoparticles R-18 after being incubated in serum for different times in Embodiment 11 of the disclosure.

Fig. 36 shows an electrophoresis detection result of RNA nanoparticles R-19 after being incubated in serum for different times in Embodiment 11 of the disclosure.

Fig. 37 shows an electrophoresis detection result of RNA nanoparticles R-20 after being incubated in serum for different times in Embodiment 11 of the disclosure.

Fig. 38 shows an electrophoresis detection result of RNA nanoparticles R-21 after being incubated in serum for different times in Embodiment 11 of the disclosure.

Fig. 39 shows an electrophoresis detection result of DNA nanoparticles D-8 after being incubated in serum for different times in Embodiment 12 of the disclosure.

Fig. 40 shows an electrophoresis detection result of DNA nanoparticles D-9 after being incubated in serum for different times in Embodiment 12 of the disclosure.

Fig. 41 shows an electrophoresis detection result of DNA nanoparticles D-10 after being incubated in serum for different times in Embodiment 12 of the disclosure.

Fig. 42 shows an electrophoresis detection result of DNA nanoparticles D-11 after being incubated in serum for

different times in Embodiment 12 of the disclosure.

Fig. 43 shows an electrophoresis detection result of DNA nanoparticles D-12 after being incubated in serum for different times in Embodiment 12 of the disclosure.

Fig. 44 shows an electrophoresis detection result of DNA nanoparticles D-13 after being incubated in serum for different times in Embodiment 12 of the disclosure.

Fig. 45 shows an electrophoresis detection result of DNA nanoparticles D-14 after being incubated in serum for different times in Embodiment 12 of the disclosure.

Fig. 46a, Fig. 46b, Fig. 46c, Fig. 46d, Fig. 46e, Fig. 46f, Fig. 46g, and Fig. 46h respectively show cell survival rate curves corresponding to DMSO and original drug doxorubicin, D-8 and D-8-doxorubicin, D-9 and D-9-doxorubicin, D-10 and D-10-doxorubicin, D-11 and D-11-doxorubicin, D-12 and D-12-doxorubicin, D-13 and D-13-doxorubicin, and D-14 and D-14-doxorubicin in Embodiment 15 of the disclosure.

Fig. 47 shows a standard curve of a daunorubicin absorbance used in a loading rate detection process of Embodiment 16.

Fig. 48a and Fig. 48b, Fig. 49, Fig. 50a and Fig. 50b, Fig. 51, Fig. 52, Fig. 53, Fig. 54a and Fig. 54b, and Fig. 55 successively show standard curves of absorbance of epirubicin, methotrexate, pirarubicin, daunorubicin, pentafluorouracil, 10-hydroxycamptothecin, aspirin and gemcitabine in the loading rate detection process in Embodiment 17 of the present application.

Fig. 56 to Fig. 63 respectively show binding and internalization ability of the nucleic acid nanoparticles loaded with the epirubicin, the methotrexate, the pirarubicin, the daunorubicin, the pentafluorouracil, the 10-hydroxycamptothecin, the aspirin and the gemcitabine to cells.

Fig. 64 to Fig. 81 respectively show stability of the nucleic acid nanoparticles loaded with the epirubicin (Fig. 64 to Fig. 66), the methotrexate (Fig. 67 to Fig. 68), the pirarubicin (Fig. 69, 70 and 71), the daunorubicin (Fig. 72 and 73), the pentafluorouracil (Fig. 74 and 75), the 10-hydroxycamptothecin (Fig. 76 and 77), the aspirin (Fig. 78 and 79) and the gemcitabine (Fig. 80 and 81) in serum.

Fig. 82 to Fig. 101 respectively show toxicity of the nucleic acid nanoparticles loaded with the epirubicin (Fig. 82, Fig. 83, Fig. 84a to 84d and Fig. 85a to 85d), the methotrexate (Fig. 86 and 87), the pirarubicin (Fig. 88, 89, and 91a to 91d), the daunorubicin (Fig. 92 and 93), the pentafluorouracil (Fig. 94 and 95), the 10-hydroxycamptothecin (Fig. 96 and 97), the aspirin (Fig. 98 and 99) and the gemcitabine (Fig. 100 and 101) to the cells.

## Detailed Description of the Embodiments

It is to be noted that embodiments in the present application and features in the embodiments may be combined with each other under a condition without conflicting. The present application is described in detail below with reference to the embodiments.

Term explanation:

Blank carrier: refers to a blank nucleic acid nanoparticle carrier without containing any bioactive substances, such as an RNAh or a DNAh.
Targeting carrier: refers to a nucleic acid nanoparticle carrier which contains a target head but does not contain a fluorescent substance, such as a Biotin-RNAh or a Biotin-DNAh.
Fluorescent carrier: refers to a nucleic acid nanoparticle carrier which contains the fluorescent substance but does not contain the target head, such as a Cy5-RNAh or a Cy5-DNAh.
Targeted fluorescent carrier: refers to a nucleic acid nanoparticle carrier containing the target head and the fluorescent substance, such as a Biotin-Cy5-RNAh or a Biotin-Cy5-DNAh.
Targeted drug: refers to a nucleic acid nanoparticle carrier containing the target head, the fluorescent substance and a chemical drug, such as a tacrine-Biotin-Cy5-RNAh or a tacrine-Biotin-Cy5-DNAh.
It is to be noted that there is no special format for a naming rule of each carrier or bioactive substance in the present application, and front and rear positions thereof in the expression do not mean that it is at a 5'end or a 3'end of

the RNAh or the DNAh, but only mean that the bioactive substance is contained.

As mentioned in the background, although there are a variety of drug carriers for improving drug delivery efficiency in the prior art, it is still difficult to solve the problem of the limited clinical applications of the drugs. In order to improve this situation, the inventor of the present application researches on all existing materials which may be used as the drug carriers, and deeply investigates and analyzes the various carriers in aspects, such as cell/tissue targeting of the carrier, stability in a delivery process, activity and efficiency of entering target cells, drug release ability after reaching the target cells and toxicity to cells, it is discovered that an emerging nanostructure formed by self-assembly of DNA and/or RNA molecules, such as a DNA in a self-assembly system of DNA dendrimers, is used to have a significant obstruction effect to degradation of a nuclease, and have a very important application value in the fields of gene therapy and biomedicine.

Through analyzing the existing reported nanoparticle formed by the self-assembly of the DNA and RNA, it is discovered that, compared with the relatively rigid DNA nanoparticle, the RNA nanoparticle has larger flexibility and stronger tension because there are a large number of stem-loop structures within or between molecules, so it has more advantages in an aspect as a candidate drug carrier. However, the RNA nanoparticle in a natural state are relatively poor in stability, and the existing improvements based on application aspects of the RNA nanocarriers are mostly focused on improving the stability and reliability thereof. Although research results at present provide the possibility of loading the drug to a certain extent, they are more focused on researching the possibility and effectiveness of loading nucleic acid drugs, especially a siRNA drug or a miRNA drug and the like. There are few reports at present on whether non-nucleic acid drugs are equally effective. In addition, the existing self-assembled nanoparticle, especially the self-assembled nanoparticle used as carriers, are self-assembled by using a RNA strand, and a very few is self-assembled in a mode of a RNA strand and DNA strand combination, but the self-assembly is achieved without using a pure DNA strand.

In order to provide a new RNA nanoparticle carrier with good reliability and self-assembly, the existing RNA nanoparticle are compared and improved by the applicant, a series of new RNA nanoparticle are developed, and in view of improving applicability and reducing cost, the self-assembly is further tried to be performed by using the pure DNA strand, it is unexpectedly discovered that after being changed, these DNA single strands may not only self-assemble into the DNA nanoparticle, but also have the same excellent performance as the RNA nanoparticle. In addition, the self-assembly of the DNA nanoparticle also has advantages of low price and easy operation. After being verified by experiments, both the RNA nanoparticle and the DNA nanoparticle improved by the inventor may be loaded with various drugs, and may exist stably in serum; and further verified by the experiments, it may carry the drugs into the cells, and the separate carrier is non-toxic to the cells. However, the drug-carried carrier may have alleviating and treating effects to corresponding diseases.

On the basis of the above research result, the applicant provides a technical scheme of the present application. The present application provides a nucleic acid nanocarrier drug, the nucleic acid nanocarrier drug includes nucleic acid nanoparticle and a drug, the drug is loaded on the nucleic acid nanoparticle, and the drug includes one or more of tacrine, epirubicin, methotrexate, pirarubicin, daunorubicin, pentafluorouracil, 10-hydroxycamptothecin, aspirin and gemcitabine; and the nucleic acid nanoparticle includes a nucleic acid domain, the nucleic acid domain includes a sequence a, a sequence b and a sequence c, the sequence a includes a sequence al or a sequence in which at least one base is inserted, deleted or substituted in the sequence al, the sequence b includes a sequence b1 or a sequence in which at least one base is inserted, deleted or substituted in the sequence b1, and the sequence c includes a sequence c1 or a sequence in which at least one base is inserted, deleted or substituted in the sequence c1, herein the sequence a1 is SEQ ID NO:1: 5'-CCAGCGUUCC-3'or SEQ ID NO:2: 5'-CCAGCGTTCC-3'; the sequence b1 is SEQ ID NO:3: 5'-GGUUCGCCG-3'or SEQ ID NO:4: 5'-GGTTCGCCG-3'; and the sequence c1 is SEQ ID NO:5: 5'-CGGCCAUAGCGG-3'or SEQ ID NO:6: 5'-CGGCCATAGCGG-3'.

The nucleic acid nanocarrier drug provided by the present application includes the nucleic acid nanoparticle and the drug, and one or more of the above drugs are loaded on the nucleic acid nanoparticle. The nucleic acid nanoparticle, through including the above three sequences or the variant sequences thereof, not only may be self-assembled to form the nucleic acid domain, but also may be served as a carrier to link the drug at the arbitrary 5'-end and/or 3'-end of the three strands, or enable the drug to be stably inserted between the strands of the nucleic acid domain. The nucleic acid nanocarrier drug provided by the present application is capable of, through loading the above drug on the nucleic acid nanoparticle, because the nucleic acid nanoparticle are hydrophobic in the interior, hydrophilic in the exterior and have a stacking effect on the base, it is equivalent to a "coating effect" to the drug, and the drug may not be dissolved within a certain period of time through the coating or the covalent linkage, improving the delivery stability. In addition, when the nucleic acid domain is modified by the target head, it may have the better targeting property, and may deliver the drug stably, the reliability is very high; at the same time, it may reduce a chance of the drug in contact with non-target cells or tissues, toxic side effects are reduced.

The above self-assembly refers to a technology that basic structural units spontaneously form an ordered structure. In a process of the self-assembly, the basic structural units spontaneously organize or aggregate into a stable structure with a certain regular geometric appearance under an interaction based on a non-covalent bond. The self-assembly process is not a simple superposition of weak interaction forces (herein the "weak interaction forces" refer to a hydrogen bond, a Van der Waals force, an electrostatic force, a hydrophobic action force and the like) between a large number of atoms, ions or molecules, but a tight and orderly whole formed by simultaneously spontaneously parallel connection and aggregation between a plurality of individuals, and is an overall complicated synergistic effect.

The production of the self-assembly requires two conditions: self-assembly power and guiding effect. The self-assembly power refers to the synergistic effect of the weak interaction forces between the molecules, and it provides energy for molecular self-assembly. The guiding effect of the self-assembly refers to complementary of the molecules in space, namely the production of the self-assembly needs to meet requirements of molecular rearrangement in size and direction of the space.

A DNA nanotechnology is a bottom-up molecular self-assembly mode, a stable structure is spontaneously formed by using a molecular structure as a starting point on the basis of physical and chemical properties of the nucleic acid nanoparticle, and a strict nucleic acid base pairing principle is followed. Multiple DNA fragments are linked together in vitro in a correct sequence, a sub-assembly structure is established through the base complementary pairing principle, and finally a complicated multi-level structure is formed. Unlike the DNA, the structure of the RNA may exceed limitation of double-helix. The RNA may form a series of different base pairs, and at least two hydrogen bonds are formed between the base pairs. The different bases may be divided into two types, including a standard Waston-Crick base pair type and a non-Waston-Crick base pair type, so that the RNA forms a large number and multiple types of cyclic structure modules, and these modules are basic units for forming a folded RNA three-level structure. The RNA nanotechnology may make use of these natural existing 3D modules and predictable interactions thereof, herein, many RNA structures with biological activity may have an atomic-level resolution, such as a ribosome, various ribozymes and a natural RNA aptamer existing in a riboswitch. A superior feature of the RNA nanotechnology is that a structure which is comparable in size and complexity to a natural RNA substance may be designed. A unique assembly property of the RNA in a natural RNA complex may also be utilized.

The above nucleic acid nanoparticle of the present application include three sequences shown in sequences SEQ ID NO:1, SEQ ID NO:3 and SEQ ID NO:5 or variant sequences thereof, or include three sequences shown in sequences SEQ ID NO:2, SEQ ID NO:4 and SEQ ID NO:6 or variant sequences thereof, and all of the sequences are subject to an ability to form the nucleic acid nanoparticle through the self-assembly, the specific variant sequence may be obtained on the basis of the sequences of the SEQ ID NO:1, the SEQ ID NO:2, the SEQ ID NO:3, the SEQ ID NO:4, the SEQ ID NO:5 and the SEQ ID NO:6 by rationally selecting a variant site and a variant type thereof, or obtained by extending a suitable fragment.

The nanoparticle formed by the self-assembly of the SEQ ID NO:1, the SEQ ID NO:3 and the SEQ ID NO:5 are the RNA nanoparticle, and the nanoparticle formed by the self-assembly of the SEQ ID NO:2, the SEQ ID NO:4 and the SEQ ID NO:6 are the DNA nanoparticle. In a preferred embodiment, when the above nucleic acid nanoparticle are the RNA nanoparticle, and at least one of the sequence a, the sequence b and the sequence c includes the sequence in which at least one base is inserted, deleted or substituted, a specific position and a base type of the variant sequence in the RNA nanoparticle may be modified into the nanoparticle of improving a drug loading amount or improving stability according to the needs under a precondition of achieving the self-assembly.

In order to make the prepared nucleic acid nanoparticle have the relatively higher stability, when the base insertion, deletion or substitution is performed on the sequences shown in the above SEQ ID NO:1/2, SEQ ID NO:3/4 and SEQ ID NO:5/6, it may be performed on bases in some specific positions of the above sequences. On the one hand, the variant sequence is the same as the original sequence, and may be self-assembled into the nanoparticle, and on the other hand, the variation retains at least 50%, 55%, 60%, 65%, 70%, 75%, 80%, 85% 90% or 95% of homology with the original sequence, so that it has the same drug-loading features and similar stability as the nanoparticle formed by the self-assembly of the above sequences, the drug may be loaded and delivered well.

In a preferred embodiment, the above base insertion, deletion or substitution is generated: (1) on 1, 2, 4 or 5-th base starting from a 5 ’-end of the sequence a shown in the SEQ ID NO:1 or 2; and/or (2) between 8-th and 10-th bases starting from the 5 ’-end of the sequence a shown in the SEQ ID NO:1 or 2; and/or (3) between 1-th and 3-th bases starting from a 5 ’-end of the sequence b shown in the SEQ ID NO:3 or 4; and/or (4) between 6-th and 9-th bases starting from the 5 ’-end of the sequence b shown in the SEQ ID NO:3 or 4; and/or (5) between 1-th and 4-th bases starting from a 5 ’-end of the sequence c shown in the SEQ ID NO:5 or 6; and/or (6) between 9-th and 12-th bases starting from the 5 ’-end of the sequence c shown in the SEQ ID NO:5 or 6.

In the above preferred embodiment, the defined base position in which the variation happens is a non-classical Watson-Crick paired base position or a bulged unpaired base position in the nanostructure formed by the sequences shown in the SEQ ID NO:1, SEQ ID NO:2, SEQ ID NO:3, SEQ ID NO:4, SEQ ID NO:5 and SEQ ID NO:6, thus the formation of these bulges or loop structures is not affected, thereby the flexibility and tension of the nanostructure formed by the above sequences are maintained, and it is helpful to maintain the stability thereof as a carrier.

In order to further improve the stability of the above nucleic acid nanoparticle, and improve the stability of the drug after drug loading, in a preferred embodiment, the sequence a, the sequence b and the sequence c are self-assembled into a structure shown in Formula (1):

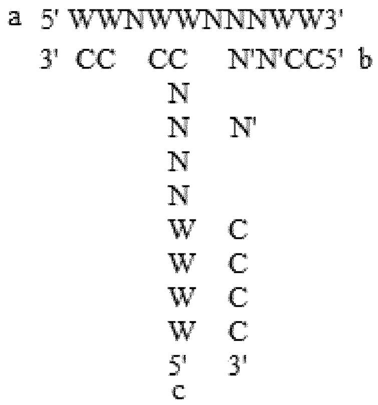


Formula (1)

herein, W-C represents Watson-Crick pairing, N and N' represent non-Watson-Crick pairing, the W-C in any one position is independently selected from C-G or G-C; in the sequence a, the first N from the 5'-end is A, the second N is G, the third N is U or T, and the fourth N is any one of U, T, A, C or G; in the sequence b, the first N' from the 5'-end is any one of U, T, A, C or G, the second N' is U or T, and the third N' is C; and in the sequence c, a sequence NNNN along a direction from the 5'-end to the 3'-end is CAUA or CATA.

In the above preferred embodiment, the sequences a, b and c are self-assembled to form the nucleic acid domain shown in Formula (1), herein, except the non-Watson-Crick paired bases defined by N and N', the bases in the rest positions all form classical Watson-Crick pairing, and the bases of the above Watson-Crick pairing all choose G-C or C-G base pairs. Because an action force of hydrogen bonds between the G-C or C-G base pairs is greater than an action force of hydrogen bonds between the A-U/T or U/T-A base pairs, the nucleic acid nanostructure is more stable. Rather than the bulges or loop structures formed by the non-Watson-Crick paired bases, the greater tension is brought to the nucleic acid nanocarrier, so that it has the stronger adaptation to a micro-environmental change, thus the stability of the nucleic acid nanoparticle is higher.

In the nanoparticle in the above structure of Formula (1), the specific sequence formation of the sequence a, the sequence b and the sequence c is not specially limited, as long as the above structure may be formed. In view of the self-assembly of the nucleic acid sequence, in order to further improve the efficiency of the self-assembly of the above three sequences into the nanoparticle in the above structure of Formula (1), when the Watson-Crick paired bases are selected, the selection of the bases in the different positions is best to follow the following principles: (1) the sequence a, the sequence b and the sequence c, when a single sequence is selected, it is not complementary-paired by itself to form a two-level structure; and (2) the sequence a, the sequence b and the sequence c, one end of arbitrary two sequences is complementary-paired to form a double-strand, and the other end is not complementary-paired, to form a Y-type or T-type structure. The above principle of the base selection is to most efficiently enable two ends of any one strand to be respectively complementary-paired with two ends of the other two strands, thereby the self-assembly efficiency is improved. Certainly, in addition to the Y-type or T-type structure, it may also be other deformation modes such as a quadrangle rather than a trigeminal shape, as long as it meets the principle that one end of arbitrary two sequences is complementary-paired to form the double-strand, and the other end is not complementary-paired.

In the nanoparticle in the above structure of Formula (1), in the non-Watson-Crick paired bases, the fourth N starting from the 5'-end in the sequence a and the first N' starting from the 5'-end paired in the sequence b may be non-Watson-Crick paired U-U, or may be T, A, C or G improved for following the Watson-Crick pairing principle. A binding force between the strands is relatively improved by the Watson-Crick pairing, the stability is improved, and the nanoparticle are endowed with the larger softness and flexibility by the non-Watson-Crick pairing, in the face of the micro-environmental change, it is also helpful to improve the stability of the nanoparticle.

In a preferred embodiment, the sequence a, the sequence b and the sequence c are any one of the following groups: (1) sequence a (SEQ ID NO:7): 5'-GGAGCGUUGG-3', sequence b (SEQ ID NO:8): 5'-CCUUCGCCG-3', sequence c (SEQ ID NO:9): 5'-CGGCCAUAGCCC-3'; (2) sequence a (SEQ ID NO:10): 5'-GCAGCGUUCG-3', sequence b (SEQ ID NO:11): 5'-CGUUCGCCG-3', sequence c (SEQ ID NO:12): 5'-CGGCCAUAGCGC-3'; (3) sequence a (SEQ ID NO:13): 5'-CGAGCGUUGC-3', sequence b (SEQ ID NO:14): 5'-GCUUCGCCG-3', sequence c (SEQ ID NO:15): 5'-CGGCCAUAGCCG-3'; (4) sequence a (SEQ ID NO:16): 5'-GGAGCGUUGG-3', sequence b (SEQ ID NO:17): 5'-CCUUCGGGG-3', sequence c (SEQ ID NO:18): 5'-CCCCCAUAGCCC-3'; (5) sequence a (SEQ ID NO:19): 5'-GCAGCGUUCG-3', sequence b (SEQ ID NO:20): 5'-CGUUCGGCG-3', sequence c (SEQ ID NO:21): 5'-CGCCCAUAGCGC-3'; (6) sequence a (SEQ ID NO:22): 5'-GCAGCGUUCG-3', sequence b (SEQ ID NO:23): 5'-CGUUCGGCC-3', sequence c

(SEQ ID NO:24): 5'-GGCCCAUAGCGC-3'; (7) sequence a (SEQ ID NO:25): 5'-CGAGCGUUGC-3', sequence b (SEQ ID NO:26): 5'-GCUUCGGCG-3', sequence c (SEQ ID NO:27): 5'-CGCCCAUAGCCG-3'; (8) sequence a (SEQ ID NO:28): 5'-GGAGCGTTGG-3', sequence b (SEQ ID NO:29): 5'-CCTTCGCCG-3', sequence c (SEQ ID NO:30): 5'-CGGCCATAGCCC-3'; (9) sequence a (SEQ ID NO:31): 5'-GCAGCGTTCG-3', sequence b (SEQ ID NO:32): 5'-CGTTCGCCG-3', sequence c (SEQ ID NO:33): 5'-CGGCCATAGCGC-3'; (10) sequence a (SEQ ID NO:34): 5'-CGAGCGTTGC-3', sequence b (SEQ ID NO:35): 5'-GCTTCGCCG-3', sequence c (SEQ ID NO:36): 5'-CGGCCATAGCCG-3'; (11) sequence a (SEQ ID NO:37): 5'-GGAGCGTTGG-3', sequence b (SEQ ID NO:38): 5'-CCTTCGGGG-3', sequence c (SEQ ID NO:39): 5'-CCCCCATAGCCC-3'; (12) sequence a (SEQ ID NO:40): 5'-GCAGCGTTCG-3', sequence b (SEQ ID NO:41): 5'-CGTTCGGCG-3', sequence c (SEQ ID NO:42): 5'-CGCCCATAGCGC-3'; (13) sequence a (SEQ ID NO:43): 5'-GCAGCGTTCG-3', sequence b (SEQ ID NO:44): 5'-CGTTCGGCC-3', sequence c (SEQ ID NO:45): 5'-GGCCCATAGCGC-3'; and (14) sequence a (SEQ ID NO:46): 5'-CGAGCGTTGC-3', sequence b (SEQ ID NO:46): 5'-GCTTCGGCG-3', sequence c (SEQ ID NO:48): 5'-CGCCCATAGCCG-3'.

The nucleic acid nanoparticle formed by the self-assembly of fourteen groups of the above sequences not only have the higher stability, but also have the higher self-assembly efficiency.

The nucleic acid nanoparticle mentioned above may not only be self-assembled for forming, but also have the ability to carry or load the drugs. According to the different positions of G-C or C-G base pairs in the above nucleic acid nanoparticle and differences of types or natures of the drugs to be loaded, the amounts of the loaded drugs are also different.

In order to enable the above nucleic acid domain to load more drugs and other bioactive substances (the introduction of the bioactive substance is described below), in a preferred embodiment, the above nucleic acid domain further includes a first extension fragment, the first extension fragment is an extension fragment of Watson-Crick pairing, and the first extension fragment is located at the 5'-end and/or 3'-end of any one of the sequences a, b, and c. A certain matching relation is required between the carrier and the loaded substance. While a molecular weight of the carrier is too small and a molecular weight of the loaded substance is too large, in view of mechanics, carrying or delivering capacity of the carrier to the loaded substance is relatively reduced. Therefore, based on the above nucleic acid nanostructure, through adding the first extension fragment at the 5'-end and/or 3'-end of any one sequence of the sequence a, the sequence b and the sequence c, the carrier matched with the size of the loaded substance may be acquired.

A specific length of the above first extension fragment may be determined according to the size of the substance to be loaded. In a preferred embodiment, the first extension fragment is selected from any one of the following groups: (1): a-strand 5'-end: 5'-CCCA-3', c-strand 3'-end: 5'-UGGG-3'; (2): a-strand 3'-end: 5'-GGG-3', b-strand 5'-end: 5'-CCC-3'; (3): b-strand 3'-end: 5'-CCA-3', c-strand 5'-end: 5'-UGG-3'; (4): a-strand 5'-end: 5'-CCCG-3', c-strand 3'-end: 5'-CGGG-3'; (5): a-strand 5'-end: 5'-CCCC-3', c-strand 3'-end: 5'-GGGG-3'; (6): b-strand 3'-end: 5'-CCC-3', c-strand 5'-end: 5'-GGG-3'; (7): b-strand 3'-end: 5'-CCG-3', c-strand 5'-end: 5'-CGG-3'; (8): a-strand 5'-end: 5'-CCCA-3', c-strand 3'-end: 5'-TGGG-3'; (9): b-strand 3'-end: 5'-CCA-3', c-strand 5'-end: 5'-TGG-3'; (10): a-strand 5'-end: 5'-GCGGCGAGCGGCGA-3'(SEQ ID NO:162), c-strand 3'-end: 5'-UCGCCGCUCGCCGC-3'(SEQ ID NO:163); (11): a-strand 3'-end: 5'-GGCCGGAGGCCGG-3'(SEQ ID NO:164), b-strand 5'-end: 5'-CCGGCCUCCGGCC-3'(SEQ ID NO:165); (12): b-strand 3'-end: 5'-CCAGCCGCC-3'(SEQ ID NO:166), c-strand 5'-end: 5'-GGCGGCAGG-3'(SEQ ID NO:167); (13): a-strand 5'-end: 5'-GCGGCGAGCGGCGA-3'(SEQ ID NO:168), c-strand 3'-end: 5'-TCGCCGCTCGCCGC-3'(SEQ ID NO:169); and (14): a-strand 3'-end: 5'-GGCCGGAGGCCGG-3'(SEQ ID NO:170), b-strand 5'-end: 5'-CCGGCCTCCGGCC-3'(SEQ ID NO:171).

The above first extension fragment not only increases a length of any one or more of the three sequences for forming the nucleic acid nanostructure, but also the first extension fragment formed by the GC base further improves the stability of the formed nanoparticle. In addition, the first extension fragment formed by the above sequences also enables the sequence a, the sequence b and the sequence c to maintain the higher self-assembly activity and efficiency.

In view of the size of the formed nucleic acid nanoparticle and the stability thereof when delivered as a drug delivery carrier in vivo, it is necessary not to be filtered out by kidneys before reaching the target cells when the drugs may be delivered. In a preferred embodiment, the the nucleic acid domain further includes a second extension fragment, the second extension fragment is positioned at the 5'-end and/or the 3'-end of any one sequence of the sequence a, the sequence b, or the sequence c, and the second extension fragment is an extension fragment of Watson-Crick pairing; more preferably, the second extension fragment is an extension sequence of a CG base pair; and further preferably, the second extension fragment is an extension sequence of 1-10 CG base pairs. The second extension fragment is the extension fragment further added on the basis of the first extension fragment.

In a preferred embodiment, the above nucleic acid domain further includes at least one group of the following second extension fragments: first group: a-strand 5'-end: 5'-CGCGCG-3', c-strand 3'-end: 5'-CGCGCG-3'; second group: a-strand 3'-end: 5'-CGCCGC-3', b-strand 5'-end: 5'-GCGGCG-3'; and third group: b-strand 3'-end: 5'-GGCGGC-3', c-strand 5'-end: 5'-GCCGCC-3'. Such a second extension fragment enables the nanoparticle not to have immunogenicity, and there is not a situation of the two-level structure in which each strand is folded and linked by itself.

It is to be noted that the above first extension fragment and/or second extension fragment may also be separated

by an unpaired base pair.

In order to enable the above nucleic acid nanoparticle to load the bioactive substance (the introduction of the bioactive substance is described below) with the larger molecular weight, increase the drug loading amount and maintain the necessary stability, in a preferred embodiment, the second extension fragment is an extension sequence containing both CG base pair and AT/AU base pair, and preferably the second extension fragment is an extension sequence of 2-50 base pairs. Here, "/" in the "AT/AU base" is a relation of or, specifically, the second extension fragment is an extension sequence containing both CG base pair and AT base pair, or the second extension fragment is an extension sequence containing both CG base pair and AU base pair.

More specifically, the sequences a, b and c after the above second extension fragment is added may be the following sequences respectively:

The sequence a is (SEQ ID NO:49):

5'-CGCGCGAAAAAACGCGCGAAAAAACGCGCGCCCACCAGCGMMCCGGGCGCGCGAAA AAACGCGCGAAAAAACGCGCG-3'.

The sequence b is (SEQ ID NO:50):

5'-CGCGCGMMMMMMCGCGCGMMMMMMCGCGCGCCCGGMMCGCCGCCAGCCGCCM MMMMMGCCGCCMMMMMMGCCGCC-3'.

The sequence c is (SEQ ID NO:51):

5'-GGCGGCAAAAAAGGCGGCAAAAAAGGCGGCAGGCGGCAMAGCGGMGGGCGCGCGM MMMMMCGCGCGMMMMMMCGCGCG-3'.

M in the above sequence a, sequence b and sequence c is U or T, when the M is the T, a synthetic cost of the above sequences is greatly reduced.

In practical applications, specific setting positions of the extension sequences of the above CG base pair and AT/AU base pair may be rationally adjusted according to actual needs. In a more preferred embodiment, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

Specifically, positions of the extension fragment CGCGCG and the extension fragment CGCCGC in the sequence a as shown in the above SEQ ID NO:49 are interchanged with a position of the extension fragment AAAAAA, positions of the extension fragment GCGGCG and the extension fragment GGCGGC in the sequence b shown in the above SEQ ID NO:50 are interchanged with a position of the extension fragment TTTTTT, the extension fragment GCCGCC in the sequence c shown in the above SEQ ID NO:51 is interchanged with the extension fragment AAAAAA, and the extension fragment CGCCGC is interchanged with the extension fragment TTTTTT at the same time. The nucleic acid nanoparticle formed by the self-assembly of the above sequences are suitable for loading bioactive substences in indole molecular structures (indole drug molecules are preferably linked with A).

In the past few years, three major challenges of the RNA as a widely used construction material includes 1) sensitivity to RNA enzymatic degradation; 2) sensitivity to dissociation after systemic injection; and 3) toxicity and adverse immune response. At present, the three challenges are overcome to a large extent already: 1) 2'-fluoro(2'-F) or 2'-O-methyl(2'-OMe) modification of a ribose-OH group may make the RNA chemically stable in serum; 2) some natural existing linking sequence motifs are thermodynamically stable, and may keep the overall RNA nanoparticle to be integral at an ultra-low concentration; and 3) the immunogenicity of the RNA nanoparticle is sequence and shape-dependent, and may be adjusted, so that the RNA nanoparticle stimulate generation of inflammatory cytokines, or the RNA nanoparticle have non-immunogenicity and non-toxicity when administered by repeated intravenous injection of 30 mg/kg.

Therefore, in order to further reduce the sensitivity of the above nucleic acid nanoparticle to the RNA enzymatic degradation, and improve the stability in the delivery process, in a preferred embodiment, a base, a ribose and a phosphate in the sequence a, the sequence b and the sequence c have at least one modifiable site, and any one of the modifiable sites is modified by any one of the following modification linkers: -F, a methyl, an amino, a disulfide, a carbonyl, a carboxyl, a sulfhydryl and a formyl; and preferably, the base C or U in the sequence a, the sequence b and the sequence c has 2'-F modification. While the modification linker is the sulfhydryl, it belongs to a thio modification, modification strength is weaker, and a cost is low.

The above drug may be loaded in the modes of the physical linkage and/or the covalent linkage. While the drug is linked with the nucleic acid domain by using two modes of the physical insertion and the covalent linkage simultaneously, the physical insertion is usually inserted between the GC base pairs, the number of the preferred insertion sites is based on the different numbers of the GC base pairs on the the nucleic acid domain, and the insertion is performed according to the ratio of 1 to 100:1. While the covalent linkage mode is used for linkage, the above drug usually chemically reacts with a G-exocyclic amino to form the covalent linkage. More preferably, a molar ratio between the drug and the nucleic acid nanoparticle is 2 to 300:1, preferably 2 to 290:1, more preferably 2 to 29:1, further preferably 10 to 50:1, and most preferably 15 to 25:1.

In the nucleic acid nanocarrier drug provided in the present application, the nucleic acid nanoparticle are served as a drug delivery carrier. In addition, according to different drug purposes, in a preferred embodiment, the above nucleic acid nanoparticle also include the bioactive substance, and the bioactive substance is linked with the nucleic acid domain. The bioactive substance is one of more of a target head, a fluorescein, an interfering nucleic acid siRNA, an miRNA, a ribozyme, a riboswitch, an aptamer, a RNA antibody, a protein, a peptide, a flavonoid, a glucose, a natural salicylic acid, a monoclonal antibody, a vitamin, a phenol, a lecithin and a small molecular drug, herein the small molecular drug does not include the tacrine, the epirubicin, the methotrexate, the pirarubicin, the daunorubicin, the pentafluorouracil, the 10-hydroxycamptothecin, the aspirin and the gemcitabine.

In order to improve loading efficiency and delivering efficiency of the nucleic acid nanoparticle to the loaded bioactive substance, a relative molecular weight of the the nucleic acid domain and a total relative molecular weight of the drug and the bioactive substance should preferably have a certain matching relation. In a preferred embodiment, the relative molecular weight of the nucleic acid domain is marked as N1, and the total relative molecular weight of the drug and the bioactive substance is marked as N2, N1/ N2≥1:1.

According to the different types of the bioactive substances loaded specifically, performance optimization effects thereof on the nucleic acid nanoparticle of the present application are not the same. For example, when the bioactive substance is a biotin or a folic acid, a function thereof is to make the nucleic acid nanoparticle have a targeting property, for example, specifically targeted to cancer cells. While the bioactive substance is the fluorescein, a function thereof is to make the nucleic acid nanoparticle have a luminous tracking effect. While the bioactive substance is some siRNA, miRNA, protein, peptide, RNA antibody and small molecular drug, according to the different biological functions, the nucleic acid nanocarrier drug may be made into a new product with a specific therapeutic effect, such as a drug with more excellent performance. In addition, according to the different types of the bioactive substances loaded specifically, the DNA nanoparticle and the RNA nanoparticle are specifically preferably used, and may be rationally selected according to the actual needs. For example, when the bioactive substance is the drug, the DNA nanoparticle and the RNA nanoparticle are preferably used for loading, and there is no special requirement for a length of the single strand of the nanoparticle formed by assembly.

In a preferred embodiment, the bioactive substance is the target head, the fluorescein and the miRNA, herein, the target head is positioned on any one sequence of the sequences a, b and c, preferably the 5'-end or the 3'-end of any one sequence of the sequences a, b and c, or inserted between GC bonds of the the nucleic acid domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c; and preferably, the target head is the folic acid or the biotin, the fluorescein is any one or more of FAM, CY5 and CY3, and the anti-miRNA is anti-miR-21.

The above target head may be linked on any one sequence of the sequences a, b and c in a mode of linker covalent linkage, the available linker is selected from a disulfide bond, a p-phenylazide, bromopropyne or PEG. Here, the "on any one sequence" refers to on a base in any one position of any one sequence of the sequences a, b and c, and it is more convenient to be linked at the 5'-end or 3'-end, the application is wider. Folic acid modification may be physical insertion mode linkage or physical insertion + covalent linkage.

The above fluorescein may be a commonly used fluorescein, and preferably any one of more of FAM, CY5 and CY3.

The above miRNA may be a miRNA with a cancer suppression effect, or an anti-miRNA which may suppress a corresponding disease, and it may be rationally selected in practical applications according to medical needs. The above anti-miRNA may be synthesized at any one or more positions of the 3'-end of the above sequence a, the 5'-end and 3'-end of the sequence c. While the anti-miRNA is synthesized in the above three positions, the anti-miRNA has a relatively stronger suppression effect on the corresponding miRNA.

It is anti-miR-21 preferably, the miR-21 participates in initiation and progression of multiple types of the cancers, and is a main oncogene for invasion and metastasis. The anti-miR-21 may effectively regulate a wide range of target genes at the same time, and is beneficial to solve a problem of cancer heterogeneity. Therefore, in the above preferred nucleic acid nanoparticle, the target head, such as the folic acid or the biotin, may be specifically targeted to the cancer cells, and after being linked and internalized with the cancer cells, the anti-miR-21 is complemented with a miR-21 base in very high affinity and specificity, thereby the expression of the oncogenic miR-21 is effectively reduced. Therefore,

according to the actual needs, the above anti-miR-21 may be synthesized at any one or more positions of the 3'-end of the above sequence a, the 5'-end and the 3'-end of the sequence c. While the anti-miR-21 is synthesized in the above three positions, the anti-miR-21 has a relatively stronger suppression effect on the miR-21.

While the above bioactive substance capable of loading is other small molecular drugs except the tacrine, the epirubicin, the methotrexate, the pirarubicin, the daunorubicin, the pentafluorouracil, the 10-hydroxycamptothecin, the aspirin and the gemcitabine, the nucleic acid nanocarrier drug, according to types of diseases which may be treated by the different drugs, includes but not limited to drugs for treating liver cancer, gastric cancer, lung cancer, breast cancer, head and neck cancer, uterine cancer, ovarian cancer, melanoma, leukemia, Alzheimer's disease, ankylosing spondylitis, malignant lymphoma, bronchial cancer, rheumatoid arthritis, HBV hepatitis B, multiple myeloma, pancreatic cancer, non-small cell lung cancer, prostate cancer, nasopharyngeal cancer, esophageal cancer, oral cancer, lupus erythematosus; and preferably, the head and neck cancer is brain cancer, neuroblastoma or glioblastoma.

While the above bioactive substance capable of loading is other small molecular drugs except the above tacrine and the like, according to a difference of molecular structures of the drugs or a difference of characteristic groups which it has, the drug includes but not limited to a drug containing any one or more of the following groups: an amino group, a hydroxyl group, a carboxyl group, a mercapto group, a benzene ring group and an acetamido group.

In a preferred embodiment, the above protein is one or more of antibodies or aptamers of superoxide dismutase (SOD), survivin, human telomerase reverse transcriptase (hTERT), epidermal growth factor receptor (EGFR) and prostate-specific membrane antigen (PSMA); the above vitamin is L-Vc and/or esterified Vc; and the above phenol is a tea polyphenol and/or a grape polyphenol.

In a preferred embodiment, a particle size of the nucleic acid nanoparticle is 1-100 nm, preferably 5-50 nm; more preferably 10-30 nm; and further preferably 10-15 nm. The size is appropriate within this range, it may not only enter a cell membrane through cell phagocytosis mediated by a cell surface receptor, but also avoid non-specific cell penetration so as to be filtered and removed by the kidneys. Therefore, the favorable particle size is helpful to improve pharmacokinetics, pharmacodynamics, biological distribution and toxicological distribution.

According to a second aspect of the present application, a preparation method for the above nucleic acid nanocarrier drug is further provided, and the preparation method includes the following steps: any one of the above nucleic acid nanoparticle is provided; a drug is loaded on the nucleic acid nanoparticle in modes of physical linkage and/or covalent linkage, to obtain the nucleic acid nanocarrier drug.

While the physical linkage mode is used, the drug may be usually formed and inserted between GC base pairs in a physical insertion form. While the covalent linkage mode is used for linkage, the drug usually chemically reacts with a G-exocyclic amino to form the covalent linkage. The nucleic acid nanocarrier drug prepared by using the above method may have the better targeting property after it is modified by the target head, the drug may be stably delivered, and the reliability is very high.

In a preferred embodiment, the step of loading the drug in the physical linkage mode includesenabling the drug, the nucleic acid nanoparticle and a first solvent to be mixed and stirring, to obtain a premixed system; and precipitating the premixed system, to obtain the nucleic acid nanocarrier drug. Specific dosages of the drug and the nucleic acid nanoparticle may be adjusted according to a change of the loading amount, this may be understood by those skilled in the art, and it is not repeatedly described here.

In order to improve the efficiency and the stability of the physical linkage, an amount of the drug added per liter of the first solvent is preferably 0.1 to 1 g. Preferably, the first solvent is selected from one or more of DCM, DCC, DMAP, Py, DMSO, PBS and glacial acetic acid. Preferably, the step of precipitating the premixed system, to obtain the nucleic acid nanocarrier drug includes precipitating the premixed system, to obtain a precipitation; and washing the precipitation to remove impurities, to obtain the nucleic acid nanocarrier drug. More preferably, the premixed system is mixed with absolute ethyl alcohol, and precipitated at a temperature condition lower than 10 DEG C, to obtain the precipitation, further preferably, the precipitation is obtained by precipitating at a temperature condition of 0-5 DEG C. More preferably, the precipitation is washed to remove the impurities with 6-12 times of the absolute ethyl alcohol in volume, as to obtain the nucleic acid nanocarrier drug.

In a preferred embodiment, the step of loading the drug in the covalent linkage mode includes drug solution is prepared; the drug solution reacts with the G-exocyclic amino of the nucleic acid nanoparticle under a mediating effect of the formaldehyde, to obtain a reaction system; and the reaction system is purified, to obtain the nucleic acid nanocarrier drug.

Through a formaldehyde-mediated form, the following reaction may occur:

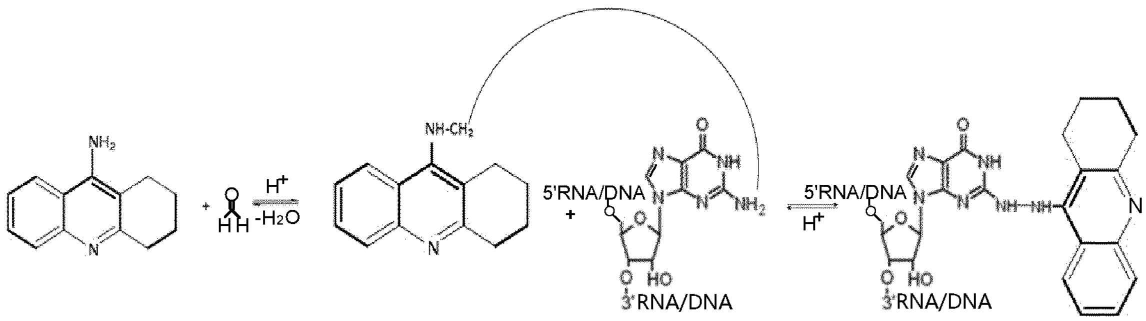

Pref erably, the above reaction step includes the drug solution is mixed with paraformaldehyde solution and the nucleic acid nanoparticle, and it is reacted in a dark condition, to obtain the reaction system; herein the concentration of the paraformaldehyde solution is preferably 3.7-4wt%, and the paraformaldehyde solution is preferably solution formed by mixing paraformaldehyde and a second solvent, and the second solvent is one or more of DCM, DCC, DMAP, Py, DMSO, PBS and glacial acetic acid.

In the above preparation method, the nucleic acid nanoparticle may be prepared in a mode of self-assembly, for example: (1) enabling the RNA or DNA single strands a, b and c to be simultaneously mixed and dissolved in DEPC water or TMS buffer solution; (2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min; (3) enabling a product to be loaded on 8% (m/v) of non-denaturing PAGE gel and placed in TBM buffer solution under a condition of 4 DEG C, purifying a complex by 100 v of electrophoresis; and (4) cutting a target band and eluting in RNA/DNA elution buffer solution at 37 DEG C, after that, precipitating overnight in ethanol, volatilizing under reduced pressure and low temperature, to obtain a self-assembly product, namely the the nucleic acid domain, and then acquiring the nucleic acid nanoparticle.

In order to make the above nucleic acid nanocarrier drug have other functions, in a preferred embodiment, after the nucleic acid domain is obtained, the preparation method further includesenabling the bioactive substance as mentioned above to be loaded on the the nucleic acid domain in the modes of physical linkage and/or covalent linkage, to obtain the nucleic acid nanoparticle. The loading mode of the bioactive substance may also be the physical linkage and/or the covalent linkage. The covalent linkage mode includes but not limited to the solvent covalent linkage, the linker covalent linkage or the click-linkage; preferably, a third solvent used in the solvent covalent linkage is served as a linkage medium, and the third solvent is selected from one or more of paraformaldehyde, DCM, DCC, DMAP, Py, DMSO, PBS and glacial acetic acid; preferably, the linker is selected from a disulfide bond, a p-phenylazide, bromopropyne or a PEG; and preferably, the click-linkage is that alkynyl or azide modification is simultaneously performed on a bioactive substance precursor and the the nucleic acid domain, and then they are linked through the click-linkage.

It is to be noted that the above classification does not mean that there is only one linkage mode to link a certain bioactive substance with the nucleic acid nanocarrier. However, some bioactive substances may be linked with the nucleic acid nanocarrier in the physical insertion mode, or may be linked with the nucleic acid nanocarrier in the physical insertion and covalent linkage modes, or may be linked by using the click-linkage mode at the same time. However, for a certain specific bioactive substance, there may be only one linkage mode, or there may be multiple linkage modes, but it may be that the efficiency of certain linkage has an advantageous practical value.

In the above linkage modes, when the different drugs are linked with the the nucleic acid domain in the physical insertion mode, insertion linkage sites and numbers are also different slightly. For example, when anthracycline and acridine drugs are inserted, they are usually inserted between the GC base pairs, the preferred number of the insertion sites is based on the different numbers of the GC base pairs in the the nucleic acid domain, and the insertion is performed in a ratio of 1 to 100:1. While a naphthylamide drug is inserted, it is usually inserted between the AA base pairs, the preferred number of the insertion sites is based on the different numbers of the AA base pairs in the the nucleic acid domain, and pyridocarbazoles are inserted according to the different numbers of the AA base pairs in a ratio of 1 to 200:1.

Specifically, according to the different types of the bioactive substances, the lengths of the sequences a, b and c for forming the the nucleic acid domain in the nucleic acid nanoparticle and the number of GC complementary base pairs thereof, the physical insertion may be performed by rationally selecting a molar ratio of the bioactive substance and the the nucleic acid domain.

In a preferred embodiment, when the bioactive substance is linked with the nucleic acid domain in the physical insertion mode and the covalent linkage mode, a molar ratio of the bioactive substance linked in the physical insertion mode and the drug linked in the covalent linkage mode is 1 to 200:1. The linkage mode is suitable for the anthracycline and acridine drugs. A proportion of the drugs linked in the above different linkage modes is not limited to the above range, as long as it may meet a requirement of high-efficient loading, there is a non-toxic effect on the cells, and the

effective release of the drug is achieved after reaching a target.

While a bioactive substance precursor and the the nucleic acid domain are simultaneously modified by an alkynyl or an azide, and linked in the click-linkage mode, the different click-linkages are selected according to changes of the different structures of the drugs. In addition, along with the different structures of the bioactive substances, the linkage positions may also be changed correspondingly, this may be understood by those skilled in the art.

In a preferred embodiment, when the bioactive substance is linked with the the nucleic acid domain in the click-linkage mode, a site, for performing the alkynyl or azide modification, of the bioactive substance precursor is selected from a hydroxyl, a carboxyl, a mercapto or an amino, and a site, for performing the alkynyl or azide modification, of the the nucleic acid domain is selected from the amino, an imino or the hydroxyl.

It is to be noted that when the above nucleic acid domain is linked with the drug, the nucleic acid domain is water-soluble, and most of the drugs have poor water solubility. After it is linked with the nucleic acid domain, the water solubility is improved. When the above drugs are anthracyclines, these drugs are covalent-linked with the nucleic acid domain through a -NH bond (under a suitable pH value condition, the activity of the -NH group is hundreds of times greater than the activity of other groups which may be covalent-linked with the drugs) on a nucleotide guanosine, thereby the the nucleic acid domain for loading the drugs is formed. Therefore, according to a size of a specific drug molecule and the number of the GC base pairs on the sequence a, the sequence b and the sequence c in the specifically designed the nucleic acid domain, when linked, a linking reaction is performed theoretically according to 1.1-1.3 times of a supersaturated linking amount, and at most 35 to 45 drugs may be linked in one the nucleic acid domain. While the above drugs are other structures, the loading amount is related to an occupation situation (including but not limited to a molecular structure, a morphology, a shape and a molecular weight) of the specific drug. Therefore, a linkage condition of an activity site of the drug and the -NH bond on the nucleotide guanosine of the the nucleic acid domain is relatively harsh, and it may also be loaded but it is relatively difficult to cause a situation of excessive linkage. According to a third aspect of the present application, a pharmaceutical composition is further provided, and the pharmaceutical composition includes any one of the above nucleic acid nanocarrier drugs. Specifically, suitable combination drugs or auxiliaries may be selected according to actual needs to form a drug combination that has combined efficacy or may improve a certain aspect of the drug properties (such as stability).

According to a fourth aspect of the present application, an application of any one of the above nucleic acid nanocarrier drugs in preparing a drug for treating an Alzheimer's disease, a tumor, an autoimmune disease or a heart disease. For specific applications, a new drug may be obtained by improving the drug itself on the basis of the drug of the present application, or the drug of the present application is served as a main active ingredient and prepared into a preparation in a suitable dosage form and the like.

Specifically, according to the different drugs in the nucleic acid nanocarrier drugs, the diseases which may be treated are also different. While the drug in the nucleic acid nanocarrier drug includes the tacrine, it may be used to prepare a drug for Alzheimer's disease. While the drug includes the epirubicin, the above nucleic acid nanocarrier drug may be used to prepare a drug for the treatment of a tumor, and the tumor may be any one of more of acute leukemia, malignant lymphoma, breast cancer, bronchial lung cancer, ovarian cancer, Wilms tumor, soft tissue sarcoma, primary hepatocellular carcinoma, metastatic liver cancer, and medullary thyroid carcinoma.

While the drug includes methotrexate, the above nucleic acid nanocarrier drug may be used to prepare a drug for preventing and/or treating the tumor or the autoimmune disease. Preferably, the tumor targeted is any one of more of acute leukemia, breast cancer, choriocarcinoma, malignant hydatidiform mole, head and neck tumors, bone tumors, leukemia meninges spinal cord infiltration, lung cancer, reproductive system tumors, and liver cancer, and the autoimmune disease is any one or more of refractory psoriasis, systemic lupus erythematosus, mandatory spondylitis and dermatomyositis.

While the drug includes the pirarubicin, the above nucleic acid nanocarrier drug may be used to prepare a drug for treating the tumor. Preferably, the tumor is any one or more of the breast cancer, head and neck cancer, bladder cancer, ureteral cancer, renal pelvis cancer, ovarian cancer and cervical cancer.

While the drug includes the daunorubicin, the above nucleic acid nanocarrier drug may also be used to prepare a drug for treating the tumor. Preferably, the tumor is the acute lymphocytic leukemia or granulocytic leukemia.

While the drug includes the pentafluorouracil, the above nucleic acid nanocarrier drug may also be used to prepare a drug for treating the tumor. Preferably, it may be used to prepare drugs for treating the liver cancer, colon cancer, rectal cancer, stomach cancer, breast cancer, ovarian cancer, choriocarcinoma, malignant hydatidiform mole, head and neck squamous cell carcinoma, skin cancer, lung cancer, cervical cancer, pancreatic cancer or bladder cancer.

While the drug is the 10-hydroxycamptothecin, the above nucleic acid nanocarrier drug may also be used to prepare a drug for treating the liver cancer, stomach cancer, head and neck cancer or leukemia.

While the drug is the aspirin, the above nucleic acid nanocarrier drug may be used to prepare drugs for antipyretic and analgesic, preventing the heart disease and cerebral thrombosis, anti-inflammatory and anti-rheumatic, treating arthritis, alleviating skin mucosal lymph node syndrome in a patient with a Kawasaki disease, resisting the cancer, and preventing digestive tract tumor.

While the drug includes the gemcitabine, the above nucleic acid nanocarrier drug may also be used to prepare a drug for treating the tumor. Preferably, it may be used to prepare drugs for treating the pancreatic cancer, non-small cell lung cancer, ovarian cancer, breast cancer, bladder cancer, cervical cancer, liver cancer, biliary tract cancer, nasopharyngeal cancer, testicular tumor, lymphoma, mesothelioma or head and neck cancer.

According to a fifth aspect of the present application, a method for preventing and/or treating an Alzheimer's disease, a tumor, an autoimmune disease or a heart disease is further provided, the method includes: any one of the above nucleic acid nanocarrier drugs or pharmaceutical compositions is provided; and an effective dosage of the above nucleic acid nanocarrier drug or pharmaceutical composition is administered to a patient with the Alzheimer's disease, the tumor, the autoimmune disease or the heart disease. The effective dosage herein includes a prophylactically effective dosage and/or a therapeutically effective dosage. The therapeutically effective dosage refers to a dosage that is effective to achieve a desired therapeutic result, such as a reduction of the Alzheimer's disease, within a necessary dosage and time period. In a specific implementation mode, the dosage may be adjusted to provide the optimal therapeutic response dosage, and the therapeutically effective dosage may be varied according to the following factors: a disease state, an age, a gender, and a weight of an individual and an ability of a preparation which causes a desired response in the individual. The meaning of the therapeutically effective dosage also includes a dosage of which beneficial effects of treatment exceed its toxic or harmful effects. The prophylactically effective dosage refers to a dosage that is effective to achieve the desired preventive result, such as preventing or inhibiting the occurrence of the Alzheimer's disease, within the necessary dosage and time period. The prophylactically effective dosage may be determined according to the above description of the therapeutically effective dosage. For any specific subjects, the specific dosage may be adjusted along with time according to individual needs and the professional judgment of an administering person.

It is to be noted that the nucleic acid nanoparticle formed by the self-assembly of the sequences or sequence variations provided by the present application may also be used as basic structural units, and may be further polymerized to form multimers, such as a dimer, a trimer, a tetramer, a pentamer, a hexamer or a heptamer, according to the actual application needs.

The beneficial effects of the present application are further described below in combination with specific embodiments.

Assembly of nucleic acid nanoparticles

**Embodiment 1**

**I. RNA and DNA nanoparticle carrier:**

(1) Three polynucleotide base sequences for assembling RNA nanoparticles are specifically shown in Table 1:

Table 1:

| Name | Sequence sense (5'-3') | Base number | Molecular weight | Chemical modification | Fluorescence mark | Special modification | Total molecular weight |
|---|---|---|---|---|---|---|---|
| a-str and (SEQ ID NO: 52) | cGcGcGcccAccAGc GuuccGGGcGccGc | 29 | 9678. 7 | C/U base 2'Fmodification | / | 5'Biotin | 29524.9 |
| b-strand (SEQ ID NO: 53) | GcGGcGcccGGuuc GccGccAGGcGGc | 27 | 9116. 8 | C/U base 2'F modification | / | 5'Biotin | |
| c-strand (SEQ ID NO: 54) | GccGccAGGcGGcc AuAGcGGuGGGcG cGcG | 31 | 10729 .4 | C/U base 2'F modification | 5'CY3 | | |

(2) Three polynucleotide base sequences of DNA nanoparticles

DNA uses the same sequence as the above RNA, except that U is replaced by T. Herein, a molecular weight of the a-strand is 8802.66, a molecular weight of the b-strand is 8280.33, and a molecular weight of the c-strand is 9605.2.

The strands a, b and c of the above RNA nanoparticles and DNA nanoparticles are all commissioned to be synthesized by Sangon Bioengineering (Shanghai) Co., Ltd.

II. Self-assembly experiment steps:

(1) according to a molar ratio of 1:1:1, enabling the RNA or DNA single strands a, b and c to be simultaneously mixed and dissolved in DEPC treated water or TMS buffer solution;

(2) heating mixed solution to 80/95 DEG C (herein a RNA assembly temperature is 80 DEG C, and a DNA assembly temperature is 95 DEG C), after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA/DNA elution buffer solution at 37 DEG C, after that, precipitating overnight in ethanol, volatilizing under reduced pressure and low temperature, to obtain a self-assembly product; and

(5) electrophoresis analysis detection and laser scanning observation.

III. Self-assembly experiment result

Electrophoresis detection result

An electrophoresis detection result of the RNA self-assembly product is shown in Fig. 1. In Fig. 1, lanes 1 to 3 from left to right are successively: the a-strand, the b-strand, and the RNA self-assembly product. It may be seen from the figure that although the RNA self-assembly product is slightly diffused, it may be apparently seen that it is a single band. In addition, because the molecular weight is a molecular weight after assembly, it is larger than a single-stranded molecular weight, a band position is behind the a-strand and the b-strand, and the actual situation is consistent with a theory, it is proved that a stable composite structure is formed between the above RNA single strands by the self-assembly, and RNA nanoparticles are formed.

An electrophoresis detection result of the DNA self-assembly product is shown in Fig. 2. In Fig. 2, lanes 1 to 3 from left to right are successively: the a-strand, the b-strand, and the DNA self-assembly product. It may be seen from the figure that a band of the DNA self-assembly product is bright and clear, and is a single band, it is proved that a stable composite structure is formed between the above DNA single strands by the self-assembly, and DNA nanoparticles are formed.

In the embodiment, it is verified by gel electrophoresis that the sequences a, b and c including the RNA core sequences SEQ ID NO:1, SEQ ID NO:3 and SEQ ID NO:5 may be successfully self-assembled into the RNA nanoparticles. The sequences a, b and c including the DNA core sequences SEQ ID NO:2, SEQ ID NO:4 and SEQ ID NO:6 may also be successfully self-assembled into the DNA nanoparticles.

In addition to the core sequences which form a nucleic acid structural domain, the sequences a, b and c of the above RNA nanoparticles and DNA nanoparticles also have various extension sequences (including a drug loading linking sequence) which promote a loading function of the nucleic acid structural domain and a target head or a fluorescein which is linked with the nucleic acid structural domain. It may be seen that the presence of substances other than these core sequences does not affect the formation of the nucleic acid structural domain and the successful self-assembly of the nucleic acid nanoparticles. The self-assembled nucleic acid nanoparticles may have a targeting property under the guidance of the target head, and the fluorescein may make the nucleic acid nanoparticles have visibility and traceability.

**Embodiment 2**

I. 7 groups of short-sequence RNA nanoparticle carriers:

(1) 7 groups of three polynucleotide base sequences for forming RNA nanoparticles are shown in Table 2 to Table 8:

Table 2: R-1:

| Name | Sequence sense (5'-3') | Base number | Chemical modification | Molecular weight | Total molecular weight |
|---|---|---|---|---|---|
| a-strand (SEQ ID NO: 55) | **GG**AGcGuu**GG** | 10 | c/u base 2'F modification | 3262.9 | 9828.7 |
| b-strand (SEQ ID NO: 56) | **cc**uuc**GCCG** | 9 | c/u base 2'F modification | 2780.6 | |
| c-strand (SEQ ID NO: 57) | **cGGcc**AuAGc**cc** | 12 | c/u base 2'F modification | 3785.2 | |

Table 3: R-2:

| Name | Sequence sense (5'-3') | Base number | Chemical modification | Molecular weight | Total molecular weight |
|---|---|---|---|---|---|
| a-strand (SEQ ID NO: 58) | **Gc**AGcGuu**cG** | 10 | c/u base 2'F modification | 3186.7 | 9829.4 |
| b-strand (SEQ ID NO: 59) | **cG**uuc**GccG** | 9 | c/u base 2'F modification | 2820.2 | |
| c-strand (SEQ ID NO: 60) | **cGGc**cAuAGc**Gc** | 12 | c/u base 2'F modification | 3822.5 | |

Table 4: R-3:

| Name | Sequence sense (5'-3') | Base number | Chemical modification | Molecular weight | Total molecular weight |
|---|---|---|---|---|---|
| a-strand (SEQ ID NO: 61) | **cG**AGcGuu**Gc** | 10 | c/u base 2'F modification | 3187.5 | 9829.9 |
| b-strand (SEQ ID NO: 62) | **Gc**uuc**GccG** | 9 | c/u base 2'F modification | 2819.2 | |
| c-strand (SEQ ID NO: 63) | **cGGc**cAuAGc**cG** | 12 | c/u base 2'F modification | 3823.2 | |

Table 5: R-4:

| Name | Sequence sense (5'-3') | Base number | Chemical modification | Molecular weight | Total molecular weight |
|---|---|---|---|---|---|
| a-strand (SEQ ID NO: 64) | **GG**AGcGuu**GG** | 10 | c/u base 2'F modification | 3263.7 | 9830.9 |
| b-strand (SEQ ID NO: 65) | **cc**uuc**GGGG** | 9 | c/u base 2'F modification | 2858.2 | |
| c-strand (SEQ ID NO: 66) | **cccc**cAuAGc**cc** | 12 | c/u base 2'F modification | 3709.0 | |

Table 6: R-5:

| Name | Sequence sense (5'-3') | Base number | Chemical modification | Molecular weight | Total molecular weight |
|---|---|---|---|---|---|
| a-strand (SEQ ID NO: 67) | **Gc**AGcGuu**cG** | 10 | c/u base 2'F modification | 3187.5 | 9830.2 |
| b-strand (SEQ ID NO: 68) | **cG**uuc**GGcG** | 9 | c/u base 2'F modification | 2857.5 | |
| c-strand (SEQ ID NO: 69) | **cGcc**cAuAGc**Gc** | 12 | c/u base 2'F modification | 3785.2 | |

Table 7: R-6:

| Name | Sequence sense (5'-3') | Base number | Chemical modification | Molecular weight | Total molecular weight |
|---|---|---|---|---|---|
| a-strand (SEQ ID NO: 70) | **Gc**AGcGuu**cG** | 10 | c/u base 2'F modification | 3187.2 | 9830.5 |
| b-strand (SEQ ID NO: 71) | **cG**uuc**GGcc** | 9 | c/u base 2'F modification | 2820.4 | |
| c-strand (SEQ ID NO: 72) | **GGcc**cAuAGc**Gc** | 12 | c/u base 2'F modification | 3822.9 | |

Table 8: R-7:

| Name | Sequence sense (5'-3') | Base number | Chemical modification | Molecular weight | Total molecular weight |
|---|---|---|---|---|---|
| a-strand (SEQ ID NO: 73) | **cG**AGcGuu**Gc** | 10 | c/u base 2'F modification | 3187.6 | 9830.7 |
| b-strand (SEQ ID NO: 74) | **Gc**uuc**GGcG** | 9 | c/u base 2'F modification | 2857.8 | |
| c-strand (SEQ ID NO: 75) | **cGcc**cAuAGc**cG** | 12 | c/u base 2'F modification | 3785.3 | |

The single strands of the above 7 groups of the short-sequence RNA nanoparticle carriers are all commissioned to be synthesized by Sangon Bioengineering (Shanghai) Co., Ltd.

II. Self-assembly experiment steps:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, volatilizing under reduced pressure and low temperature, to obtain a short-sequence RNA self-assembly product;

(5) electrophoresis analysis detection and laser scanning observation; and

(6) electric potential detection.

III. Self-assembly experiment result

(1) Electrophoresis detection result

A 2% agarose gel electrophoresis diagram of the 7 groups of the short-sequence RNA self-assembly products is shown in Fig. 3. From left to right, lanes 1 to 7 in Fig. 3 are successively: short-sequences R-1, R-2, R-3, R-4, R-5, R-6 and R-7.

A 4% agarose gel electrophoresis diagram of the 7 groups of the short-sequence RNA self-assembly products is shown in Fig. 4. From left to right, lanes 1 to 7 in Fig. 4 are successively: short-sequences R-1, R-2, R-3, R-4, R-5, R-6 and R-7.

It may be clearly seen from results of Fig. 3 and Fig. 4 that bands of the R-2, R-3, R-5 and R-7 in the 7 groups of the short-sequence self-assembly products are bright and clear, although the R-1, R-4 and R-6 are relatively diffused, it may still be seen as a single band, it is indicated that the 7 groups of the short-sequences may be self-assembled better into a RNA nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 7 groups of the short-sequence RNA nanoparticles are shown below in Table 9 to Table 15:

Table 9:

| Sample name | Detection times | Potential ZP (mV) |
|---|---|---|
| R-1 | 1 | -33.7 |
| | 2 | -35.6 |
| | 3 | -34.6 |
| Experiment result | -34.65 mV. (±0.95mV) | |

Table 10:

| Sample name | Detection times | Potential ZP (mV) |
|---|---|---|
| R-2 | 1 | -37.9 |
| | 2 | -37.3 |
| | 3 | -36.8 |
| Experiment result | -37.35 mV. (±0.55mV) | |

Table 11:

| Sample name | Detection times | Potential ZP (mV) |
|---|---|---|
| R-3 | 1 | -31.5 |
| | 2 | -33 |
| | 3 | -32.7 |

(continued)

| Sample name | Detection times | Potential ZP (mV) |
|---|---|---|
| Experiment result | -32.425 mV. (±0.75mV) | |

Table 12:

| Sample name | Detection times | Potential ZP (mV) |
|---|---|---|
| R-4 | 1 | -35.40 |
| | 2 | -36.00 |
| | 3 | -35.50 |
| Experiment result | -35.7 mV. (±0.3mV) | |

Table 13:

| Sample name | Detection times | Potential ZP (mV) |
|---|---|---|
| R-5 | 1 | -34.00 |
| | 2 | -33.30 |
| | 3 | -34.90 |
| Experiment result | -34.1 mV. (±0.8mV) | |

Table 14:

| Sample name | Detection times | Potential ZP (mV) |
|---|---|---|
| R-6 | 1 | -33.10 |
| | 2 | -36.10 |
| | 3 | -35.60 |
| Experiment result | -34.6 mV. (±1.5mV) | |

Table 15:

| Sample name | Detection times | Potential ZP (mV) |
|---|---|---|
| R-7 | 1 | -35.60 |
| | 2 | -34.80 |
| | 3 | -34.00 |
| Experiment result | -34.80 mV. ±0.8mV | |

It may be seen from the above potential detection data that the 7 groups of the short-sequence RNA self-assembly products all have good stability, and it is further indicated that the nanoparticles formed by the self-assembly of each short-sequence RNA have a relatively stable self-assembly structure.

It is indicated from the embodiment that: different combinations of the core sequences a, b and c may form the RNA nanoparticles with the nucleic acid structural domain through the self-assembly, and the structure is stable. It may be seen on the basis of Embodiment 1 that the RNA nanoparticles may also be successfully assembled by adding various functional extension fragments or a linkage target head, a fluorescein and the like on the basis of these different core sequence combinations, and have functions such as drug loading, cell targeting, visibility and traceability.

In order to further verify these performances, the extension fragment is added on the basis of Embodiment 2,

and it is specifically described in Embodiment 3. On the basis of the DNA core sequence corresponding to the RNA core sequence of Embodiment 2, the extension fragment is added, and the target head is connected or unconnected at the same time, it is specifically described in Embodiment 4.

**Embodiment 3**

I. 7 groups of conventional-sequence RNA nanoparticle carriers:

(1) 7 groups of three polynucleotide base sequences for forming RNA nanoparticles are shown in Table 16 to Table 22:

Table 16: R-8

| Name | Sequence sense (5'-3') | Base number | Chemical modification | Molecular weight | Total molecular weight |
|---|---|---|---|---|---|
| a (SEQ ID NO: 76) | cGcGcGcccA**GG**AGcGuu**GG**cGGGcGGcG | 29 | C/U 2'F modification | 9462.78 | 28084.13 |
| b (SEQ ID NO: 77) | cGccGcccG**cc**uuc**GccG**ccAGccGcc | 27 | C/U 2'F modification | 8522.18 | |
| c (SEQ ID NO: 78) | GGcGGcAGG**cGGc**cAuAGc**cc**uGGGcGcGcG | 31 | C/U 2'F modification | 10099.17 | |

Table 17: R-9

| Name | Sequence sense (5'-3') | Base number | Chemical modification | Molecular weight | Total molecular weight |
|---|---|---|---|---|---|
| a (SEQ ID NO: 79) | cGcGcGcccA**Gc**AGcGuu**cG**cGGGcGGcG | 29 | C/U 2'F modification | 9386.73 | 28084.13 |
| b (SEQ ID NO: 80) | cGccGcccG**cG**uuc**GccG**ccAGccGcc | 27 | C/U 2'F modification | 8560.20 | |
| c (SEQ ID NO: 81) | GGcGGcAGG**cGGc**cAuAGc**Gc**uGGGcGcGcG | 31 | C/U 2'F modification | 10137.20 | |

Table 18: R-10

| Name | Sequence sense (5'-3') | Base number | Chemical modification | Molecular weight | Total molecular weight |
|---|---|---|---|---|---|
| a (SEQ ID NO: 82) | cGcGcGcccA**cG**AGcGuu**Gc**GGGGcGGcG | 29 | C/U 2'F modification | 9424.75 | 28084.13 |
| b (SEQ ID NO: 83) | cGccGcccc**Gc**uuc**GccG**ccAGccGcc | 27 | C/U 2'F modification | 8522.18 | |
| c (SEQ ID NO: 84) | GGcGGcAGG**cGGc**cAuAGc**cG**uGGGcGcGcG | 31 | C/U 2'F modification | 10137.20 | |

Table 19: R-11

| Name | Sequence sense (5'-3') | Base number | Chemical modification | Molecular weight | Total molecular weight |
|---|---|---|---|---|---|
| a (SEQ ID NO: 85) | cGcGcGcccA**GG**AGCGuu**GG**cccGcGGcG | 29 | C/U 2'F modification | 9386.73 | 28084.13 |
| b (SEQ ID NO: 86) | cGccGcGGG**cc**uuc**GGGG**ccAGccGcc | 27 | C/U 2'F modification | 8674.28 | |
| c (SEQ ID NO: 87) | GGcGGcAGG**ccccc**AuAGc**cc**uGGGcGcGcG | 31 | C/U 2'F modification | 10023.12 | |

Table 20: R-12

| Name | Sequence sense (5'-3') | Base number | Chemical modification | Molecular weight | Total molecular weight |
|---|---|---|---|---|---|
| a (SEQ ID NO: 88) | cGcGcGcccA**Gc**AGcGuu**cG**ccccGccGc | 29 | C/U 2'F modification | 9234.62 | 28084.18 |
| b (SEQ ID NO: 89) | GcGGcGGGG**cG**uuc**GGcG**GcAGGcGGc | 27 | C/U 2'F modification | 8864.41 | |
| c (SEQ ID NO: 90) | GccGccAGc**cGcc**cAuAGc**Gc**uGGGcGcGcG | 31 | C/U 2'F modification | 9985.10 | |

Table 21: R-13

| Name | Sequence sense (5'-3') | Base number | Chemical modification | Molecular weight | Total molecular weight |
|---|---|---|---|---|---|
| a (SEQ ID NO: 91) | cGcGcGcccA**Gc**AGcGuu **cG**GGGcGccGc | 29 | C/U 2'F modification | 9348.70 | 28736.53 |
| b (SEQ ID NO: 92) | GcGGcGccc**cG**uuc**GGcc** GGcAGGcGGc | 28 | C/U 2'F modification | 9057.52 | |
| c (SEQ ID NO: 93) | GccGccAGcc**GGccc**AuA Gc**Gc**uGGGcGcGcG | 32 | C/U 2'F modification | 10330.31 | |

Table 22: R-14 (uGAcAGAuAAGGAAccuGcudTdT in the following a-strand is survivin siRNA)

| Name | Sequence sense (5'-3') | Base number | Chemical modification | Molecular weight | Total molecular weight |
|---|---|---|---|---|---|
| a (SEQ ID NO: 94) | cGcGcG**cG**AGcGuu**Gc**A AuGAcAGAuAAGGAAcc uGcudTdT | 39 | C/U 2'F modification | 12901.91 | 31832.42 |
| b (SEQ ID NO: 95) | GGcAGGuuccuuAucuGu cAAA**Gc**uuc**GGcG**GcAG c | 36 | C/U 2'F modification | 11555.97 | |
| c (SEQ ID NO: 96) | GcAGc**cGccc**AuAGc**cG**c GcGcG | 23 | C/U 2'F modification | 7374.54 | |

The single strands of the above 7 groups of conventional-sequence RNA nanoparticle carriers are all commissioned to be synthesized by Suzhou Gima Company, herein the sequence a, the sequence b and the sequence c in R-8 to R-14 are respectively extended RNA oligonucleotide sequences formed by adding the extension fragments on the basis of the sequence a, the sequence b and the sequence c in R-1 to R-7, a targeting module fragment is not extended, and C/U base 2'F modification (resistance to enzyme digestion and stability are enhanced) is performed. In addition, a siRNA nucleic acid interference therapeutic fragment of a survivin is modified in the above RNA nanoparticle R-14, specifically a sense strand (see an underlined part of the a-strand) of Survivin siRNA is extended at a-strand 3'-end, and an antisense strand (see an underlined part of the b-strand) is extended and connected at b-strand 5'-end, so base pair complementary is formed.

II. Self-assembly experiment steps:

(1) according to a molar ratio of 1:1:1, enabling the RNA single strands a, b and c to be simultaneously mixed and

dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, volatilizing under reduced pressure and low temperature;

(5) electrophoresis analysis detection and laser scanning observation;

(6) electric potential detection.

III. Self-assembly experiment result

(1) Electrophoresis detection result

A 2% agarose gel electrophoresis diagram of the 7 groups of the conventional-sequence RNA self-assembly products is shown in Fig. 5. From left to right, lanes 1 to 7 in Fig. 5 are successively: conventional-sequence RNA self-assembly products R-8, R-9, R-10, R-11, R-12, R-13 and R-14.

A 4% agarose gel electrophoresis diagram of the 7 groups of the conventional-sequence RNA self-assembly products is shown in Fig. 6. From left to right, lanes 1 to 7 in Fig. 6 are successively: conventional-sequence RNA self-assembly products R-8, R-9, R-10, R-11, R-12, R-13 and R-14.

It may be clearly seen from results of Fig. 5 and Fig. 6 that bands of the 7 groups of the conventional-sequence self-assembly products are bright and clear single bands, it is indicated that the 7 groups of the conventional-sequences may be self-assembled into a nanostructure. Herein after a Survivin siRNA nucleic acid interference therapeutic fragment is modified in the conventional-sequence RNA self-assembly product R-14, it still has the stable self-assembly structure, it is also indicated that the nucleic acid nanoparticles of the disclosure may load a nucleic acid drug, and have a delivery carrier function of the nucleic acid drug.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 7 groups of the conventional-sequence RNA nanoparticles are shown below in Table 23 to Table 29:

Table 23:

| Sample name | Detection times | Potential ZP (mV) |
|---|---|---|
| R-8 | 1 | -18.00 |
| | 2 | -16.10 |
| | 3 | -18.20 |
| Experiment result | -17.43 mV (±1.33mV) | |

Table 24:

| Sample name | Detection times | Potential ZP (mV) |
|---|---|---|
| R-9 | 1 | -16.90 |
| | 2 | -17.50 |
| | 3 | -20.20 |
| Experime nt result | -18.20 mV (±1.3mV) | |

Table 25:

| Sample name | Detection times | Potential ZP (mV) |
|---|---|---|
| R-10 | 1 | -10.40 |
| | 2 | -11.80 |
| | 3 | -10.40 |
| Experiment result | -10.87 mV (±0.47mV) | |

Table 26:

| Sample name | Detection times | Potential ZP (mV) |
|---|---|---|
| R-11 | 1 | -28.30 |
| | 2 | -26.00 |
| | 3 | -33.10 |
| Experiment result | -29.13mV (±3.13mV) | |

Table 27:

| Sample name | Detection times | Potential ZP (mV) |
|---|---|---|
| R-12 | 1 | -7.59 |
| | 2 | -7.80 |
| | 3 | -17.20 |
| Experiment result | -10.86 mV (±3.27mV) | |

Table 28:

| Sample name | Detection times | Potential ZP (mV) |
|---|---|---|
| R-13 | 1 | -9.64 |
| | 2 | -15.60 |
| | 3 | -21.10 |
| Experiment result | -15.45 mV (±5.81 mV) | |

Table 29:

| Sample name | Detection times | Potential ZP (mV) |
|---|---|---|
| R-14 | 1 | -21.40 |

(continued)

| Sample name | Detection times | Potential ZP (mV) |
|---|---|---|
| | 2 | -21.20 |
| | 3 | -28.00 |
| Experiment result | -23.53 mV. ±2.33mV | |

It may be seen from the above potential detection data that the 7 groups of the conventional-sequence RNA self-assembly products all have good stability, and it is further indicated that the nanoparticles formed by the self-assembly of each conventional-sequence RNA have a relatively stable self-assembly structure.

It is indicated from the embodiment that: on the basis of different combinations of the RNA core sequences, the RNA nanoparticles with the stable structure may also be successfully self-assembled by adding the extension fragments. At the same time, the added extension fragments make the RNA nanoparticles have superior drug loading performance (specifically described in Embodiment 5).

**Embodiment 4**

I. 7 groups of conventional-sequence DNA nanoparticle carriers:

(1) 7 groups of three polynucleotide base sequences for forming DNA nanoparticles are shown below in Table 30 to Table 36.

An EGFRapt target head or a PSMAapt(A9L) target head is extended in a part of a-strands in the table:

EGFRapt (SEQ ID NO:97): GCCTTAGTAACGTGCTTTGATGTCGATTCGACAGGAGGC;

PSMAapt (A9L, SEQ ID NO: 98):

GGGCCGAAAAAGACCTGACTTCTATACTAAGTCTACGTCCC.

Table 30: D-1

| Name | Sequence sense (5'-3') | Base number | Chemical modification | Molecular weight | Total molecular weight |
|---|---|---|---|---|---|
| a (SEQ ID NO: 99) | CGCGCGCCCAGGAGCGTTGGCGGGCGGCG<u>GCCTTAGTAACGTGCTTTGATGTCGATTCGACAGGAGGC</u> | 68 | 3 bases before 5'-end and 3'-end, thio modificatio n | 21214.63 | 39092.09 |
| b (SEQ ID NO: 100) | CGCCGCCCGCCTTCGCCGCCAGCCGCC | 27 | 3 bases before 5'-end and 3'-end, thio modification | 8176.24 | |
| c (SEQ ID NO: 101) | GGCGGCAGGCGGCCATAGCCCTGGGCGCGCG | 31 | 3 bases before 5'-end and 3'-end, thio modification | 9701.22 | |

Table 31: D-2

| Name | Sequence sense (5'-3') | Base number | Chemical modification | Molecular weight | Total molecular weight |
|---|---|---|---|---|---|
| a (SEQ ID NO: 102) | CGCGCGCCCAGCAGCGTTCGCGGGCGGCGGCCTTAGTAACGTGCTTTGATGTCGATTCGACAGGAGGC | 68 | 3 bases before 5'-end and 3'-end, thio modification | 21134.59 | 39092.11 |
| b (SEQ ID NO: 103) | CGCCGCCCGCGTTCGCCGCCAGCCGCC | 27 | 3 bases before 5'-end and 3'-end, thio modification | 8216.27 | |
| c (SEQ ID NO: 104) | GGCGGCAGGCGGCCATAGCGCTGGGCGCGCG | 31 | 3 bases before 5'-end and 3'-end, thio modification | 9741.25 | |

Table 32: D-3

| Name | Sequence sense (5'-3') | Base number | Chemical modification | Molecular weight | Total molecular weight |
|---|---|---|---|---|---|
| a (SEQ ID NO: 105) | CGCGCGCCCACGAGCGTTGCGGGGCGGCGGCCTTAGTAACGTGCTTTGATGTCGATTCGACAGGAGGC | 68 | 3 bases before 5'-end and 3'-end, thio modification | 21174.60 | 39092.09 |
| b (SEQ ID NO: 106) | CGCCGCCCCGCTTCGCCGCCAGCCGCC | 27 | 3 bases before 5'-end and 3'-end, thio modification | 8176.24 | |
| c (SEQ ID NO: 107) | GGCGGCAGGCGGCCATAGCCGTGGGCGCGCG | 31 | 3 bases before 5'-end and 3'-end, thio modification | 9741.25 | |

Table 33: D-4

| Name | Sequence sense (5'-3') | Base number | Chemical modification | Molecular weight | Total molecular weight |
|---|---|---|---|---|---|
| a (SEQ ID NO: 108) | CGCGCGCCCAGGAGCGTTGGCCCGCGGCGTGGGCCGAAAAAGACCTGACTTCTATACTAAGTCTACGTCCC | 71 | 3 bases before 5'-end and 3'-end, thio modification | 21923.12 | 39780.63 |
| b (SEQ ID NO: 109) | CGCCGCGGGCCTTCGGGGCCAGCCGCC | 27 | 3 bases before 5'-end and 3'-end, thio modification | 8236.34 | |
| c (SEQ ID NO: 110) | GGCGGCAGGCCCCCATAGCCCTGGGCGCGCG | 31 | 3 bases before 5'-end and 3'-end, thio modification | 9621.17 | |

Table 34: D-5

| Name | Sequence sense (5'-3') | Base number | Chemical modification | Molecular weight | Total molecular weight |
|---|---|---|---|---|---|
| a (SEQ ID NO: 111) | CGCGCGCCCAGCAGCGTTCGCCCCGCCGCTGGGCCGAAAAAGACCTGACTTCTATACTAAGTCTACGTCCC | 71 | 53 bases before 5'-end and 3'-end, thio modification | 21763.03 | 39880.64 |
| b (SEQ ID NO: 112) | GCGGCGGGGCGTTCGGCGGCAGGCGGC | 27 | 3 bases before 5'-end and 3'-end, thio modification | 8536.46 | |
| c (SEQ ID NO: 113) | GCCGCCAGCCGCCCATAGCGCTGGGCGCGCG | 31 | 3 bases before 5'-end and 3'-end, thio modification | 9581.15 | |

Table 35: D-6

| Name | Sequence sense (5'-3') | Base number | Chemical modification | Molecular weight | Total molecular weight |
|---|---|---|---|---|---|
| a (SEQ ID NO: 114) | CGCGCGCCCAGCAGCG TTCGGGGCGCCGC | 29 | 3 bases before 5'-end and 3'-end, thio modification | 8978.76 | 27594.69 |
| b (SEQ ID NO: 115) | GCGGCGCCCCGTTCGG CCGGCAGGCGGC | 28 | 3 bases before 5'-end and 3'-end, thio modification | 8705.57 | |
| c (SEQ ID NO: 116) | GCCGCCAGCCGGCCCA TAGCGCTGGGCGCGCG | 32 | 3 bases before 5'-end and 3'-end, thio modification | 9910.36 | |

Table 36: D-7

| Name | Sequence sense (5'-3') | Base number | Chemical modification | Molecular weight | Total molecular weight |
|---|---|---|---|---|---|
| a (SEQ ID NO: 117) | CGCGCGCCCACGAGCGTT GCGGGCGCCGC | 29 | 3 bases before 5'-end and 3'-end, thio modification | 8978.76 | 26976.30 |
| b (SEQ ID NO: 118) | GCGGCGCCCGCTTCGGCG GCAGGCGGC | 27 | 3 bases before 5'-end and 3'-end, thio modification | 8416.39 | |
| c (SEQ ID NO: 119) | GCCGCCAGCCGCCCATAG CCGTGGGCGCGCG | 31 | 3 bases before 5'-end and 3'-end, thio modification | 9581.15 | |

Single strands of the above 7 groups of the conventional-sequence DNA nanoparticles are all commissioned to be synthesized by Suzhou Hongxun Company, herein:

D-1 is the conventional-sequence DNA nanoparticles formed after adding an extension sequence containing the EGFRapt target head (see an underlined part) on the basis of the above core sequences (8) (sequence a: 5'-GGAGCGTTGG-3', sequence b: 5'-CCTTCGCCG-3', and sequence c: 5'-CGGCCATAGCCC-3');
D-2 is the conventional-sequence DNA nanoparticles formed after adding an extension sequence containing the EGFRapt target head (see an underlined part) on the basis of the above core sequences (9) (sequence a: 5'-GCAGCGTTCG-3', sequence b: 5'-CGTTCGCCG-3', and sequence c: 5'-CGGCCATAGCGC-3');
D-3 is the conventional-sequence DNA nanoparticles formed after adding an extension sequence containing the EGFRapt target head (see an underlined part) on the basis of the above core sequences (10) (sequence a: 5'-CGAGCGTTGC-3', sequence b: 5'-GCTTCGCCG-3', and sequence c: 5'-CGGCCATAGCCG-3');
D-4 is the conventional-sequence DNA nanoparticles formed after adding an extension sequence containing the PSMAapt target head (see an underlined part) on the basis of the above core sequences (11) (sequence a: 5'-GGAGCGTTGG-3', sequence b: 5'-CCTTCGGGG-3', and sequence c: 5'-CCCCCATAGCCC-3');
D-5 is the conventional-sequence DNA nanoparticles formed after adding an extension sequence containing the PSMAapt target head (see an underlined part) on the basis of the above core sequences (12) (sequence a: 5'-GCAGCGTTCG-3', sequence b: 5'-CGTTCGGCG-3', and sequence c: 5'-CGCCCATAGCGC-3');
D-6 is the conventional-sequence DNA nanoparticles formed after adding an extension sequence without containing a target head structure on the basis of the above core sequences (13) (sequence a: 5'-GCAGCGTTCG-3', sequence b: 5'-CGTTCGGCC-3', and sequence c: 5'-GGCCCATAGCGC-3'); and
D-7 is the conventional-sequence DNA nanoparticles formed after adding an extension sequence without containing a target head structure on the basis of the above core sequences (14) (sequence a: 5'-CGAGCGTTGC-3', sequence b: 5'-GCTTCGGCG-3', and sequence c: 5'-CGCCCATAGCCG-3').

II. Self-assembly experiment steps:

(1) According to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in DNA elution buffer solution at 37 DEG C, after that, precipitating overnight in ethanol, volatilizing under reduced pressure and low temperature, to obtain a conventional-sequence DNA self-assembly product;

(5) electrophoresis analysis detection and laser scanning observation;

(6) electric potential detection;

(7) particle size measurement; and

(8) observing by a transmission electron microscope.

III. Self-assembly experiment result

(1) Electrophoresis detection result

A 2% agarose gel electrophoresis diagram of the 7 groups of the conventional-sequence DNA self-assembly products is shown in Fig. 7. From left to right, lanes 1 to 7 in Fig. 7 are successively: conventional -sequence DNA self-assembly products D-1, D-2, D-3, D-4, D-5, D-6 and D-7.

A 4% agarose gel electrophoresis diagram of the 7 groups of the conventional-sequence DNA self-assembly products is shown in Fig. 8. From left to right, lanes 1 to 7 in Fig. 8 are successively: conventional-sequence DNA self-assembly products D-1, D-2, D-3, D-4, D-5, D-6 and D-7.

It may be clearly seen from results of Fig. 7 and Fig. 8 that bands of the 7 groups of the conventional-sequence DNA self-assembly products are bright and clear, it is indicated that the 7 groups of the conventional-sequence DNA strands are all self-assembled to form stable nanoparticle structures. Herein two groups of the self-assembly structures D-6 and D-7 carry the EGFRapt or PSMAapt target head, and the molecular weights are slightly low, positions of bands thereof are apparently in front of the other bands, actual and theoretical conditions are completely consistent, so the stability of the self-assembly structures is further proved.

It is indicated from the embodiment: on the base of combinations of these different DNA core sequences, when various functional extension fragments are added or the target head is linked at the same time, the DNA nanoparticles may also be successfully assembled, and also have functions such as drug loading, cell targeting, visibility and traceability (specifically described in Embodiment 6 and Embodiment 8).

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears; setting a software detection parameter;
and then clicking Ok to complete the settings, after a measurement dialog box appears, clicking Start to start.

Measurement result: potential detection results of the 3 groups of the conventional-sequence DNA nanoparticles are shown below in Table 37 to 39:

Table 37:

| Sample name | Detection times | Potential ZP (mV) |
| --- | --- | --- |
| D-2 | 1 | -36.00 |
| | 2 | -35.80 |
| | 3 | -37.60 |
| Experiment result | -36.47 mV (±0.67mV) | |

Table 38:

| Sample name | Detection times | Potential ZP (mV) |
| --- | --- | --- |
| D-6 | 1 | -31.70 |
| | 2 | -32.10 |
| | 3 | -31.90 |
| Experiment result | -31.90 mV (±0.2mV) | |

Table 39:

| Sample name | Detection times | Potential ZP (mV) |
| --- | --- | --- |
| D-7 | 1 | -32.10 |
| | 2 | -31.70 |
| | 3 | -32.80 |
| Experiment result | -32.20 mV (±0.0.5mV) | |

It may be seen from the above potential detection data that the 3 groups of the conventional-sequence RNA self-assembly products have good stability, it is further indicated that the nanoparticles formed by the self-assembly of each conventional-sequence RNA have a relatively stable self-assembly structure.

(3) Particle size measurement

1. Preparing a potential sample (conventional-sequence DNA self-assembly product D-7) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, when a measurement dialog box appears, clicking Start to start, a DLS measurement value result of a hydrodynamic size of the self-assembly product D-7 is shown below in Table 40:

Table 40:

| Sample name | Detection times | Particle size Z-Ave (d.nm) |
| --- | --- | --- |
| 1 | 1 | 10.76 |
| | 2 | 13.9 |
| | 3 | 10.36 |
| Experiment result | 12.33 d.nm (±1.57 d.nm) | |

(4) Observed result of transmission electron microscope

The above conventional-sequence DNA self-assembly product D-7 is irradiated by a transmission electron

microscope, and steps are as follows:

1. taking a drop of a sample and suspending on 400 meshes of a carbon-coated film copper grid, keeping for 1 min at a room temperature;

2. absorbing liquid by filter paper;

3. staining for 1 min by 2% uranyl acetate;

4. absorbing by the filter paper, and drying at the room temperature; and

5. observing and taking a picture by a transmission electron microscope JEM-1400 at 120 kv.

The result is as shown in Fig. 9, it may be apparently seen from the figure that the above conventional-sequence DNA self-assembly product D-7 is an integral structure, and it may be clearly seen that it has a T-type structure.

**Embodiment 5**

**Tacrine loading experiment**

Chemical loading:

I. Experiment material and experiment method

1. Experiment materials and reagents:

(1) Nucleic acid nanoparticles: RNA nanoparticles from Embodiment 1.

(2) DEPC treated water: Biyuntian Company.

(3) PBS buffer solution: cellgro.

(4) 4% paraformaldehyde

(5) Tacrine.

(6) Chloroform: Beijing Beihua Fine Chemicals Co., Ltd.

(7) Anhydrous ethanol: Beijing Beihua Fine Chemicals Co., Ltd.

2. Experiment method:

(1) Accurately weighing the tacrine (0.32 mg, 1.354μmol) and dissolving in the DEPC water (1.0 mL) and the PBS buffer solution (1.25 mL), adding 4% paraformaldehyde aqueous solution (0.4 mL) under cooling of an ice-water bath and uniformly mixing, enabling the mixed solution to be totally uniformly mixed with the RNA nanoparticles (1 mg, 33.84 nmol), and reacting for 72 hours at 4 DEG C under a dark condition.

(2) Taking 10μL of reaction solution and diluting for 10 times, using 50 μM of the tacrine aqueous solution and 310 ng/μL of the RNA nanoparticles as controls, and performing HPLC analysis according to equal volume injection. According to a peak area ratio of each component, it may be adjusted that reaction conversion is basically completed.

(3) Extracting the reaction solution by the chloroform (10mLx3), and then adding 25 mL of the anhydrous ethanol, after uniformly mixing, keeping at 4 DEG C in the dark and enabling a product to be adequately precipitated (4 hours). Centrifuging (4000/min), transferring supernatant, and washing the solid product again by the ethanol (50

mL), evaporating a solvent under reduced pressure and lower temperature, to obtain a loading product.

(4) Loading rate calculation:

1. preparing a known concentration of tacrine-HCl standard solution (the HCl concentration is 0.1 M): 2 μM, 4 μM, 6 μM, 8 μM, and 10 μM, 100 μL each;

2. enabling tacrine-RNAh particles to be dissolved in 100 μL of the PBS;

3. putting the standard solution and the tacrine-RNAh particles into a PCR plate, heating at 85 DEG C for 5 min, and then cooling to the room temperature;

4. measuring an absorbance of the tacrine at 240 nm by using a microplate reader, drawing a standard curve (as shown in Fig. 10), and calculating the molar concentration of the tacrine in the loaded product;

5. measuring an absorbance of RNA at 260 nm by using a spectrophotometer, to obtain the mass concentration of the tacrine-RNAh particles contained in each sample; and

6. according to the tacrine molar concentration and the mass concentration of the RNAh particles obtained by measuring, calculating a loading rate.

A specific calculation process is as follows:

$$C_{RNAh\text{-}1} = 76.0\ \mu g/\mu L,\ M_{RNAh} \approx 30000,\ 100\mu L;\ C\ t_{acrine\ \text{-}1} = 42.24\ \mu M,\ 100\mu L;$$

$$C_{RNAh\text{-}2} = 52.0\mu g/\mu L,\ M_{RNAh} \approx 30000,\ 100\mu L;\ C\ _{tacrine\ \text{-}1} = 24.0\ \mu M,\ 100\mu L;$$

$$N\text{-}1 = \frac{42.24 \times 10\text{-}6 \times 100 \times 10\text{-}6}{0.0760 \times 100 \times 10\text{-}6/30000} = 16.7\ ; \quad N\text{-}2 = \frac{24.0 \times 10\text{-}6 \times 100 \times 10\text{-}6}{0.0520 \times 100 \times 10\text{-}6/30000} = 13.8\ .$$

An average value of N-1 and N-2 is taken so that the loading rate of RNAh-tacrine is about 15, and it means that about 15 tacrine molecules may be loaded on each nucleic acid nanoparticle carrier.

In addition, on the basis of the tacrine-loaded RNA nanoparticles, other small molecular drugs may be further loaded for the second time in the same way as the tacrine loading. For example, the present application is further loaded with a folic acid to obtain the RNA nanoparticles co-loaded with two small molecular drugs of the tacrine and the folic acid, and the loading rates of the two drugs may be detected by referring to the above method (values are not shown).

It is indicated from Embodiment 5 that the RNA nanoparticles (in Embodiment 1) with the extension fragment, the target head and the fluorescein have a function of loaded drugs, may achieve the loading with the small molecular drug tacrine in a mode of covalent linkage (paraformaldehyde-solvent covalence), and may also achieve the co-loading with other small molecular drugs.

Embodiment 6

**Cell binding ability of drug-loaded RNA nanoparticles detected by confocal microscopy experiment**

I. Experiment material and experiment method:

1. Samples to be tested are as shown in Table 41:

Table 41:

| Nanoparticles | | MW | Dissolution solvent |
|---|---|---|---|
| 1 | RNAh-Biotin-quasar670 | 29552.6 | PBS |

(continued)

| Nanoparticles | | MW | Dissolution solvent |
|---|---|---|---|
| 2 | RNAh-Biotin-quasar670-tacrine | 36591.738 | PBS |
| Note: the RNAh-Biotine-quasar670 in the table is served as a control, and refers to the nanoparticle formed by performing the biotin modification at the 5'-end of the a-strand and b-strand prepared according to the self-assembly method in Embodiment 1, and performing the quasar670 fluorescein modification at the 3'-end of the c-strand, and the RNAh-Biotin-quasar670-tacrine refers to the nanoparticle formed after further loading the tacrine (loaded according to the chemical method in Embodiment 5). | | | |

2. Experiment reagents used and sources thereof are as follows:

RPMI-1640 medium (Gibco, C11875500BT-500mL); DMEM (Gibco, C11995500BT-500 mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Trypsin-EDTA (Stemcell, 07901-500mL); DMSO (Sigma, D5879-1L); Prolong Gold Antifade Mountant (Thermo, P36941-2 mL); and DAPI (Yeasen, 36308ES11-4 mL).

3. Experiment instruments used are as follows:

Inverted Microscope (Olympus BX53, U-RFL-T); BD Falcon (Corning, 354118); and Cytospin (TXD3).

4. Experiment method:

(1) Culturing SH-SY5Y cells (neuroblastoma cell line) in a RPMI1640+10%FBS+1%PS medium under a condition of 37 DEG C and 5% $CO_2$.

(2) Trypsin-digesting the SH-SY5Y cells, washing once with the PBS, and adding to a cell culture glass slide in $1x10^5$ cells per well.

(3) After the cells are adhered to a wall, rinsing the glass slide with the culture medium.

(4) Incubating the cells with 200 nM and 400 nM of the RNAh-Biotin-quasar670 and RNAh-Biotin-quasar670-tacrine nanoparticles under 37 DEG C and 5% $CO_2$ for 1 h and 4 hrs.

(5) After the adherent cells are washed with the PBS, treating with the Prolong Gold Antifade Mountant and keeping overnight at a room temperature.

(6) Staining with the DAPI for 5 min at the room temperature, and then sealing the glass slide.

(7) Taking pictures under the microscope and saving.

II. Experiment result

An experiment result is shown in Fig. 11. It may be seen from Fig. 11 that the results of cell binding and internalization experiments show that the RNAh-Biotin-quasar670 and RNAh-Biotin-quasar670-tacrine nanoparticles may be both bound and internalized with the cells because they both carry the target head---Biotin. This result shows that the drug RNAh-Biotin-quasar670-tacrine nanoparticles containing the tacrine have a strong ability to bind and internalize with the SH-SY5Y cells.

**Embodiment 7**

**Stability detection of tacrine-containing drug loaded on nucleic acid nanoparticles in serum**

I. Experiment material and experiment method

1. Samples to be tested: RNAh-Biotin-quasar670-tacrine nanoparticles prepared in Embodiment 5 dissolved in PBS solution.

2. Experiment reagents:
RPMI-1640 medium (Gibco, C11875500BT-500 mL); Fetal bovine serum (FBS) (ExCell Bio, FNA500-500 mL); Penicillin/Streptomycin (PS) (Gibco, 15140 -122-100 mL); PBS buffer solution (Gibco, C20012500BT-500 mL); Novex™ Tris-Glycine Native Sample Buffer (2X) (Invitrogen, LC2673-20 mL); Novex™ 8% Tris-Glycine Mini Gels (Invitrogen , XP00080BOX-1.0 mm); Tris-Glycine Native Running buffer (10x) (Life science, LC2672-500 mL); and G250 staining solution (Beyotime, P0017-250 mL).

3. Experiment instrument:
Spectrophotometer (Thermo, ND2000C); Mini Gel Tank (Invitrogen, PS0301); and Imaging System (Bio-Rad, ChemiDoc MP).

4. Experiment method:

(1) Enabling the RNAh-Biotin-quasar670-tacrine nanoparticles to be prepared to 100 μM, and adequately mixing uniformly.

(2) Taking 1μL of solution and placing in 99 μL of a RPMI 1640 medium containing 10% serum and incubating.

(3) After being incubated at 37 DEG C for 10 min, 1 h, 12 h, and 36 h, respectively taking samples.

(4) After using NanoDrop for quantification, taking 200 ng of the RNA nanoparticles, adding the same volume of Tris-Glycine SDS sample buffer solution (2X), and adequately mixing uniformly.

(5) Taking a piece of Novex™ 8% Tris-Glycine Mini gel, loading the samples in order, setting a program at 200 V, 30 min, and starting electrophoresis.

(6) After the electrophoresis is finished, performing G250 staining, placing on a horizontal shaker for 30 min-1 h, taking pictures and imaging.

II. Experiment result

Table 42: quantitative result and loading volume

| Sample | RNAh-Biotin-quasar670-tacrine (ng/μL) | 200ng RNAh-Biotin-quasar670-tacrine (μL) | Buffer solution (μL) |
|---|---|---|---|
| 0 | 95.2 | 2.10 | 2.10 |
| 10 min | 96.0 | 2.08 | 2.08 |
| 1 h | 95.3 | 2.10 | 2.10 |
| 12 h | 96.0 | 2.08 | 2.08 |
| 36 h | 124.8 | 1.60 | 1.60 |

The electrophoresis detection results are shown in Fig. 12 and Fig. 13. Herein, Fig. 12 shows the electrophoresis

result of 8% non-denaturing gel (Coomassie Blue program), and Fig. 13 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Biotin-quasar670-tacrine nanoparticles, it is indicated that RNAh-Biotin-quasar670-tacrine nanoparticles are relatively stable in the 1640 medium with the 10% FBS without significant degradation.

**Embodiment 8**

**Cytotoxicity research of RNAh-Biotin-quasar670-tacrine nanoparticles in SH-SY5Y cells**

I. Experiment material and experiment method

1. Samples to be tested are a DMSO control, a small molecular drug tacrine and RNAh-Biotin-quasar670-tacrine nanoparticles.

2. Experiment reagent:
RPMI-1640 medium (Gibco, C11875500BT-500 mL); DMEM (Gibco, C11995500BT-500 mL); Fetal bovine serum (FBS) (ExCell Bio, FNA500-500 mL); Penicillin/Streptomycin (Penicillin/Streptomycin, PS) (Gibco, 15140-122-100 mL); PBS buffer solution (Gibco, C20012500BT-500 mL); Trypsin-EDTA (Stemcell, 07901-500 mL); DMSO (Sigma, D5879-1 L) ; Dox (HISUN Pharm, H33021980-10 mg); and Cell Titer-Glo Luminescent Cell Viability Assay kit (CTG) (Promega, G7572-100 mL).

3. Experiment instrument:
Inverted Microscope (Olympus IX71, TH4-200); and 96-well Plate Reader (Molecular Devices, Flexstation 3).

4. Experiment method:

(1) Using the RPMI1640+10%FBS+1%PS medium to culture the SH-SY5Y cells at 37 DEG C and 5% CO2.

(2) Collecting the cells, centrifuging at 800 rpm for 5 minutes, resuspending the medium, adjusting the cell concentration, and adding to the 96-well plate in a volume of 5000 cells per 90 μL.

(3) Diluting a sample to be tested with the culture medium on the next day, respectively adding 200 nM to each sample, herein each sample has 4 replicate wells for replication.

(4) After being cultured for 72 hours, adding 100 μL of the CTG reagent to each well, shaking for 2 minutes, and standing at the room temperature for 10 minutes, herein a whole process is protected from light.

(5) Finally using Soft Max Pro5 software to read.

II. Experiment result:

Table 43: cell proliferation rate (%)

| Cell line | Treatment time | Tacrine | RNAh-Biotin-quasar670-tacrine |
|---|---|---|---|
| SH-SY5Y | 72h | 39.75 | 13.04 |

The experiment results are shown in Table 43 and Fig. 14. It may be seen from Table 43 and Fig. 14 that 200 nM of the RNA nanoparticle RNAh-Biotin-quasar670-tacrine carrying the tacrine has the apparent cytotoxicity to the SH-SY5Y cells (P< 0.0001), and it is unpredictable that: compared with the small molecular drug tacrine on cell proliferation inhibition, 200 nM of the RNAh-Biotin-quasar670-tacrine has the more significant inhibition to the proliferation of the SH-SY5Y cells, and the cell proliferation rate is further reduced by at least 2/3 (to 13.04%) on the basis of 39.75% of the proliferation rate of the cells after being treated with the small molecular drug tacrine.

In order to further determine that the RNA nanoparticles without carrying the tacrine have no apparent cytotoxicity to the SH-SY5Y cells, the inventor further designs a toxicity experiment of a targeted fluorescent carrier RNAh-Biotin-Cy5 to SH-SY5Y cells, and uses another small molecular drug Cisplatin as a control (the highest administration concentration of the drug in the experiment is 5 μM). The results thereof are shown in Table 44 and Fig. 15. It can be seen from an $IC_{50}$ value in Table 44 and Fig. 15 that the targeted fluorescent carrier without carrying the tacrine has no apparent toxicity to the experimental cells.

Table 44:

| | Bio-Cy5-RNAh | Cisplatin |
|---|---|---|
| IC50 (μM) | >5 | 0.51 |

**Assembly of nucleic acid nanoparticles**

**Embodiment 9**

I. 7 groups of extension fragment deformation + core short-sequence RNA nanoparticle carriers:

(1) 7 groups of three polynucleotide base sequences for forming extension fragment deformation + core short-sequence RNA nanoparticles:

Table 45: R-15

| Name | Sequence sense (5'-3') | Base number | Chemical modification | Molecular weight | Total molecular weight |
|---|---|---|---|---|---|
| a (SEQ ID NO: 120) | GCGGCGAGCGGCGAGGAGCGUUGGGGCCGGAGGCCGG | 37 | 3'-end is linked with a biotin (Bio); bases C and U, and 2'F modificatio n | 12668.8 | 33713 |
| b (SEQ ID NO: 121) | CCGGCCUCCGGCC**CCUUCGGGG**CCAGCCGCC | 31 | bases C and U, and 2'F modificatio n | 9866.8 | |
| c (SEQ ID NO: 122) | GGCGGCAGG**CCCCCAUAGCCC**UCGCCGCUCGCCGC | 35 | bases C and U, and 2'F modificatio n | 11177.4 | |

Table 46: R-16:

| Name | Sequence sense (5'-3') | Base number | Chemical modification | Molecular weight | Total molecular weight |
|---|---|---|---|---|---|
| a (SEQ ID NO: 123) | GCGGCGAGCGGCGAGCAGCGU UCGGGCCGGAGGCCGG | 37 | 3'-end is linked with a biotin (Bio); bases C and U, and 2'F modification | 12591.6 | 33709.8 |
| b (SEQ ID NO: 124) | CCGGCCUCCGGCCCGUUCGCC GCCAGCCGCC | 31 | bases C and U, and 2'F modification | 9827.6 | |
| c (SEQ ID NO: 125) | GGCGGCAGGCGGCCAUAGCGC UCGCCGCUCGCCGC | 35 | bases C and U, and 2'F modification | 11290.6 | |

Table 47: R-17:

| Name | Sequence sense (5'-3') | Base number | Chemical modification | Molecular weight | Total molecular weight |
|---|---|---|---|---|---|
| a (SEQ ID NO: 126) | GCGGCGAGCGGCGA**GGAGCGU UGG**GGCCGGAGGCCGG | 37 | 3'-end is linked with a biotin (Bio); bases C and U, and 2'F modification | 12668.9 | 33713 |
| b (SEQ ID NO: 127) | CCGGCCUCCGGCC**CCUUCGCC G**CCAGCCGCC | 31 | bases C and U, and 2'F modification | 9790.6 | |
| c (SEQ ID NO: 128) | GGCGGCAGG**CGGCCAUAGCCC** UCGCCGCUCGCCGC | 35 | bases C and U, and 2'F modification | 11253.5 | |

Table 48: R-18:

| Name | Sequence sense (5'-3') | Base number | Chemical modification | Molecular weight | Total molecular weight |
|---|---|---|---|---|---|
| a (SEQ ID NO: 129) | GCGGCGAGCGGCGAGCAGCGU UCGGGCCGGAGGCCGG | 37 | 3'-end is linked with a biotin (Bio); bases C and U, and 2'F modification | 12591.6 | 33709.8 |
| b (SEQ ID NO: 130) | CCGGCCUCCGGCCCGUUCGGC GCCAGCCGCC | 31 | bases C and U, and 2'F modificatio n | 9865.7 | |
| c (SEQ ID NO: 131) | GGCGGCAGGCGCCCAUAGCGC UCGCCGCUCGCCGC | 35 | bases C and U, and 2'F modification | 11252.5 | |

Table 49: R-19:

| Name | Sequence sense (5'-3') | Base number | Chemical modification | Molecular weight | Total molecular weight |
|---|---|---|---|---|---|
| a (SEQ ID NO: 132) | GCGGCGAGCGGCGAGCAGCGU UCGGGCCGGAGGCCGG | 37 | 3'-end is linked with a biotin (Bio); bases C and U, and 2'F modification | 12591.6 | 33709.8 |
| b (SEQ ID NO: 133) | CCGGCCUCCGGCCCGUUCGGC CCCAGCCGCC | 31 | bases C and U, and 2'F modification | 9827.6 | |
| c (SEQ ID NO: 134) | GGCGGCAGGGGCCCAUAGCGC UCGCCGCUCGCCGC | 35 | bases C and U, and 2'F modification | 11290.6 | |

Table 50: R-20:

| Name | Sequence sense (5'-3') | Base number | Chemical modification | Molecular weight | Total molecular weight |
|---|---|---|---|---|---|
| a (SEQ ID NO: 135) | GCGGCGAGCGGCGACGAGCGU UGCGGCCGGAGGCCGG | 37 | 3'-end is linked with a biotin (Bio); bases C and U, and 2'F modification | 12591.6 | 33709.8 |
| b (SEQ ID NO: 136) | CCGGCCUCCGGCCGCUUCGCC GCCAGCCGCC | 31 | bases C and U, and 2'F modification | 9827.6 | |

(continued)

| Name | Sequence sense (5'-3') | Base number | Chemical modification | Molecular weight | Total molecular weight |
|---|---|---|---|---|---|
| c (SEQ ID NO: 137) | GGCGGCAGGCGGCCAUAGCCG UCGCCGCUCGCCGC | 35 | bases C and U, and 2'F modification | 11290.6 | |

Table 51: R-21:

| Name | Sequence sense (5'-3') | Base number | Chemical modification | Molecular weight | Total molecular weight |
|---|---|---|---|---|---|
| a (SEQ ID NO: 138) | GCGGCGAGCGGCGACGAGCGU UGCGGCCGGAGGCCGG | 37 | 3'-end is linked with a biotin (Bio); bases C and U, and 2'F modification | 11290.6 | 32408.8 |
| b (SEQ ID NO: 139) | CCGGCCUCCGGCCGCUUCGGC GCCAGCCGCC | 31 | bases C and U, and 2'F modification | 9865.7 | |
| c (SEQ ID NO: 140) | GGCGGCAGGCGCCCAUAGCCG UCGCCGCUCGCCGC | 35 | bases C and U, and 2'F modification | 11252.5 | |

II. Self-assembly experiment steps:

(1) according to a molar ratio of 1:1:1, enabling the RNA single strands a, b and c to be simultaneously mixed and dissolved in DEPC treated water or TMS buffer solution;

(2) heating mixed solution to 80 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in RNA elution buffer solution at 37 DEG C, after that, precipitating overnight in ethanol, and volatilizing under reduced pressure and low temperature; and

(5) electrophoresis analysis detection and laser scanning observation.

III. Self-assembly experiment result

(1) Electrophoresis detection

Main reagents and instruments are as follows:

Table 52:

| Reagent name | Article number | Manufacturer |
|---|---|---|
| 6×DNA Loading buffer | TSJ010 | Qingke Biotechnology Co., Ltd. |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |

Table 53:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Steps:

① The RNA nanoparticles are diluted with ultrapure water by using a method shown belowin Table 54.

Table 54:

| Serial number | Measured concentration (μg/mL) |
|---|---|
| R-15 | 165.937 |
| R-16 | 131.706 |
| R-17 | 144.649 |
| R-18 | 164.743 |
| R-19 | 126.377 |
| R-20 | 172.686 |
| R-21 | 169.455 |

② 10μL (500ng) of the processed sample is taken and uniformly mixed with 2μL of 6xDNA Loading Buffer, it is operated on ice, and a label is made.
③ A 8% non-denaturing PAGE gel is taken, a piece of the gel is applied to samples with different incubation times, 12 μL of the processed samples are all loaded, and a program is set to run the gel at 100V for 40 minutes.
④ After running the gel, dyeing is performed, it is placed on a horizontal shaker for 30 minutes, and pictures are taken for imaging.

Detection result:

Non-denaturing PAGE gel running results of the 7 groups of extension fragment deformation + core short-sequence RNA self-assembly products are shown in Fig. 16. Lanes 1 to 7 in Fig. 16 from left to right are successively: the 7 groups of extension fragment deformation + core short-sequence RNA self-assembly products R-15, R-16, R-17, R-18, R-19, R-20 and R-21.

It may be clearly seen from the results in Fig. 16 that the bands of the 7 groups of extension fragment deformation + core short-sequence RNA self-assembly products are bright and clear, it is indicated that the 7 groups of extension fragment deformation + core short-sequence RNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears;
setting a software detection parameter;
and then clicking Ok to complete the settings, after a measurement dialog box appears, clicking Start to start.

Measurement result: potential detection results of the 7 groups of the extension fragment deformation + core short-sequence RNA nanoparticles at 25 DEG C are as follows:

Table 55:

| Sample name | Detection times | Potential ZP (mV) |
|---|---|---|
| R-15 | 1 | -22.50 |
| | 2 | -23.90 |
| | 3 | -23.30 |
| Experiment result | -23.23 mV | |

Table 56:

| Sample name | Detection times | Potential ZP (mV) |
|---|---|---|
| R-16 | 1 | -21.10 |
| | 2 | -19.80 |
| | 3 | -21.90 |
| Experiment result | -20.93 mV | |

Table 57:

| Sample name | Detection times | Potential ZP (mV) |
|---|---|---|
| R-17 | 1 | -24.90 |
| | 2 | -20.90 |
| | 3 | -24.70 |
| Experiment result | -23.50 mV | |

Table 58:

| Sample name | Detection times | Potential ZP (mV) |
|---|---|---|
| R-18 | 1 | -20.80 |
| | 2 | -21.30 |
| | 3 | -21.70 |
| Experiment result | -21.27 mV | |

Table 59:

| Sample name | Detection times | Potential ZP (mV) |
|---|---|---|
| R-19 | 1 | -16.80 |
| | 2 | -16.90 |
| | 3 | -21.20 |
| Experiment result | -18.30 mV | |

Table 60:

| Sample name | Detection times | Potential ZP (mV) |
|---|---|---|
| R-20 | 1 | -16.00 |
| | 2 | -16.90 |
| | 3 | -21.20 |
| Experiment result | -17.90 mV | |

Table 61:

| Sample name | Detection times | Potential ZP (mV) |
|---|---|---|
| R-21 | 1 | -15.20 |
| | 2 | -16.70 |
| | 3 | -16.40 |
| Experiment result | -16.10 mV | |

It may be seen from the above potential detection data that: the 7 groups of extension fragment deformation + core short-sequence RNA nanoparticles all have the good stability, and it is further indicated that the nanoparticles formed by the self-assembly of each extension fragment deformation + core short-sequence RNA have a relative stable self-assembly structure.

(3) Particle size measurement

1. Preparing a potential sample (7 groups of extension fragment deformation + core short-sequence RNA) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, when a measurement dialog box appears, clicking Start to start, a DLS measurement value result of a hydrodynamic size of the 7 groups of extension fragment deformation + core short-sequence RNA is as follows:

Table 62:

| Serial number | Mean particle size (nm) |
|---|---|
| R-15 | 6.808 |
| R-16 | 6.978 |
| R-17 | 7.592 |
| R-18 | 7.520 |
| R-19 | 6.936 |

(continued)

| Serial number | Mean particle size (nm) |
|---|---|
| R-20 | 7.110 |
| R-21 | 6.720 |

(4) TM value detection

A solubility curve method is used to detect TM values of the 7 groups of the extension fragment deformation + core short-sequence RNA nanoparticles, and the samples are consistent with the potential samples.

Reagents and instruments are as follows:

Table 63:

| Reagent name | Article number | Manufacturer |
|---|---|---|
| AE buffer | / | Takara |
| SYBR Green I Dye | / | Self-made |

Table 64:

| Name | Model | Manufacturer |
|---|---|---|
| Real-Time System | CFX Connect | Bio-rad |
| Clean bench | HDL | Beijing Donglian Haer Instrument Manufacturing Co., Ltd. |

Steps:

① After the sample is diluted with ultrapure water, 5μg of the diluted sample is mixed with 2μL of SYBR Green I dye (diluted by 1:200), a final volume is 20μL, and the dilution concentration is as follows:

Table 65:

| Serial number | Measured concentration (μg/mL) |
|---|---|
| R-15 | 773.009 |
| R-16 | 782.098 |
| R-17 | 740.607 |
| R-18 | 806.163 |
| R-19 | 829.996 |
| R-20 | 723.082 |
| R-21 | 721.674 |

② It is incubated for 30 min in the dark at a room temperature; and

③ On-machine detection is performed, a program is set to start at 20 DEG C, the temperature rises per second from 0.1 DEG C to 95 DEGC, and reading is performed every 5 s.

Detection result:

The TM values of the 7 groups of extension fragment deformation + core short-sequence RNA nanoparticles are as follows, a solubility curve diagram of the R-15 is shown in Fig. 17, a solubility curve diagram of the R-16 is shown in Fig. 18, a solubility curve diagram of the R-17 is shown in Fig. 19, a solubility curve diagram of the R-18 is shown in Fig. 20, a solubility curve diagram of the R-19 is shown in Fig. 21, a solubility curve diagram of the R-20 is shown in Fig.

22, and a solubility curve diagram of the R-21 is shown in Fig. 23. Due to the particularity of the RNA samples, a temperature corresponding to 1/2 RFUmax in a range of 20 to 90 DEG C is used as a sample Tm value in this test.

Table 66:

| | TM value (°C) |
|---|---|
| R-15 | 57.5°C |
| R-16 | 53.8°C |
| R-17 | 55.2°C |
| R-18 | 54.5°C |
| R-19 | 54.0°C |
| R-20 | 59.5°C |
| R-21 | 65.0°C |

The TM values of the 7 groups of extension fragment deformation + core short-sequence RNA nanoparticles are all high, it is indicated that the self-assembly products have the good structural stability.

**Embodiment 10**

**I. 7 groups of extension fragment deformation + core short-sequence DNA nanoparticle carriers:**

(1) 7 groups of three polynucleotide base sequences for forming extension fragment deformation + core short-sequence DNA nanoparticles:

Table 67: D-8:

| Name | Sequence sense (5'-3') | Base number | Chemical modification | Molecular weight | Total molecular weight |
|---|---|---|---|---|---|
| a (SEQ ID NO: 141) | GCGGCGAGCGGCGA**GGAGCGTTGG**GGCCGGAGGCCGG | 37 | 3'-end is linked with a biotin (Bio) | 12087.3 | 32081.8 |
| b (SEQ ID NO: 142) | CCGGCCTCCGGCC**CCTTCGGGG**CCAGCCGCC | 31 | | 9375.1 | |
| c (SEQ ID NO: 143) | GGCGGCAGG**CCCCCATAGCCC**TCGCCGCTCGCCGC | 35 | | 10619.4 | |

Table 68: D-9:

| Name | Sequence sense (5'-3') | Base number | Chemical modification | Molecular weight | Total molecular weight |
|---|---|---|---|---|---|
| a (SEQ ID NO: 144) | GCGGCGAGCGGCGAGCAGCGTTCGGGCCGGAGGCCGG | 37 | 3'-end is linked with a biotin (Bio) | 12000.4 | 32071.6 |
| b (SEQ ID NO: 145) | CCGGCCTCCGGCCCGTTCGCCGCCAGCCGCC | 31 | | 9333.2 | |

(continued)

| Name | Sequence sense (5'-3') | Base number | Chemical modification | Molecular weight | Total molecular weight |
|---|---|---|---|---|---|
| c (SEQ ID NO: 146) | GGCGGCAGGCGGCCATAGCGC TCGCCGCTCGCCGC | 35 | | 10738 | |

Table 69: D-10:

| Name | Sequence sense (5'-3') | Base number | Chemical modification | Molecular weight | Total molecular weight |
|---|---|---|---|---|---|
| a (SEQ ID NO: 147) | GCGGCGAGCGGCGA**GGAGCGT TGG**GGCCGGAGGCCGG | 37 | 3'-end is linked with a biotin (Bio) | 12087.7 | 32081.6 |
| b (SEQ ID NO: 148) | CCGGCCTCCGGCC**CCTTCGCC G**CCAGCCGCC | 31 | | 9294.3 | |
| c (SEQ ID NO: 149) | GGCGGCAGG**CGGCCATAGCCC** TCGCCGCTCGCCGC | 35 | | 10699.6 | |

Table 70: D-11:

| Name | Sequence sense (5'-3') | Base number | Chemical modification | Molecular weight | Total molecular weight |
|---|---|---|---|---|---|
| a (SEQ ID NO: 150) | GCGGCGAGCGGCGAGCAGCGT TCGGGCCGGAGGCCGG | 37 | 3'-end is linked with a biotin (Bio) | 12000.4 | 32071.6 |
| b (SEQ ID NO: 151) | CCGGCCTCCGGCCCGTTCGGC GCCAGCCGCC | 31 | | 9333.2 | |
| c (SEQ ID NO: 152) | GGCGGCAGGCGCCCATAGCGC TCGCCGCTCGCCGC | 35 | | 10738 | |

Table 71: D-12:

| Name | Sequence sense (5'-3') | Base number | Chemical modification | Molecular weight | Total molecular weight |
|---|---|---|---|---|---|
| a (SEQ ID NO: 153) | GCGGCGAGCGGCGAGCAGCGT TCGGGCCGGAGGCCGG | 37 | 3'-end is linked with a biotin (Bio) | 12000.4 | 32071.6 |
| b (SEQ ID NO: 154) | CCGGCCTCCGGCCCGTTCGGC CCCAGCCGCC | 31 | | 9333.2 | |

(continued)

| Name | Sequence sense (5'-3') | Base number | Chemical modification | Molecular weight | Total molecular weight |
|---|---|---|---|---|---|
| c (SEQ ID NO: 155) | GGCGGCAGGGGCCCATAGCGC TCGCCGCTCGCCGC | 35 | | 10738 | |

Table 72: D-13:

| Name | Sequence sense (5'-3') | Base number | Chemical modification | Molecular weight | Total molecular weight |
|---|---|---|---|---|---|
| a (SEQ ID NO: 156) | GCGGCGAGCGGCGACGAGCGT TGCGGCCGGAGGCCGG | 37 | 3'-end is linked with a biotin (Bio) | 12000.4 | 32071.6 |
| b (SEQ ID NO: 157) | CCGGCCTCCGGCCGCTTCGCC GCCAGCCGCC | 31 | | 9333.2 | |
| c (SEQ ID NO: 158) | GGCGGCAGGCGGCCATAGCCG TCGCCGCTCGCCGC | 35 | | 10738 | |

Table 73: D-14:

| Nam e | Sequence sense (5'-3') | Base number | Chemical modification | Molecular weight | Total molecular weight |
|---|---|---|---|---|---|
| a (SEQ ID NO: 159) | GCGGCGAGCGGCGACGAGCGT TGCGGCCGGAGGCCGG | 37 | 3'-end is linked with a biotin (Bio) | 12000.4 | 32071.6 |
| b (SEQ ID NO: 160) | CCGGCCTCCGGCCGCTTCGGC GCCAGCCGCC | 31 | | 9373.2 | |
| c (SEQ ID NO: 161) | GGCGGCAGGCGCCCATAGCCG TCGCCGCTCGCCGC | 35 | | 10698 | |

II. Self-assembly experiment steps:

(1) according to a molar ratio of 1:1:1, enabling the DNA single strands a, b and c to be simultaneously mixed and dissolved in DEPC treated water or TMS buffer solution;

(2) heating mixed solution to 95 DEG C, after keeping for 5 min, slowly cooling to a room temperature at a rate of 2 DEG C/min;

(3) enabling a product to be loaded on 8% (m/v) of non-denaturing PAGE gel and placed in TBM buffer solution under a condition of 4 DEG C, purifying a complex by 100 v of electrophoresis;

(4) cutting a target band and eluting in DNA elution buffer solution at 37 DEG C, after that, precipitating overnight

in ethanol, and volatilizing under reduced pressure and low temperature, to obtain the DNA self-assembly products;

(5) electrophoresis analysis detection and laser scanning observation;

(6) electric potential detection;

(7) particle size detection; and

(8) TM value detection.

III. Self-assembly experiment result

(1) Electrophoresis detection

Main reagents and instruments are as follows:

Table 74:

| Reagent name | Article number | Manufacturer |
|---|---|---|
| 6×DNA Loading buffer | TSJ010 | Qingke Biotechnology Co., Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |

Table 75:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | Tanon 3500 | Tanon |

Steps:

① The DNA nanoparticles are diluted with ultrapure water by using a method shown in Table 76 below.

Table 76:

| | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

② 10μL (500ng) of the processed sample is taken and uniformly mixed with 2μL of 6xDNA Loading Buffer, it is operated

on ice, and a label is made.

③ A 8% non-denaturing PAGE gel is taken, a piece of the gel is applied to samples with different incubation times, 12 μL of the processed samples are all loaded, and a program is set to run the gel at 100V for 40 minutes.

④ After running the gel, dyeing is performed, it is placed on a horizontal shaker for 30 minutes, and pictures are taken for imaging.

Detection result:

Non-denaturing PAGE gel running results of the 7 groups of extension fragment deformation + core short-sequence DNA self-assembly products are shown in Fig. 24. Lanes 1 to 7 in Fig. 24 from left to right are successively: the 7 groups of extension fragment deformation + core short-sequence DNA self-assembly products D-8, D-9, D-10, D-11, D-12, D-13 and D-14.

It may be clearly seen from the results in Fig. 24 that the bands of the 7 groups of extension fragment deformation + core short-sequence DNA self-assembly products are bright and clear, it is indicated that the 7 groups of extension fragment deformation + core short-sequence DNA strands are all self-assembled completely, to form a stable nanoparticle structure.

(2) Electric potential measurement

Measurement method: preparing a potential sample (the self-assembly product is dissolved in ultrapure water) and putting into a sample pool, opening a sample pool cover of an instrument, and placing the instrument;
opening software, clicking a menu MeasUre€ManUal, so a manual measurement parameter setting dialog box appears; setting a software detection parameter; and then
clicking Ok to complete the settings, after a measurement dialog box appears, clicking Start to start.

Measurement result: potential detection results of the 7 groups of the extension fragment deformation + core short-sequence DNA nanoparticles at 25 DEG C are as follows:

Table 77:

| Sample name | Detection times | Potential ZP (mV) |
|---|---|---|
| D-8 | 1 | -29.20 |
| | 2 | -32.90 |
| | 3 | -28.60 |
| Experiment result | -30.23 mV | |

Table 78:

| Sample name | Detection times | Potential ZP (mV) |
|---|---|---|
| D-9 | 1 | -39.20 |
| | 2 | -34.20 |
| | 3 | -31.80 |
| Experiment result | -35.07 mV | |

Table 79:

| Sample name | Detection times | Potential ZP (mV) |
|---|---|---|
| D-10 | 1 | -27.90 |
| | 2 | -28.30 |
| | 3 | -21.10 |
| Experiment result | -25.77 mV | |

Table 80:

| Sample name | Detection times | Potential ZP (mV) |
|---|---|---|
| D-11 | 1 | -29.70 |
| | 2 | -24.80 |
| | 3 | -27.80 |
| Experiment result | -27.43 mV | |

Table 81:

| Sample name | Detection times | Potential ZP (mV) |
|---|---|---|
| D-12 | 1 | -33.50 |
| | 2 | -29.80 |
| | 3 | -34.20 |
| Experiment result | -32.50 mV | |

Table 82:

| Sample name | Detection times | Potential ZP (mV) |
|---|---|---|
| D-13 | 1 | -26.80 |
| | 2 | -27.80 |
| | 3 | -26.40 |
| Experiment result | -27.00 mV | |

Table 83:

| Sample name | Detection times | Potential ZP (mV) |
|---|---|---|
| D-14 | 1 | -31.70 |
| | 2 | -32.10 |
| | 3 | -22.40 |
| Experiment result | -28.73 mV | |

It may be seen from the above potential detection data that: the 7 groups of extension fragment deformation + core short-sequence DNA nanoparticles all have the good stability, and it is further indicated that the nanoparticles formed by the self-assembly of each extension fragment deformation + core short-sequence DNA have a relative stable self-assembly structure.

(3) Particle size measurement

1. Preparing a potential sample (7 groups of extension fragment deformation + core short-sequence DNA) and putting into a sample pool, opening a sample pool cover of an instrument, and placing the instrument;
2. opening software, and clicking a menu, so a manual measurement parameter setting dialog box appears;
3. setting a software detection parameter; and then
4. clicking Ok to complete the settings, when a measurement dialog box appears, clicking Start to start, a DLS measurement value result of a hydrodynamic size of the 7 groups of extension fragment deformation + core short-sequence DNA is as follows:

Table 84:

| Serial number | Mean particle size (nm) |
|---|---|
| D-8 | 7.460 |
| D-9 | 7.920 |
| D-10 | 7.220 |
| D-11 | 7.472 |
| D-12 | 6.968 |
| D-13 | 7.012 |
| D-14 | 6.896 |

(4) TM value detection

A solubility curve method is used to detect TM values of the 7 groups of the extension fragment deformation + core short-sequence DNA nanoparticles, and the samples are consistent with the potential samples.

Reagents and instruments are as follows:

Table 85:

| **Reagent name** | **Article number** | **Manufacturer** |
|---|---|---|
| AE buffer | / | Takara |
| SYBR Green I Dye | / | Self-made |

Table 86:

| **Name** | **Model** | **Manufacturer** |
|---|---|---|
| Real-Time System | CFX Connect | Bio-rad |
| Clean bench | HDL | Beijing Donglian Haer Instrument Manufacturing Co., Ltd. |

Steps:

① After the sample is diluted with ultrapure water, 5μg of the diluted sample is mixed with 2μL of SYBR Green I dye (diluted by 1:200), a final volume is 20μL, and the dilution concentration is as follows:

Table 87:

| Serial number | Measured concentration (μg/mL) |
|---|---|
| D-8 | 2890.932 |
| D-9 | 2238.682 |
| D-10 | 2075.084 |
| D-11 | 3117.389 |
| D-12 | 2880.939 |
| D-13 | 2704.757 |
| D-14 | 3216.917 |

② It is incubated for 30 min in the dark at a room temperature; and

③ On-machine detection is performed, a program is set to start at 20 DEG C, the temperature rises per second from

0.1 DEG C to 95 DEGC, and reading is performed every 5 s.

Detection result:

The TM values of the 7 groups of extension fragment deformation + core short-sequence DNA nanoparticles are as follows, a solubility curve diagram of the D-8 is shown in Fig. 25, a solubility curve diagram of the D-9 is shown in Fig. 26, a solubility curve diagram of the D-10 is shown in Fig. 27, a solubility curve diagram of the D-11 is shown in Fig. 28, a solubility curve diagram of the D-12 is shown in Fig. 29, a solubility curve diagram of the D-13 is shown in Fig. 30, and a solubility curve diagram of the D-14 is shown in Fig. 31.

Table 88:

| Serial number | TM value (°C) |
|---|---|
| D-8 | 48.5 |
| D-9 | 52.5 |
| D-10 | 54.5~55.0 |
| D-11 | 48.7 |
| D-12 | 51.5 |
| D-13 | 51.0 |
| D-14 | 49.2 |

It may be seen from Fig. 25 to 31 that the TM values of the 7 groups of extension fragment deformation + core short-sequence DNA nanoparticles are all high, it is indicated that the self-assembly products have the good structural stability.

**Stability detection of nucleic acid nanoparticles in serum**

**Embodiment 11**

A non-denaturing PAGE method is used to characterize the stability of 7 groups of extension fragment deformation + core short-sequence RNA nanoparticles in serum.

Main reagents and instruments are as follows:

Table 89:

| Reagent name | Article number | Manufacturer |
|---|---|---|
| 6×DNA Loading buffer | TSJ010 | Qingke Biotechnology Co., Ltd |
| 20bp DNA Ladder | 3420A | TAKARA |
| 10000*SolarGelRed nucleic acid dye | E1020 | solarbio |
| 8% non-denaturing PAGE gel | / | Self-made |
| 1× TBE Buffer (RNA enzyme-free) | / | Self-made |
| Serum (FBS) | / | Excel |
| RPMI 1640 | / | GBICO |

Table 90:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |

(continued)

| Name | Model | Manufacturer |
|---|---|---|
| Gel imager | GenoSens1880 | Shanghai Qinxiang Scientific Instrument Co., Ltd. |

Steps:

① The RNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 91:

| Sample name | Sample concentratio n (μg/mL) | Sample volume | 50%FBS-1640 / μL | 1640/ μL | Diluted concentration (μg/mL) |
|---|---|---|---|---|---|
| R-15 | 773.009 | 1.3 | 4 | 14.7 | 50 μg/mL |
| R-16 | 782.098 | 1.3 | 4 | 14.7 | 50 μg/mL |
| R-17 | 740.607 | 1.4 | 4 | 14.6 | 50 μg/mL |
| R-18 | 806.163 | 1.2 | 4 | 14.8 | 50 μg/mL |
| R-19 | 829.996 | 1.2 | 4 | 14.8 | 50 μg/mL |
| R-20 | 723.082 | 1.4 | 4 | 14.6 | 50 μg/mL |
| R-21 | 721.674 | 1.4 | 4 | 14.6 | 50 μg/mL |

② 10μL of the processed sample is taken and uniformly mixed with 2μL of 6xDNA Loading Buffer, it is operated on ice, and a label is made;
③ a 8% non-denaturing PAGE gel is taken, a piece of the gel is applied on the samples with different incubation times, 12 μL of the processed samples are all loaded, and a program is set to run the gel at 100V for 40 min; and
④ after running the gel, dyeing is performed, it is placed on a horizontal shaker and shaken slowly for 30 min, pictures are taken for imaging.

An electrophoresis detection result of the R-15 is shown in Fig. 32, an electrophoresis detection result of the R-16 is shown in Fig. 33, an electrophoresis detection result of the R-17 is shown in Fig. 34, an electrophoresis detection result of the R-18 is shown in Fig. 35, an electrophoresis detection result of the R-19 is shown in Fig. 36, an electrophoresis detection result of the R-20 is shown in Fig. 37, and an electrophoresis detection result of the R-21 is shown in Fig. 38. In Fig. 32 to 38, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of RNA nanoparticle samples at different times, it is indicated that RNA nanoparticles R-15 to R-21 are relatively stable in a 1640 medium of 50% FBS without apparent degradation.

**Embodiment 12**

A non-denaturing PAGE method is used to characterize the stability of 7 groups of extension fragment deformation + core short-sequence DNA nanoparticles in serum.

Main reagents and instruments are as follows:

Table 92:

| Reagent name | Article number | Manufacturer |
|---|---|---|
| 6×DNA Loading buffer | TSJ010 | Qingke Biotechnology Co.,Ltd |
| 20bp DNA Ladder | 3420A | TAKARA |
| 10000*SolarGelRed nucleic acid dye | E1020 | solarbio |
| 8% non-denaturing PAGE gel | / | Self-made |

(continued)

| Reagent name | Article number | Manufacturer |
|---|---|---|
| 1× TBE Buffer (RNA enzyme-free) | / | Self-made |
| Serum (FBS) | / | Excel |
| RPMI 1640 | / | GBICO |

Table 93:

| Name | Model | Manufacturer |
|---|---|---|
| Electrophoresis apparatus | PowerPac Basic | Bio-rad |
| Vertical electrophoresis cell | Mini PROTEAN Tetra Cell | Bio-rad |
| Discoloration shaker | TS-3D | orbital shaker |
| Gel imager | GenoSens1880 | Shanghai Qinxiang Scientific Instrument Co., Ltd. |

Steps:

① The DNA nanoparticles are prepared to the concentration in the table below, and then the prepared sample is diluted according to a method in the table below. It is diluted by 5 tubes. After being diluted, the sample is placed in a water bath at 37 DEG C for different times (0, 10 min, 1 h, 12 h, and 36 h);

Table 94:

| Sample name | Sample concentration (μg/mL) | Sample volume | 50%FBS-1640/μL | 1640/μL | Diluted concentration (μg/mL) |
|---|---|---|---|---|---|
| D-8 | 2890.932 | 1.4 | 8 | 30.6 | 100 |
| D-9 | 2238.682 | 1.8 | 8 | 30.2 | 100 |
| D-10 | 2075.084 | 1.9 | 8 | 30.1 | 100 |
| D-11 | 3117.389 | 1.3 | 8 | 30.7 | 100 |
| D-12 | 2880.939 | 1.4 | 8 | 30.6 | 100 |
| D-13 | 2704.757 | 1.5 | 8 | 30.5 | 100 |
| D-14 | 3216.917 | 1.2 | 8 | 30.8 | 100 |

② 10μL of the processed sample is taken and uniformly mixed with 2μL of 6xDNA Loading Buffer, it is operated on ice, and a label is made;
③ a 8% non-denaturing PAGE gel is taken, a piece of the gel is applied on the samples with different incubation times, 6 μL of the processed samples are all loaded, and a program is set to run the gel at 100V for 40 min; and
④ after running the gel, dyeing is performed, it is placed on a horizontal shaker and shaken slowly for 30 min, pictures are taken for imaging.

An electrophoresis detection result of the D-8 is shown in Fig. 39, an electrophoresis detection result of the D-9 is shown in Fig. 40, an electrophoresis detection result of the D-10 is shown in Fig. 41, an electrophoresis detection result of the D-11 is shown in Fig. 42, an electrophoresis detection result of the D-12 is shown in Fig. 43, an electrophoresis detection result of the D-13 is shown in Fig. 44, and an electrophoresis detection result of the D-14 is shown in Fig. 45. In Fig. 39 to 45, lanes from left to right are respectively M: marker; 1: 36 h; 2: 12 h; 3: 1 h; 4: 10 min; 5: 0 min. It may be seen from the results of the serum stability test that: the results of the non-denaturing gel at 10 min, 1 h, 12 h and 36 h show that there is no significant difference between bands of DNA nanoparticle samples at different times, it is indicated that DNA nanoparticles D-8 to D-14 are relatively stable in a 1640 medium of 50% FBS without apparent degradation.

Drug loading test of nucleic acid nanoparticle

**Embodiment 13**

**Doxorubicin loading experiment:**

According to the chemical loading method (unless otherwise specified, the method is the same as that of Embodiment 5) of Embodiment 5, the RNA nanoparticles formed by the self-assembly of the previous R-15, R-16, R-17, R-18, R-19, R-20 and R-21 in Embodiment 9, and the DNA nanoparticles formed by the self-assembly of the D-8, D-9, D-10, D-11, D-12, D-13 and D-14 in Embodiment 10 are respectively used as doxorubicin loading carriers, doxorubicin loading rates measured are respectively as follows:

The doxorubicin loading rate of RNA nanoparticles R-15 is 20.5.

The doxorubicin loading rate of RNA nanoparticles R-16 is 29.4.

The doxorubicin loading rate of RNA nanoparticles R-17 is 30.9.

The doxorubicin loading rate of RNA nanoparticles R-18 is 34.1.

The doxorubicin loading rate of RNA nanoparticles R-19 is 27.1.

The doxorubicin loading rate of RNA nanoparticles R-20 is 30.2.

The doxorubicin loading rate of RNA nanoparticles R-21 is 20.1.

The doxorubicin loading rate of DNA nanoparticles D-8 is 28.0.

The doxorubicin loading rate of DNA nanoparticles D-9 is 27.9.

The doxorubicin loading rate of DNA nanoparticles D-10 is 18.9.

The doxorubicin loading rate of DNA nanoparticles D-11 is 26.8.

The doxorubicin loading rate of DNA nanoparticles D-12 is 27.6.

The doxorubicin loading rate of DNA nanoparticles D-13 is 31.8.

The doxorubicin loading rate of DNA nanoparticles D-14 is 32.

Cell binding ability of DNA nanoparticles and carrier drugs detected by Fluorescence Activated Cell Sorter (FACS) Experiment

**Embodiment 14**

I. Cell information

HepG2 (from Concord Cell Bank), a medium is DMEM+10% FBS+1% double antibody (gibco, 15140-122), and culture conditions are 37 DEG C, 5% $CO_2$ and saturated humidity.

II. Substances to be tested

Blank carrier: DNA nanoparticle carriers formed by the self-assembly of the previous D-8, D-9, D-10, D-11, D-12, D-13 and D-14 in Embodiment 12.
Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 12 are used to load the doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

III. Main devices and consumables

Table 95:

| | **Manufacturer** | **Model** |
|---|---|---|
| Biosafety cabinet | Beijing Donglian Haar Instrument Manufacturing Company | BSC-1360IIA2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Fluorescence activated cell sorter | BD | BD FACSCalibur™ |

IV. Main reagent

Table 96:

| Reagent name | Manufacturer | Article number | Remark |
|---|---|---|---|
| DMEM (biotin-free) | Provided by Baiyao Zhidao Nano-biotechnology Co., Ltd. | YS3160 | |
| 1%BSA-PBS | Self-made | - | |

V. Experiment method

1. A cell state is adjusted to a logarithmic growth phase, the medium is changed into a medium without biotin and folic acid, and it is incubated overnight in an incubator at 37 DEG C.

2. After incubation, cell suspension is trypsinized and collected, and centrifuged at 1000 rpm for 5 min, after the concentration is adjusted, a $2\times10^5$-$5\times10^5$ cell/EP tube is taken and washed twice with 1 mL/tube of 1% BSA-PBS, and cells at the bottom of the tube is observed to prevent them from being absorbed.

3. A substance to be tested is dissolved, and the substance to be tested is diluted to a use concentration.

4. Cell supernatant is absorbed, 100μL of a corresponding sample is added to each tube in order, light is avoided, and it is incubated at 37 DEG C for 2 h.

5. It is washed twice with 1% BSA-PBS; and centrifuged at 1000 rpm for 5min.

6. Finally, it is precipitated with 300 μL of PBS cell resuspension, and flow cytometric on-machine detection (the blank carrier used in the present embodiment is labeled by Quasar670, and the doxorubicin in the carrier drug has its own fluorescence, so the detection may be performed through FL4-APC and FL2-PE respectively) is performed.

7. Data analysis.

VI. Experiment result

1. An experiment result is shown in the table below:

Table 97:

| Detected substance | | Experiment cell | Positive rate |
|---|---|---|---|
| PBS | | HepG2 | 2.11% |
| D-8-doxorubicin (carrier drug) | 1μM | HepG2 | 93.1% |
| | 2μM | HepG2 | 96.3% |
| D-8 (blank carrier) | 1μM | HepG2 | 96.9% |
| | 2μM | HepG2 | 98.4% |
| D-9-doxorubicin (carrier drug) | 1μm | HepG2 | 88.6% |
| | 2μM | HepG2 | 95.4% |
| D-9 (blank carrier) | 1μM | HepG2 | 96.7% |
| | 2μM | HepG2 | 98.1% |
| D-10-doxorubicin (carrier drug) | 1μM | HepG2 | 89.4% |
| | 2μM | HepG2 | 95.5% |
| D-10 (blank carrier) | 1μM | HepG2 | 97.9% |
| | 2μM | HepG2 | 98.3% |
| D-11-doxorubicin (carrier drug) | 1μM | HepG2 | 89.3% |
| | 2μM | HepG2 | 95.5% |
| D-11 (blank carrier) | 1μM | HepG2 | 97.7% |
| | 2μM | HepG2 | 97.8% |
| D-12-doxorubicin (carrier drug) | 1μM | HepG2 | 94.6% |
| | 2μM | HepG2 | 95.9% |
| D-12 (blank carrier) | 1μM | HepG2 | 96.9% |
| | 2μM | HepG2 | 97.1% |
| D-13-doxorubicin (carrier drug) | 1μM | HepG2 | 89.6% |
| | 2μM | HepG2 | 94.0% |
| D-13 (blank carrier) | 1μM | HepG2 | 97.6% |
| | 2μM | HepG2 | 98.2% |
| D-14-doxorubicin (carrier drug) | 1μM | HepG2 | 90.3% |
| | 2μM | HepG2 | 96.1% |
| D-14 (blank carrier) | 1μM | HepG2 | 97.4% |
| | 2μM | HepG2 | 98.3% |

2. Conclusion

1. After the HepG2 cells are incubated with D-8-doxorubicin (carrier drug) and D-8 (blank carrier), the binding rates are very high (93.1%-98.4%).

2. After the HepG2 cells are incubated with D-9-doxorubicin (carrier drug) and D-9 (blank carrier), the binding rates are very high (88.6%-98.1%).

3. After the HepG2 cells are incubated with D-10-doxorubicin (carrier drug) and D-10 (blank carrier), the binding rates are very high (89.4%-98.3%).

4. After the HepG2 cells are incubated with D-11-doxorubicin (carrier drug) and D-11 (blank carrier), the binding rates are very high (89.3%-97.8%).

5. After the HepG2 cells are incubated with D-12-doxorubicin (carrier drug) and D-12 (blank carrier), the binding rates are very high (94.6%-97.1%).

6. After the HepG2 cells are incubated with D-13-doxorubicin (carrier drug) and D-13 (blank carrier), the binding rates are very high (89.6%-98.2%).

7. After the HepG2 cells are incubated with D-14-doxorubicin (carrier drug) and D-14 (blank carrier), the binding rates are very high (90.3%-98.3%).

Cytotoxicity research of DNA nanoparticles and carrier drugs in HepG2 cells

**Embodiment 15**

A CCK8 method is used to detect the toxicity of DNA naoparticles and carrier drugs to HepG2.

I. Main reagent

Table 98:

| Reagent name | Manufacturer | Article number |
|---|---|---|
| PBS | - | - |
| DMSO | SIGMA | D2650 |
| DMEM(biotin-free) | Provided by Baiyao Zhidao Nano-biotechnology Co., Ltd. | YS3160 |
| FBS | Excell Bio | FSP500 |
| Double-antibody | gibco | 15140-122 |
| pancreatin | gibco | 25200-056 |
| CCK8 kit | Biyuntian Company | C0038 |

II. Main consumables and instruments

Table 99:

| **Name** | **Manufacturer** | **Model** |
|---|---|---|
| 96-well cell culture plate | NEST | 701001 |
| Biosafety cabinet | Beijing Donglian Haer Instrument Manufacturing Company | BSC-1360IIA2 |
| Low-speed centrifuge | Zhongke Zhongjia Instrument Co., Ltd. | SC-3612 |
| $CO_2$ Incubator | Thermo | 3111 |
| Inverted microscope | UOP | DSZ2000X |
| Microplate reader | Shanghai Ouying Experimental Equipment Co., Ltd. | K3 |

III. Cell information

HepG2 (from Concord Cell Bank), a medium is DMEM+10% FBS+1% double antibody (gibco, 15140-122), and culture conditions are 37 DEG C, 5% $CO_2$ and saturated humidity.

IV. Experiment material

1. Samples to be tested

Blank carrier: DNA nanoparticle carriers formed by the self-assembly of the previous D-8, D-9, D-10, D-11, D-12, D-13 and D-14 in Embodiment 10, respectively marked as: D-8, D-9, D-10, D-11, D-12, D-13 and D-14.

Carrier drug: according to the chemical loading method (unless otherwise specified, the method is the same as that of Embodiment 5) of Embodiment 5, the DNA nanoparticles formed by the self-assembly of the previous D-8, D-9, D-10, D-11, D-12, D-13 and D-14 in Embodiment 10 are used to load a doxorubicin, they are respectively marked as D-8-doxorubicin, D-9-doxorubicin, D-10-doxorubicin, D-11-doxorubicin, D-12-doxorubicin, D-13-doxorubicin and D-14-doxorubicin.

Original drug doxorubicin.

DMSO.

V. Experiment procedure

1. HepG2 cells in a logarithmic growth phase are taken, trypan blue is used for staining and the cell viability is counted to be 98.3%, plating is performed with 5000 Cell/well, a volume is 100 μL/well, 8 96-well plates are paved with 57 wells per plate, and it is incubated overnight at 37 DEG C.
2. A sample to be tested is diluted according to the following table and added: the original culture medium is removed and 100 μL of a culture medium of the sample to be tested with the different concentration is added, and there are 3 replicate wells in each group.

Table 100:

| Well number | C9 | C8 | C7 | C6 | C5 | C4 | C3 | C2 | C1 |
|---|---|---|---|---|---|---|---|---|---|
| Final concentration of loaded drug | 10μM | 3.16μM | 1μM | 316nM | 100nM | 31.6nM | 10nM | 3.16nM | 1nM |
| Final concentration of blank carrier | 1μM | 316nM | 100nM | 31.6nM | 10nM | 3.16nM | 1nM | 0.316nM | 0.1nM |
| Final concentration of original drug doxorubicin | 10μM | 3.16μM | 1μM | 316nM | 100nM | 31.6nM | 10nM | 3.16nM | 1nM |
| DMSO (%) | 0.1 | 0.0316 | 0.01 | 0.00316 | 0.001 | 0.00036 | 0.0001 | 0.000036 | 0.00001 |

In the present embodiment, the loaded drug and the blank carrier are firstly prepared into 100 μM of stock solution using the PBS, and then diluted with the complete culture medium (biotin-free DMEM). The original drug doxorubicin is firstly prepared into 100 μM of stock solution by using the DMSO, and then diluted with the complete culture medium (biotin-free DMEM). The DMSO is directly diluted with the complete medium (biotin-free DMEM).
3. After the sample to be tested is added, the 96-well plate is placed in an incubator under 37 DEG C and 5% $CO_2$ and incubated for 72 hours.
4. A kit is taken out and melted at a room temperature, 10 μL of CCK-8 solution is added to each well, or the CCK8 solution is mixed with the culture medium at a ratio of 1:9, and then added to the well in an amount of 100 μL/well.
5. It is continuously incubated in the cell incubator for 4 hours, and a length of time depends on experimental conditions such as a cell type and a cell density.
6. A microplate reader is used to measure an absorbance at 450 nm.
7. Calculation: cell viability (%)=(OD experimental group-OD blank group)×100%/(OD control group-OD blank group), $IC_{50}$ is calculated by GraphPad Prism 5.0.

VI. Experiment result

Table 101:

| Cell line | Detected sample | $IC_{50}$ (μM) |
|---|---|---|
| HepG2 | Original drug doxorubicin | 0.2725 |
| | D-8-doxorubicin (loaded drug) | 0.05087 |
| | D-8 (blank carrier) | >1 |
| | D-9-doxorubicin (loaded drug) | 0.0386 |
| HepG2 | D-9 (blanking carrier) | >1 |
| | D-10-doxorubicin (loaded drug) | 0.03955 |
| | D-10 (blank carrier) | >1 |
| | D-11-doxorubicin (loaded drug) | 0.04271 |
| HepG2 | D-11 (blank carrier) | >1 |
| | D-12-doxorubicin (loaded drug) | 0.02294 |
| | D-12 (blank carrier) | >1 |
| | D-13-doxorubicin (loaded drug) | 0.03017 |
| HepG2 | D-13 (blank carrier) | >1 |
| | D-14-doxorubicin (loaded drug) | 0.03458 |
| | D-14 (blank carrier) | >1 |
| | DMSO | >0.1% |

Conclusion:

It may be seen from the above table and Fig. 46a, Fig. 46b, Fig. 46c, Fig. 46d, Fig. 46e, Fig. 46f. Fig. 46g and Fig. 46h that the $IC_{50}$ of the original drug doxorubicin and the loaded drugs D-8-doxorubicin, D-9-doxorubicin, D-10-doxorubicin, D-11-doxorubicin, D-12-doxorubicin, D-13-doxorubicin and D-14-doxorubicin acting on the HepG2 cells is 0.2725 μM, 0.05087 μM, 0.0386, 0.03955, 0.04271, 0.02294, 0.03017 and 0.03458 respectively; the $IC_{50}$ of the DMSO acting on the HepG2 cells is >0.1%; the $IC_{50}$ of the D-8 (blank carrier), D-9 (blank carrier), D-10 (blank carrier), D-11 (blank carrier), D-12 (blank carrier), D-13 (blank carrier) and D-14 (blank carrier) acting on the HepG2 cells is >1μM. It is indicated that for the HepG2 cell line, compared with the simple blank carriers D-8, D-9, D-10, D-11, D-12, D-13 and D-14, the original drug doxorubicin of small molecular drug and the loaded drugs D-8-doxorubicin, D-9-doxorubicin, D-10-doxorubicin, D-11-doxorubicin, D-12-doxorubicin, D-13-doxorubicin Both D-14-doxorubicin and D-14-doxorubicin are toxic to the HepG2 cells, and compared with the original drug doxorubicin, the loaded drugs D-8-doxorubicin, D-9-doxorubicin, D-10-doxorubicin, and D-11-doxorubicin, D-12-doxorubicin, D-13-doxorubicin and D-14-doxorubicin have

an apparent synergistic effect.

Embodiment 16

According to the chemical loading method (unless otherwise specified, the method is the same as that of Embodiment 5) of Embodiment 5, the DNA nanoparticles formed by the self-assembly of the previous D-10 and D-14 in Embodiment 10 are used as a daunorubicin loading carrier. A microplate reader is used to measure an absorbance of the daunorubicin at 492 nm, and a standard curve (as shown in Fig. 47) is drawn.

Measured loading rates of the daunorubicin are respectively as follows:

The daunorubicin loading rate of the DNA nanoparticles D-10 is 24.0.

The daunorubicin loading rate of the DNA nanoparticles D-14 is 25.1.

**Embodiment 17**

**Epirubicin loading experiment**

(I) Loading of RNA nanoparticles

According to a chemical loading method of Embodiment 5 (unless otherwise specified, the method is the same as that of Embodiment 5, the same molar number of the epirubicin is used as a loading amount), nucleic acid nanoparticles (a molecular weight is 29550, it is similar to the RNA nanoparticles in Embodiment 1, and a difference is that a fluorescent label on a c-strand is Cy5) are used as a carrier, and respectively loaded with: epirubicin, methotrexate, pirarubicin, daunorubicin, pentafluorouracil, 10-hydroxycamptothecin, aspirin and gemcitabine.

Herein, when a standard curve is drawn, the absorbance of the above drugs on the microplate reader is measured respectively. The absorbance of the epirubicin, methotrexate, pirarubicin, daunorubicin, pentafluorouracil, 10-hydroxycamptothecin, aspirin and gemcitabine is respectively detected at the following wavelengths: 492 nm, 303 nm, 492 nm, 492 nm, 265 nm , 384 nm, 225 nm and 268 nm. The standard curves obtained correspondingly are respectively shown in Fig. 48a, Fig. 49, Fig. 50a, Fig. 51, Fig. 52, Fig. 53, Fig. 54a and Fig. 55.

Each loading rate measured is respectively as follows:

Epirubicin:

$C_{RNAh-1}$ = 21.0 μg/ml, $M_{RNAh}$ ≈30000, 100μl; $C_{epirubicin-1}$ = 7.158 μM, 100μl;

$C_{RNAh-2}$ = 33.5 μg/ml, $M_{RNAH}$ ≈30000, 100μl; $C_{epirubicin-2}$=9.263 μM, 100μl;

$$N\text{-}1=\frac{7.158\times 10\text{-}6\times 100\times 10\text{-}6}{0.0210\times 100\times 10\text{-}6/30000}=10.2, \quad N\text{-}2=\frac{9.263\times 10\text{-}6\times 100\times 10\text{-}6}{0.0335\times 100\times 10\text{-}6/30000}=8.3.$$

An average value thereof is taken so that the loading rate of epirubicin-RNAh nucleic acid nanoparticles is about 9.3, and it means that about 9.3 epirubicin molecules may be loaded on each nucleic acid nanoparticle carrier.

Methotrexate:

CRNAh-1 = 45.0 μg/μL, $M_{RNAh}$ ≈30000, 100μL; $C_{methotrexate-1}$ = 16.9 μM, 100μL;

CRNAh-2 = 36.0 μg/μL, $M_{RNAh}$ ≈30000, 100μL; $C_{methotrexate-2}$ = 10.85 μM, 100μL;

$$N\text{-}2=\frac{10.85\times 10\text{-}6\times 100\times 10\text{-}6}{0.0360\times 100\times 10\text{-}6/30000}=9.04\ ;\quad N\text{-}1=\frac{16.9\times 10\text{-}6\times 100\times 10\text{-}6}{0.0450\times 100\times 10\text{-}6/30000}=11.3\ .$$

An average value thereof is taken so that the loading rate of methotrexate-RNAh nucleic acid nanoparticles is about 10, and it means that about 10 methotrexate molecules may be loaded on each nucleic acid nanoparticle carrier.

Pirarubicin:

$C_{RNAh\text{-}1}$ = 23.2 μg/ml, $M_{RNAh}$ ≈30000, 100μL; C $_{pirarubicin\ \text{-}1}$ =8.500 μM, 100μL;

$C_{RNAh\text{-}2}$ = 48.1 μg/ml, $M_{RNAh}$ ≈30000, 100μL; C $_{pirarubicin\text{-}2}$ = 19.24 μM, 100μL;

$$N\text{-}1=\frac{8.500\times 10\text{-}6\times 100\times 10\text{-}6}{0.0232\times 100\times 10\text{-}6/30000}=11\ ,\quad N\text{-}2=\frac{19.24\times 10\text{-}6\times 100\times 10\text{-}6}{0.0481\times 100\times 10\text{-}6/30000}=12\ .$$

An average value thereof is taken so that the loading rate of pirarubicin-RNAh nucleic acid nanoparticles is about 11.5, and it means that about 11.5 pirarubicin molecules may be loaded on each nucleic acid nanoparticle carrier.

Daunorubicin:

$C_{RNAh\text{-}1}$ = 58.8 μg/ml, $M_{RNAh}$≈30000, 100μL, C $_{daunorubicin\text{-}1}$ = 11.76 μM, 100μL;

$C_{RNAh\text{-}2}$ = 39.8 μg/ml, $M_{RNAh}$ ≈30000, 100μL; C $_{daunorubicin\text{-}2}$ = 7.506 μM, 100μL;

$$N\text{-}1=\frac{11.76\times 10\text{-}6\times 100\times 10\text{-}6}{0.0588\times 100\times 10\text{-}6/30000}=6\ ,\quad N\text{-}2=\frac{7.506\times 10\text{-}6\times 100\times 10\text{-}6}{0.0398\times 100\times 10\text{-}6/30000}=5.7\ .$$

An average value thereof is taken so that the loading rate of daunorubicin-RNAh nucleic acid nanoparticles is about 6, and it means that about 6 daunorubicin molecules may be loaded on each nucleic acid nanoparticle carrier.

Pentafluorouracil:

The loading rate of the RNAh-pentafluorouracil obtained by calculating is about 0.31, and it is represented that about 0.31 pentafluorouracil molecules may be loaded on each nucleic acid nanoparticle carrier.

Through changing a relative dosage of the pentafluorouracil and the RNA nanoparticles, the RNAh-pentafluorouracil particles of which the loading rates is 10, 20, 28, and 50 and the like may also be acquired, it is not repeatedly described here.

10-hydroxycamptothecin:

$C_{RNAh\text{-}1}$ = 73.3 μg/ml, $M_{RNAh}$ ≈30000, 100μL; $C_{10\text{-}hydroxycamptothecin\text{-}1}$ = 28.88 μM, 100μL;

$C_{RNAh\text{-}2}$ = 65.8 μg/ml, $M_{RNAh}$ ≈30000, 100μL; $C_{10\text{-}hydroxycamptothecin\ \text{-}2}$ = 15.2 μM, 100μL;

$$N\text{-}2=\frac{15.2\times 10\text{-}6\times 100\times 10\text{-}6}{0.0658\times 100\times 10\text{-}6/30000}=6.9\ ,\quad N\text{-}1=\frac{28.88\times 10\text{-}6\times 100\times 10\text{-}6}{0.0733\times 100\times 10\text{-}6/30000}=11.8\ .$$

An average value thereof is taken so that the loading rate of 10-hydroxycamptothecin-RNAh is about 9, and it means that about 16 10-hydroxycamptothecin molecules may be loaded on each nucleic acid nanoparticle carrier.

Through changing a relative dosage of the 10-hydroxycamptothecin and the RNA nanoparticles, the RNAh-10-hydroxycamptothecin particles of which the loading rates is 10, 20, 28, 50, 70, 80, 100, and 200 and the like may also be acquired, it is not repeatedly described here.

Aspirin:

$C_{RNAh-1}$ = 68.4 μg/ml, $M_{RNAH}$ ≈30000, 100μl; C $_{aspirin-1}$ = 52.5 μM, 100μl;

$C_{RNAh-2}$ = 26.8 μg/ml, $M_{RNAh}$ ≈30000, 100μl; C $_{aspirin-2}$ = 18.4 μM, 100μl;

$$N\text{-}2=\frac{18.4\times 10\text{-}6\times 100\times 10\text{-}6}{0.0268\times 100\times 10\text{-}6/30000}=20.59 \quad , \quad N\text{-}1=\frac{52.5\times 10\text{-}6\times 100\times 10\text{-}6}{0.0684\times 100\times 10\text{-}6/30000}=23.02 .$$

An average value of the N-1 and N-2 is taken so that the loading rate of aspirin-RNAh is about 22, and it means that about 22 aspirin molecules may be loaded on each nucleic acid nanoparticle carrier.

Gemcitabine:

$C_{RNAh-1}$ = 26.9 μg/ml, $M_{RNAh}$ ≈30000, 100μL; C $_{gemcitabine-1}$ = 18.23 μM, 100μL;

$C_{RNAh-2}$ = 29.8 μg/μL, $M_{RNAh}$ ≈30000, 100μLμL; C $_{gemcitabine\ -2}$ = 21.65 μM, 100μLμL;

$$N\text{-}2=\frac{21.65\times 10\text{-}6\times 100\times 10\text{-}6}{0.0298\times 100\times 10\text{-}6/30000}=21.8 \quad , \quad N\text{-}1=\frac{18.23\times 10\text{-}6\times 100\times 10\text{-}6}{0.0269\times 100\times 10\text{-}6/30000}=20.4 .$$

An average value of the N-1 and N-2 is taken so that the loading rate of gemcitabine-RNAh is about 21, and it means that about 21 gemcitabine molecules may be loaded on each nucleic acid nanoparticle carrier.

(II) Loading experiment of DNA nucleic acid nanoparticles

The loading method and the calculation method of the loading rate are the same as the above RNA nucleic acid nanoparticles. The specific nucleic acid nanoparticles used are:
DNAh-Bio-EGFRapt-Cy5, herein the three strands of DNAh are respectively as follows:

a-strand: (SEQ ID NO:172:) 5'-CGCGCGCCCACGAGCGTTCCGGGCGC**GCCTTAGTAACGTGCTTTGATGTCGATTCGACAG GAGGC-**3'; the first three bases at the 5'-end and the last three bases at the 3'-end are thio-modified, and the 5'-end is linked with Biotin, a bolded part is the EGFRapt sequence;

b-strand (SEQ ID NO: 173:): 5'-GCGCCCGGTTCGCCGCCAGCCGCCGC-3', the first three bases at the 5'-end and the last three bases at the 3'-end are thio-modified; and

c-strand (SEQ ID NO: 174:): 5'-GCGGCGGCAGGCGGCCATAGCCGTGGGCGCGCG-3'; the first three bases at the 5'-end and the last three bases at the 3'-end are respectively thio-modified, and the 3'-end is linked with a Cy5 fluorescent label.

The standard curve of the above epirubicin-loaded DNA nucleic acid nanoparticles is shown in Fig. 48b, and a specific calculation process is as follows:

$C_{DNAh-1}$ = 22.19 ug/ml, $M_{DNAh}$ ≈39500, 100μL; C $_{epirubicin-1}$ = 17.06 μM, 100μL;

$C_{DNAh-2}$ = 32.57 ug/ml, $M_{DNAh}$ ≈39500, 100μL; $C_{epirubicin\ -2}$ = 20.40 μM, 100μL;

$$N\text{-}1= \frac{17.06\times10\text{-}6\times100\times10\text{-}6}{0.2219\times100\times10\text{-}6/39500}\approx 30.4 \qquad N\text{-}2= \frac{20.40\times10\text{-}6\times100\times10\text{-}6}{0.3257\times100\times10\text{-}6/39500}\approx 24.7$$

;

An average value thereof is taken so that the loading rate of epirubicin-DNAh is about 27.6, and it means that about 27.6 epirubicin molecules may be loaded on each DNA nanoparticle carrier.

The standard curve of the above pirarubicin-loaded DNA nucleic acid nanoparticles is shown in Fig. 50b, and a specific calculation process is as follows:

$C_{DNAh-1}$ = 18.64 ug/ml, $M_{DNAh}$ ≈39500, 100μL; $C_{pirarubicin-1}$ = 11.7 μM, 100μL;

$C_{DNAh-2}$ = 41.23 ug/ml, $M_{DNAh}$ ≈39500, 100μL; $C_{pirarubicin\ -2}$ = 19.73 μM, 100μL;

$$N\text{-}1= \frac{11.7\times10\text{-}6\times100\times10\text{-}6}{0.1864\times100\times10\text{-}6/39500}\approx 24.9 \qquad N\text{-}2= \frac{19.73\times10\text{-}6\times100\times10\text{-}6}{0.4123\times100\times10\text{-}6/39500}\approx 18.9$$

.

An average value thereof is taken so that the loading rate of pirarubicin-DNAh is about 21.9, and it means that about 21.9 pirarubicin molecules may be loaded on each DNA nanoparticle carrier.

The standard curve of the above aspirin-loaded DNA nucleic acid nanoparticles is shown in Fig. 54b, and a specific calculation process is as follows:

$C_{DNAh-1}$ = 10.97 μg/ml, $M_{DNAh}$ ≈39500, 100μl; $C_{aspirin\ -1}$ = 3.7 μM, 100μl;

$C_{DNAh-2}$ = 21.56 μg/ml, $M_{DNAh}$ ≈39500, 100μl; $C_{aspirin\ -2}$ = 7.67 μM, 100μl;

$$N\text{-}1= \frac{3.7\times10\text{-}6\times100\times10\text{-}6}{0.1097\times100\times10\text{-}6/39500}\approx 13.4 \qquad N\text{-}2= \frac{7.67\times10\text{-}6\times100\times10\text{-}6}{0.2156\times100\times10\text{-}6/39500}\approx 14.05$$

An average value thereof is taken so that the loading rate of aspirin-DNAh is about 14, and it means that about 14 aspirin molecules may be loaded on each DNA nanoparticle carrier.

In addition, on the basis of the above RNA nanoparticles and DNA nanoparticles loaded with each of the above drugs such as the epirubicin, other small molecular drugs may be further loaded for the second time in the same way as the epirubicin loading. For example, the present application is further loaded with a folic acid to obtain the RNA nanoparticles and DNA nanoparticles co-loaded with two small molecular drugs of the epirubicin and the folic acid, and the loading rates of the two drugs may be detected by referring to the above method (values are not shown).

It is indicated from the present embodiment that the RNA nanoparticles (in Embodiment 1) and DNA nanoparticles with the extension fragment, the target head and the fluorescein have a function of loaded drugs, may achieve the loading with the small molecular drug epirubicin in a mode of covalent linkage (paraformaldehyde-solvent covalence), and may also achieve the co-loading with other small molecular drugs.

**Embodiment 18**

**Cell binding ability of drug-loaded RNA nanoparticles detected by confocal microscopy experiment**

I. Experiment material and experiment method:

1. Samples to be tested as shown in Table 102:

Table 102:

| Nanoparticles | MW | Dissolution reagent |
|---|---|---|
| RNAh-Biotin-quasar670, RNAh-Bio-670 for short | 29552.6 | PBS |
| RNAh-Biotin-quasar670-EPB, RNAh-Bio-670-EPB for short | 35352.4 | PBS |
| RNAh-Biotin-quasar670-MTX, RNAh-Bio-670-MTX for short | 35352.4 | PBS |
| RNAh-Biotin-quasar670-THP, RNAh-Bio-670-THP for short | 35352.4 | PBS |
| RNAh-Biotin-quasar670-DNR, RNAh-Bio-670-DNR for short | 35352.4 | PBS |
| RNAh-Biotin-quasar670-flu | 32934.68 | PBS |
| RNAh-Biotin-quasar670-hdcp | 33196.1 | PBS |
| RNAh-Biotin-quasar670-aspirin, RNAh-Bio-670-aspirin for short, also called RNAh-Bio-aspirin | 34056.35 | PBS |
| RNAh-Biotin-quasar670-gemcitabine, RNAh-Bio-670-gemcitabine for short, also called RNAh-Bio-gemcitabine | 35079.8 | PBS |
| Note: the RNAh-Bio-670 in the table is served as a control, and refers to the nanoparticles formed by performing the Biotin modification at the 5'-end of the a-strand and b-strand prepared according to the self-assembly method in Embodiment 1, and performing the quasar670 fluorescein modification at the 3'-end of the c-strand, and the RNAh-Bio-670-EPB and the like refer to the nanoparticles formed after further loading the epirubicin (loaded according to the chemical method in Embodiment 5). | | |

2. Experiment reagents used and sources thereof are as follows:
RPMI-1640 medium (Gibco, C11875500BT-500mL); Fetal bovine serum (FBS) (ExCell Bio, FNA500-500mL); Penicillin/Streptomycin (PS) (Gibco, 15140-122 -100mL); PBS buffer solution (Gibco, C20012500BT-500mL); Trypsin-EDTA (Stemcell, 07901-500mL); DMSO (Sigma, D5879-1L); Prolong Gold Antifade Mountant (Thermo, P36941-2 mL); and DAPI (Yeasen, 36308ES11-4 mL).
3. Experiment instruments used are as follows:
Inverted Microscope (Olympus BX53, U-RFL-T); BD Falcon (Corning, 354118); and Cytospin (TXD3).
4. Experiment method:

(1) Respectively placing cells used by each drug in a RPMI1640+10%FBS+1%PS medium, and culturing under a condition of 37 DEG C and 5% $CO_2$;
Epirubicin: HL60 cells, acute leukemia cell line;
Methotrexate: MOLT4 cells (human acute lymphoblastic leukemia line)
Pirubicin: MCF-7 cells (human breast cancer cell line)
Daunorubicin: MCF-7 cells
Pentafluorouracil: HepG2 cells;
10-hydroxycamptothecin: SGC7901;
Aspirin: SH-SY5Y cells;
Gemcitabine: BxPC-3 cells.

(2) Collecting the cells, washing with the PBS, and adding to the 48-well plate in $1x10^5$ cells per well.

(3) Incubating the cells with 200 nM and 400 nM of the RNAh-Bio-670 and RNAh-Bio-670-EPB nanoparticles at 37 DEG C and 5% $CO_2$ for 1-2 h and 4 h.

(4) After the cells are washed with the PBS, adding the cells to a glass slide by a centrifugal picture method, treating with the Prolong Gold Antifade Mountant, and keeping overnight at a room temperature.

(5) Staining with DAPI for 5 min at the room temperature, and then sealing the glass slide.

(6) Using DAPI and FAM channels of the laser scanning confocal microscope to evaluate the cell binding and internalization, taking pictures under the microscope and saving.

II. Experiment result

Experiment results are shown in Fig. 56 to Fig. 63. It may be seen from Fig. 56 that the RNAh-Bio-670 and RNAh-Bio-670-EPB nanoparticles may be both bound and internalized with the cells because they both carry the target head---Biotin. It may be seen that the drug RNAh-Bio-670-EPB nanoparticles containing the epirubicin have a relatively strong binding and internalization ability to the HL60 cells.

It may be seen from Fig. 57 that the RNAh-Bio-670 and RNAh-Bio-670-MTX nanoparticles may be both bound and internalized with the cells because they both carry the target head---Biotin. This result shows that the drug RNAh-Bio-670-MTX nanoparticles containing the methotrexate have a relatively strong binding and internalization ability to the MOLT4 cells.

It may be seen from Fig. 58 that the cell binding and internalization experiment results show that the RNAh-Bio-670 and RNAh-Bio-670-THP nanoparticles may be both bound and internalized with the cells because they both carry the target head---Biotin. This result shows that the drug RNAh-Bio-670-THP nanoparticles containing the pirarubicin have a relatively strong binding and internalization ability to the MCF-7 cells.

It may be seen from Fig. 59 that the cell binding and internalization experiment results show that the RNAh-Bio-670 and RNAh-Bio-670-DNR nanoparticles may be both bound and internalized with the cells because they both carry the target head---Biotin. This result shows that the drug RNAh-Bio-670-DNR nanoparticles containing the daunorubicin have a relatively strong binding and internalization ability to the MCF-7 cells.

It may be seen from Fig. 60 that the cell binding and internalization experiment results show that the RNAh-Biotin-quasar670 and RNAh-Biotin-quasar670-flu nanoparticles may be both bound and internalized with the cells because they both carry the target head---Biotin.

It may be seen from Fig. 61 that the cell binding and internalization experiment results show that the RNAh-Biotin-quasar670 and RNAh-Biotin-quasar670-hdcp nanoparticles may be both bound and internalized with the cells because they both carry the target head---Biotin.

It may be seen from the cell binding and internalization experiment results in Fig. 62 and Fig. 63 that the aspirin-RNA nanoparticles have a relatively strong binding and internalization ability to the SH-SY5Y cells, and the gemcitabine-RNA nanoparticles have a relatively strong binding and internalization ability to the BxPC-3 cells.

### Embodiment 19

#### (I) Binding ability of DNAh-Bio-EGFRapt-Cy5-EPB nanoparticles and cells detected by flow cytometry experiment

I. Cell information

MCF-7 (source: ATCC, and article number: HTB-22), NCI-H1975 (source: ATCC, and article number: CRL-5908); a culture medium is MEM + 10% FBS, and culture conditions are 37 DEG C, 5% $CO_2$, and saturated humidity.

II. Substances to be tested

Targeted drug: DNAh-Bio-EGFRapt-Cy5-EPB (loaded according to the DNA nanoparticle loading method in Embodiment 5).

Targeted fluorescent carrier: DNAh-Bio-EGFRapt-Cy5.

III. Devices and consumables

Table 103:

| Name | Brand | Article number / Model |
|---|---|---|
| 24-well plate | Corning | 3526 |
| Centrifuge | Gineek | LD5-2B |
| $CO_2$ incubator | Thermo | 3111 |
| Microwell plate shaker | QILINBEIER | QB-9001 |
| Microscope | Olympus | IX53 |
| Multifunctional microplate reader | Bio Tek | Synergy H1 |
| Flow cytometer | ACEA | Novo Cyte |

IV. Reagents

Table 104:

| Name | Brand | Article number |
|---|---|---|
| RPMI 1640 medium (without folic acid and biotin) | Provided by Baiyao Zhidao Nano-biotechnolog y Co., Ltd | YS3160-500 |
| MEM medium (without folic acid and biotin) | Provided by Baiyao Zhidao Nano-biotechnolog y Co., Ltd | YS4150-500 |
| DMEM medium (without folic acid and biotin) | Provided by Baiyao Zhidao Nano-biotechnolog y Co., Ltd | YS1200-500 |
| FBS | Cegrogen | A0500-3018 |
| GlutaMax | Gibco | 35050-061 |
| trypsin-EDTA digestive juice (0.25%) | Solarbio | T1320-100ml |
| Penicillin-streptomycin (100X) | Solarbio | P1400 |
| CellTiter-Glo® 2.0 | Promega | G9243 |

V. Experiment method:

1. Adjusting a cell state to a logarithmic growth phase, changing the medium to a medium without biotin and folic acid, and incubating overnight in the incubator at 37 DEG C;

2. dissolving the substances to be tested and preparing the to-be-tested substance stock solution;

3. digesting and collecting the single-cell suspension and counting, adjusting the cell density to $2\times10^5$/mL, planting 1 mL/well into the 24-well plate;

4. respectively adding the substances to be tested to the corresponding cell wells, herein the final concentration is 2 $\mu$M, shaking and mixing uniformly;

5. placing the cell plate in the incubator at 37 DEG C and incubating for 2 hours;

6. after the incubation, pancreatin-digesting and collecting the cell suspension;

7. centrifugally collecting a cell precipitation, and washing twice with the PBS;

8. finally, resuspending the cell precipitation with 300 $\mu$L of the PBS, and performing a flow cytometric on-machine detection;

9. fluorescence carrier or epirubicin detection channels: excitation light wavelength: 488 nm, and emission light channel: 560nm; and

10. data analysis.

VI. Experiment result

Table 105:

| Cell line | Test sample | Binding rate (%) |
|---|---|---|
| MCF-7 | DNAh-Bio-EGFRapt-Cy5-EPB | 99.89 |
| | DNAh-Bio-EGFRapt-Cy5 | 99.97 |
| | Blank control (medium only) | 0.16 |
| NCI-H1975 | DNAh-Bio-EGFRapt-Cy5-EPB | 100 |
| | DNAh-Bio-EGFRapt-Cy5 | 100 |
| | Blank control (medium only) | 0.34 |

It may be seen from the above table that the epirubicin targeted drug DNAh-Bio-EGFRapt-Cy5-EPB may be bound with the MCF-7 cells and NCI-H1975 cells, and the binding rates are both close to 100%; and the targeted fluorescent carrier DNAh- Bio-Cy5 may also be bound with the MCF-7 cells and NCI-H1975 cells, and the binding rates are also 100%.

**(II) Cell binding ability of RNAh-Biotin-Cy5-DNR nanoparticles detected by flow cytometer**

I. Samples to be tested

Targeted drug: RNAh-Biotin-Cy5-DNR, herein a preparation method of the RNAh-Biotin-Cy5 is the same as that of the RNAh-Biotin-quasar670, and a difference is that the fluorescent substance is replaced by the Cy5 from the quasar670. The RNAh-Biotin-Cy5-DNR is the nanoparticles formed by further loading the DNR on the RNAh-Biotin-Cy5 (loaded according to the method in Embodiment 5).

II. Experiment cells and culture conditions (MCF-7 cells, the details are the same as the above, and it is not repeatedly described here)

III. Fluorescence detection

Conditions of a fluorescence detection are as follows:
Excitation light is 640 nm, emission light is 675 nm, a detection height is 7 mm, measured value/data point=10, detection speed: normal, and extension: 100 ms.

IV. Detection result

Table 106:

| | Test sample | Binding rate (%) | | Binding rate (%) | |
|---|---|---|---|---|---|
| | | Treatment time 1h | | Treatment time 2h | |
| | | 0.2μM | 0.4μM | 0.2μM | 0.4μM |
| MCF-7 | RNAh-Biotin-Cy5-DNR | 39.41% | 84.22% | 87.14% | 94.53% |
| | Blank control (medium only) | 0.45% | 0.45% | 0.24% | 0.24% |

It may be seen from the above table that the binding rate of RNAh-Biotin-Cy5-DNR nanoparticles and MCF-7 cells may be as high as 84% or more in the case that the treatment time and concentration are appropriate. Compared with the blank control containing the medium only, the RNA drug-loaded particles have a strong ability to bind and internalize with the MCF-7 cells.

**(III) Cell binding ability of pirarubicin-loaded DNA nanoparticles detected by flow cytometer**

I. Cell information

Table 107:

| Cell line | Source | Product model or article number | Medium | Culture condition |
|---|---|---|---|---|
| SKOV3 | ATCC | HTB-77 | MEM + 10% FBS | 37°C, 5%$CO_2$, |
| SGC-7901 | Bolise | SGC-7901 | DMEM + 10% FBS | Saturation humidity |

II. Samples to be tested

Pirubicin targeted drug: DNAh-Biotin-EGFRapt-Cy5-THP; (loaded according to the loading method of the DNA nanoparticles in Embodiment 5).

Targeted fluorescent carrier: DNAh-Bio-EGFRapt-Cy5.

III. Information about instruments, devices and related reagents (same as above)

IV. Experiment method:

1). Adjusting a cell state to a logarithmic growth phase, changing the medium to a medium without biotin and folic acid, and incubating overnight in the incubator at 37 DEG C;

2). dissolving the substances to be tested and preparing the to-be-tested substance stock solution;

3). digesting and collecting the single-cell suspension and counting, adjusting the cell density to $2\times10^5$/mL, planting 1 mL/well into the 24-well plate;

4). respectively adding the substances to be tested to the corresponding cell wells, herein the final concentration is 2 $\mu$M, shaking and mixing uniformly;

5). placing the cell plate in the incubator at 37 DEG C and incubating for 2 hours;

6). after the incubation, pancreatin-digesting and collecting the cell suspension;

7). centrifugally collecting a cell precipitation, and washing twice with the PBS;

8). finally, resuspending the cell precipitation with 300 $\mu$L of the PBS, and performing a flow cytometric on-machine detection; herein detection channels of fluorescence carrier or pirarubicin targeted drug: excitation light wavelength: 488 nm, and emission light channel: 560 nm; and

9). data analysis. An analysis result is shown in the following table.

Table 108:

| Cell line | Test sample | Binding rate (%) |
|---|---|---|
| SGC-7901 | DNAh-Bio-EGFRapt-Cy5-THP | 100 |
| | DNAh-Bio-EGFRapt-Cy5 | 99.99 |
| | Blank control (medium only) | 0.34 |

(continued)

| Cell line | Test sample | Binding rate (%) |
|---|---|---|
| SKOV3 | DNAh-Bio-EGFRapt-Cy5-THP | 99.98 |
| | DNAh-Bio-EGFRapt-Cy5 | 100 |
| | Blank control (medium only) | 0.11 |

It may be seen from the above table that the DNA nucleic acid nanoparticles carrying the target head and the small molecular drug pirarubicin have a high binding rate to the cells, and it may be apparently seen that it may be bound and internalized with the corresponding tumor cell line cells. In addition, it may also be seen from the above table that the DNAh-Bio-EGFRapt-Cy5-THP may not only efficiently bind and internalize with the human gastric cancer cell line SGC-7901 cells, but also may bind and internalize with the human ovarian cancer cell line SKOV3 cells. It may be seen that the DNAh-Bio-EGFRapt-Cy5-THP, a pirarubicin targeted drug, has both application prospects for the treatment of gastric cancer and ovarian cancer.

**(IV) Cell binding ability of RNAh-Biotin-Cy5-THP nanoparticles detected by flow cytometer**

I. Samples to be tested

Targeted drug: RNAh-Biotin-Cy5-THP, herein a preparation method of the RNAh-Biotin-Cy5 is the same as that of the RNAh-Biotin-quasar670, and a difference is that the fluorescent substance is replaced by the Cy5 from the quasar670. The RNAh-Biotin-Cy5-THP is the nanoparticles formed by further loading the THP on the RNAh-Biotin-Cy5 (loaded according to the method in Embodiment 5).

II. Experiment cells and culture conditions (MCF-7 cells, the details are the same as the above confocal microscopy experiment in Embodiment 6, and it is not repeatedly described here)

III. Fluorescence detection

Conditions of a fluorescence detection are as follows:
Excitation light is 640 nm, emission light is 675 nm, a detection height is 7 mm, measured value/data point=10, detection speed: normal, and extension: 100 ms.

IV. Detection result

Table 109:

| Cell line | Test sample | Binding rate (%) | | Binding rate (%) | |
|---|---|---|---|---|---|
| | | Treatment time 1h | | Treatment time 2h | |
| | | 0.2μM | 0.4μM | 0.2μM | 0.4μM |
| MCF-7 | RNAh-Biotin-Cy5-THP | 98.69% | 99.55% | 96.65% | 99.23% |
| | Blank control (medium only) | 0.45% | 0.45% | 0.24% | 0.24% |

It may be seen from the above table that the binding rate of RNAh-Biotin-Cy5-THP nanoparticles and MCF-7 cells may be as high as 96% or more. Compared with the blank control containing the medium only, the RNA drug-loaded particles have a strong ability to bind and internalize with the MCF-7 cells.

**(V) Cell binding ability of RNAh-Biotin-Cy5-gemcitabine nanoparticles detected by flow cytometer**

I. Samples to be tested

Targeted drug: RNAh-Biotin-Cy5-gemcitabine, herein a preparation method of the RNAh-Biotin-Cy5 is the same as that of the RNAh-Biotin-quasar670, and a difference is that the fluorescent substance is replaced by the Cy5 from the quasar670. The RNAh-Biotin-Cy5-gemcitabine is the nanoparticles formed by further loading the gemcitabine on the RNAh-Biotin-Cy5 (loaded according to the method in Embodiment 5).

II. Experiment cells and culture conditions (BxPC-3 cells, same as above)

III. Fluorescence detection

Conditions of a fluorescence detection are as follows:
Excitation light is 640 nm, emission light is 675 nm, a detection height is 7 mm, measured value/data point=10, detection speed: normal, and extension: 100 ms.

IV. Detection result

Table 110:

| | Test sample | Binding rate (%) | | Binding rate (%) | |
|---|---|---|---|---|---|
| | | Treatment time 1h | | Treatment time 2h | |
| | | 0.2μM | 0.4μM | 0.2μM | 0.4μM |
| BxPC-3 | RNAh-Biotin-Cy5-gemcitabine | 95.10% | 98.51% | 98.17% | 99.59% |
| | Blank control (medium only) | 0.14% | 0.14% | 0.16% | 0.16% |

It may be seen from the above table that the binding rate of RNAh-Biotin-Cy5-gemcitabine nanoparticles and BxPC-3 cells may be as high as 98% or more in the case that the treatment time and concentration are appropriate. Compared with the blank control containing the medium only, the RNA drug-loaded particles have a strong ability to bind and internalize with the BxPC-3 cells.

**Embodiment 20**

**Stability detection of nanoparticles in serum**

(I) Stability of RNAh-Bio-670-EPB nanoparticles in serum

I. Experiment material and experiment method

1. Samples to be tested: RNAh-Bio-670-EPB nanoparticles dissolved in PBS solution.

2. Experiment reagents:
RPMI-1640 medium (Gibco, C11875500BT-500 mL); Fetal bovine serum (FBS) (ExCell Bio, FNA500-500 mL); Penicillin/Streptomycin (PS) (Gibco, 15140 -122-100 mL); PBS buffer solution (Gibco, C20012500BT-500 mL); Novex™ Tris-Glycine Native Sample Buffer (2X) (Invitrogen, LC2673-20 mL); Novex™ 8% Tris-Glycine Mini Gels (Invitrogen , XP00080BOX-1.0 mm); Tris-Glycine Native Running buffer (10x) (Life science, LC2672-500 mL); and G250 staining solution (Beyotime, P0017-250 mL).

3. Experiment instrument:
Spectrophotometer (Thermo, ND2000C); Mini Gel Tank (Invitrogen, PS0301); and Imaging System (Bio-Rad, Chemi-Doc MP).

4. Experiment method:

(1) Taking 10 μL of the 10 μM RNAh-Bio-670-EPB nanoparticles and placing in 90 μL of a RPMI 1640 medium containing 10% serum and incubating.

(2) After being incubated at 37 DEG C for 10 min, 1 h, 12 h, and 36 h, respectively taking samples.

(3) After using NanoDrop for quantification, taking 200 ng of the RNA nanoparticles, adding the same volume of Tris-Glycine SDS sample buffer solution (2X), and adequately mixing uniformly.

(4) Taking a piece of Novex™ 8% Tris-Glycine Mini gel, loading the samples in order, setting a program at 200 V, 30 min, and starting electrophoresis.

(5) After the electrophoresis is finished, performing G250 staining, placing on a horizontal shaker for 30 min, taking pictures and imaging.

II. Experiment result

Table 111: quantitative result and loading volume

| Sample | RNAh-Bio-670-EPB (ng/μL) | 200ng RNAh-Bio-670-EPB (μL) | Buffer solution (μL) |
|---|---|---|---|
| 0 | 89.7 | 2.2 | 2.2 |
| 10 min | 91.6 | 2.2 | 2.2 |
| 1 h | 89.0 | 2.2 | 2.2 |
| 12 h | 88.6 | 2.3 | 2.3 |
| 36 h | 89.4 | 2.2 | 2.2 |

The electrophoresis detection results are shown in Fig. 64 and Fig. 65. Herein, Fig. 64 shows the electrophoresis result of 8% non-denaturing gel (Coomassie Blue program), and Fig. 65 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Bio-670-EPB nanoparticles, it is indicated that it is relatively stable in the 1640 medium with the 10% FBS without significant degradation.

**(II) Stability of DNAh-Bio-EGFRapt-Cy5-EPB nanoparticles in serum**

I. Experiment materials, reagents and devices

1. Experiment material

DNAh-Bio-EGFRapt-Cy5-EPB nanoparticles

2. Experiment reagent

6×DNA loading buffer solution (TSJ010, Geosciences), 100bp DNA molecular marker (TSJ010, Geosciences); 10000*SolarGelRed nucleic acid dye (E1020, solarbio); 8% non-denaturing polyacrylamide gel (self-made); 1× TBE Buffer (without RNA enzyme) (self-made); serum (FBS) (Excel); and RPMI 1640 (GBICO).

PowerPac Basic (Bio-rad), Mini PROTEAN Tetra Cell (Bio-rad), orbital shaker (TS-3D), and Tanon (Tanon 3500).

II. Experiment method

(1) Taking 6μL of the DNAh-Bio-EGFRapt-Cy5-EPB nanoparticles, diluting with 6μL of the RPMI 1640 medium containing 10% serum, herein the concentration may reach 900μg/ml after dilution, diluting by 5 tubes respectively, and placing the diluted sample at 37 DEG C of a water bath for different times (0, 10 min, 1 h, 12 h, and 36 h).

(2) Taking the treated sample and mixing with the 6×DNA Loading Buffer, operating on ice, and making a label.

(3) Taking 8% Native PAGE, applying a piece of the gel to the nanoparticle samples with the different incubation times, herein a loading amount is 20μL/well/sample, setting a program at 90-100 V, and performing electrophoresis for 50 min.

(4) After the electrophoresis, staining, placing in a horizontal shaker for 30 minutes, taking pictures and imaging.

III. Experiment result

The result of the non-denaturing PAGE gel electrophoresis is shown in Fig. 66, herein 1 represents 0 min, 2 represents 10 min, 3 represents 1 h, 4 represents 12 h, and 5 represents 36 h. The target band of the DNAh-Bio-EGFRapt-Cy5-EPB nanoparticles is about 200 bp. It may be seen from Fig. 66 that the epirubicin-DNAh nanoparticles are basically stable after being incubated at 37 DEG C for 36 h.

**(III) Stability detection of methotrexate-containing drug loaded on nucleic acid nanoparticles in serum**

I. Except a sample to be tested: RNAh-Bio-670-MTX nanoparticles, the rest are the same as (I).

II. Experiment result

Table 112: quantitative result and loading volume

| Sample | RNAh-Bio-670-MTX (ng/μL) | 200ng RNAh-Bio-670-MTX (μL) | Buffer solution (μL) |
|---|---|---|---|
| 0 | 95.2 | 2.10 | 2.10 |
| 10 min | 96.0 | 2.08 | 2.08 |
| 1 h | 95.3 | 2.10 | 2.10 |
| 12 h | 96.0 | 2.08 | 2.08 |
| 36 h | 124.8 | 1.60 | 1.60 |

The electrophoresis detection results are shown in Fig. 67 and Fig. 68. Herein, Fig. 67 shows the electrophoresis result of 8% non-denaturing gel (Coomassie Blue program), and Fig. 68 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Bio-670-MTX nanoparticles, it is indicated that it is relatively stable in the 1640 medium with the 10% FBS without significant degradation.

**(IV) Stability detection of targeted drug RNAh-Bio-670-THP nanoparticles in serum**

I. Except a sample to be tested: RNAh-Bio-670-THP nanoparticles, the rest are the same as (I).

II. Experiment result

Table 113: quantitative result and loading volume

| Sample | RNAh-Bio-670-THP (ng/μL) | 200ng RNAh-Bio-670-THP (μL) | Buffer solution (μL) |
|---|---|---|---|
| 0 | 95.2 | 2.10 | 2.10 |
| 10 min | 96.0 | 2.08 | 2.08 |
| 1 h | 95.3 | 2.10 | 2.10 |
| 12 h | 96.0 | 2.08 | 2.08 |
| 36 h | 124.8 | 1.60 | 1.60 |

The electrophoresis detection results are shown in Fig. 69 and Fig. 70 Herein, Fig. 69 shows the electrophoresis result of 8% non-denaturing gel (Coomassie Blue program), and Fig. 70 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Bio-670-THP nanoparticles, it is indicated that it is relatively stable in the 1640 medium with the 10% FBS without significant degradation.

**(V) Stability detection of DNAh-Bio-EGFRapt-Cy5-THP nanoparticles in serum**

I. Except a sample to be tested: DNAh-Bio-EGFRapt-Cy5- THP, herein the concentration is 1.8 mg/ml, the rest are the same as (I).

II. Experiment result

The electrophoresis detection result is shown in Fig. 71. Herein, 1 represents 0 min, 2 represents 10 min, 3 represents 1 h, 4 represents 12 h, and 5 represents 36 h. The target band of the DNAh-Bio-EGFRapt-Cy5-THP nanoparticles is about 200 bp. It may be seen from Fig. 71 that the DNAh-Bio-EGFRapt-Cy5-THP nanoparticles are basically stable after being incubated at 37 DEG C for 36 h.

**(VI) Stability detection of daunorubicin-containing drug loaded on nucleic acid nanoparticles in serum**

I. Except a sample to be tested: RNAh-Bio-670-DNR nanoparticles, the rest are the same as (I).

II. Experiment result

Table 114: quantitative result and loading volume

| Sample | RNAh-Bio-670-DNR (ng/μL) | 200ng RNAh-Bio-670-DNR (μL) | Buffer solution (μL) |
|---|---|---|---|
| 0 | 103.6 | 1.93 | 1.93 |
| 10 min | 105.6 | 1.89 | 1.89 |
| 1 h | 103.5 | 1.93 | 1.93 |
| 12 h | 104.5 | 1.91 | 1.91 |
| 36 h | 135.2 | 1.48 | 1.48 |

The electrophoresis detection results are shown in Fig. 72 and Fig. 73. Herein, Fig. 72 shows the electrophoresis result of 8% non-denaturing gel (Coomassie Blue program), and Fig. 73 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Bio-670-DNR nanoparticles, it is indicated that it is relatively stable in the 1640 medium with the 10% FBS without significant degradation.

**(VII) Stability detection of pentafluorouracil-containing drug loaded on nucleic acid nanoparticles in serum**

I. Except a sample to be tested: RNAh-Biotin-quasar670-flu nanoparticles, the rest are the same as (I).

II. Experiment result

Table 115: quantitative result and loading volume

| Sample | RNAh-Biotin-quasar670-flu (ng/μL) | 200ng RNAh-Biotin-quasar670-flu (μL) | Buffer solution (μL) |
|---|---|---|---|
| 0 | 104.4 | 1.9 | 1.9 |
| 10 min | 109.7 | 1.8 | 1.8 |
| 1 h | 93.4 | 2.1 | 2.1 |
| 12 h | 101.1 | 2.0 | 2.0 |
| 36 h | 100.2 | 2.0 | 2.0 |

The electrophoresis detection results are shown in Fig. 74 and Fig. 75. Herein, Fig. 74 shows the electrophoresis result of 8% non-denaturing gel (Coomassie Blue program), and Fig. 75 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Biotin-quasar670-flu nanoparticles, it is indicated that the RNAh-Biotin-quasar670-flu nanoparticles are relatively stable in the 1640 medium with the 10% FBS without significant degradation.

**(VIII) Stability detection of 10-hydroxycamptothecin-containing drug loaded on nucleic acid nanoparticles in serum**

I. Experiment material and experiment method

1. Sample to be tested: RNAh-Biotin-quasar670-hdcp nanoparticles prepared in Embodiment 5.

2. Experiment reagent: same as (I)

3. Experiment instrument: same as (I)

4. Experiment method: same as (I)

II. Experiment result

Table 116: quantitative result and loading volume

| Sample | RNAh-Bioti n-quasar670-hdcp (ng/μL) | 200ng RNAh-Biotin-quasar670-hdcp (μL) | Buffer solution (μL) |
|---|---|---|---|
| 0 | 99.4 | 2.0 | 2.0 |
| 10 min | 96.2 | 2.1 | 2.1 |
| 1 h | 100.1 | 2.0 | 2.0 |
| 12 h | 106.6 | 1.9 | 1.9 |
| 36 h | 109.3 | 1.8 | 1.8 |

The electrophoresis detection results are shown in Fig. 76 and Fig. 77. Herein, Fig. 76 shows the electrophoresis

result of 8% non-denaturing gel (Coomassie Blue program), and Fig. 77 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Biotin-quasar670-hdcp nanoparticles, it is indicated that the RNAh-Biotin-quasar670-hdcp nanoparticles are relatively stable in the 1640 medium with the 10% FBS without significant degradation.

**(IX) Stability detection of aspirin-containing drug loaded on nucleic acid nanoparticles in serum**

I. Except a sample to be tested: RNAh-Biotin-quasar670-aspirin nanoparticles, the rest are the same as (I).

II. Experiment result

Table 117: quantitative result and loading volume

| Sample | RNAh-Biotin-quasar670-aspirin (ng/μL) | 200ng RNAh-Biotin-quasar670-aspirin (μL) | Buffer solution (μL) |
|---|---|---|---|
| 0 | 113.6 | 1.8 | 1.8 |
| 10 min | 114.2 | 1.8 | 1.8 |
| 1 h | 114.2 | 1.8 | 1.8 |
| 12 h | 114.0 | 1.8 | 1.8 |
| 36 h | 117.4 | 1.7 | 1.7 |

The electrophoresis detection results are shown in Fig. 78 and Fig. 79. Herein, Fig. 78 shows the electrophoresis result of 8% non-denaturing gel (Coomassie Blue program), and Fig. 79 shows the electrophoresis result of 8% non-denaturing gel (Stain Free Gel program). The results of the serum stability test show that: 0 min, 10 min, 1 h, 12 h and 36 h, under different time lengths, there is no significant difference between the bands of RNAh-Biotin-quasar670-aspirin nanoparticles, it is indicated that the RNAh-Biotin-quasar670-aspirin nanoparticles are relatively stable in the 1640 medium with the 10% FBS without significant degradation.

**(X) Stability detection of gemcitabine-containing drug loaded on nucleic acid nanoparticles in serum**

I. Except a sample to be tested: RNAh-Bio-670-gemcitabine nanoparticles, the rest are the same as (I).

II. Experiment result

Table 118: quantitative result and loading volume

| Sample | RNAh-Bio-670-gemcitabine (ng/μL) | 200ng RNAh-Bio-670-gemcitabine (μL) | Buffer solution (μL) |
|---|---|---|---|
| 0 | 109.2 | 1.8 | 1.8 |
| 10 min | 117.0 | 1.7 | 1.7 |
| 1 h | 108.4 | 1.8 | 1.8 |
| 12 h | 122.3 | 1.6 | 1.6 |
| 36 h | 132.6 | 1.5 | 1.5 |

The electrophoresis detection results are shown in Fig. 80 and Fig. 81. Herein, Fig. 80 shows the electrophoresis result of 8% non-denaturing gel (Coomassie Blue program), and Fig. 81 shows the electrophoresis result of 8% non-denaturing gel (Stain Free Gel program). The results of the serum stability test show that: 0 min, 10 min, 1 h, 12 h and

36 h, under different time lengths, there is no significant difference between the bands of RNAh-Bio-670-gemcitabine nanoparticles, it is indicated that it is relatively stable in the 1640 medium with the 10% FBS without significant degradation.

**Embodiment 21: Toxicity research of drug-loaded nanoparticles to cells**

**(I) Cytotoxicity research of RNAh-Bio-670-EPB nanoparticles in HL60 cells**

I. Experiment material and experiment method

1. Experiment material:

Samples to be tested: small molecular drug EPB and RNAh-Bio-670-EPB nanoparticles.

Drug concentration preparation:

Preparing a freshly prepared reagent into a corresponding volume container, and adding PBS to be quantified to 10 μM.

Preparing serial dilution solvents with a culture medium, from 10 μM to 3.33 μM, 1.11 μM, 0.370 μM, 0.124 μM, 0.041 μM, 0.014 μM, 0.0046 μM, 0.0015 μM successively.

Transferring 50 μl of the solution to each well to obtain the final concentrations of 5 μM, 1.667 μM, 0.556 μM, 0.185 μM, 0.062 μM, 0.021 μM, 0.0069 μM, and 0.0023 μM, respectively.

2. Experiment reagent:

Promega; RPMI-1640 medium (Gibco, C11875500BT-500 mL); Fetal bovine serum (FBS) (ExCell Bio, FNA500-500 mL); Penicillin/Streptomycin (PS) (Gibco, 15140-122-100 mL); PBS buffer solution (Gibco, C20012500BT-500 mL); Trypsin-EDTA (Stemcell, 07901-500 mL); DMSO (Sigma, D5879-1 L); and CellTiter-Glo Luminescent Cell Viability Assay kit (CTG) (Promega, G7572-100 mL).

3. Experiment instrument:

Inverted Microscope (Olympus IX71, No.112A-1); 96-well Plate Reader (Molecular Devices, Flexstation 3); and Perkin Elmer Envision 2104 Multilabel Reader (No. 01-094- 0002).

4. Experiment method:

1) Cell culturing and plating

Cells are cultured at 37 DEG C and 5%$CO_2$ in a corresponding basal medium in which 10%FBS and 1%PS are respectively added. The cell density used in the experiment is above 80%. The cells are collected, and centrifuged at 1000 rpm for 4 minutes, the medium is resuspended, the cell concentration is adjusted, and it is added to the 96-well plate in a volume of 3000 cells per 50μL, and each group has 3 replicate wells.

2) Gradient drug concentration preparation and administration

After 24 hours, the compound solution is transferred to each well by 50 μL/well. Finally, the solution of which the final concentrations are: 5 μM, 1.667 μM, 0.556 μM, 0.185 μM, 0.062 μM, 0.021 μM, 0.0069 μM, and 0.0023 μM respectively is obtained;
Solvent control = DMSO
Medium (untreated) control: only cells without compound treatment
Blank control: without cells, used for instrument zero calibration

3) Cell culture after administration

The above cells after administration are cultured for 72 hours under a condition of 37 DEG C and 5% $CO_2$

4) Cells treated by detection kit

The well plate is brought to a room temperature in advance and stands for 30 minutes. 100μL of a CellTiter-Glo® reagent is added to each well of the well plate and mixed for 2 minutes on a shaker to promote cell lysis. The Perkin Elmer Envision 2104 Multilabel Reader is used to read values and the values are recorded.

5) Experiment data acquisition and processing

The acquired experiment data is analyzed and processed by using excel software, and GraphPad Prism 5 software is used for curve fitting analysis.

II. Experiment result:

Table 119: $IC_{50}$ value

| Cell line | Treatment time | Epirubicin (EPB) $IC_{50}$(μM) | RNAh-Bio-670-EPB $IC_{50}$(μM) |
|---|---|---|---|
| HL60 | 72h | 0.3015 | 0.06977 |

The experiment results are shown in Table 119 and Fig. 82, it may be seen from Table 119 and Fig. 82 that the epirubicin (EPB) and RNAh-Bio-670-EPB nanoparticles have a significant inhibitory effect on the proliferation of the HL60 cells, and it is unpredictable that when the concentration is 5 μM, the inhibition rates of the two drugs on the cells are 99.25% and 99.93%, respectively, and when the inhibition rate of cell proliferation is 50%, the $IC_{50}$ is 0.06977 μM and 0.3015 μM, respectively. It may be seen that the RNAh-Bio-670-EPB nanoparticles have the stronger inhibitory activity on the cell proliferation, and the drug concentration of the $IC_{50}$ thereof is almost 1/5 of the concentration of the small molecular drug EPB, so it may significantly reduce the dosage of the drug and reduce toxic side effects.

Furthermore, in order to determine that the targeted fluorescent carrier itself is not significantly toxic to the HL60 cells, the present application further designs a toxicity experiment of the RNAh-Bio-FAM targeted fluorescent carrier to HL60 cells, the toxicity of a small molecular chemical drug Cisplatin to the HL60 cells is used as a control, and a specific result is shown in Fig. 83 (herein, the highest administration concentration is 10 μM, at this time, the inhibition rate of the RNAh-Bio-FAM targeting fluorescent carrier to the HL60 cells is 8.75%, when the inhibition rate of the control cisplatin to the cells is 99.96%). It may be seen from Fig. 83 that the fluorescent carrier itself has no apparent toxicity to the HL60 cells.

**(II) Respective cytotoxicity of DNAh-Bio-EGFRapt-Cy5-EPB nanoparticles in MCF-7 and NCI-H1975 cells**

I. Experiment material

1. Cell information:

Table 120:

| Name | Source | Medium | Culture condition |
|---|---|---|---|
| NCI-H1975 | ATCC | RPMI 1640, 10%FBS | 37°C, 5%$CO_2$, saturation humidity |
| MCF-7 | ATCC | MEM, 10% FBS | 37°C, 5%$CO_2$, saturation humidity |

2. Samples to be tested

Table 121:

| Name | Carrier mass (mg) | Carrier molecular weight | Drug loaded mass (mg) | Drug molecular weight | Character | Preservation condition |
|---|---|---|---|---|---|---|
| DNAh-Bio-EGFRapt-Cy5-EPB | 0.5 | 39493 | 0.190 | 543.52 | Red Solid | -20°C |
| EPB | - | - | 2.900 | 543.52 | Red Solid | -20°C |
| DNAh-Bio-EGFRapt-Cy5 | 1.25*3 | 39485.9 | - | - | Blue Solid | -20°C |

3. Consumables and devices

Table 123:

| Name | Brand | Article number / Model |
|---|---|---|
| 96-well plate | Corning | 3599 |
| Centrifuge | Gineek | LD5-2B |
| $CO_2$ incubator | Thermo | 3111 |
| Microwell plate shaker | QILINBEIER | QB-9001 |
| Microscope | Olympus | IX53 |
| Multifunctional microplate reader | Bio Tek | Synergy H1 |

4. Reagent

Table 124:

| Name | Brand | Article number |
|---|---|---|
| RPMI 1640 medium (without folic acid and biotin) | Provided by Baiyao Zhidao Nano-biotechnolo gy Co., Ltd | YS3160-500 |
| MEM medium (without folic acid and biotin) | Provided by Baiyao Zhidao Nano-biotechnolo gy Co., Ltd | YS4150-500 |
| DMEM medium (without folic acid and biotin) | Provided by Baiyao Zhidao Nano-biotechnolo gy Co., Ltd | YS1200-500 |
| FBS | Cegrogen | A0500-3018 |
| GlutaMax | Gibco | 35050-061 |
| PBS | Gibco | C14190500BT |
| DMSO | Solarbio | D8371 |
| trypsin-EDTA digestive juice (0.25%) | Solarbio | T1320-100ml |
| CellTiter 96® AQueous One Solution | Promega | G3581 |

II. Experiment method:

1) Harvesting cells in a logarithmic growth phase, taking a small amount and staining with trypan blue for counting to ensure that the cell viability reaches more than 98%;

2) adjusting the cell density to 2.22 × $10^4$ /mL with a growth medium;
3) planting 90 μL/well of cell suspension into the 96-well plate, herein the number of cells per well in the plate is 2000;
4) placing the planted cell plate in the incubator at 37 DEG C and incubating overnight;
5) performing 3.16-fold gradient dilution on the compound at 9 concentration points;
6) taking out the cell culture plate, and adding 10 μL/well of 10X concentration drug working solution to the corresponding wells of the cell culture plate, herein three replicate holes are made for each concentration, and the final drug action concentration is shown in the table below:

Table 125:

| Test sample | Concentration gradient |
|---|---|
| EPB | 100μM, 31.6μM, 10μM, 3.16μM, 1μM, 316nM, 100nM, 31.6nM, 10nM, 0 (10%PBS) |
| DNAh-Bio-EGFRapt-Cy5-EPB | 100μM, 31,6μM, 10μM, 3.16μM, 1μM, 316nM, 100nM, 31.6nM, 10nM, 0 (10%PBS) |
| DNAh-Bio-EGFRapt-Cy5 | 10μM, 3.16μM, 1μM, 316nM, 100nM, 31.6nM, 10nM, 3.16nM, 1nM, 0 (10%PBS) |
| DMSO | 1%, 0.5%, 0.25%, 0.125%, 0.06%, 0.03%, 0 |

7) placing the cell culture plate in the incubator and continuously incubating for 96 hours;
8) placing the CellTiter 96® AQueous One Solution reagent at the room temperature and thawing for 90 minutes or thawing in a water bath at 37 DEG C, and then equilibrating at the room temperature for 30 minutes;
9) adding 20μL/well of the CellTiter 96® AQueous One Solution reagent to the cell culture plate;
10) placing the cell culture plate in the incubator at 37 DEG C and continuously incubating for 3 hours;
11) using the microplate reader to read a OD490 value of each well in the cell plate; and
12) data processing and analysis.

GraphPad Prism 5.0 software is used to process data graphically. In order to calculate the IC50, "S"-shaped non-linear regression analysis is performed on the data to match a suitable dosage-effect curve. A calculation formula of the cell survival rate is as follows, the IC50 may be automatically calculated in the GraphPad Prism 5.0.

$$\text{Cell survival rate (\%)} = (OD_{\text{test well}} - OD_{\text{blank control}}) / (OD_{\text{negative control}} - OD_{\text{blank control}}) \times 100\%.$$

III. Experiment result (as shown in Table 126, Fig. 84a to Fig. 84d and Fig. 85a to Fig. 85d)

Table 126:

| Cell line | Test sample | $IC_{50}$ (μM) |
|---|---|---|
| MCF-7 | EPB | 0.08473 |
| | DNAh-Bio-EGFRapt-Cy5-EPB | 0.04421 |
| | DNAh-Bio-EGFRapt-Cy5 | >1 |
| | DMSO | >1% |
| NCI-H1975 | EPB | 0.03062 |
| | DNAh-Bio-EGFRapt-Cy5-EPB | 0.01586 |
| | DNAh-Bio-EGFRapt-Cy5 | >0.316 |
| | DMSO | >1% |

It may be seen from Table 126 and Fig. 84a, 84b, 84c and 84d that for the MCF-7 cell line, compared with the simple DNAh targeted fluorescent carrier, the small molecular drug EPB and DNAh drug-loaded particles DNAh-Bio-EGFRapt- Cy5-EPB are both toxic to the MCF-7 cells, and the $IC_{50}$ drug concentration of the DNAh drug-loaded particles DNAh-Bio-EGFRapt-Cy5-EPB is a half of the $IC_{50}$ drug concentration of the small molecular drug EPB. Similarly, it may seen from Table 126 and Fig. 85a, 85b, 85c and 85d that for the NCI-H1975 cell line, compared with the simple DNAh targeted fluorescent carrier, the small molecular drug EPB and DNAh drug-loaded particles DNAh-Bio-EGFRapt-Cy5-EPB are both toxic to the NCI-H1975 cells, and the $IC_{50}$ drug concentration of the DNAh drug-loaded particles DNAh-Bio-EGFRapt-Cy5-EPB is a half of the $IC_{50}$ drug concentration of the small molecular drug EPB.

It may be seen from the above toxicity experiments that the drug-loaded nanoparticles of the present application have a stronger cell proliferation inhibitory effect than the small molecular drugs, and may reduce the dosage of the drug when the same drug effect is achieved, and at the same time reduce toxic side effects.

**(III) Cytotoxicity research of RNAh-Bio-670-MTX nanoparticles in MOLT4 cells**

I. Experiment material:

Samples to be tested: small molecular drug MTX and RNAh-Bio-670-MTX nanoparticles.

2. Experiment method:

1) Using the RPMI1640+10%FBS+1%PS medium to culture the MOLT4 cells at 37 DEG C and 5% $CO_2$.

2) Collecting the cells, centrifuging at 800 rpm for 5 minutes, resuspending the medium, adjusting the cell concentration, and adding to the 96-well plate in a volume of 5000 cells per 90 μL.

3) Diluting a sample to be tested with the culture medium on the next day, respectively adding 200 nM to each sample, herein each sample has 4 replicate wells for replication.

4) After being cultured for 72 hours, adding 100 μL of the CTG reagent to each well, shaking for 2 minutes, and standing at the room temperature for 10 minutes, herein a whole process is protected from light.

5) Finally using Soft Max Pro5 software to read.

II. Experiment result:

Table 127: cell survival rate (%)

| Cell line | Treatment time | Methotrexate (MTX) | RNAh-Bio-670-MTX |
|---|---|---|---|
| MOLT-4 | 72h | 2.88 | 4.72 |

The experiment results are shown in Table 127 and Fig. 86. It may be seen from Table 127 and Fig. 86 that in the MOLT4 cells in vitro, both methotrexate (MTX) and RNAh-Bio-670-MTX nanoparticles have a significant inhibitory effect on the proliferation of the MOLT4 cells, and there is no significant difference between the effects.

It is further proved through the toxicity experiments of the fluorescent targeted carrier Bio-Cy5-RNAh and the small molecular chemical drug Cisplatin to the MOLT4 cells that the fluorescent targeted carrier Bio-Cy5-RNAh has no apparent inhibitory effect on the proliferation of the MOLT4 cells (the details are shown in Fig. 87) (Only when the maximum administration concentration is 5 μm, the inhibition rate of the Bio-Cy5-RNAh to the MOLT4 cell proliferation is 47.38%, at this time, the inhibition rate of the control cisplatin to the cell proliferation is 99.94%).

**(IV) Cytotoxicity of RNAh-Bio-670-THP nanoparticles in MCF-7 cells**

I. Experiment material and experiment method

1. Samples to be tested: small molecular drug THP and RNAh-Bio-670-THP nanoparticles;

2. Experiment method:

1) Except that it is the MCF-7 cells, the rest are the same as (III).

II. Experiment result:

Table 128: cell survival rate (%)

| Cell line | Treatment time | Pirarubicin (THP) | RNAh-Bio-670-THP |
|---|---|---|---|
| MCF-7 | 72h | 13.85 | 12.93 |

The experiment results are shown in Table 128 and Fig. 88, it may be seen from Table 128 and Fig. 88 that the RNAh-Bio-670-THP nanoparticles have a significant inhibitory effect on the proliferation of the MCF-7 cells, and the inhibitory effect is slightly stronger than that of the small molecular drug pirarubicin. (THP).

Further, in order to determine that the carrier itself is not significantly toxic to the MCF-7 cells, the present application further designs a toxicity experiment of the RNAh-Bio-FAM targeted fluorescent carrier to the MCF-7 cells, the 10% PBS is used as a negative control and the medium is used as a blank control, a specific result is shown in Fig. 89. It may be seen from Fig. 89 that the targeted fluorescent carrier itself has no apparent toxicity to the MCF-7 cells.

**(V) Cytotoxicity of DNAh-Biotin-EGFRapt-Cy5-THP nanoparticles in SGC-7901 and SKOV3 cells**

I. Experiment material and method

1. Cell information

Table 129:

| Name | Source | Serial number | Medium | Culture condition |
|---|---|---|---|---|
| SGC-7901 | Bolise Co | SGC-7901 | DMEM, 10%FBS | 37°C, 5%$CO_2$, Saturation humidity |
| SKOV3 | ATCC | HTB-77 | MEM, 10% FBS | 37°C, 5%$CO_2$, Saturation humidity |

2. Samples to be tested

Table 130:

| Name | Carrier mass (mg) | Carrier molecular weight | Drug loaded mass (mg) | Drug molecular weight | Character | Preservation condition |
|---|---|---|---|---|---|---|
| DNAh-Biotin-EGFRapt -Cy5-THP | 0.5 | 39493 | 0.174 | 627.64 | Red solid | -20°C |
| THP | - | - | 0.800 | 627.64 | Red solid | -20°C |
| DNAh-Biotin-EGFRapt-C y5 | 1.25*3 | 39485.9 | - | - | Blue solid | -20°C |

II. Experiment device and method same as (II)

III. Experiment result

Table 131:

| Cell line | Test sample | $IC_{50}$(μM) |
|---|---|---|
| SKOV3 | THP | 0.02374 |
| | DNAh-Bio-EGFRapt-Cy5-THP | 0.08462 |
| | DNAh-Bio-EGFRapt-Cy5 | >1 |
| | DMSO | >1% |
| SGC-7901 | THP | 0.02739 |
| | DNAh-Bio-EGFRapt-Cy5-THP | 0.1195 |
| | DNAh-Bio-EGFRapt-Cy5 | >0.316 |
| | DMSO | >1% |

It may seen from Table 131 and Fig. 90a, 90b, 90c, and 90d that for the SKOV3 cell line, compared with the simple DNAh targeted fluorescent carrier, the small molecular drug THP and DNAh drug-loaded particles DNAh-Bio-EGFRapt-Cy5-THP are both toxic to the SKOV3 cells. Similarly, it may be seen from Table 131 and Fig. 91a, 91b, 91c, and 91d that for the SGC-7901 cell line, compared with the simple DNAh targeted fluorescent carrier, the small molecular drug THP and DNAh drug-loaded particles DNAh-Bio -EGFRapt-Cy5-THP are both toxic to the SGC-7901 cells.

**(VI) Cytotoxicity research of RNAh-Bio-670-DNR nanoparticles in MCF-7 cells**

I. Samples to be tested: small molecular drug DNR and RNAh-Bio-670-DNR nanoparticles;
II. Experiment result:

Table 132: cell survival rate (%)

| Cell line | Treatment time | Daunorubicin (DNR) | RNAh-Bio-670-DNR |
|---|---|---|---|
| MCF-7 | 72h | 17.33 | 15.36 |

The experiment results are shown in Table 132 and Fig. 92, it may be seen from Table 132 and Fig. 92 that the RNAh-Bio-670-DNR nanoparticles have a significant inhibitory effect on the proliferation of the MCF-7 cells, and the inhibitory effect is slightly stronger than that of the small molecular drug daunorubicin. (DNR).

Further, in order to determine that the carrier itself is not significantly toxic to the MCF-7 cells, the present application further designs a toxicity experiment of the RNAh-Bio-FAM targeted fluorescent carrier to the MCF-7 cells, the 10% PBS is used as a negative control and the medium is used as a blank control, a specific result is shown in Fig. 93. It may be seen from Fig. 93 that the targeted fluorescent carrier itself has no apparent toxicity to the MCF-7 cells.

**(VII) Cytotoxicity research of RNAh-Biotin-quasar670-flu nanoparticles in HepG2 cells**

I. Samples to be tested: small molecular pentafluorouracil chemical drug and RNAh-Biotin-quasar670-flu nanoparticles.
II. Experiment result:

Table 133: Cell inhibition rate (%) when administration concentration is 5 μM

| Cell line | Treatment time | small molecular pentafluorouracil chemical drug | RNAh-Biotin-quasar670 -flu |
|---|---|---|---|
| HepG2 | 72h | 39.02% | 52.98% |

The experiment results are shown in Table 133 and Fig. 94, it may be seen from Table 133 and Fig. 94 that 5μM of the RNA nanoparticles RNAh-Biotin-quasar670-flu carrying the pentafluorouracil has apparent cytotoxicity to the HepG2 cells, and it is unpredictable that: compared with the inhibitory effect of the small molecular pentafluorouracil drug to the cell proliferation, the inhibition of 5 μM of the RNAh-Biotin-quasar670-flu to the HepG2 cells is more significant, and on the basis that the cell inhibition rate after the treatment of the small molecular pentafluorouracil drug is 39.02%,

the inhibition rate thereof to the cells is further improved by at least 25% (improved to 52.98%)

In order to further determine that the RNA nanoparticles without carrying the pentafluorouracil have no apparent cytotoxicity to the HepG2 cells, the inventor further designs a toxicity experiment of the RNAh-Biotin-FAM (FAM is a fluorescent marker), a targeted fluorescent carrier, to the HepG2 cells (a drug administration gradient in the experiment is as follows: 100 μM, 31.6 μM, 10 μM, 3.16 μM, 1 μM, 316 nM, 100 nM, 31.6 nM, 10 nM, and 0 (10%PBS)), the results thereof are shown in Table 134 and Fig. 95. It may be seen from the IC50 value of Table 134 and Fig. 95 that the targeted fluorescent carrier without carrying the pentafluorouracil itself has no apparent toxicity to the experimental cells.

Table 134:

| | RNAh-Biotin-FAM |
|---|---|
| $IC_{50}$ (μM) | >10μM |

**(VIII) Cytotoxicity research of RNAh-Biotin-quasar670-hdcp nanoparticles in SGC7901 cells**

I. Samples to be tested: small molecular 10-hydroxycamptothecin chemical drug and RNAh-Biotin-quasar670-hdcp nanoparticles.

II. Experiment result:

Table 135: Cell inhibition rate (%) when administration concentration is 5 μM

| Cell line | Treatment time | small molecular 10-hydroxycamptothec in chemical drug | RNAh-Biotin-quasar670-hdcp |
|---|---|---|---|
| SGC7901 | 72h | 75.43% | 94.52% |

The experiment results are shown in Table 135 and Fig. 96, it may be seen from Table 135 and Fig. 96 that 5μM of the RNA nanoparticles RNAh-Biotin-quasar670-hdcp carrying the 10-hydroxycamptothecin has apparent cytotoxicity to the SGC7901 cells, and it is unpredictable that: compared with the inhibitory effect of the small molecular 10-hydroxycamptothecin drug to the cell proliferation, the inhibition of 5 μM of the RNAh-Biotin-quasar670-hdcp to the SGC7901 cells is more significant, and on the basis that the cell inhibition rate after the treatment of the small molecular 10-hydroxycamptothecin drug is 75.43%, the inhibition rate thereof to the cells is further improved by at least 25% (improved to 94.52%)

In order to further determine that the RNA nanoparticles without carrying the 10-hydroxycamptothecin have no apparent cytotoxicity to the SGC7901 cells, the inventor further designs a toxicity experiment of the RNAh-Biotin-FAM (FAM is a fluorescent marker), a targeted fluorescent carrier, to the SGC7901 cells (a drug administration gradient in the experiment is as follows: 100 μM, 31.6 μM, 10 μM, 3.16 μM, 1 μM, 316 nM, 100 nM, 31.6 nM, 10 nM, and 0 (10%PBS)), the results thereof are shown in Table 136 and Fig. 97. It may be seen from the $IC_{50}$ value of Table 136 and Fig. 97 that the targeted fluorescent carrier without carrying the 10-hydroxycamptothecin itself has no apparent toxicity to the experimental cells.

Table 136:

| | RNAh-Biotin-FAM |
|---|---|
| IC50 (μM) | >10μM |

**(IX) Cytotoxicity research of RNAh-Biotin-quasar670-aspirin nanoparticles in SH-SY5Y cells**

Experiment purpose: an effect of a compound on proliferation of a target tumor cell line is researched.

Experiment design: the compound is diluted in 8 concentration gradients and sequentially added to the target tumor cell line and incubated for 72 hours, the CTG kit is used to detect the effect of the compound on the cell proliferation.

I. Samples to be tested: RNAh-Biotin-quasar670-aspirin and aspirin

II. Experiment result

The $IC_{50}$ values are shown in Table 137.

Table 137:

| Cell line | Treatment time | RNAh-Biotin-quasar670-aspirin $IC_{50}$ (μM) | Aspirin $IC_{50}$ (μM) |
|---|---|---|---|
| SH-SY5Y | 72h | 0.2744 | 1430 |

The specific experiment results are shown in Fig. 98 and Table 137. It may be seen from Fig. 98 and Table 137 that the RNA carrier aspirin-loaded chemical group (RNAh-Biotin-quasar670-aspirin) has a significant inhibitory effect on the proliferation of the SH-SY5Y cells; the aspirin chemical group has no apparent inhibitory effect on the SH-SY5Y cells; when the administration concentration is 5 μM, the cell inhibition rates are 98.77% and 17.72% respectively; and the $IC_{50}$ is 0.2744 μM and 1430 μM respectively.

Furthermore, in order to determine that the targeted fluorescent carrier itself has no apparent toxicity to the SH-SY5Y cells, the present application further designs a toxicity experiment of the RNAh-Bio-Cy5 targeted fluorescent carrier to the SH-SY5Y cells, the toxicity of the small molecule chemical drug cisplatin (Cisplatin) to the SH-SY5Y cells is used as a control, and a specific result is as shown in Fig. 99 (herein, the highest administration concentration is 10μM, at this time, the inhibition rate of the RNAh-Bio-Cy5 (also written as Bio-Cy5-RNAh) targeted fluorescent carrier to the SH-SY5Y cells is 29.34%, when the inhibition rate of the control cisplatin is 99.81 %). It may be seen from Fig. 99 that the fluorescent carrier itself has no apparent toxicity to the SH-SY5Y cells.

**(X) Cytotoxicity research of RNAh-Bio-670-gemcitabine nanoparticles in BXPC3 cells**

I. Samples to be tested: small molecular drug gemcitabine and RNAh-Bio-670-gemcitabine nanoparticles

II. Experiment result

The $IC_{50}$ values are shown in Table 138.

Table 138:

| Cell line | Treatment time | RNAh-Bio-670-gemcitabine $IC_{50}$ (μM) | Gemcitabine $IC_{50}$ (μM) |
|---|---|---|---|
| BXPC3 | 72h | 0.5916 | 0.03418 |

The experiment results are shown in Table 138 and Fig. 100, it may be seen from Table 138 and Fig. 100 that the RNA carrier gemcitabine-loaded histochemical drug (RNAh-Bio-670-gemcitabine) and the gemcitabine chemical drug group both have a significant inhibitory effect on the proliferation of the BxPC3 cells; when the administration concentration is 5 μM, the cell inhibition rates are 99.68% and 82.96% respectively; and the $IC_{50}$ is 0.5916 μM and 0.0341 μM respectively.

Furthermore, in order to determine that the carrier itself is not significantly toxic to the BXPC3 cells, the present application further designs a toxicity experiment of the RNAh-Bio-FAM targeted fluorescent carrier to the BXPC3 cells, the 10% PBS is used as a negative control, and the medium is used as a blank control, a specific result is shown in Fig. 101. It may be seen from Fig. 101 that the targeted fluorescent carrier itself has no apparent toxicity to the BXPC3 cells.

It may be seen from the above description that the above embodiments of the present application achieve the following technical effects: the present application provides a series of nucleic acid nanoparticle carriers with thermodynamic stability, chemical stability, high loading rate and multivalent combination modules. A unique modular design of this type of the carriers is performed to obtain a core modular structure which not only maintains a naturally compatible affinity, but also has high stability and diverse combination characteristics. This structure may flexibly and efficiently integrate various functional modules, including a targeting module, an imaging and probe module, a treatment module and other composite intelligent modules, so that it may be used for targeted delivery in vivo to achieve precise diagnosis and treatment.

Through loading the small molecular drugs such as the tacrine on the nucleic acid nanoparticle carrier provided by the present application to form the nucleic acid nanocarrier drug, not only the delivery stability of the drug may be improved, but also in the case that the nucleic acid nanoparticles carry the target head, on the one hand, the drug is targed and delivered to the target cells, and the bioavailability of the drug is imporved; and on the other hand, the toxic and side effects to the non-target cells or tissues are reduced due to the targeted delivery, and the local drug concentration is reduced, thus the toxic and side effects caused by the high drug concentration are further reduced.

The above are only the preferred embodiments of the disclosure, and are not used to limit the disclosure, and various modifications and changes may be made to the disclosure by those skilled in the art. Any modifications, equivalent replacements, improvements and the like made within spirit and principle of the disclosure should be included in the scope of protection of the disclosure.

## Claims

1. A nucleic acid nanocarrier drug, wherein the nucleic acid nanocarrier drug comprises a nucleic acid nanoparticle and a drug loaded on the nucleic acid nanoparticle, and the drug comprises one or more of tacrine, epirubicin, methotrexate, pirarubicin, daunorubicin, pentafluorouracil, 10-hydroxycamptothecin, aspirin and gemcitabine;
wherein the nucleic acid nanoparticle comprises a nucleic acid domain, the nucleic acid domain comprises a sequence a, a sequence b and a sequence c, the sequence a comprises a sequence a1 or a sequence obtained by insertion, deletion or substitution of at least one base in the sequence a1, the sequence b comprises a sequence b1 or a sequence obtained by insertion, deletion or substitution of at least one base in the sequence b1, and the sequence c comprises a sequence c1 or a sequence obtained by insertion, deletion or substitution of at least one base in the sequence c1,
wherein the sequence a1 is SEQ ID NO:1: 5'-CCAGCGUUCC-3'or SEQ ID NO:2: 5'-CCAGCGTTCC-3';
the sequence b1 is SEQ ID NO:3: 5'-GGUUCGCCG-3'or SEQ ID NO:4: 5'-GGTTCGCCG-3'; and
the sequence c1 is SEQ ID NO:5: 5'-CGGCCAUAGCGG-3'or SEQ ID NO:6: 5'-CGGCCATAGCGG-3'.

2. The nucleic acid nanocarrier drug as claimed in claim 1, wherein when the sequence a1 is the SEQ ID NO:1, the sequence b1 is the SEQ ID NO:3, and the sequence c1 is the SEQ ID NO:5, at least one sequence of the sequence a, the sequence b and the sequence c comprises a sequence obtained by insertion, deletion or substitution of at least one base within thereof.

3. The nucleic acid nanocarrier drug as claimed in claim 1 or 2, wherein the insertion, deletion or substitution of at least one base is generated:

(1) on 1, 2, 4 or 5-th base starting from a 5 '-end of the sequence shown in the SEQ ID NO:1 or the SEQ ID NO:2; and/or
(2) between 8-th and 10-th bases starting from the 5 '-end of the sequence shown in the SEQ ID NO:1 or the SEQ ID NO:2; and/or
(3) between 1-th and 3-th bases starting from a 5 '-end of the sequence shown in the SEQ ID NO:3 or the SEQ ID NO:4; and/or
(4) between 6-th and 9-th bases starting from the 5 '-end of the sequence shown in the SEQ ID NO:3 or the SEQ ID NO:4; and/or
(5) between 1-th and 4-th bases starting from a 5 '-end of the sequence shown in the SEQ ID NO:5 or the SEQ ID NO:6; and/or
(6) between 9-th and 12-th bases starting from the 5 '-end of the sequence shown in the SEQ ID NO:5 or the SEQ ID NO:6;
preferably, the sequence a, the sequence b and the sequence c are self-assembled into a structure shown in Formula (1):

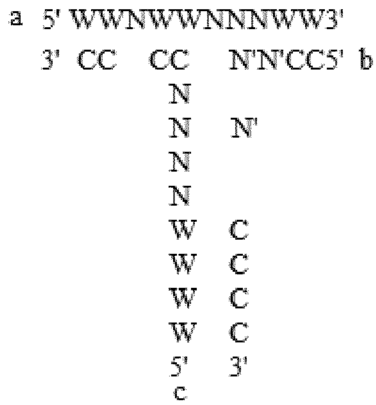


Formula (1),

wherein, W-C represents a Watson-Crick pairing, N and N' represent a non-Watson-Crick pairing, the W-C in any one position is independently selected from C-G or G-C;
in the sequence a, the first N from the 5'-end is A, the second N is G, the third N is U or T, and the fourth N is any one of U, T, A, C or G;
in the sequence b, the first N' from the 5'-end is any one of U, T, A, C or G, the second N' is U or T, and the third N' is C; and
in the sequence c, a sequence NNNN along a direction from the 5'-end to the 3'-end is CAUA or CATA;

more preferably, the sequence a, the sequence b and the sequence c are any one of the following groups:

(1)

sequence a: 5'-GGAGCGUUGG-3',
sequence b: 5'-CCUUCGCCG-3',
sequence c: 5'-CGGCCAUAGCCC-3';

(2)

sequence a: 5'-GCAGCGUUCG-3',
sequence b: 5'-CGUUCGCCG-3',
sequence c: 5'-CGGCCAUAGCGC-3';

(3)

sequence a: 5'-CGAGCGUUGC-3',
sequence b: 5'-GCUUCGCCG-3',
sequence c: 5'-CGGCCAUAGCCG-3';

(4)

sequence a: 5'-GGAGCGUUGG-3',
sequence b: 5'-CCUUCGGGG-3',
sequence c: 5'-CCCCCAUAGCCC-3';

(5)

sequence a: 5'-GCAGCGUUCG-3',
sequence b: 5'-CGUUCGGCG-3',
sequence c: 5'-CGCCCAUAGCGC-3';

(6)

sequence a: 5'-GCAGCGUUCG-3',

sequence b: 5'-CGUUCGGCC-3',
sequence c: 5'-GGCCCAUAGCGC-3';

(7)

sequence a: 5'-CGAGCGUUGC-3',
sequence b: 5'-GCUUCGGCG-3',
sequence c: 5'-CGCCCAUAGCCG-3';

(8)

sequence a: 5'-GGAGCGTTGG-3',
sequence b: 5'-CCTTCGCCG-3',
sequence c: 5'-CGGCCATAGCCC-3';

(9)

sequence a: 5'-GCAGCGTTCG-3',
sequence b: 5'-CGTTCGCCG-3',
sequence c: 5'-CGGCCATAGCGC-3';

(10)

sequence a: 5'-CGAGCGTTGC-3',
sequence b: 5'-GCTTCGCCG-3',
sequence c: 5'-CGGCCATAGCCG-3';

(11)

sequence a: 5'-GGAGCGTTGG-3',
sequence b: 5'-CCTTCGGGG-3',
sequence c: 5'-CCCCCATAGCCC-3';

(12)

sequence a: 5'-GCAGCGTTCG-3',
sequence b: 5'-CGTTCGGCG-3',
sequence c: 5'-CGCCCATAGCGC-3';

(13)

sequence a: 5'-GCAGCGTTCG-3',
sequence b: 5'-CGTTCGGCC-3',
sequence c: 5'-GGCCCATAGCGC-3'; and

(14)

sequence a: 5'-CGAGCGTTGC-3',
sequence b: 5'-GCTTCGGCG-3',
sequence c: 5'-CGCCCATAGCCG-3'.

**4.** The nucleic acid nanocarrier drug as claimed in claim 3, wherein the nucleic acid domain further comprises a first extension fragment, the first extension fragment is an extension fragment of the Watson-Crick pairing, and the first extension fragment is positioned at the 5'-end and/or the 3'-end of any one sequence of the sequence a, the sequence b or the sequence c;
preferably, the first extension fragment is selected from any one of the following groups:

(1): a-strand 5'-end: 5'-CCCA-3', c-strand 3'-end: 5'-UGGG-3';

(2): a-strand 3'-end: 5'-GGG-3', b-strand 5'-end: 5'-CCC-3';
(3): b-strand 3'-end: 5'-CCA-3', c-strand 5'-end: 5'-UGG-3';
(4): a-strand 5'-end: 5'-CCCG-3', c-strand 3'-end: 5'-CGGG-3';
(5): a-strand 5'-end: 5'-CCCC-3', c-strand 3'-end: 5'-GGGG-3';
(6): b-strand 3'-end: 5'-CCC-3', c-strand 5'-end: 5'-GGG-3';
(7): b-strand 3'-end: 5'-CCG-3', c-strand 5'-end: 5'-CGG-3';
(8): a-strand 5'-end: 5'-CCCA-3', c-strand 3'-end: 5'-TGGG-3'; and
(9): b-strand 3'-end: 5'-CCA-3', c-strand 5'-end: 5'-TGG-3'.

**5.** The nucleic acid nanocarrier drug as claimed in any one of claims 1 to 4, wherein the nucleic acid domain further comprises a second extension fragment, the second extension fragment is positioned at the 5'-end and/or the 3'-end of any one sequence of the sequence a, the sequence b, or the sequence c, and the second extension fragment is an extension fragment of a Watson-Crick pairing;
preferably, the second extension fragment is an extension sequence of a CG base pair; and
more preferably, the second extension fragment is an extension sequence of 1-10 CG base pairs.

**6.** The nucleic acid nanocarrier drug as claimed in claim 5, wherein the nucleic acid domain further comprises at least one group of the following second extension fragments:

first group: a-strand 5'-end: 5'-CGCGCG-3', c-strand 3'-end: 5'-CGCGCG-3';
second group: a-strand 3'-end: 5'-CGCCGC-3', b-strand 5'-end: 5'-GCGGCG-3'; and
third group: b-strand 3'-end: 5'-GGCGGC-3', c-strand 5'-end: 5'-GCCGCC-3'.

**7.** The nucleic acid nanocarrier drug as claimed in claim 5, wherein the second extension fragment is an extension sequence containing both CG base pair and AT/AU base pair, and preferably the second extension fragment is an extension sequence of 2-50 base pairs; and more preferably, the second extension fragment is an extension sequence in which sequences of 2-8 continuous CG base pairs and sequences of 2-8 continuous AT/AU base pairs are alternately arranged; or the second extension fragment is an extension sequence in which a sequence of 1 CG base pair and a sequence of 1 AT/AU base pair are alternately arranged.

**8.** The nucleic acid nanocarrier drug as claimed in any one of claims 1 to 7, wherein a base, a ribose and a phosphate in the sequence a, the sequence b and the sequence c have at least one modifiable site, and any one of the modifiable sites is modified by any one of the following modification adapters: -F, a methyl, an amino, a disulfide, a carbonyl, a carboxyl, a sulfhydryl and a formyl; and
preferably, the base C or U in the sequence a, the sequence b and the sequence c has 2'-F modification.

**9.** The nucleic acid nanocarrier drug as claimed in any one of claims 1 to 8, wherein the drug is loaded on the nucleic acid nanoparticle in a physical linkage mode and/or a covalent linkage mode, and a molar ratio between the drug and the nucleic acid nanoparticle is 2-300:1, preferably 10-50:1, and more preferably 15-25:1.

**10.** The nucleic acid nanocarrier drug as claimed in any one of claims 1 to 9, wherein the nucleic acid nanoparticle further comprise a bioactive substance, the bioactive substance is linked with the nucleic acid domain, and the bioactive substance is one or more of a target head, a fluorescein, an interfering nucleic acid siRNA, a miRNA, a ribozyme, a riboswitch, an aptamer, a RNA antibody, a protein, a polypeptide, a flavonoid, a glucose, a natural salicylic acid, a monoclonal antibody, a vitamin, an phenol, a lecithin, and a small molecular drug, the small molecular drug does not comprise the tacrine, the epirubicin, the methotrexate, the pirarubicin, the daunorubicin, the pentafluorouracil, the 10-hydroxycamptothecin, the aspirin and the gemcitabine;
preferably, the bioactive substance is one or more of the target head, the fluorescein and the miRNA, wherein the target head is positioned on any one sequence of the sequences a, b and c, preferably the 5'-end or the 3'-end of any one sequence of the sequences a, b and c, or inserted between GC bonds of the nucleic acid domain, the miRNA is an anti-miRNA, the fluorescein is modified at 5'-end or 3'-end of the anti-miRNA, and the miRNA is positioned in any one or more positions in the 3'-end of the sequence a, and the 5'-end and the 3'-end of the sequence c, and preferably, the target head is a folic acid or a biotin, the fluorescein is any one or more of FAM, CY5 and CY3, and the anti-miRNA is anti-miR-21;
preferably, the small molecular drug is a drug containing any one or more of the following groups: an amino group, a hydroxyl group, a carboxyl group, a mercapto group, a benzene ring group and an acetamido group; and
preferably, the protein is one or more of SOD, survivin, hTERT, EGFR and PSMA; the vitamin is L-Vc and/or esterified Vc; and the phenol is a tea polyphenol and/or a grape polyphenol.

**11.** The nucleic acid nanocarrier drug as claimed in claim 10, wherein a relative molecular weight of the nucleic acid domain is marked as $N_1$, and a total relative molecular weight of the drug and the bioactive substance is marked as $N_2$, $N_{,}/N_2 \geq 1:1$.

**12.** The nucleic acid nanocarrier drug as claimed in claim 1, wherein a particle size of the nucleic acid nanoparticle is 1-100 nm, preferably 5-50 nm; more preferably 10-30 nm; and further preferably 10-15 nm.

**13.** A method for for preparing a nucleic acid nanocarrier drug, wherein the method comprises the following steps:

providing the nucleic acid nanoparticle in the nucleic acid nanocarrier drug as claimed in any one of claims 1 to 12; and
loading the drug on the nucleic acid nanoparticle in a physical linkage mode and/or a covalent linkage mode, to obtain the nucleic acid nanocarrier drug.

**14.** The method as claimed in claim 13, wherein the step of loading the drug in the physical linkage mode comprises:

mixing and stirring the drug, the nucleic acid nanoparticle and a first solvent, to obtain a premixed system; and precipitating the premixed system, to obtain the nucleic acid nanocarrier drug;
preferably, the first solvent is selected from one or more of DCM, DCC, DMAP, Py, DMSO, PBS and glacial acetic acid;
preferably, the step of precipitating the premixed system, to obtain the nucleic acid nanocarrier drug comprises:

precipitating the premixed system, to obtain a precipitation; and
washing the precipitation to remove impurities, as to obtain the nucleic acid nanocarrier drug;
more preferably, mixing the premixed system with absolute ethyl alcohol, and precipitating at a temperature condition lower than 10 DEG C, to obtain the precipitation; and further preferably, precipitating at a temperature condition of 0-5 DEG C; and
more preferably, washing the precipitation to remove the impurities with 6-12 times of the absolute ethyl alcohol in volume, as to obtain the nucleic acid nanocarrier drug.

**15.** The method as claimed in claim 14, wherein the step of loading the drug in the covalent linkage mode comprises:

preparing a drug solution;
enabling the drug solution to react with the G-exocyclic amino of the nucleic acid nanoparticle under a mediating effect of the formaldehyde, to obtain a reaction system; and
purifying the reaction system, to obtain the nucleic acid nanocarrier drug;
preferably, the reaction step comprises:
mixing the drug solution with paraformaldehyde solution and the nucleic acid nanoparticle, and reacting in a dark condition, to obtain the reaction system; wherein the concentration of the paraformaldehyde solution is preferably 3.7-4wt%, and the paraformaldehyde solution is preferably a solution formed by mixing paraformaldehyde and a second solvent, and the second solvent is one or more of DCM, DCC, DMAP, Py, DMSO, PBS and glacial acetic acid.

**16.** The method as claimed in any one of claims 13 to 15, wherein the preparation method further comprises a step of preparing the nucleic acid nanoparticle, the step comprises: self-assembling a single strand corresponding to the nucleic acid domain in the nucleic acid nanocarrier drug as claimed in any one of claims 1 to 12, to obtain the nucleic acid domain;
preferably, after the nucleic acid domain is obtained, the method further comprises: loading the bioactive substance in the drug as claimed in claim 10 or 11 on the nucleic acid domain in the physical linkage mode and/or in the covalent linkage mode, to obtain the nucleic acid nanoparticle.

**17.** The method as claimed in claim 16, wherein in a process of loading the bioactive substance in the covalent linkage mode, the loading is performed through a solvent covalent linkage, a linker covalent linkage or a click-linkage;
preferably, a third solvent used in the solvent covalent linkage is served as a linkage medium, and the third solvent is selected from one or more of paraformaldehyde, DCM, DCC, DMAP, Py, DMSO, PBS and glacial acetic acid;
preferably, the linker is selected from a disulfide bond, a p-phenylazide, bromopropyne or a PEG;
preferably, the click-linkage is that a bioactive substance precursor and the nucleic acid domain are modified by alkynyl or azide modification simultaneously and then linked through a click reaction; and

more preferably, when the bioactive substance is linked with the nucleic acid domain in the click-linkage mode, a site, for performing the alkynyl or azide modification, of the bioactive substance precursor is selected from a 2'-hydroxyl, a carboxyl or an amino, and a site, for performing the alkynyl or azide modification, of the nucleic acid domain is selected from a G-exocyclic amino, a 2'-hydroxyl, an A-amino or a 2'-hydroxyl.

**18.** A pharmaceutical composition, wherein the pharmaceutical composition comprises the nucleic acid nanocarrier drug as claimed in any one of claims 1 to 12.

**19.** Use of the nucleic acid nanocarrier drug as claimed in any one of claims 1 to 12 in preparing a drug for treating an Alzheimer's disease, a tumor, an autoimmune disease or a heart disease.

**20.** The application as claimed in claim 19, wherein the tumor is one or more of the followings: pancreatic cancer, ovarian cancer, breast cancer, bladder cancer, cervical cancer, liver cancer, biliary tract cancer, nasopharyngeal cancer, testicular cancer, lymphoma, mesothelioma, head and neck cancer, gastric cancer, leukemia, colon cancer, rectal cancer, chorionic epithelioma, malignant hydatidiform mole, skin cancer, lung cancer, ureteral cancer, renal pelvis cancer, chorionic epithelioma, bone tumor, leukemia meningeal spinal cord infiltration, Wilms tumor, soft tissue sarcoma and medullary thyroid carcinoma;
the autoimmune disease is refractory psoriasis, systemic lupus erythematosus, mandatory spondylitis or dermatomyositis;
preferably, the leukemia is acute leukemia, more preferably the acute leukemia is acute lymphocytic leukemia or myeloid leukemia;
preferably, the lung cancer comprises bronchial lung cancer or non-small cell lung cancer; and
preferably, the liver cancer comprises primary hepatocellular carcinoma or metastatic liver cancer.

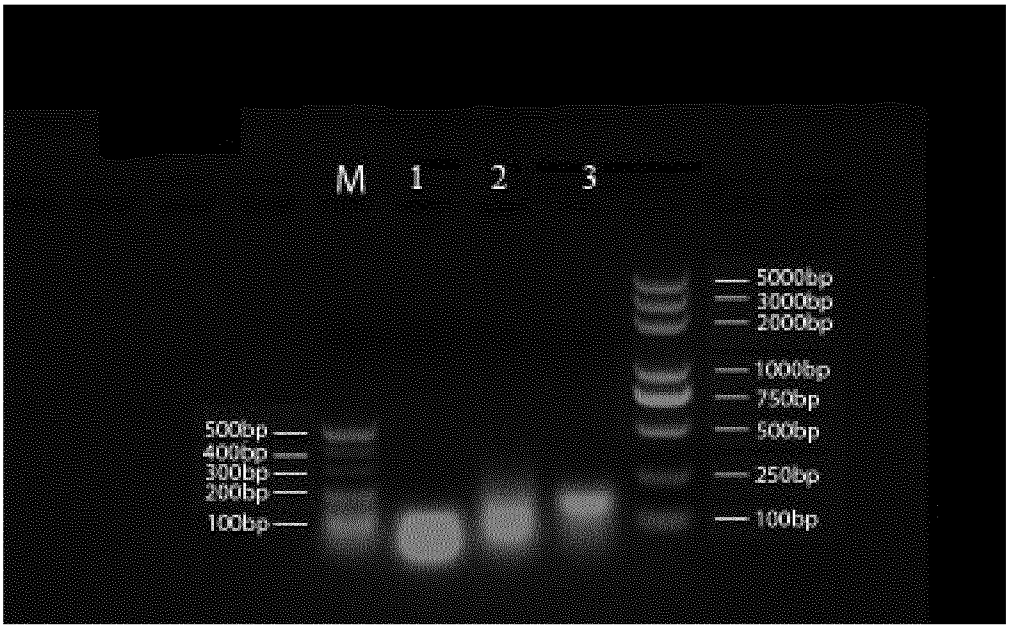
M
1
2
3
5000bp
3000bp
2000bp
1000bp
750bp
500bp
250bp
100bp
500bp
400bp
300bp
200bp
100bp

Fig. 1

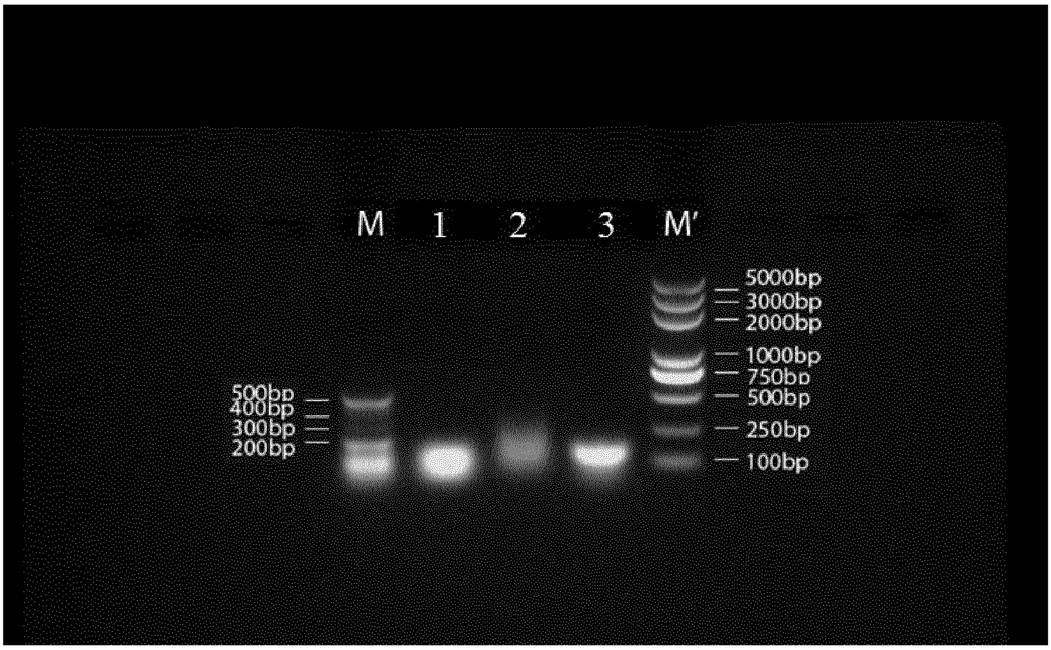
M
1
2
3
M'
5000bp
3000bp
2000bp
1000bp
750bp
500bp
250bp
100bp
500bp
400bp
300bp
200bp

Fig. 2

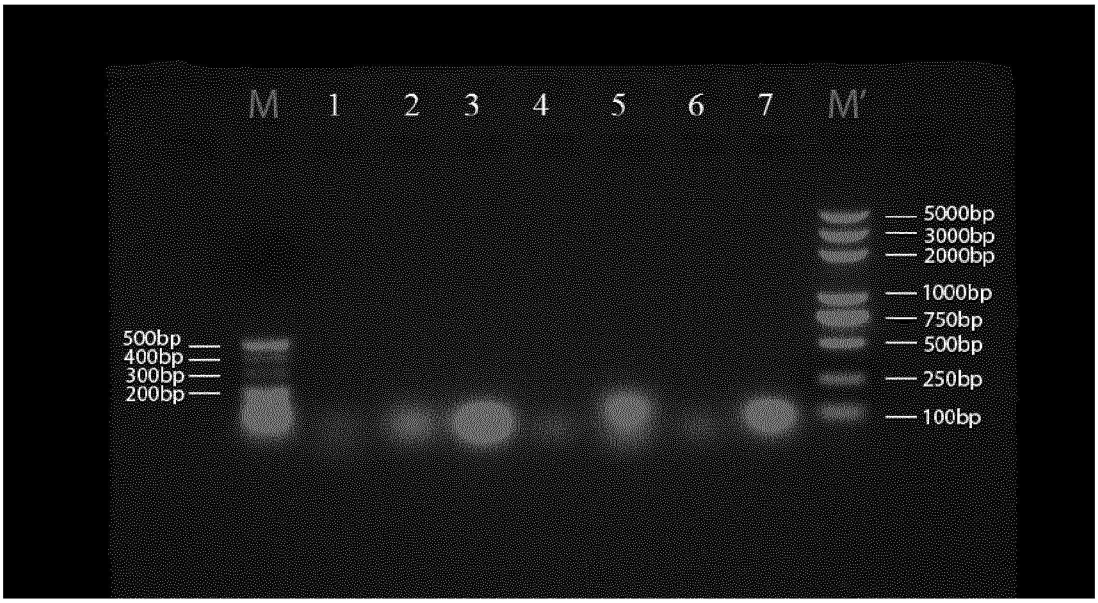
M
1
2
3
4
5
6
7
M′
5000bp
3000bp
2000bp
1000bp
750bp
500bp
250bp
100bp
500bp
400bp
300bp
200bp

**Fig. 3**

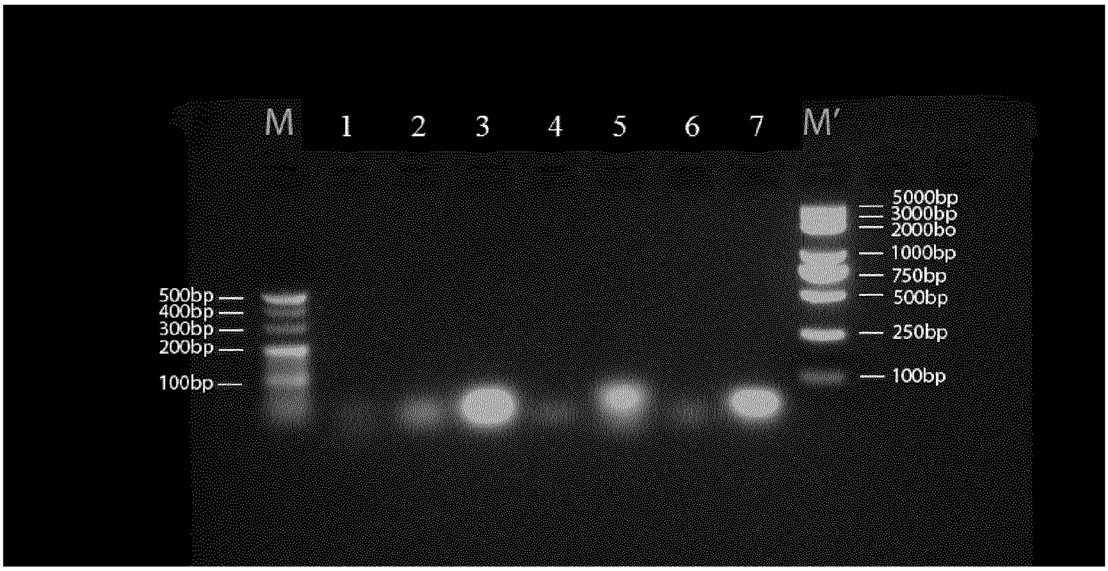
M
1
2
3
4
5
6
7
M′
5000bp
3000bp
2000bo
1000bp
750bp
500bp
250bp
100bp
500bp
400bp
300bp
200bp
100bp

**Fig. 4**

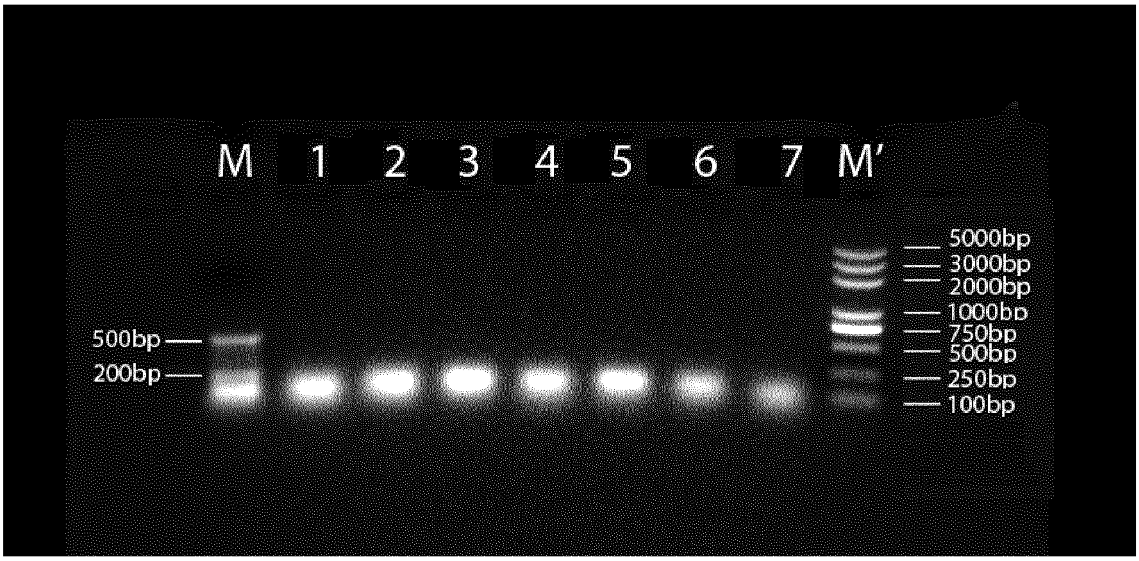
M
1
2
3
4
5
6
7
M'
500bp
200bp
5000bp
3000bp
2000bp
1000bp
750bp
500bp
250bp
100bp

Fig. 5

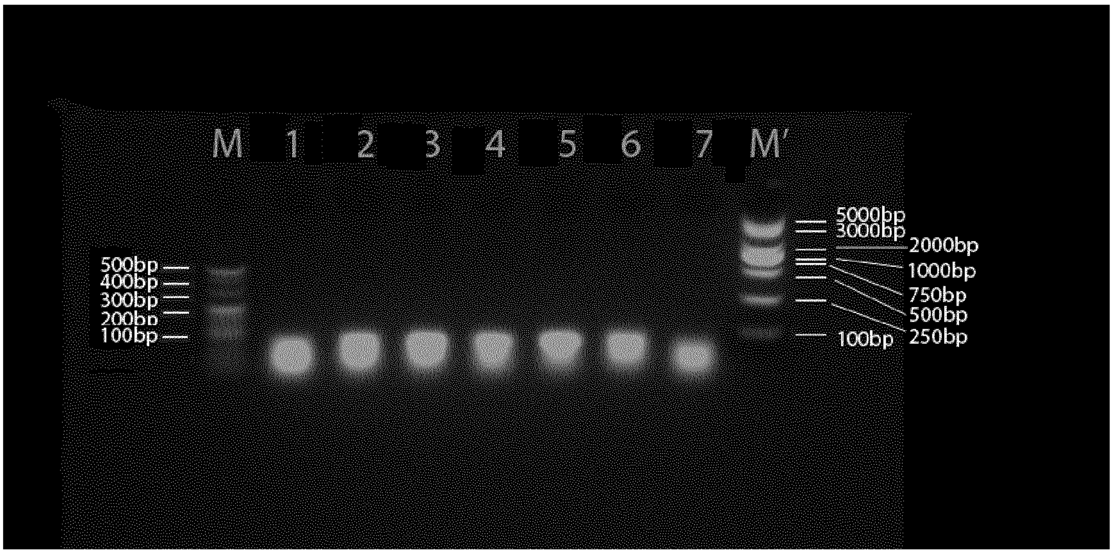
M
1
2
3
4
5
6
7
M'
500bp
400bp
300bp
200bp
100bp
5000bp
3000bp
2000bp
1000bp
750bp
500bp
100bp
250bp

Fig. 6

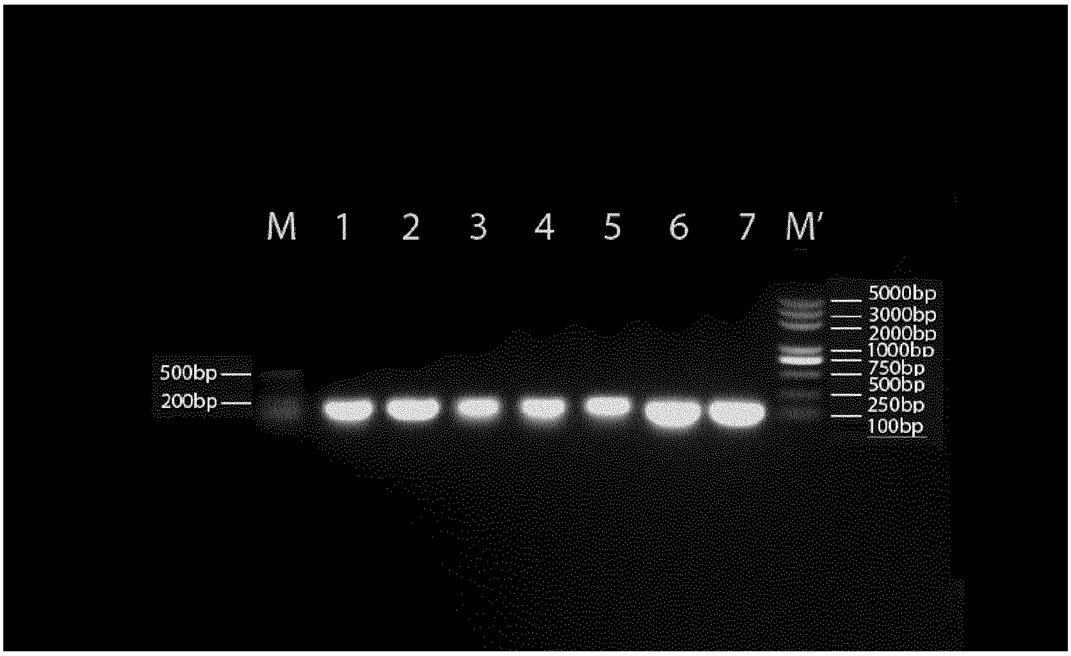
M 1 2 3 4 5 6 7 M'
500bp
200bp
5000bp
3000bp
2000bp
1000bp
750bp
500bp
250bp
100bp

Fig. 7

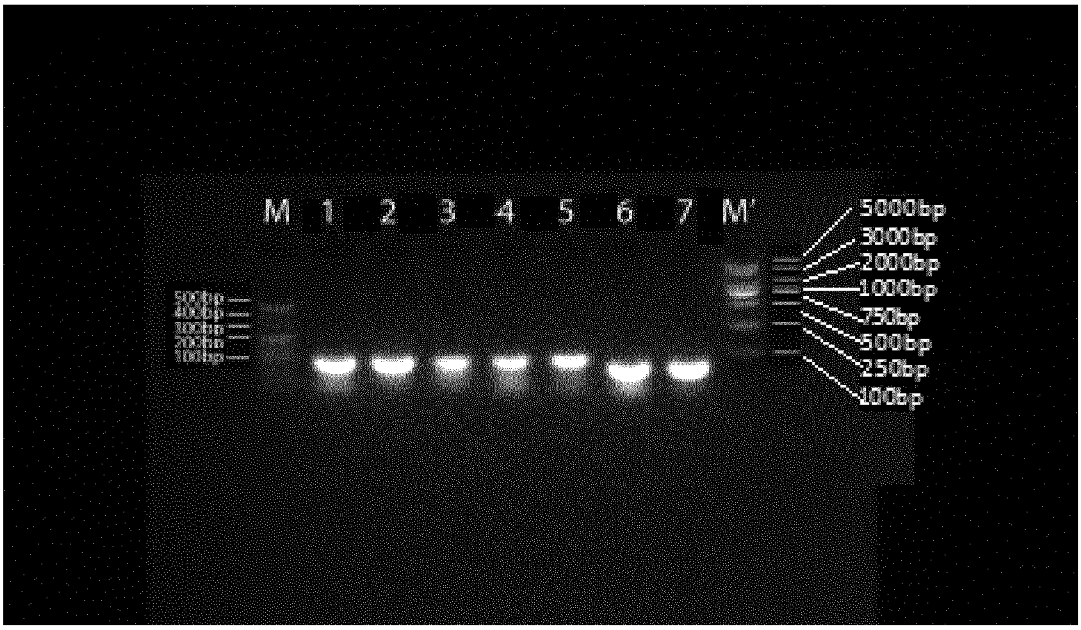
M 1 2 3 4 5 6 7 M'
5000bp
3000bp
2000bp
1000bp
750bp
500bp
250bp
100bp

Fig. 8

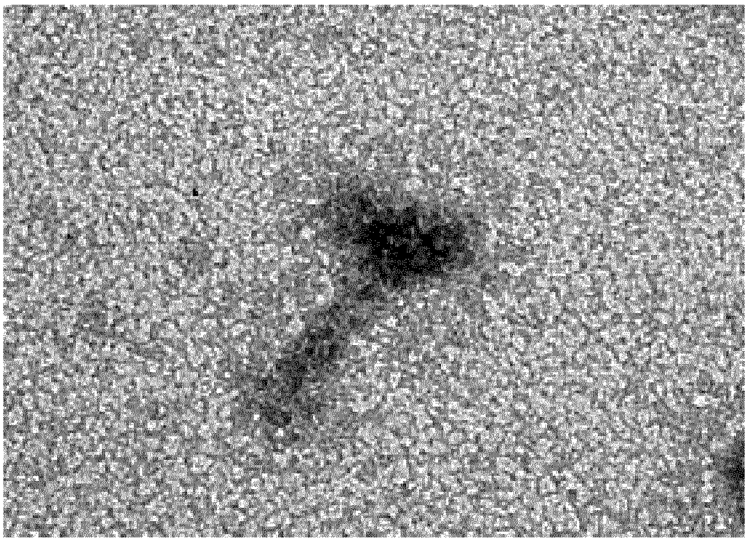

Fig. 9

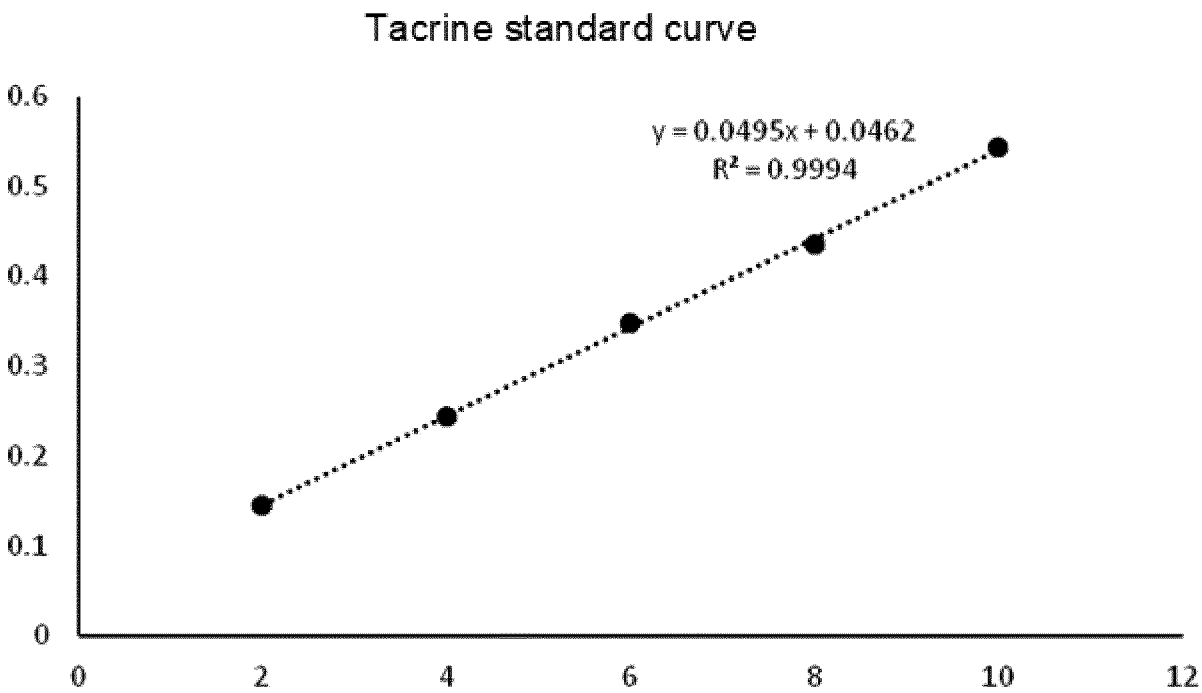
Tacrine standard curve
y = 0.0495x + 0.0462
R² = 0.9994
0.6
0.5
0.4
0.3
0.2
0.1
0
0
2
4
6
8
10
12

Fig. 10

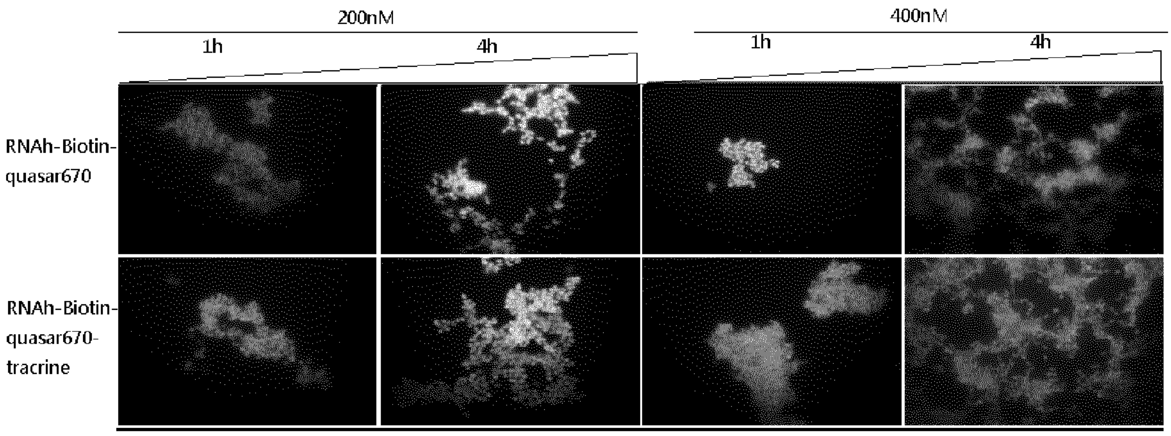
200nM
1h
4h
400nM
1h
4h
RNAh-Biotin-quasar670
RNAh-Biotin-quasar670-tracrine

Fig.11

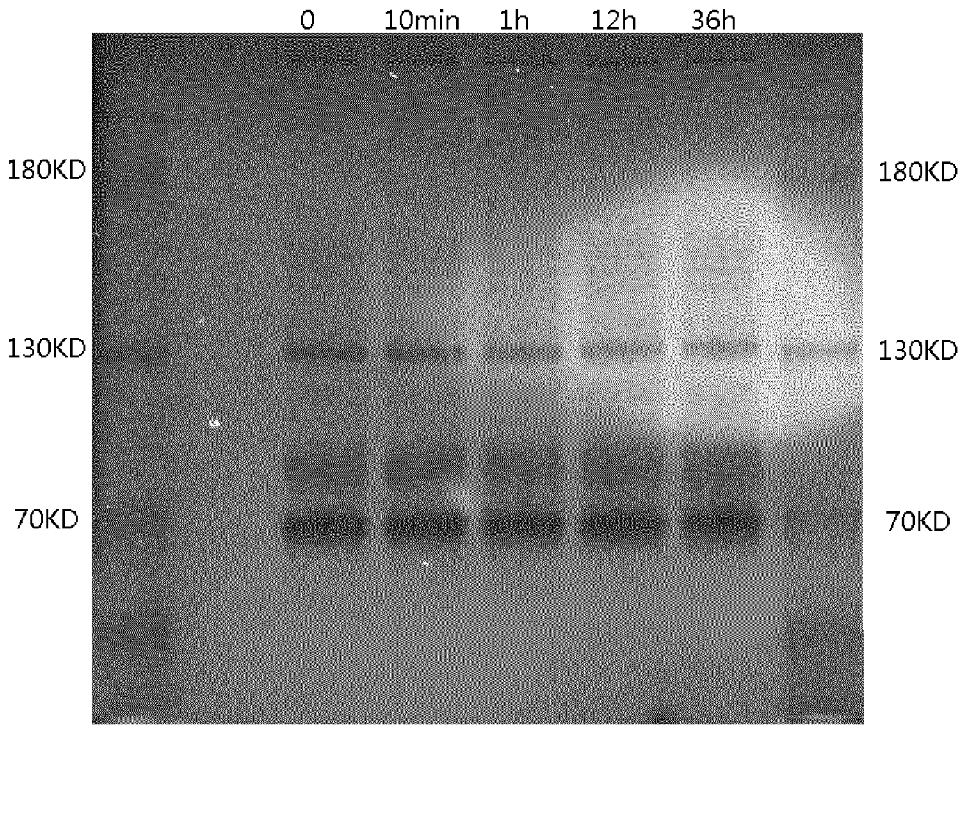
0
10min
1h
12h
36h
180KD
130KD
70KD
180KD
130KD
70KD

Fig.12

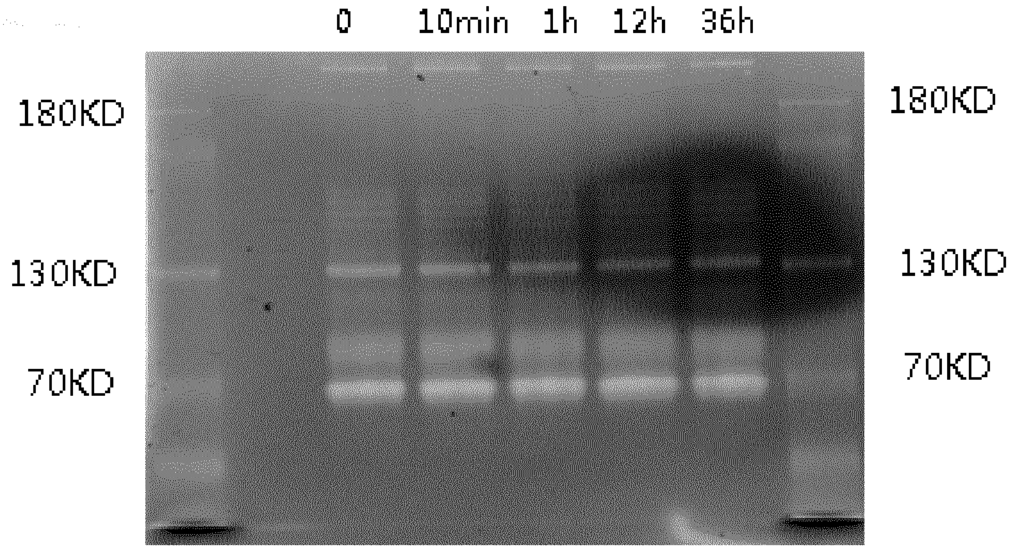
0
10min
1h
12h
36h
180KD
130KD
70KD
180KD
130KD
70KD

Fig.13

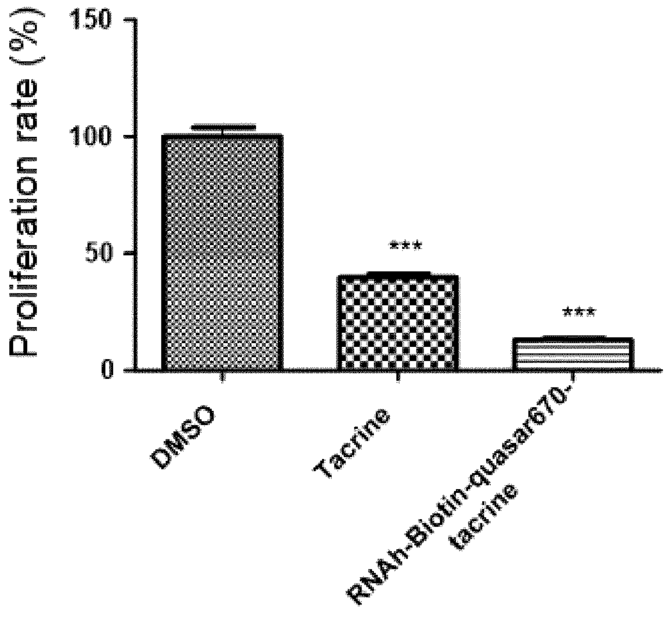
Proliferation rate (%)
150
100
50
0
***
***
DMSO
Tacrine
RNAh-Biotin-quasar670-tacrine

Fig.14

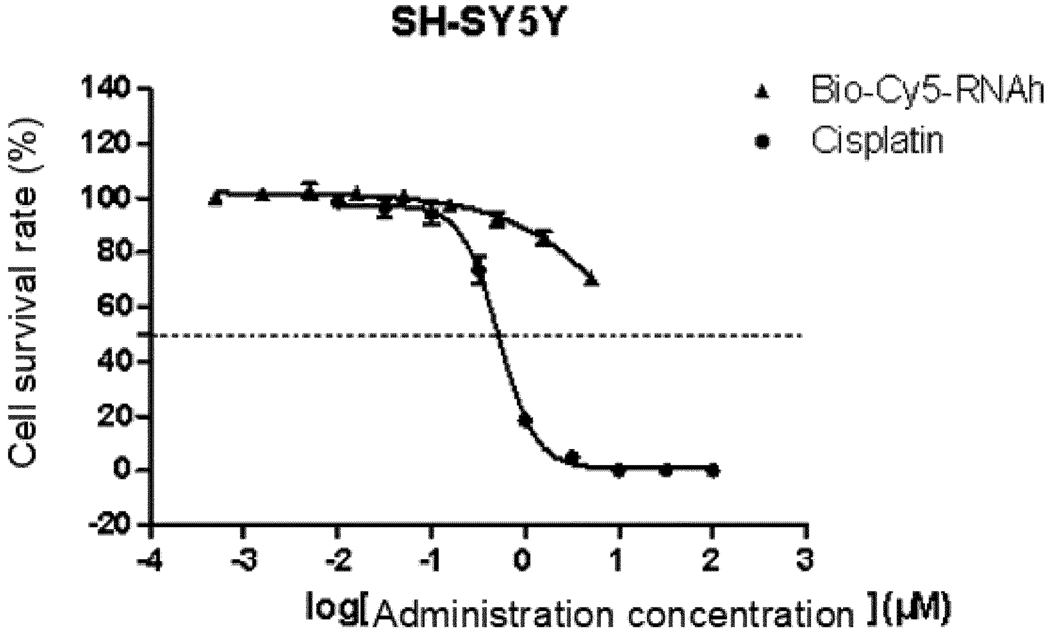
SH-SY5Y
Bio-Cy5-RNAh
Cisplatin
Cell survival rate (%)
140
120
100
80
60
40
20
0
-20
-4
-3
-2
-1
0
1
2
3
log[Administration concentration ](μM)

Fig.15

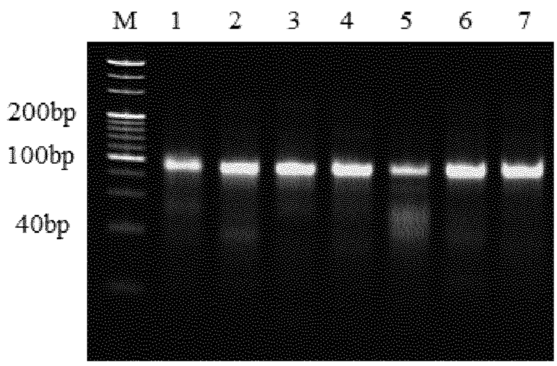
M 1 2 3 4 5 6 7
200bp
100bp
40bp

Fig.16

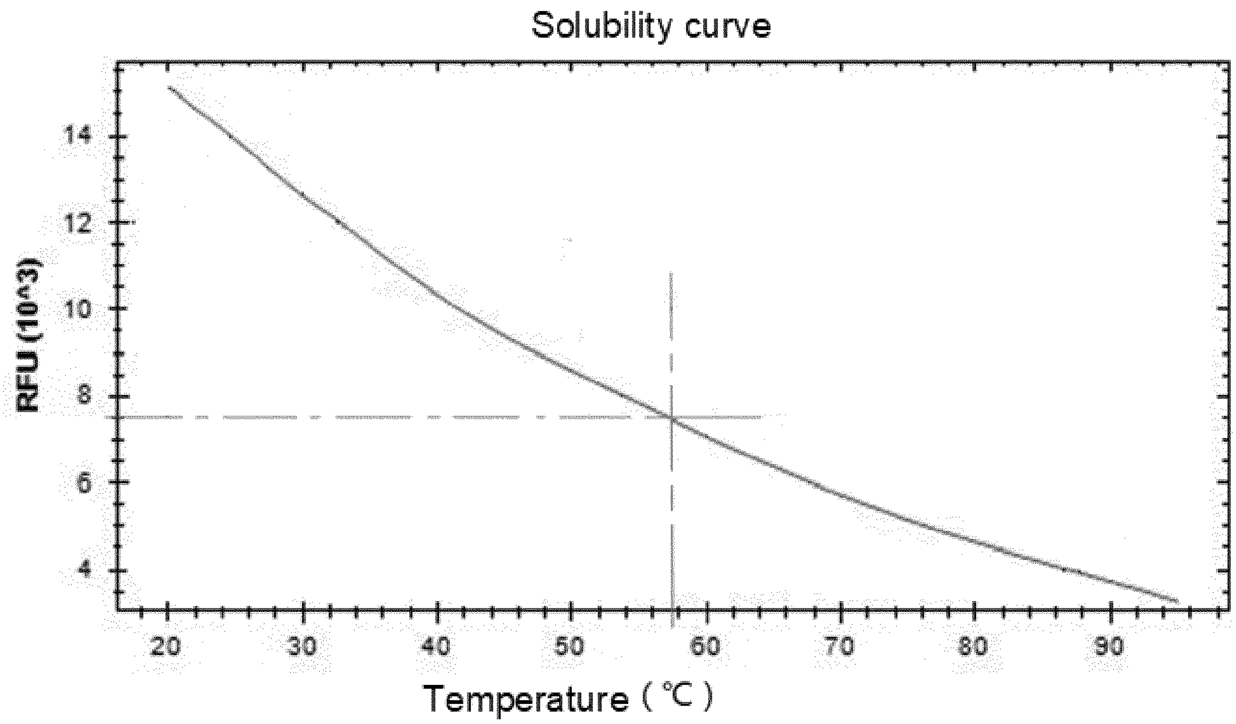
Solubility curve
RFU (10^3)
14
12
10
8
6
4
20
30
40
50
60
70
80
90
Temperature (℃)

Fig.17

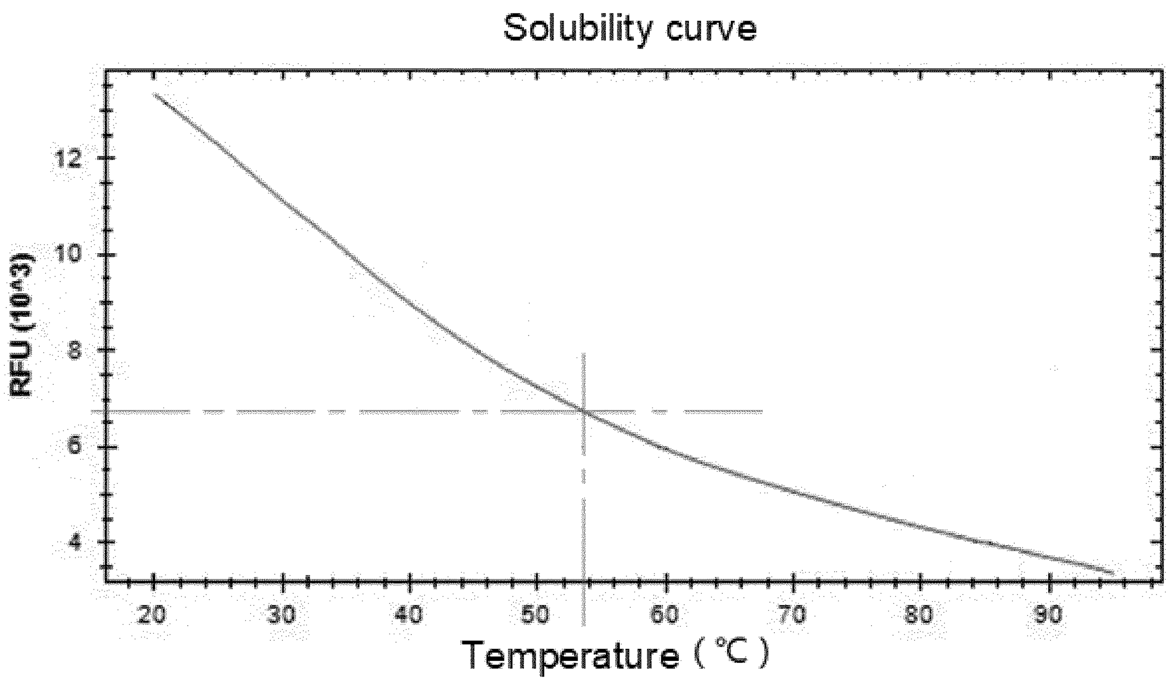
Solubility curve
RFU (10^3)
12
10
8
6
4
20
30
40
50
60
70
80
90
Temperature (℃)

Fig.18

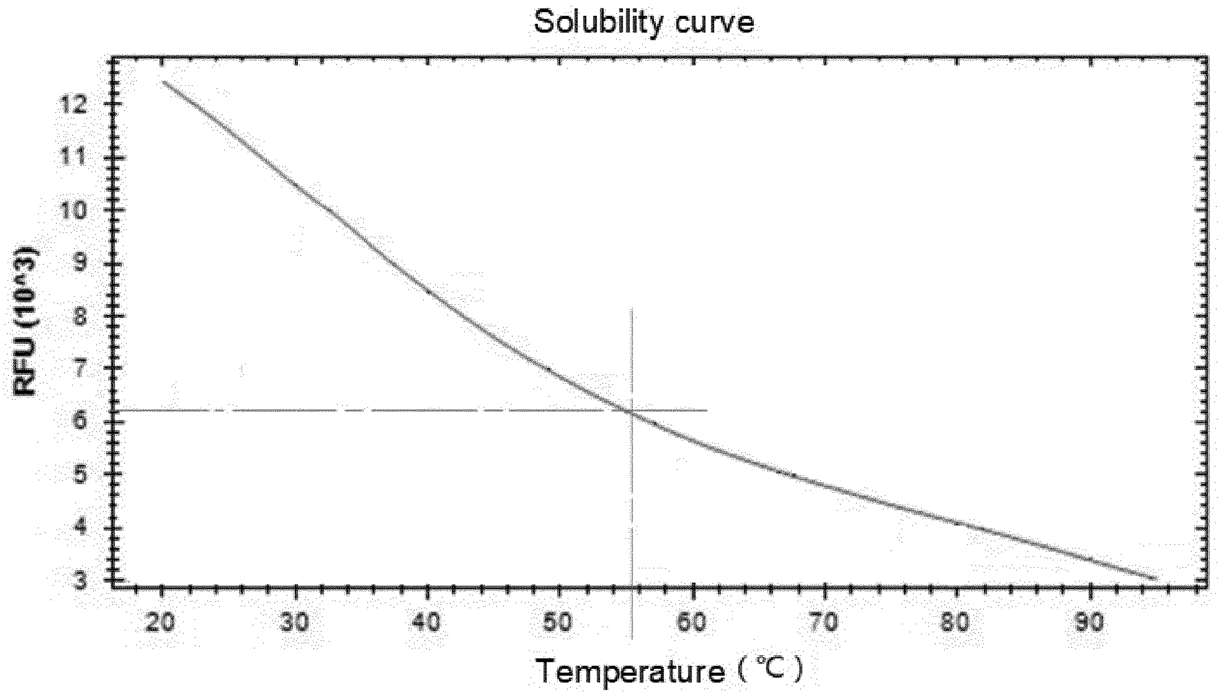
Solubility curve
RFU (10^3)
12
11
10
9
8
7
6
5
4
3
20
30
40
50
60
70
80
90
Temperature (℃)

Fig.19

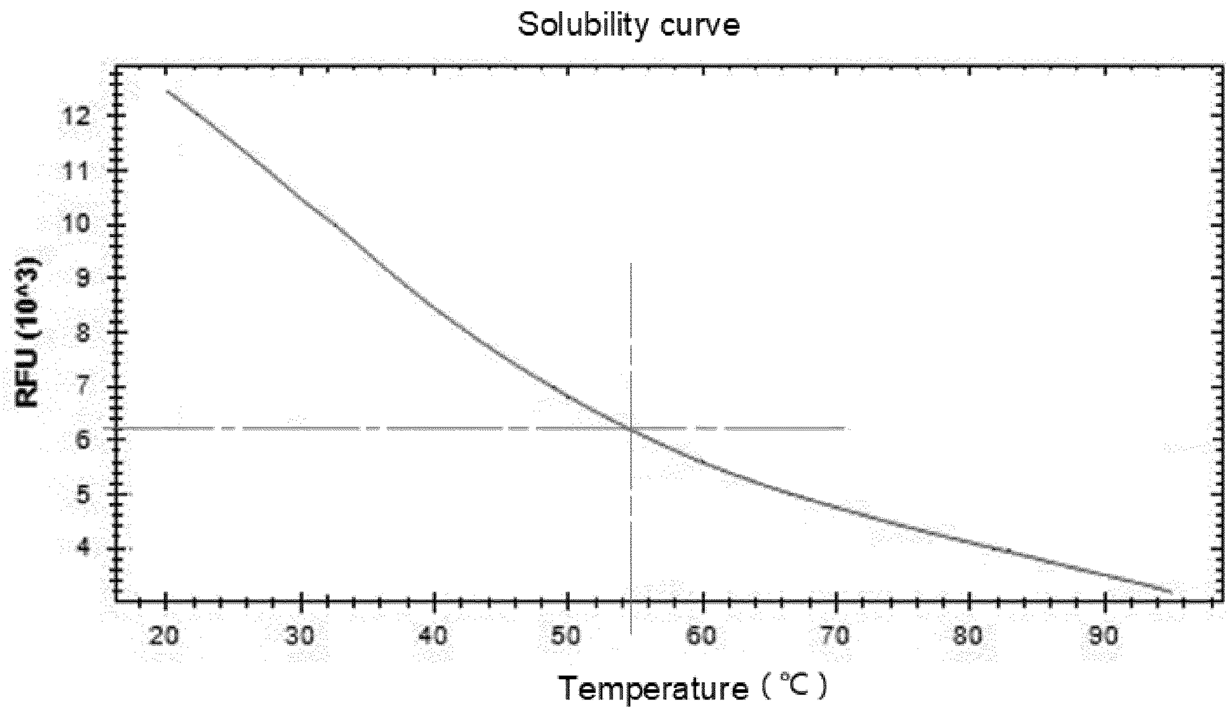
Solubility curve
RFU (10^3)
12
11
10
9
8
7
6
5
4
20
30
40
50
60
70
80
90
Temperature (℃)

Fig.20

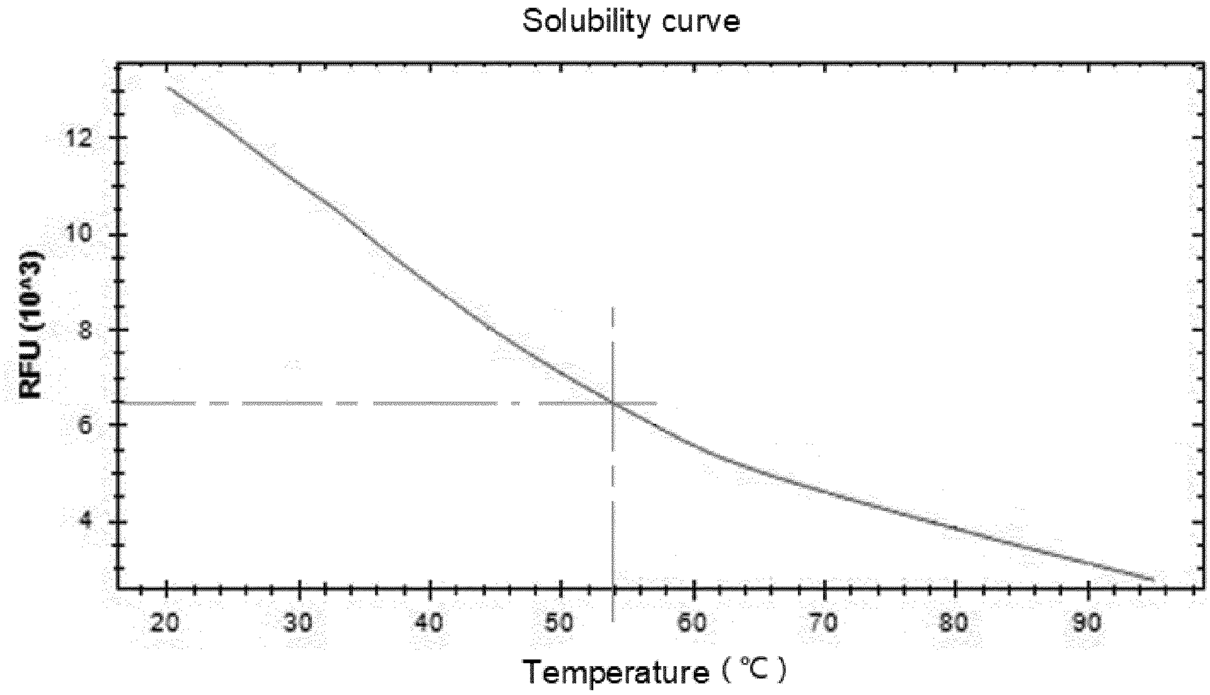
Solubility curve
RFU (10^3)
12
10
8
6
4
20
30
40
50
60
70
80
90
Temperature (℃)

Fig.21

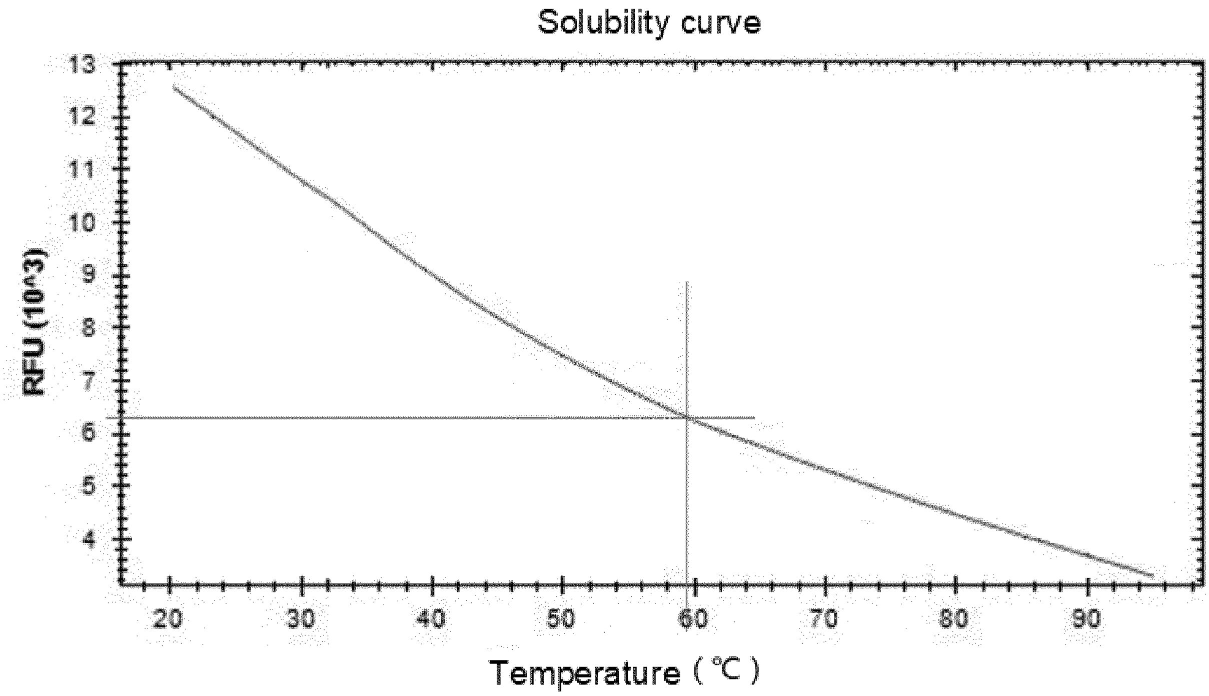
Solubility curve
RFU (10^3)
13
12
11
10
9
8
7
6
5
4
20
30
40
50
60
70
80
90
Temperature (℃)

Fig.22

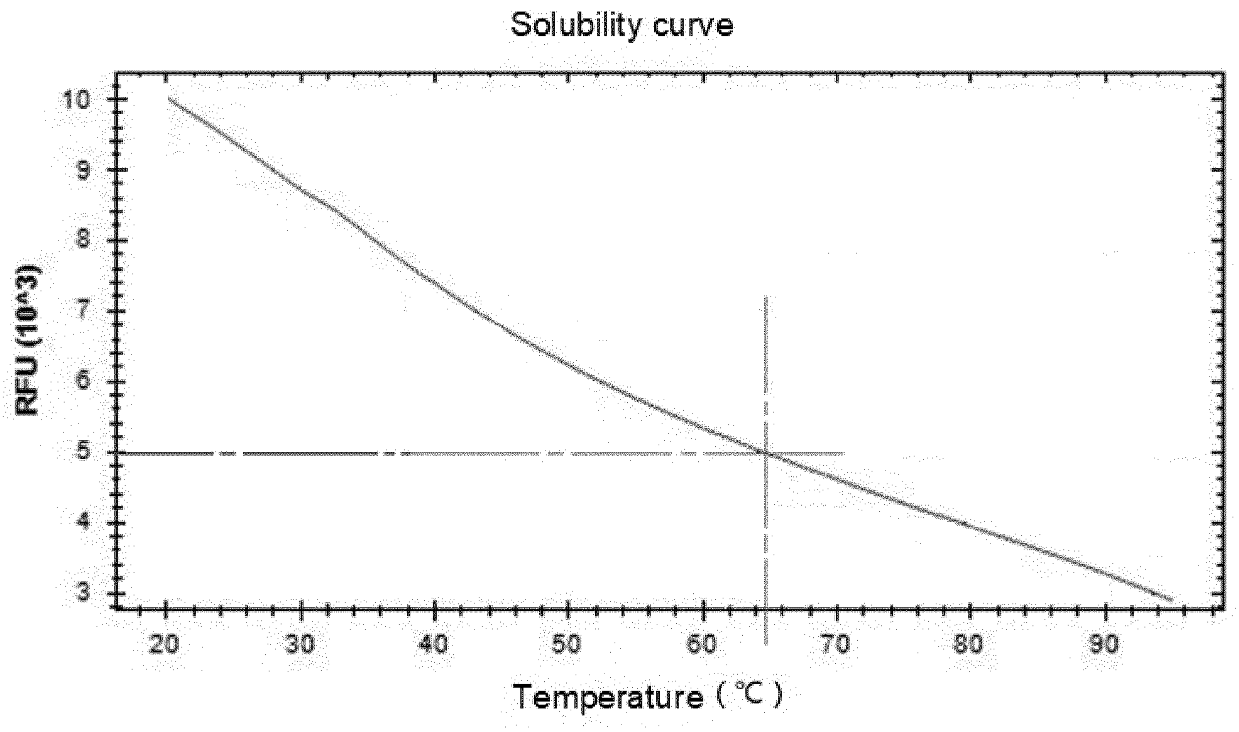
Solubility curve
RFU (10^3)
10
9
8
7
6
5
4
3
20
30
40
50
60
70
80
90
Temperature (℃)

Fig.23

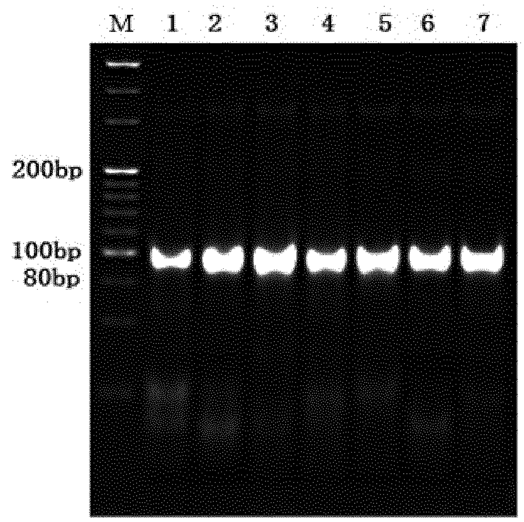
M 1 2 3 4 5 6 7
200bp
100bp
80bp

Fig.24

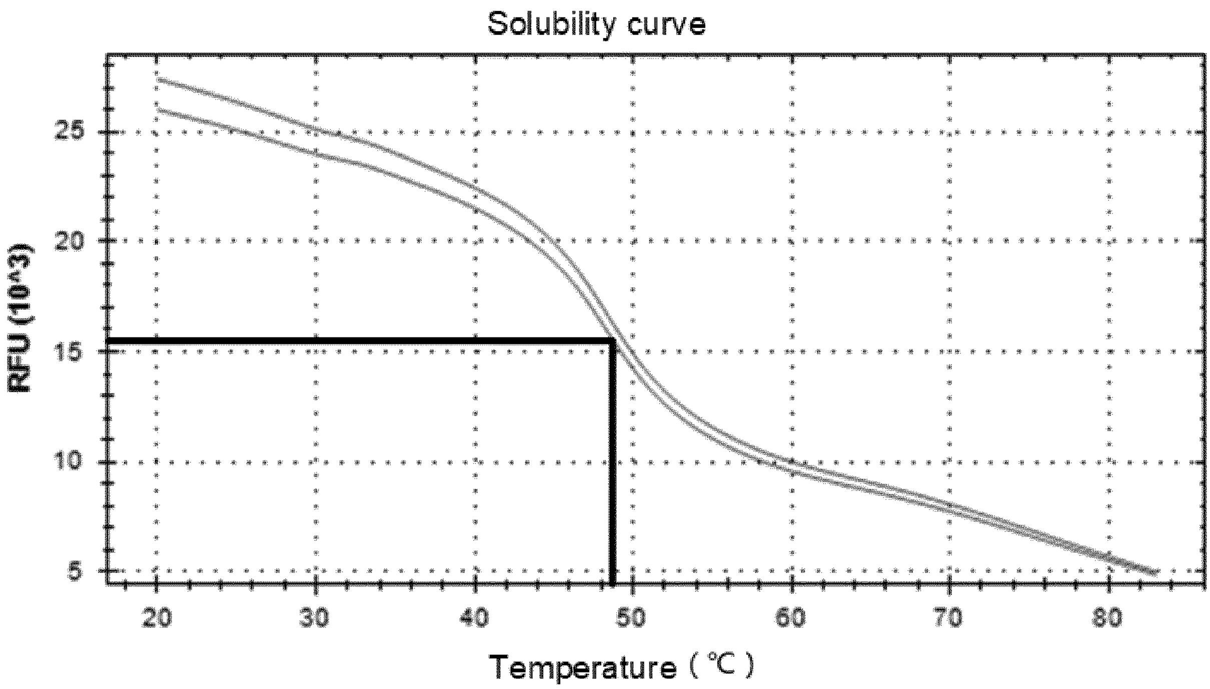

Solubility curve
RFU (10^3)
25
20
15
10
5
20
30
40
50
60
70
80
Temperature (℃)


Fig.25

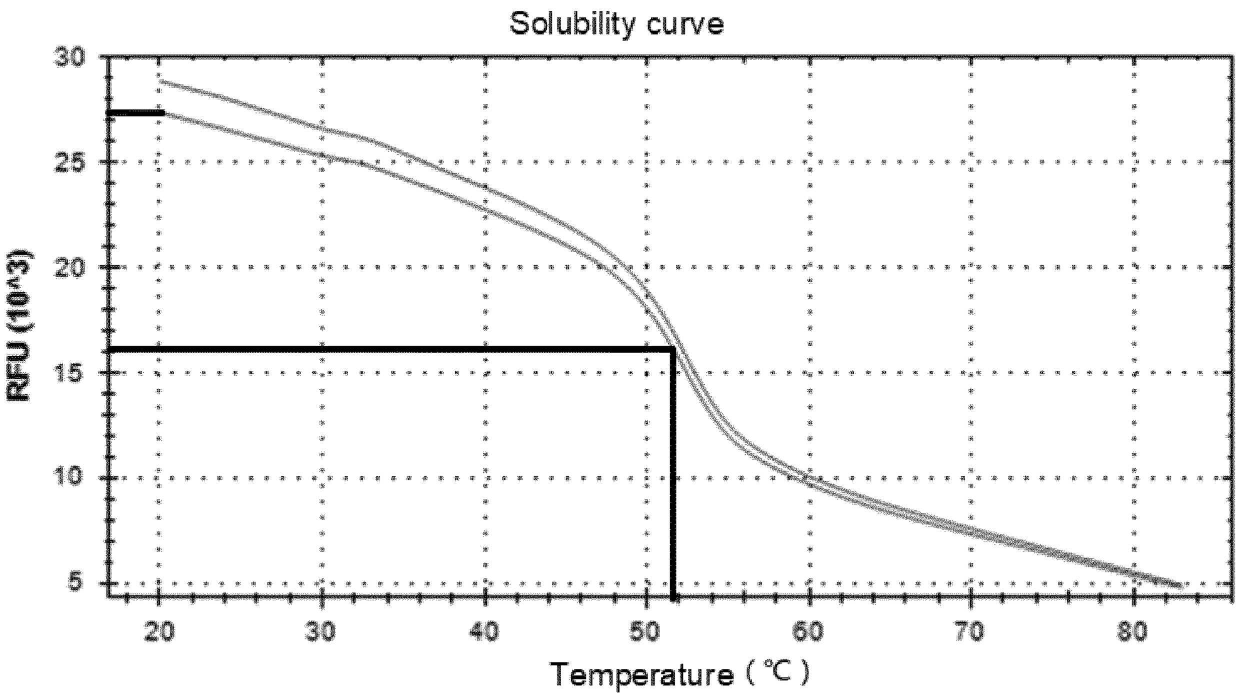

Solubility curve
RFU (10^3)
30
25
20
15
10
5
20
30
40
50
60
70
80
Temperature (℃)


Fig.26

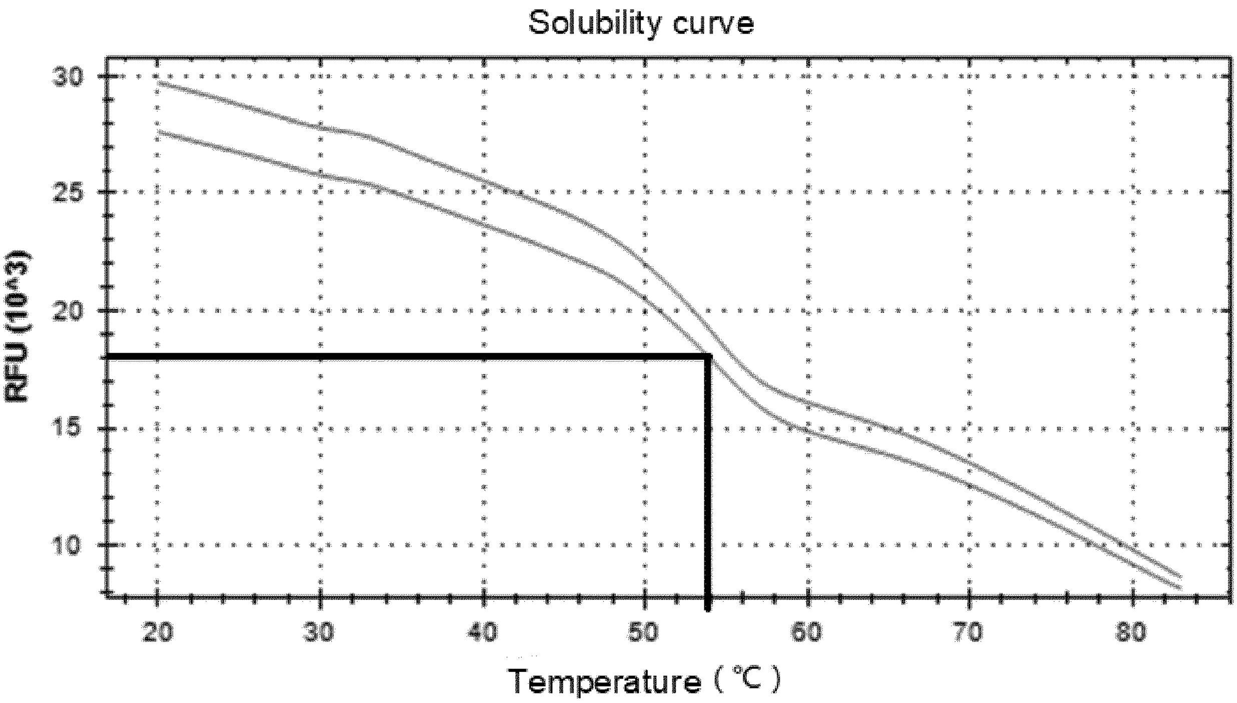
Solubility curve
RFU (10^3)
30
25
20
15
10
20
30
40
50
60
70
80
Temperature (℃)

Fig.27

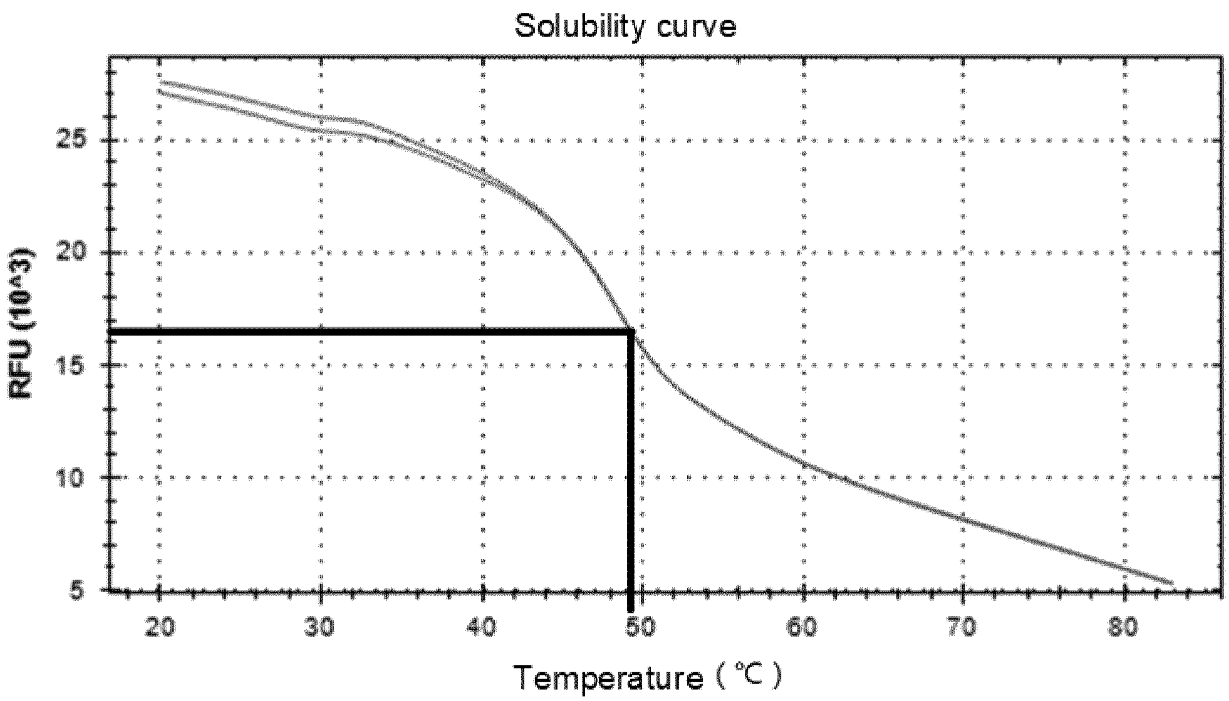
Solubility curve
RFU (10^3)
25
20
15
10
5
20
30
40
50
60
70
80
Temperature (℃)

Fig.28

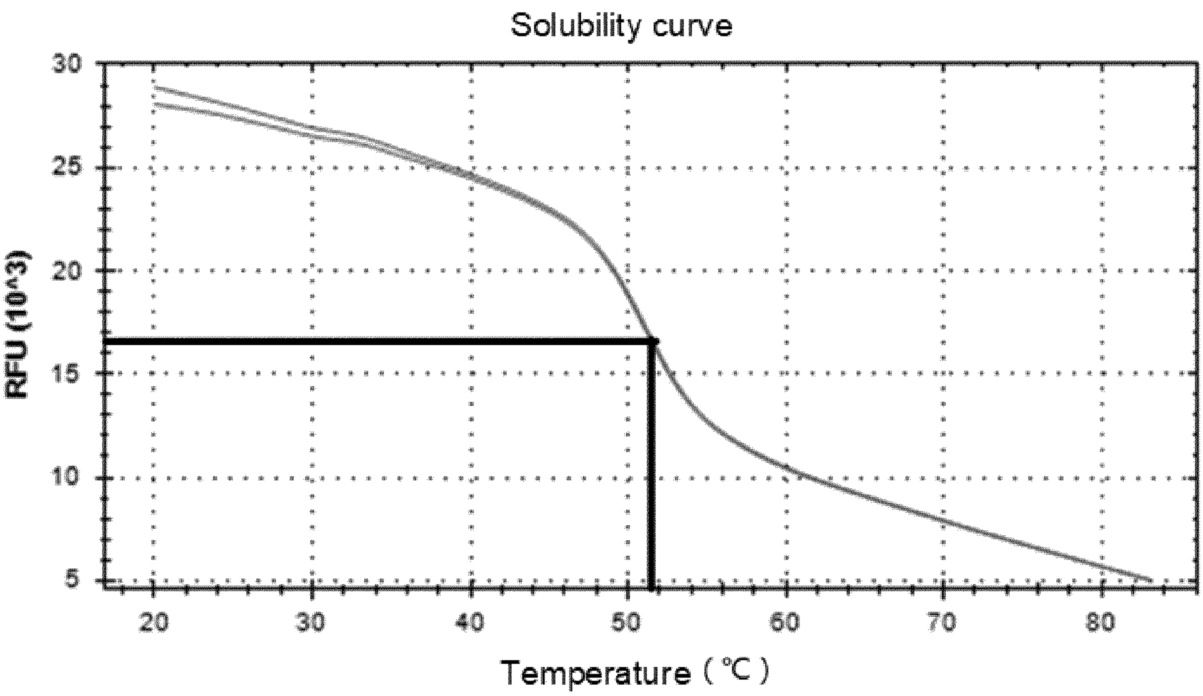
Solubility curve
30
25
20
15
10
5
RFU (10^3)
20
30
40
50
60
70
80
Temperature（℃）

Fig.29

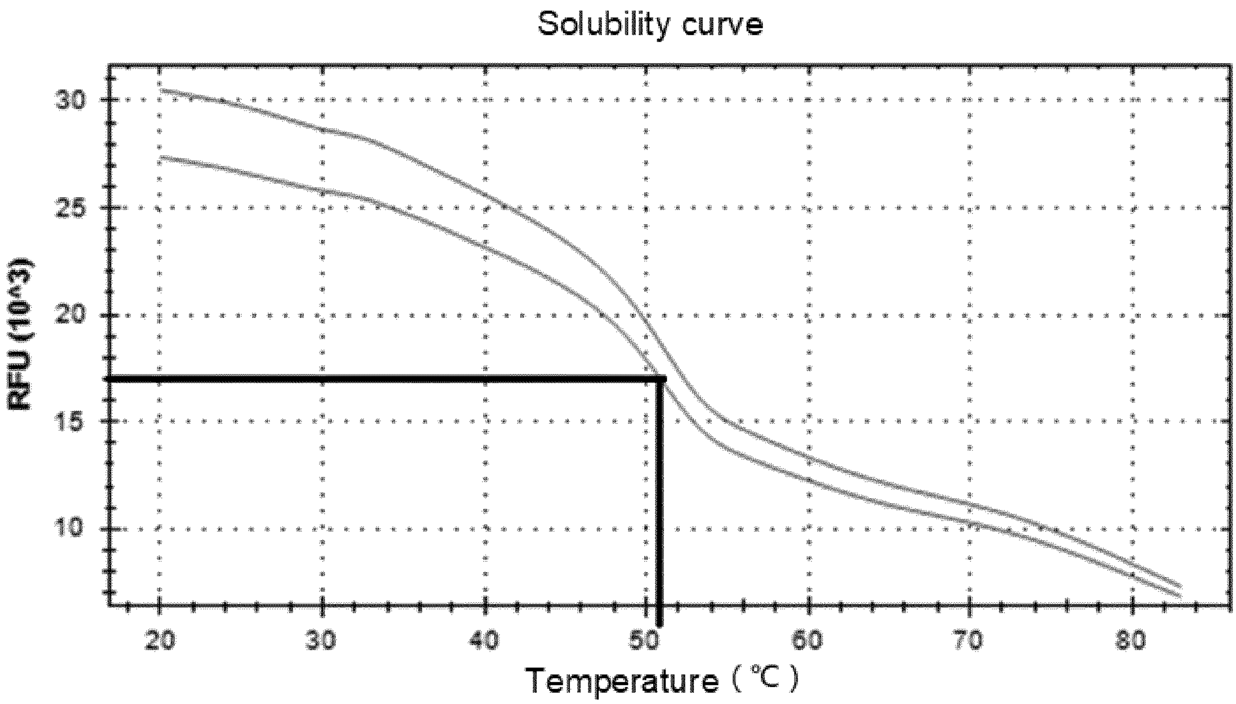
Solubility curve
30
25
20
15
10
RFU (10^3)
20
30
40
50
60
70
80
Temperature（℃）

Fig.30

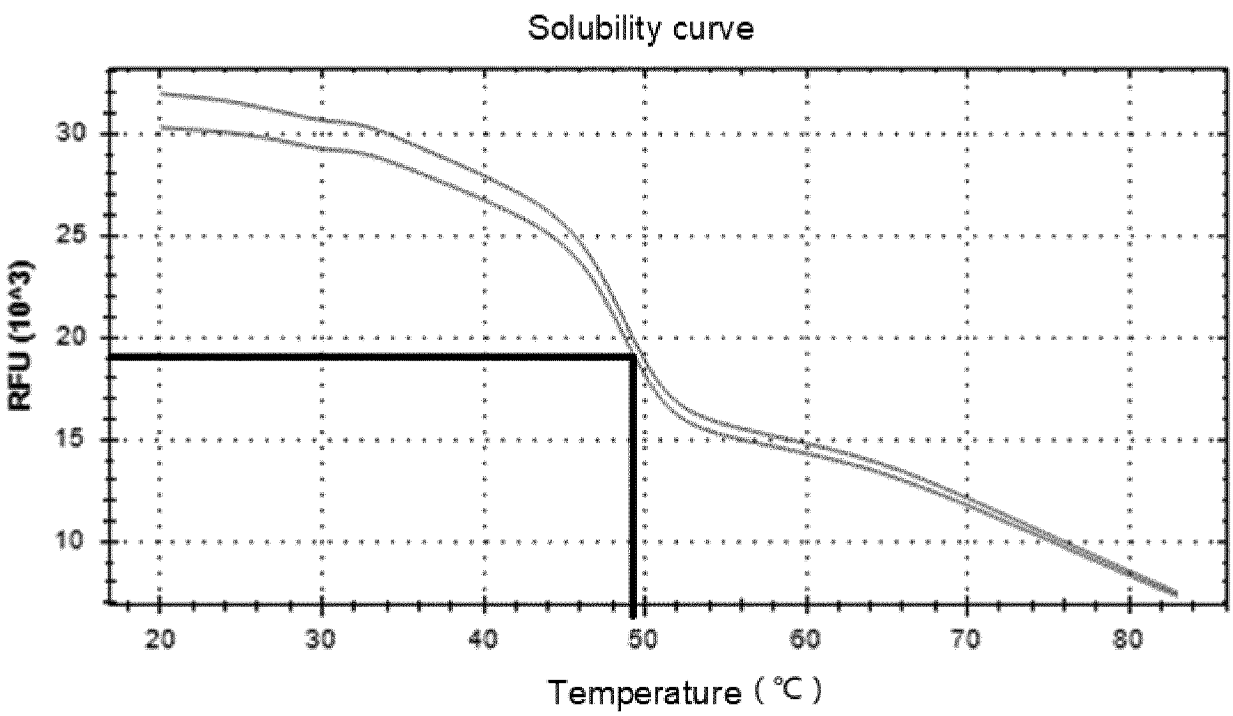
Solubility curve
RFU (10^3)
30
25
20
15
10
20
30
40
50
60
70
80
Temperature (℃)

Fig.31

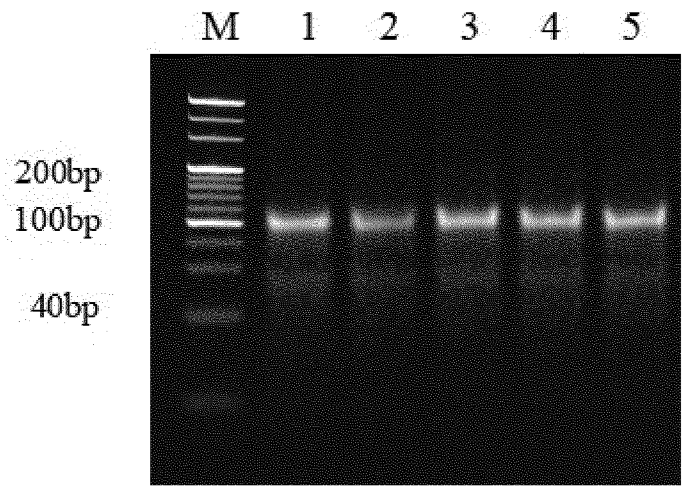
M 1 2 3 4 5
200bp
100bp
40bp

Fig.32

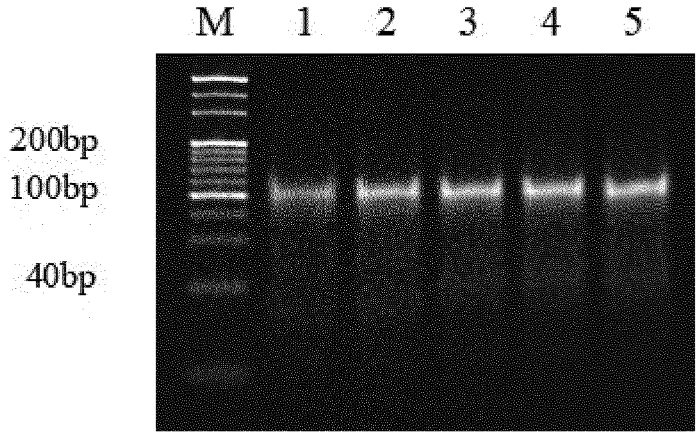


Fig.33

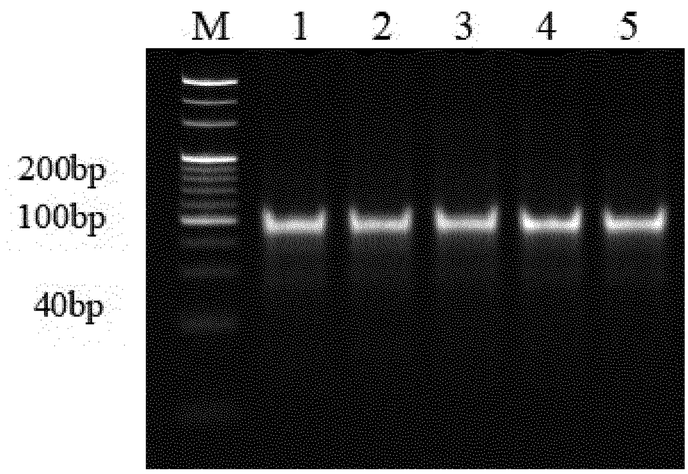


Fig.34

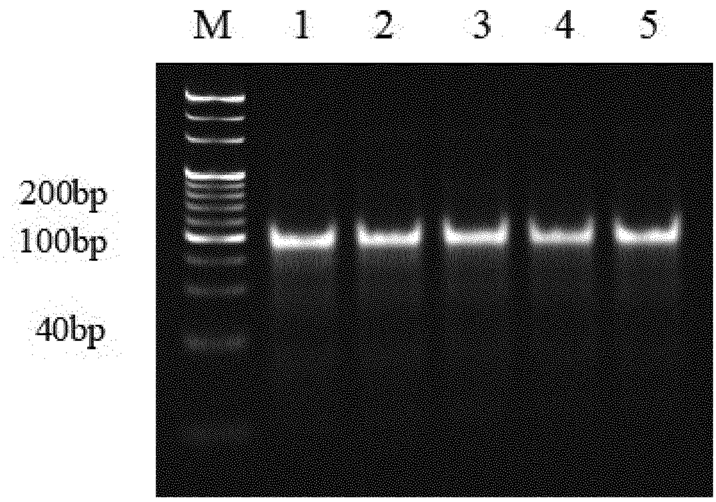


Fig.35

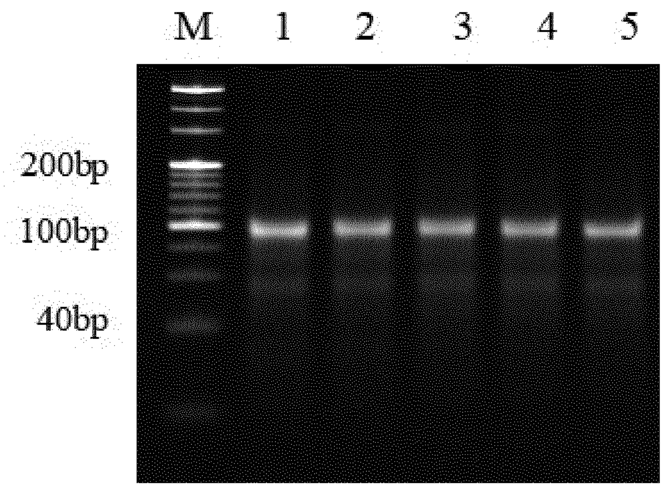


Fig.36

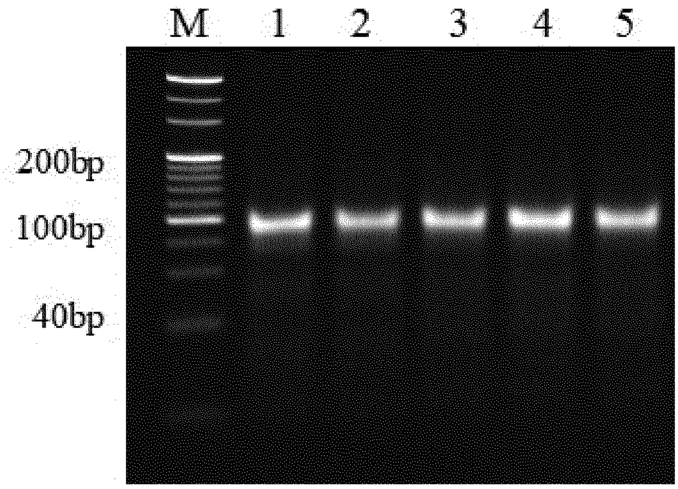


Fig.37

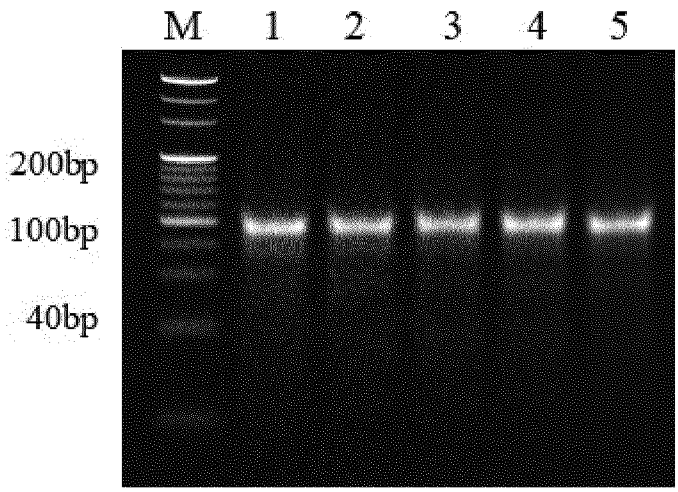


Fig.38

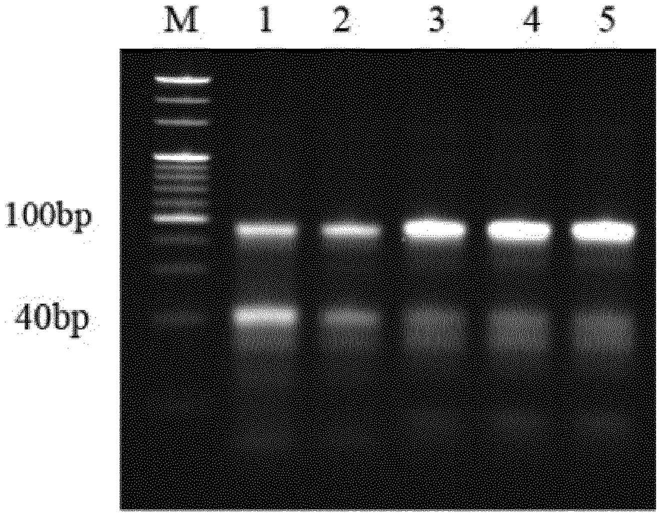


Fig.39

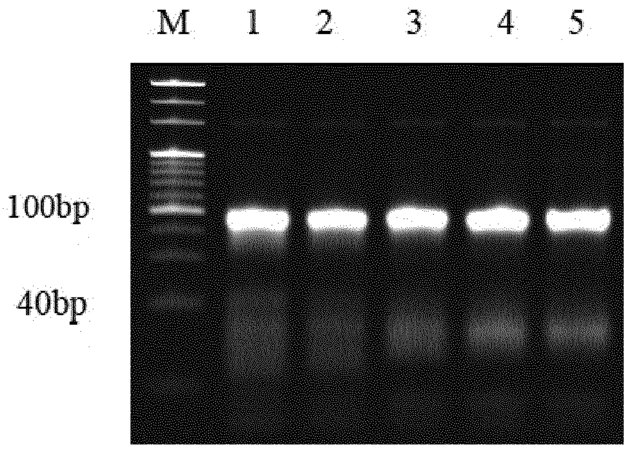


Fig.40

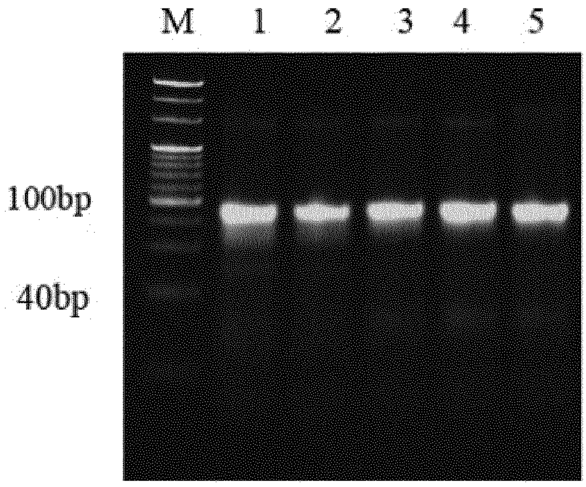


Fig.41

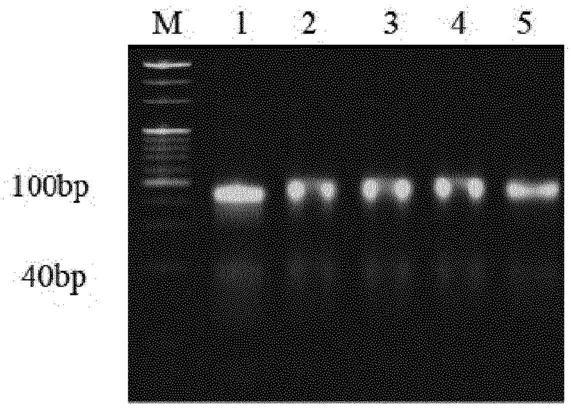


Fig.42

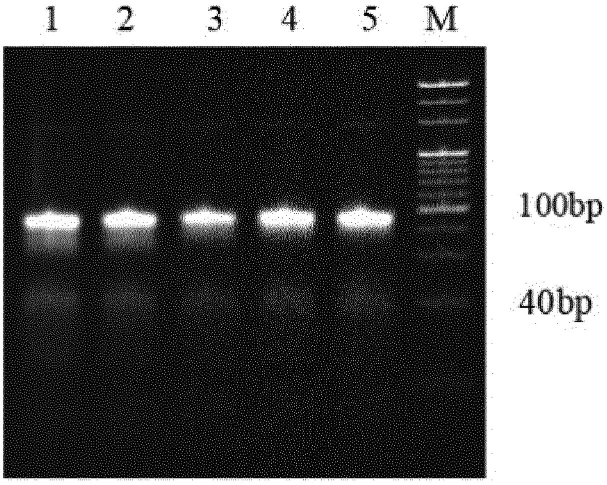


Fig.43

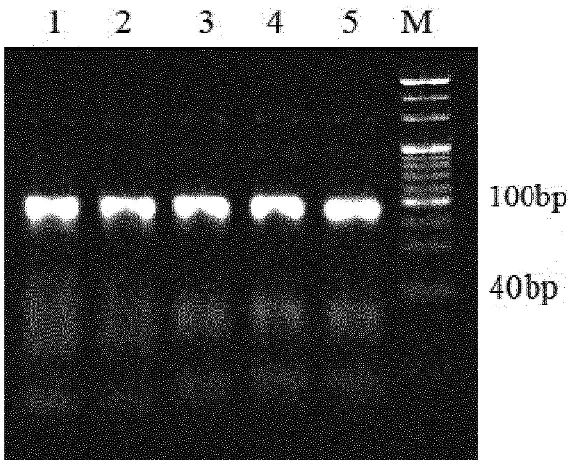


Fig.44

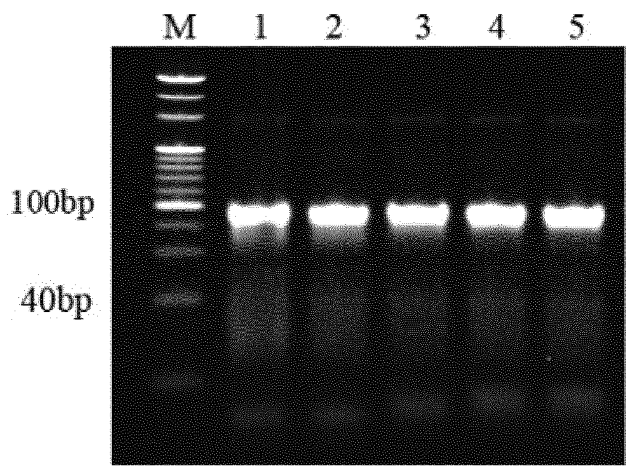


Fig.45

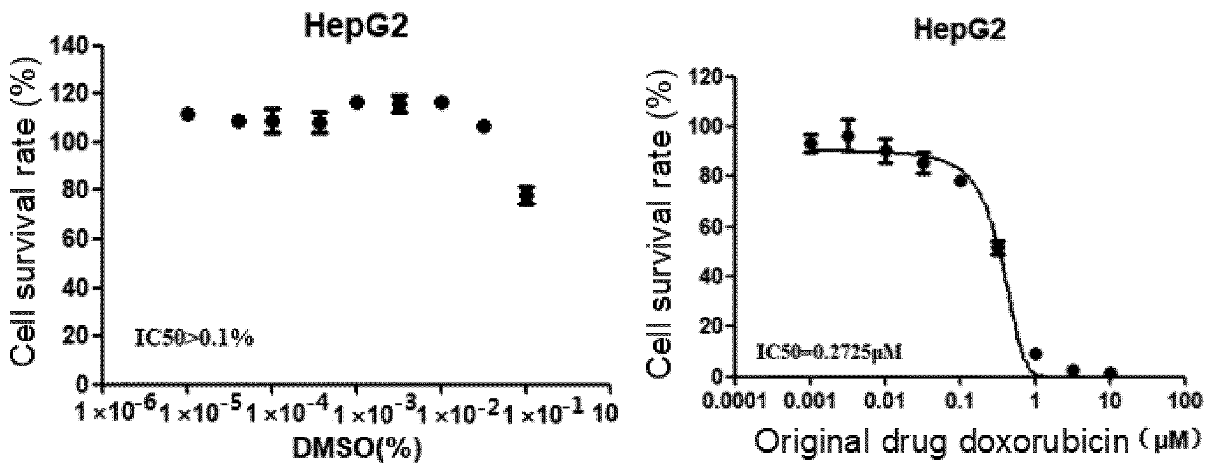


Fig.46a

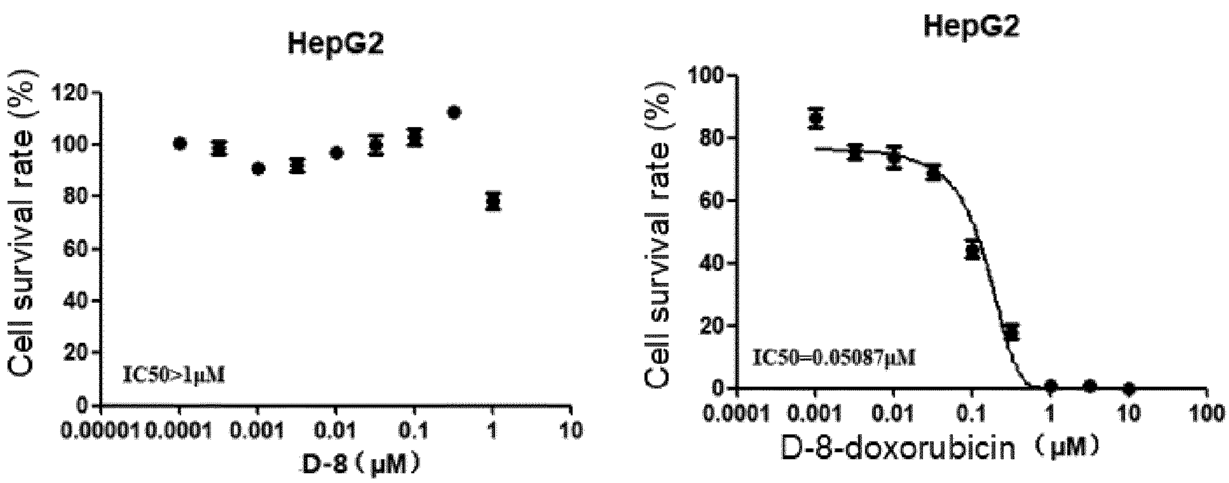


Fig.46b

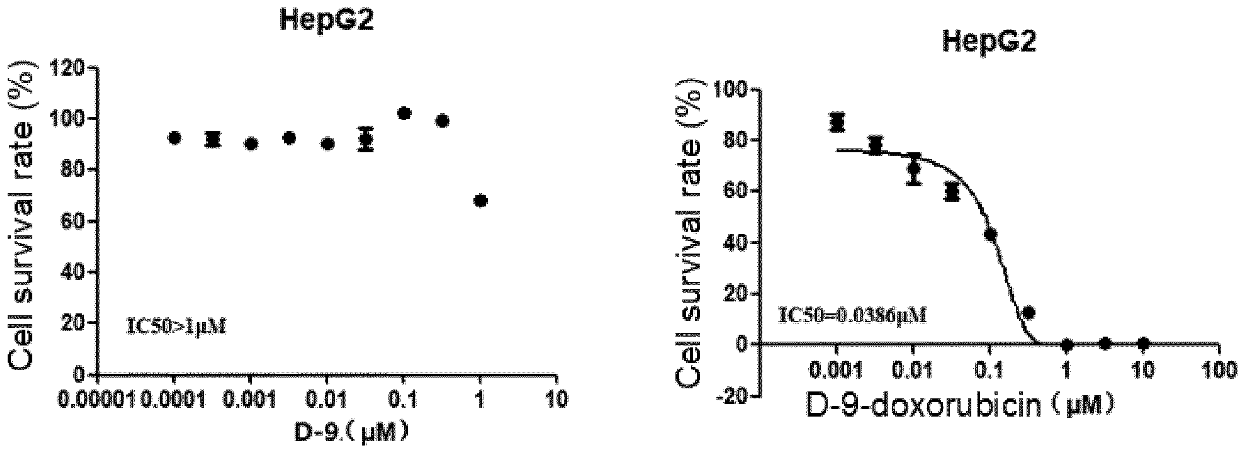


Fig.46c

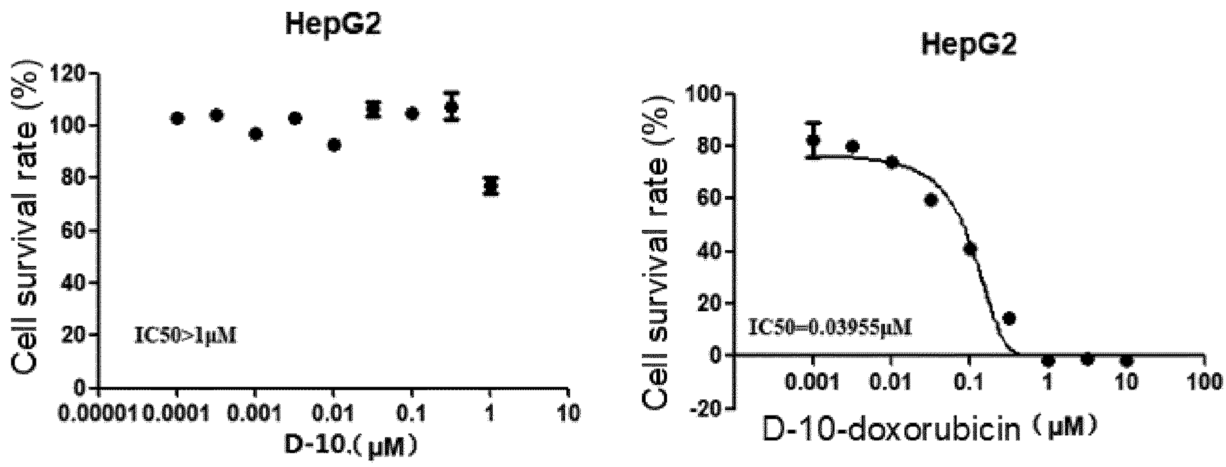


Fig.46d

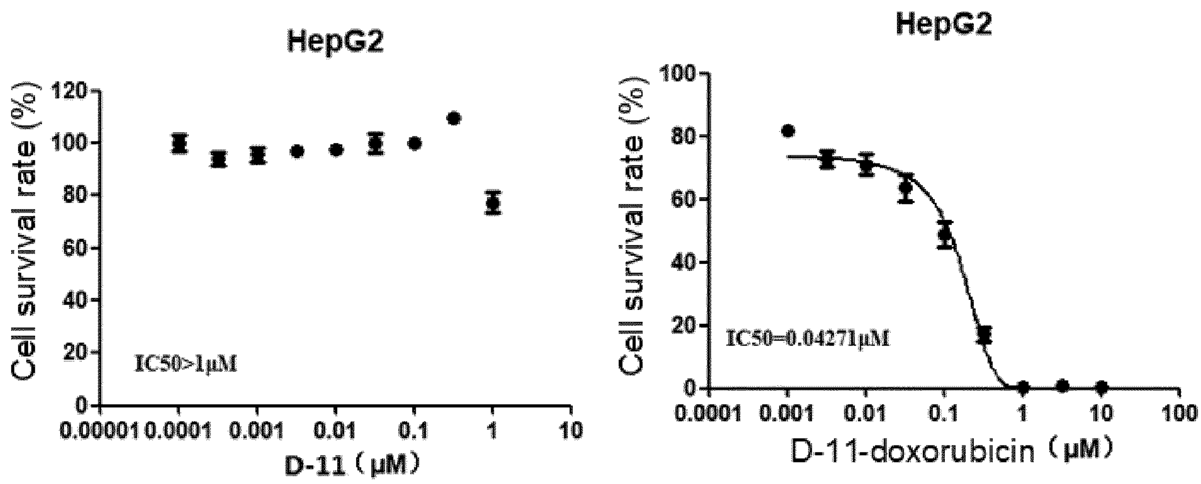


Fig.46e

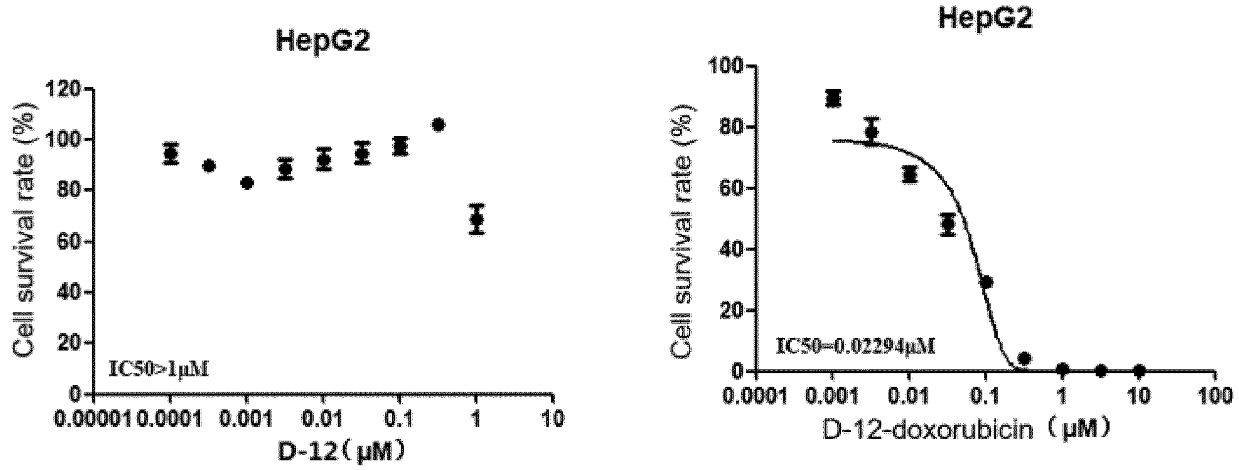


Fig.46f

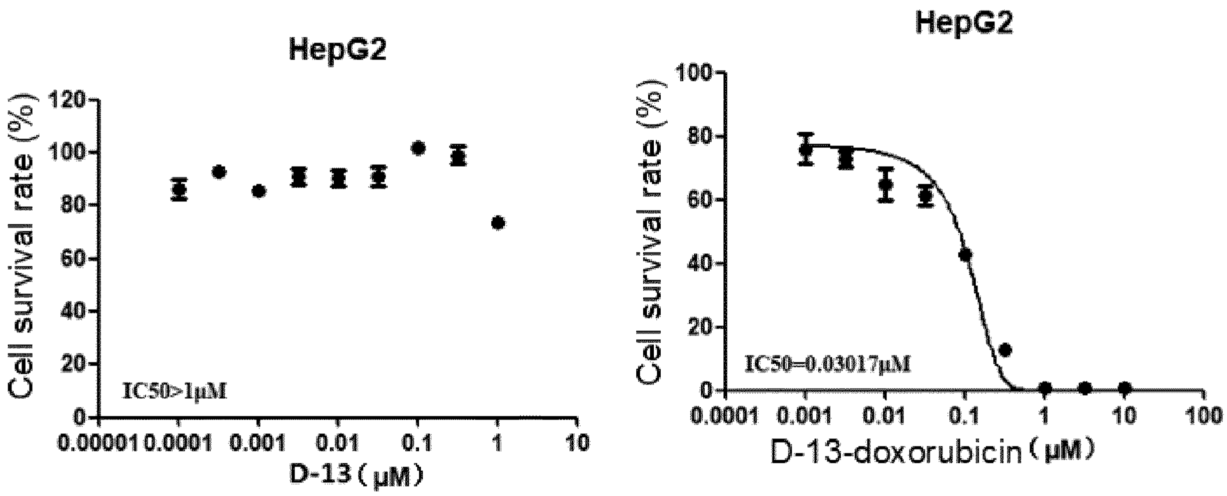


Fig.46g

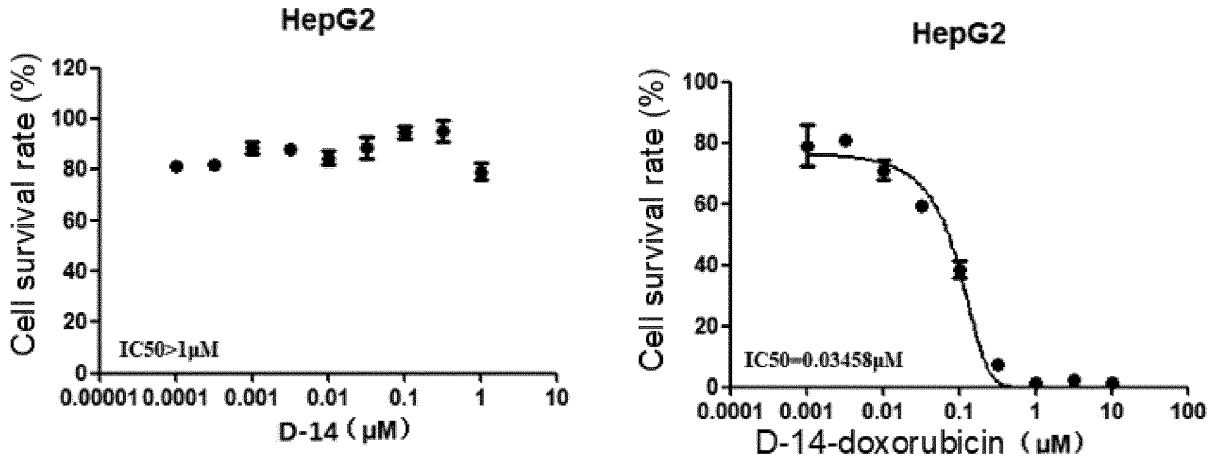


Fig.46h

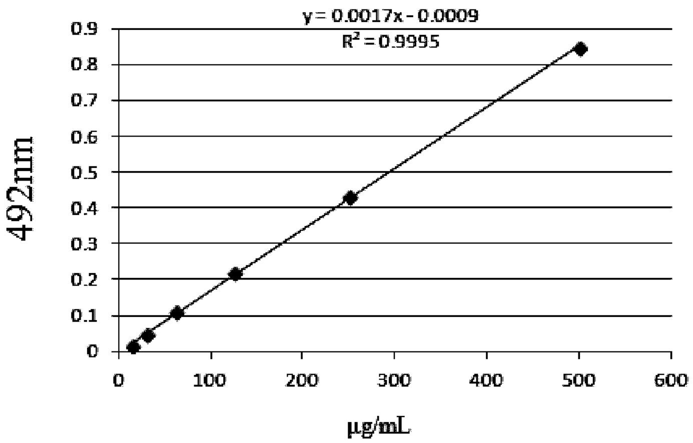


Fig.47

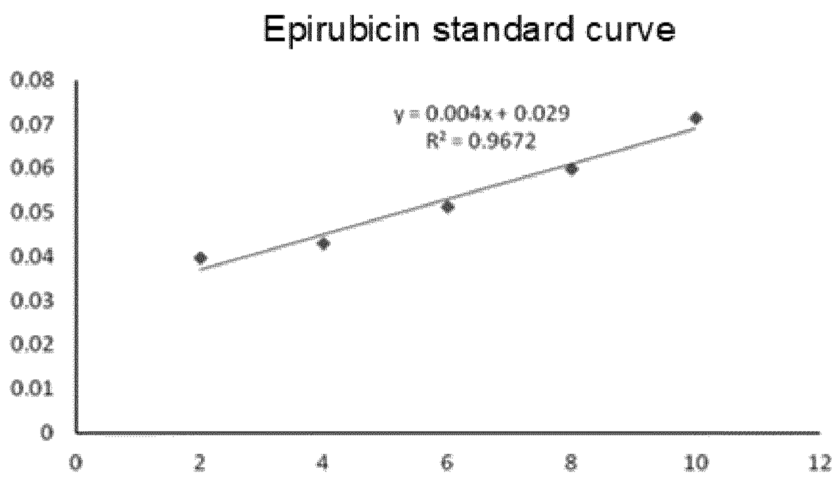


Fig.48a

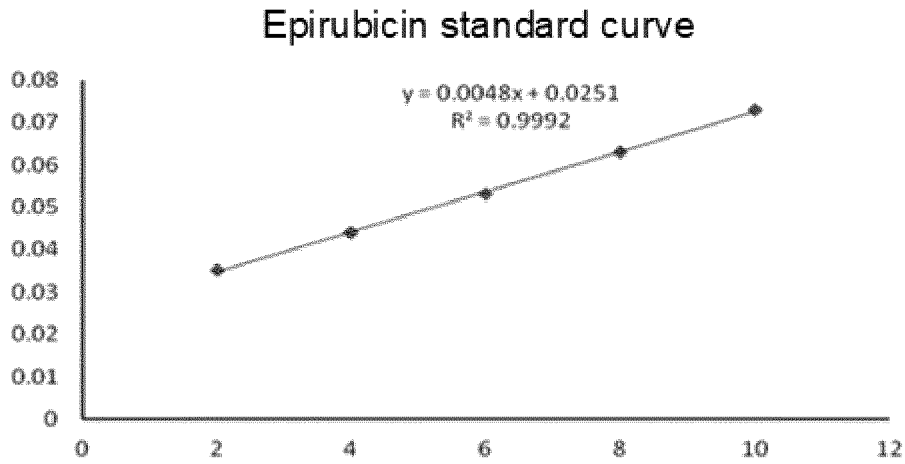


Fig.48b

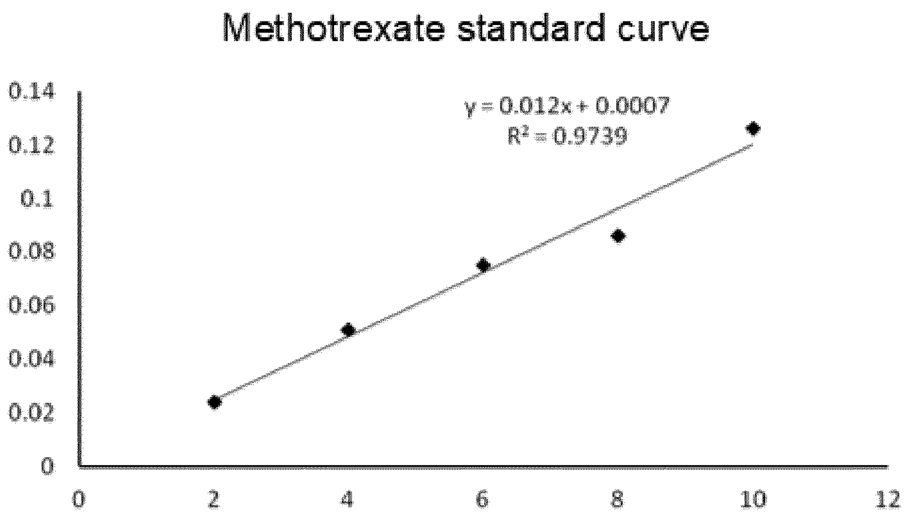


Fig.49

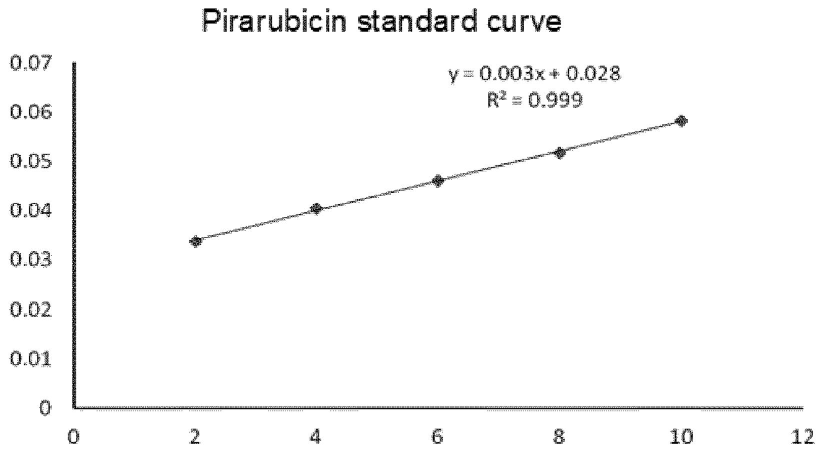


Fig.50a

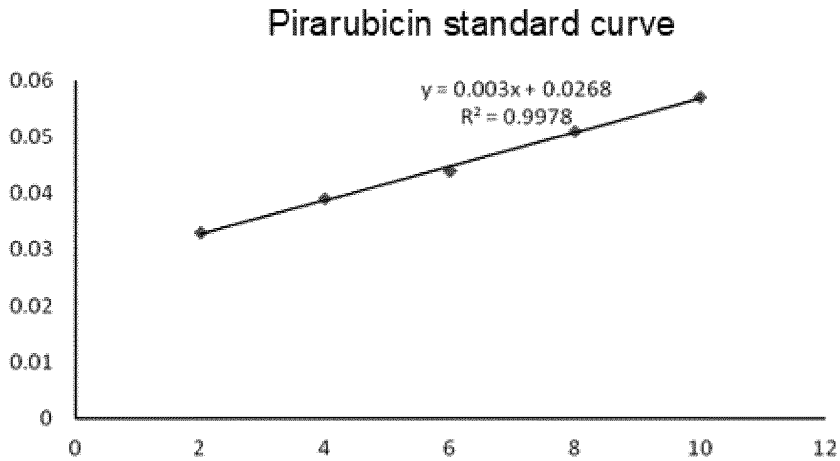


Fig.50b

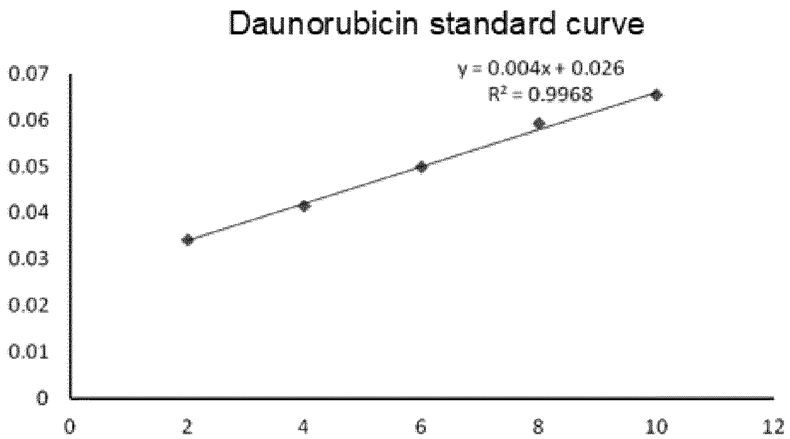


Fig.51

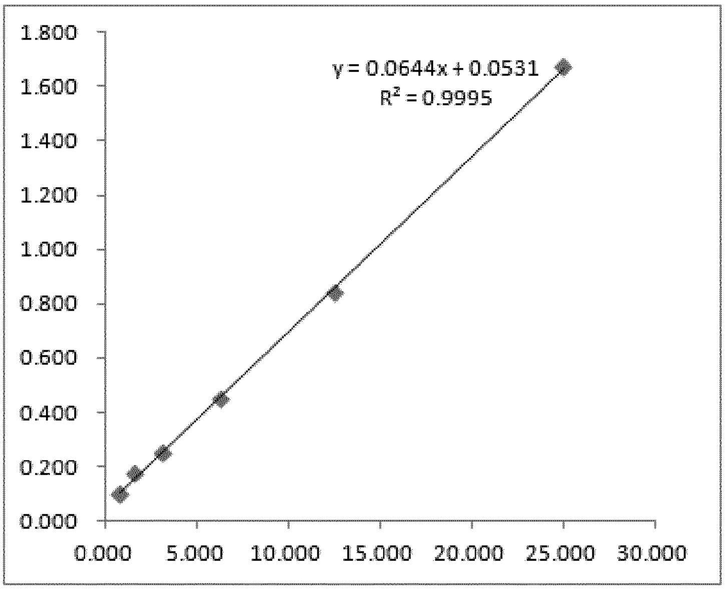
1.800
1.600
1.400
1.200
1.000
0.800
0.600
0.400
0.200
0.000
0.000
5.000
10.000
15.000
20.000
25.000
30.000
y = 0.0644x + 0.0531
R² = 0.9995

Fig.52

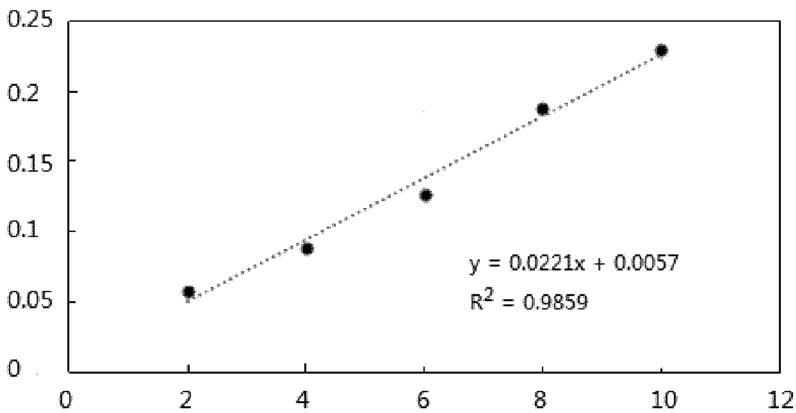
0.25
0.2
0.15
0.1
0.05
0
0
2
4
6
8
10
12
y = 0.0221x + 0.0057
R² = 0.9859

Fig.53

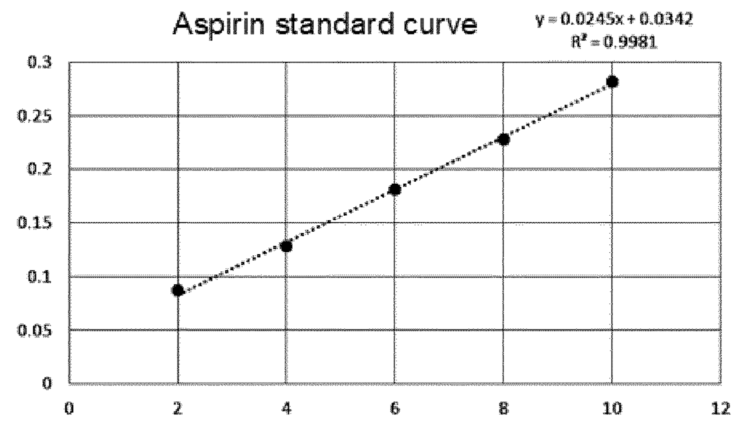


Fig.54a

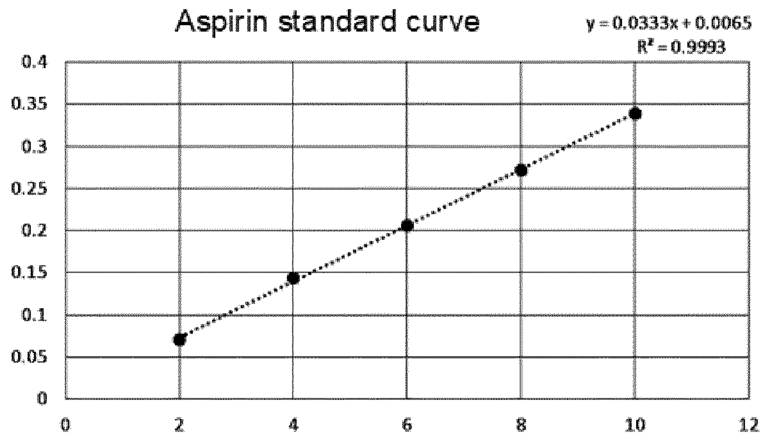


Fig.54b

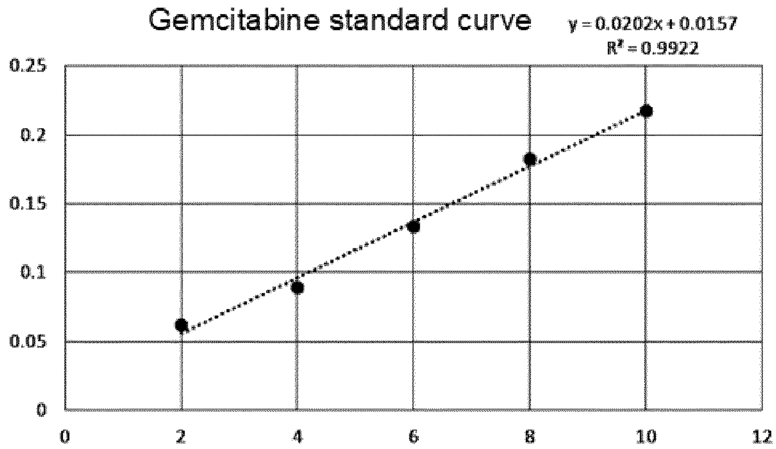


Fig.55

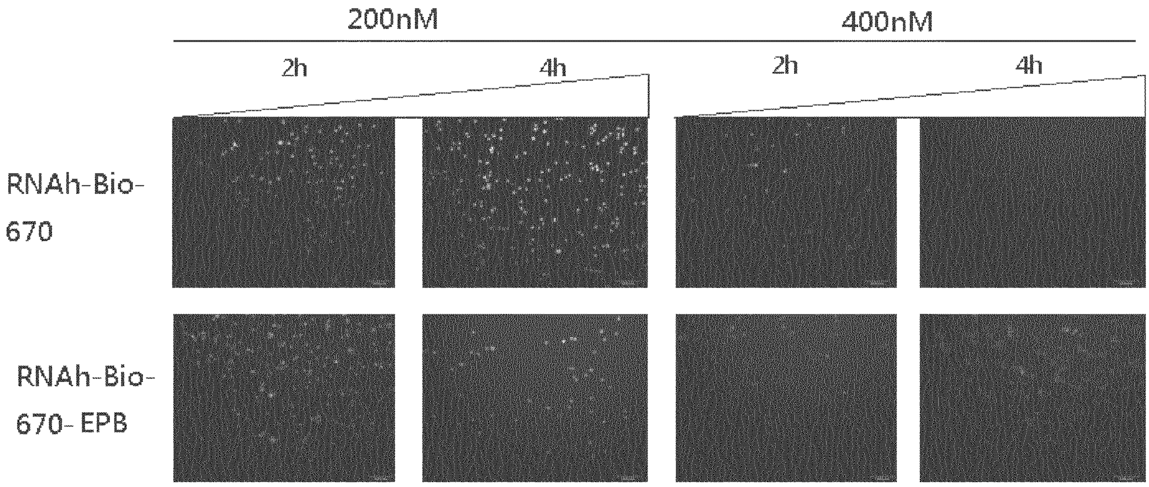


Fig.56

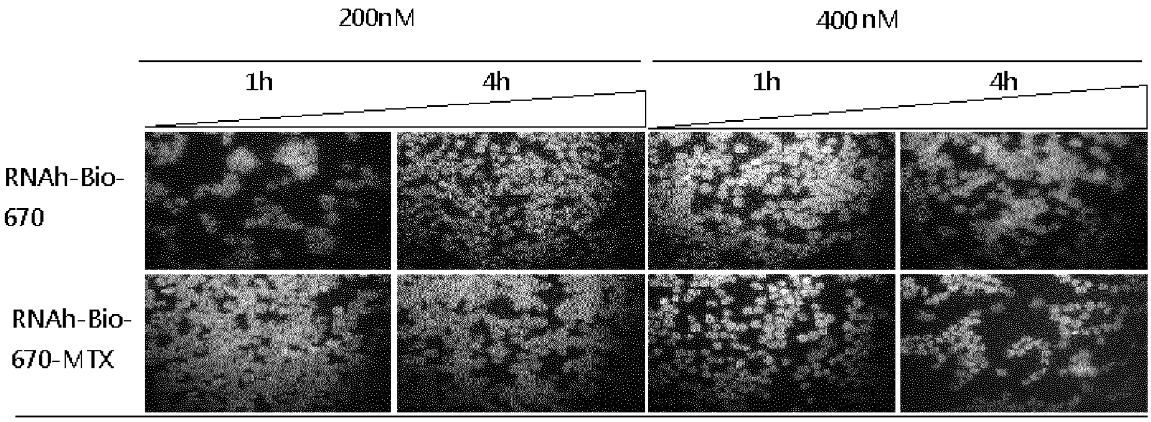


Fig.57

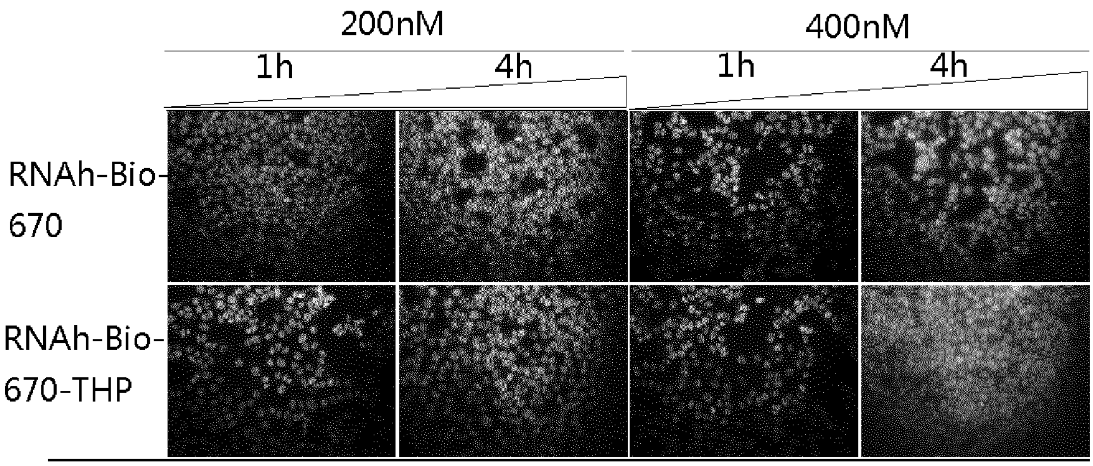


Fig.58

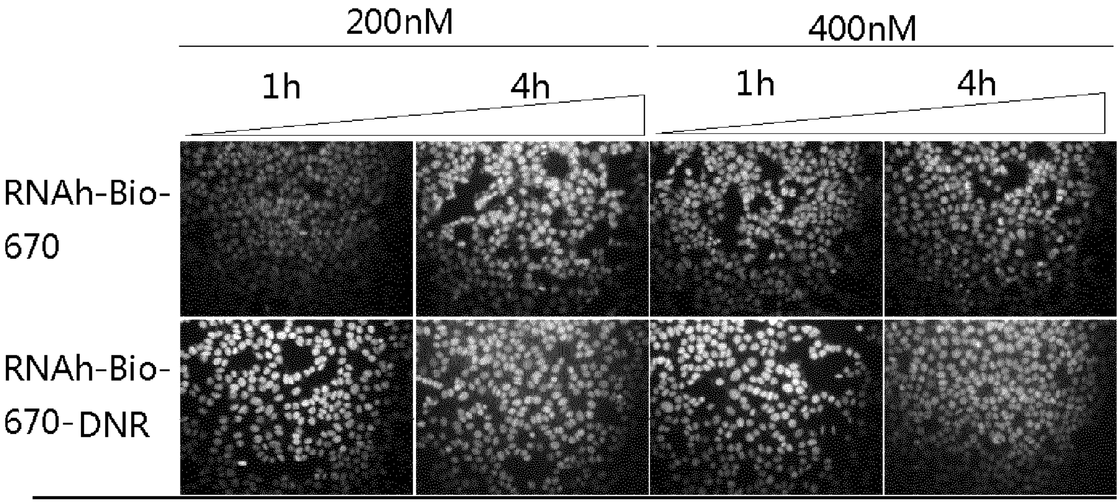


Fig.59

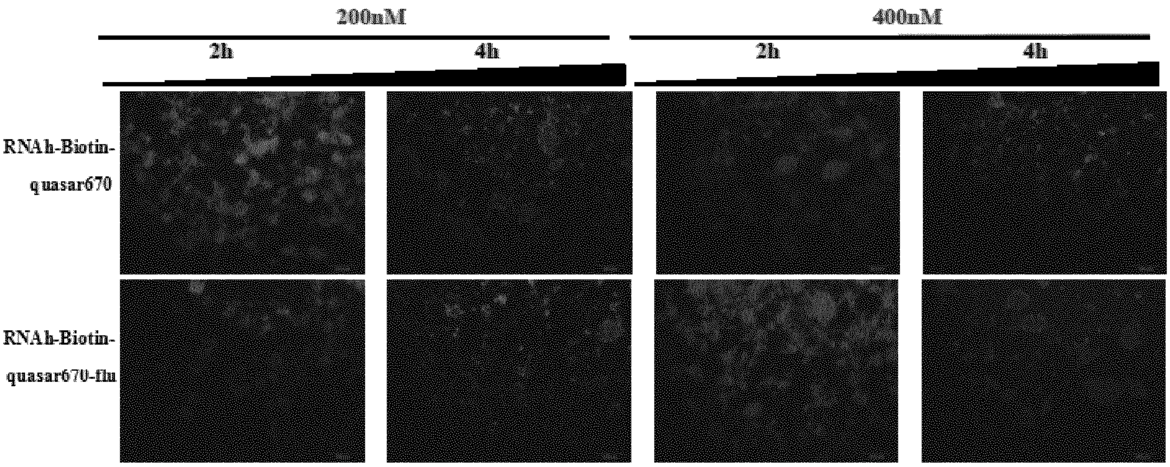


Fig.60

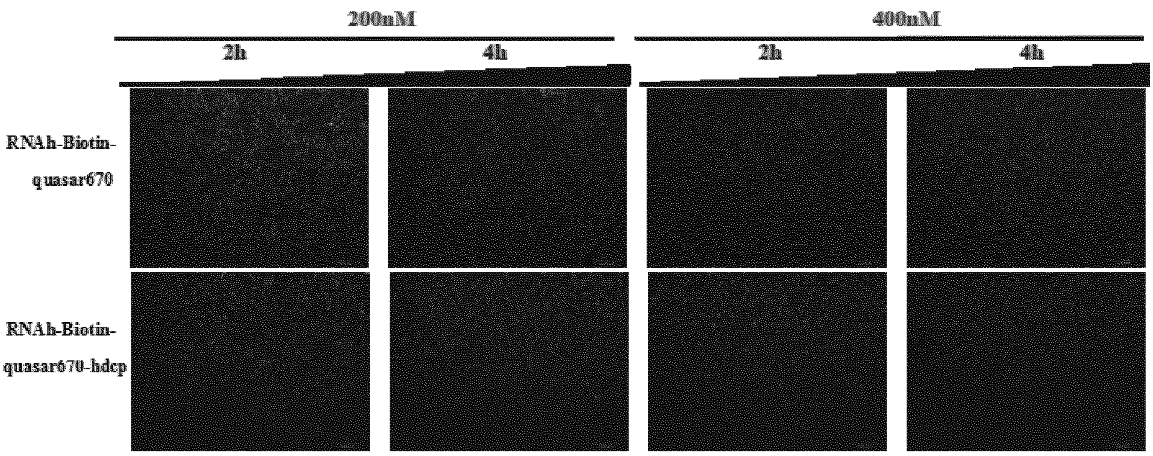


Fig.61

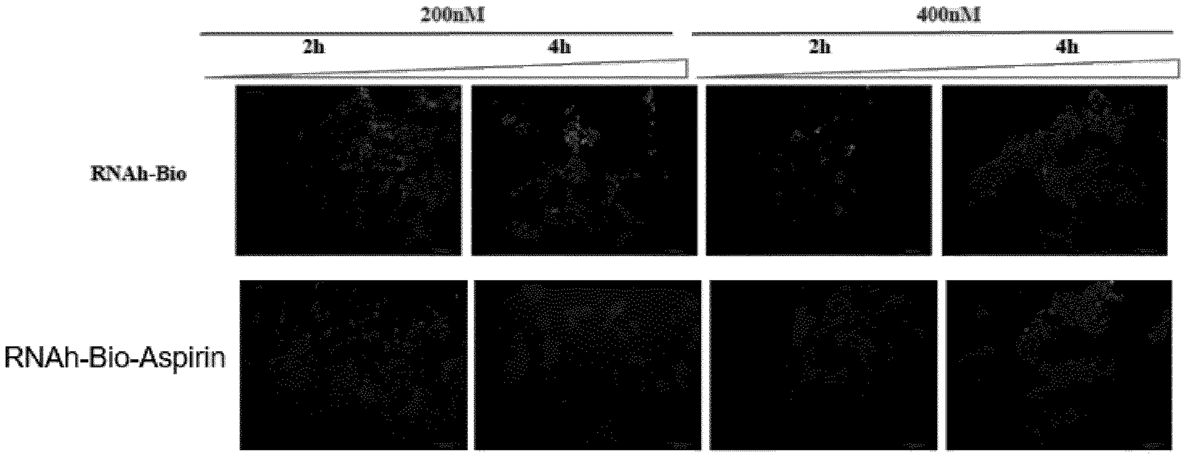
200nM
400nM
2h
4h
2h
4h
RNAh-Bio
RNAh-Bio-Aspirin

Fig.62

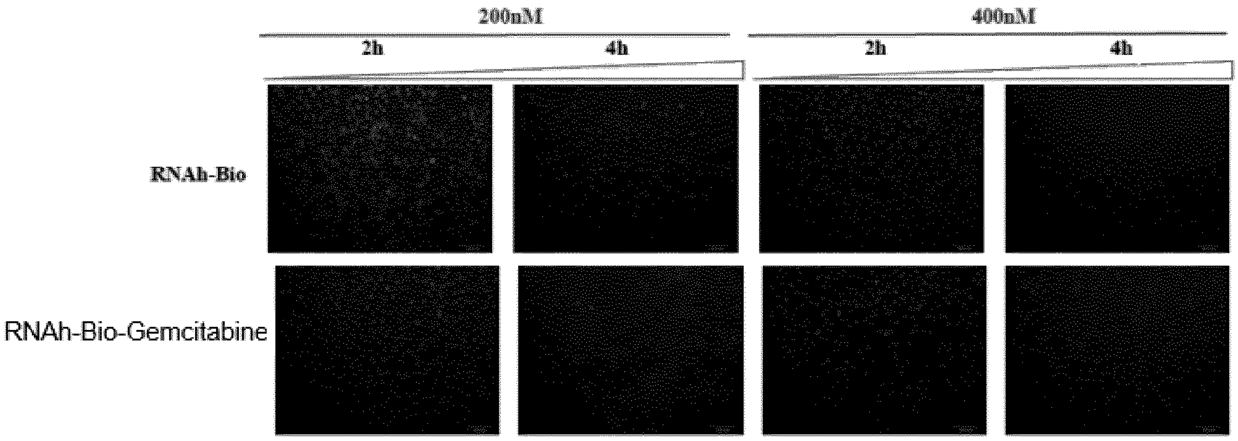
200nM
400nM
2h
4h
2h
4h
RNAh-Bio
RNAh-Bio-Gemcitabine

Fig.63

0 10min 1h 12h 36h

180KD
130KD
70KD

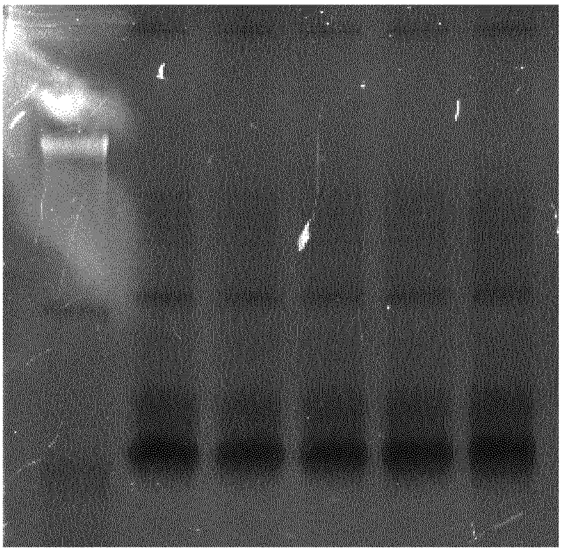

Fig.64

0 10min 1h 12h 36h

180KD
130KD
70KD

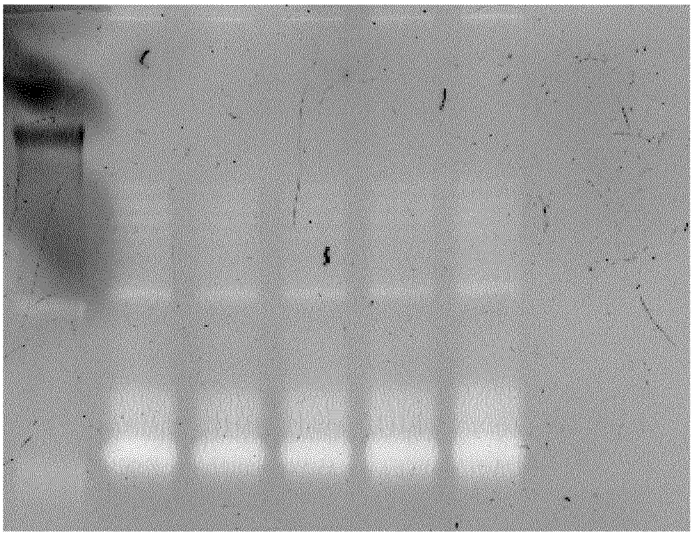

Fig.65

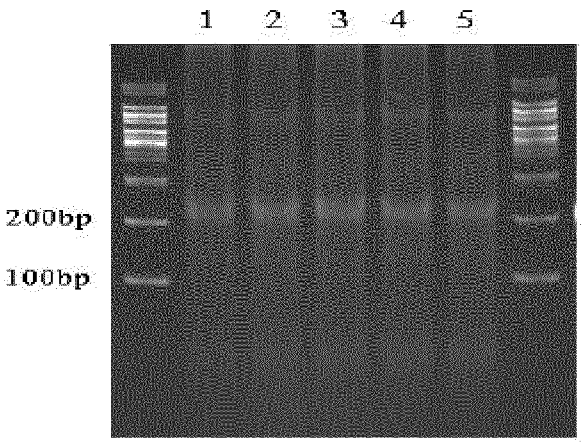
1
2
3
4
5
200bp
100bp

Fig.66

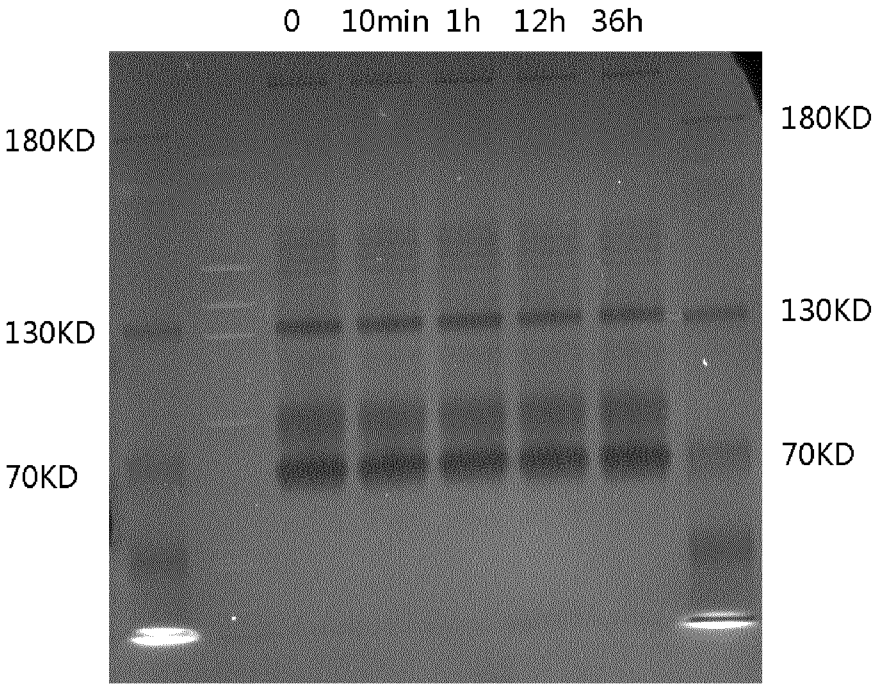
0
10min
1h
12h
36h
180KD
130KD
70KD
180KD
130KD
70KD

Fig.67

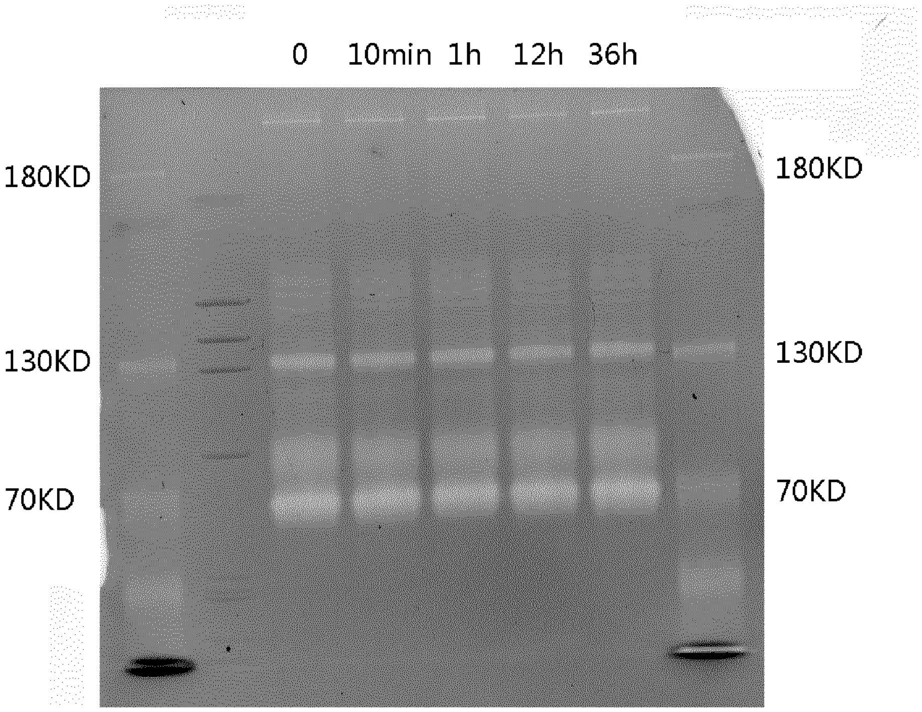
0
10min
1h
12h
36h
180KD
130KD
70KD
180KD
130KD
70KD

Fig.68

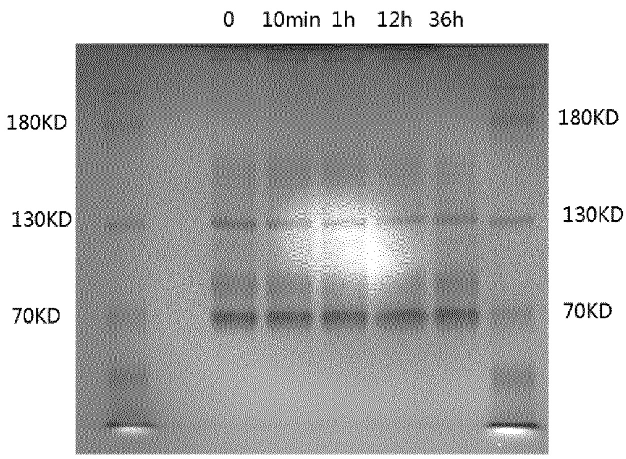
0
10min
1h
12h
36h
180KD
130KD
70KD
180KD
130KD
70KD

Fig.69

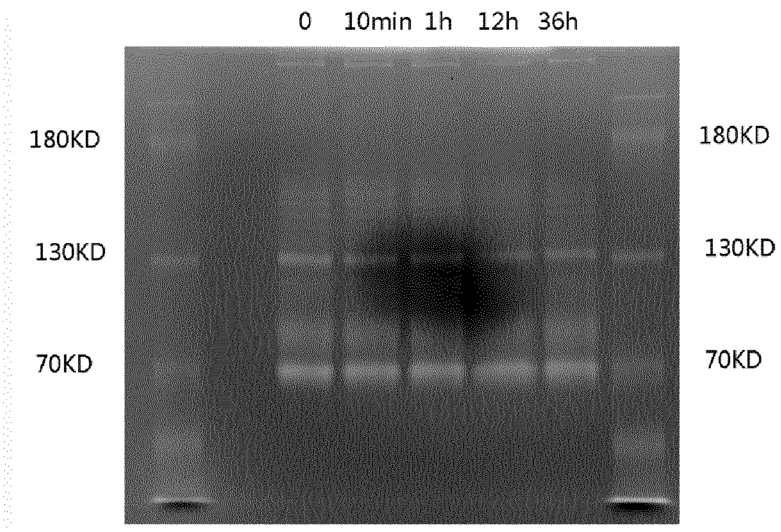
0
10min
1h
12h
36h
180KD
130KD
70KD
180KD
130KD
70KD

Fig.70

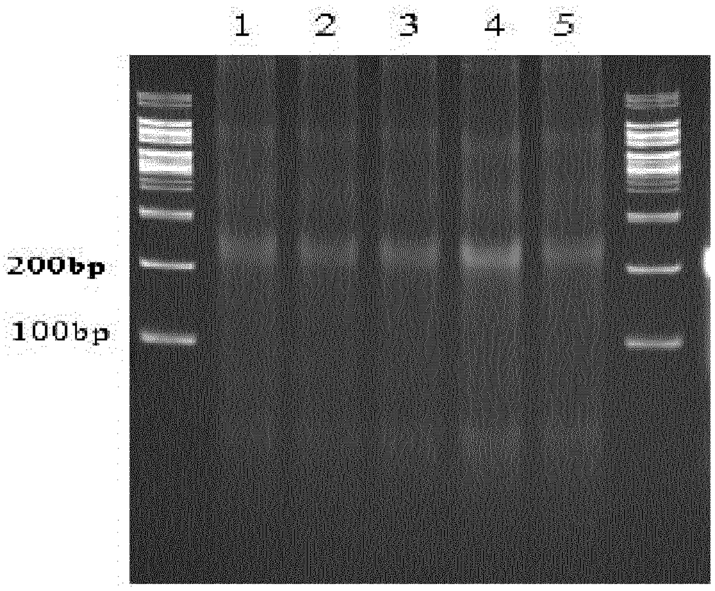
1
2
3
4
5
200bp
100bp

Fig.71

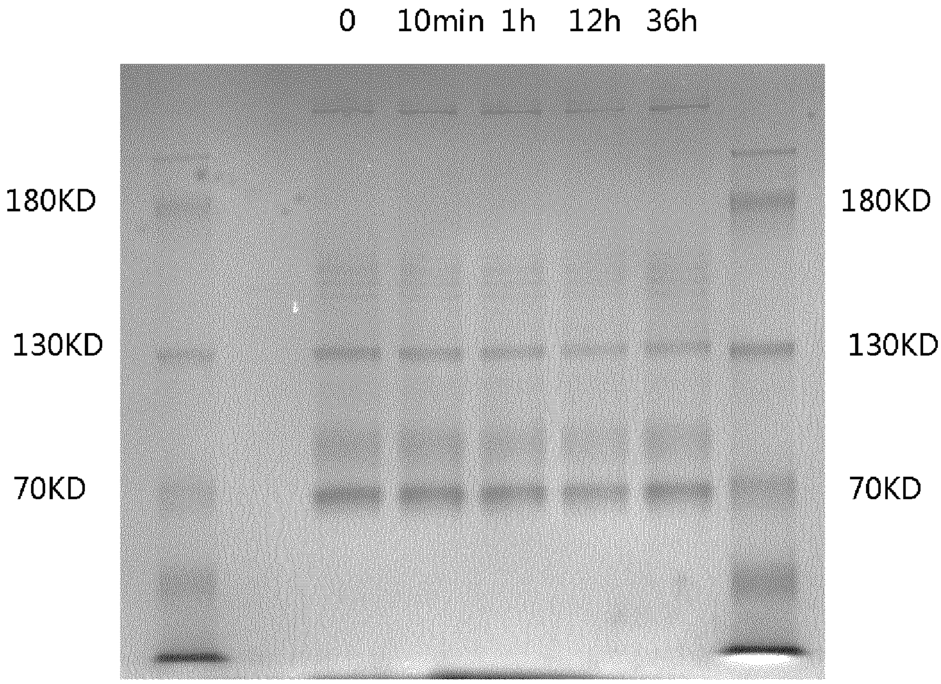
0 10min 1h 12h 36h
180KD
130KD
70KD
180KD
130KD
70KD

Fig.72

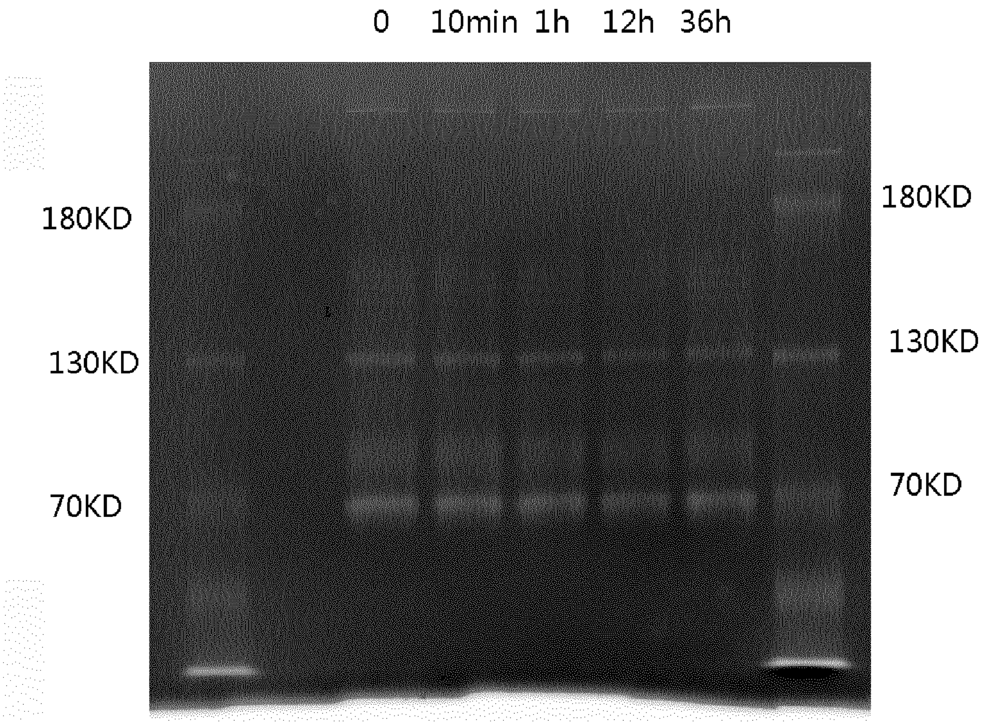
0 10min 1h 12h 36h
180KD
130KD
70KD
180KD
130KD
70KD

Fig.73

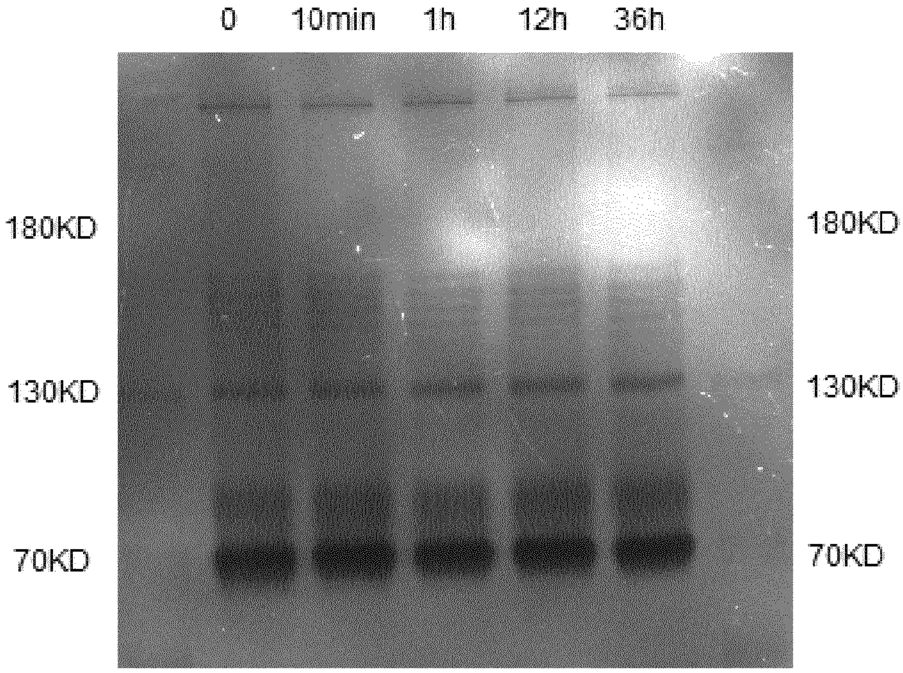
0
10min
1h
12h
36h
180KD
130KD
70KD
180KD
130KD
70KD

Fig.74

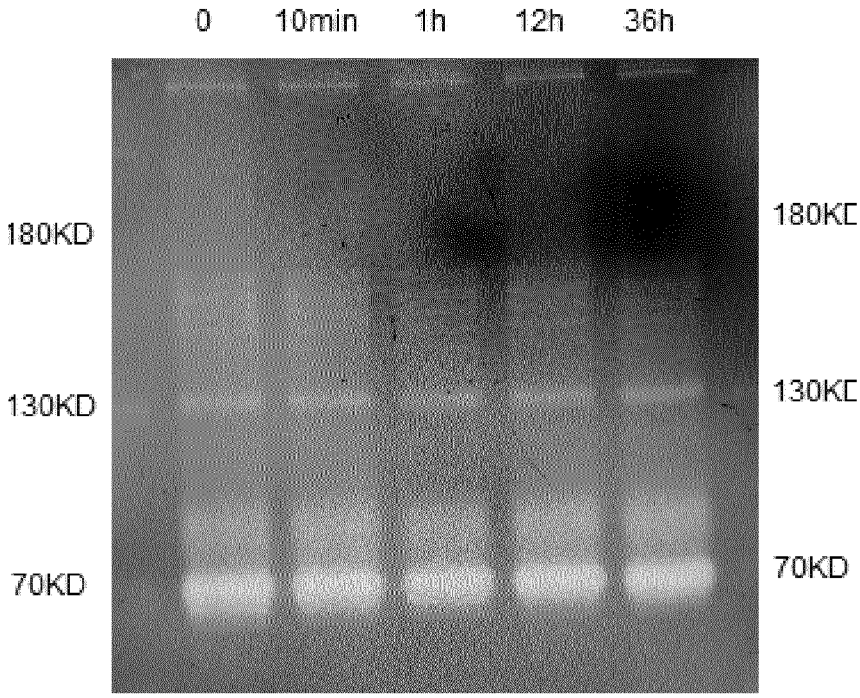
0
10min
1h
12h
36h
180KD
130KD
70KD
180KD
130KD
70KD

Fig.75

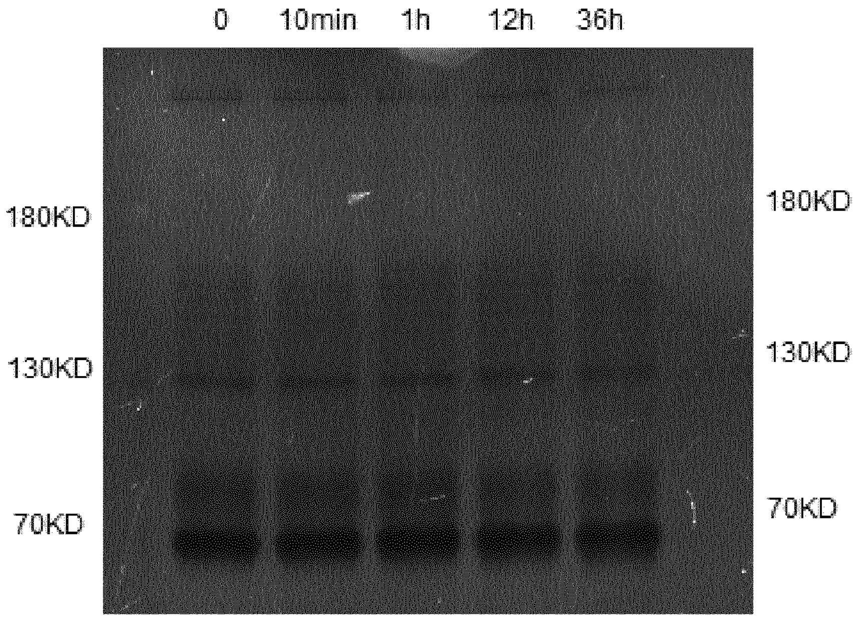
0
10min
1h
12h
36h
180KD
130KD
70KD
180KD
130KD
70KD

Fig.76

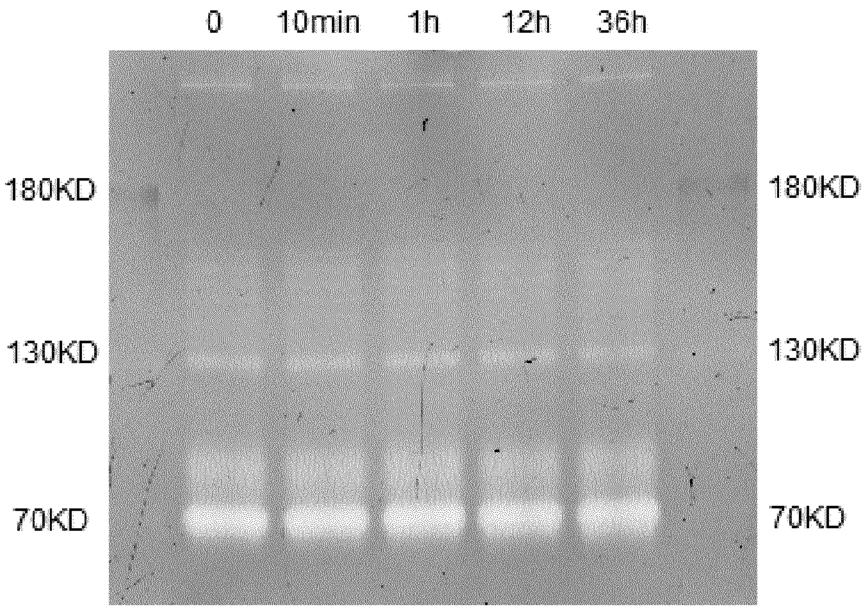
0
10min
1h
12h
36h
180KD
130KD
70KD
180KD
130KD
70KD

Fig.77

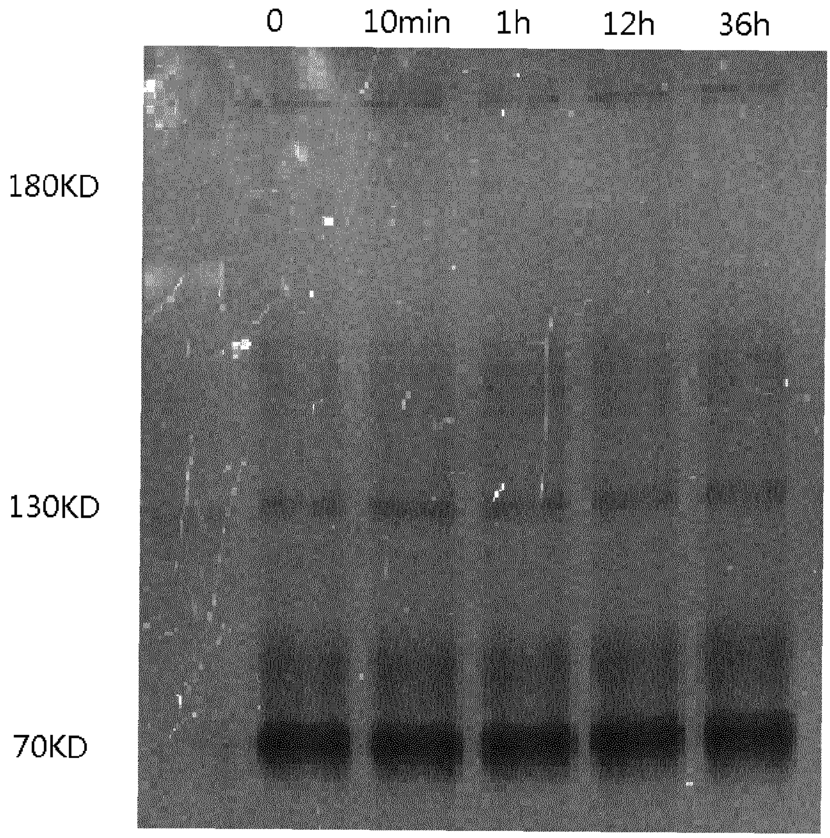
0
10min
1h
12h
36h
180KD
130KD
70KD

Fig.78

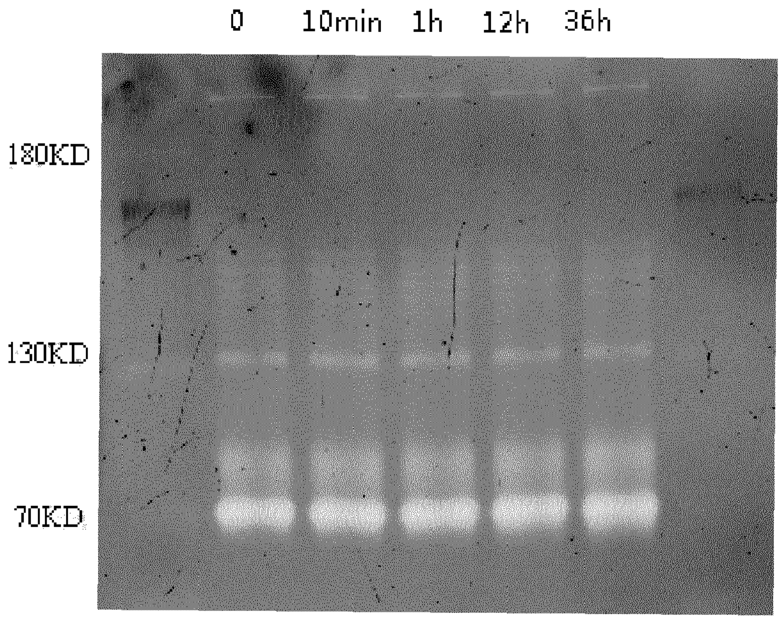
0
10min
1h
12h
36h
180KD
130KD
70KD

Fig.79

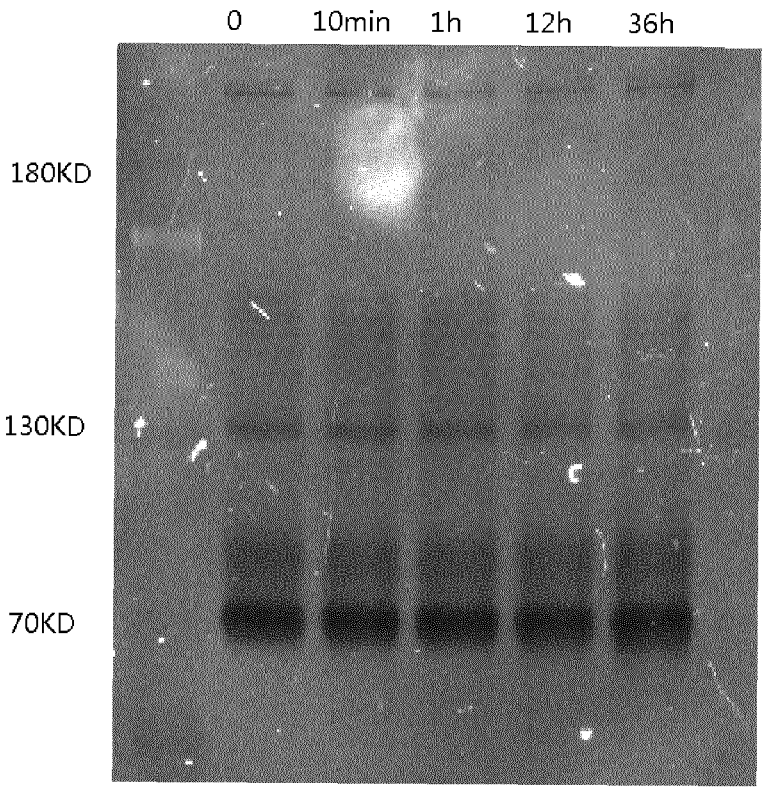
0
10min
1h
12h
36h
180KD
130KD
70KD

Fig.80

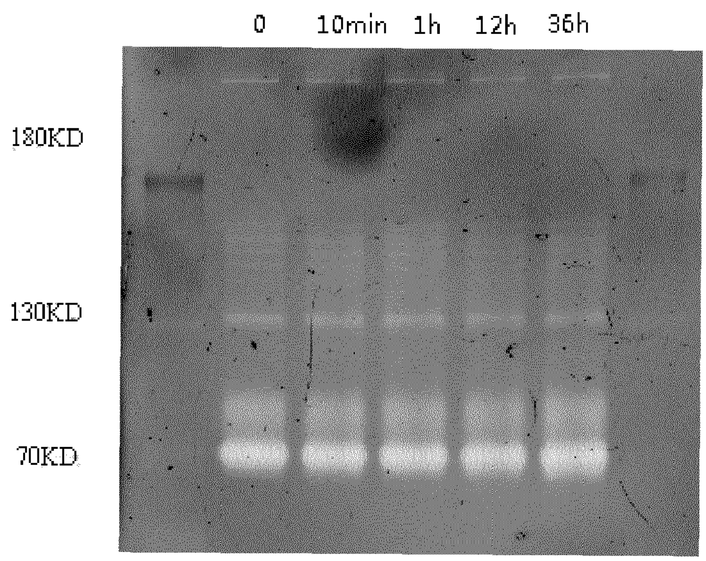
0
10min
1h
12h
36h
180KD
130KD
70KD

Fig.81

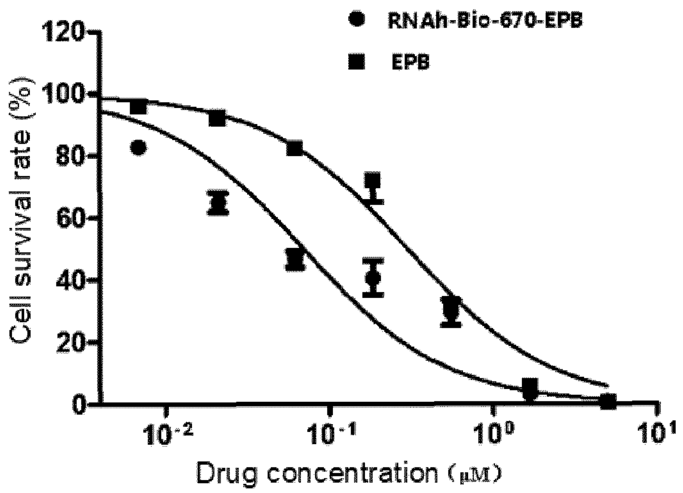
RNAh-Bio-670-EPB
EPB
Cell survival rate (%)
120
100
80
60
40
20
0
$10^{-2}$
$10^{-1}$
$10^{0}$
$10^{1}$
Drug concentration (μM)

Fig.82

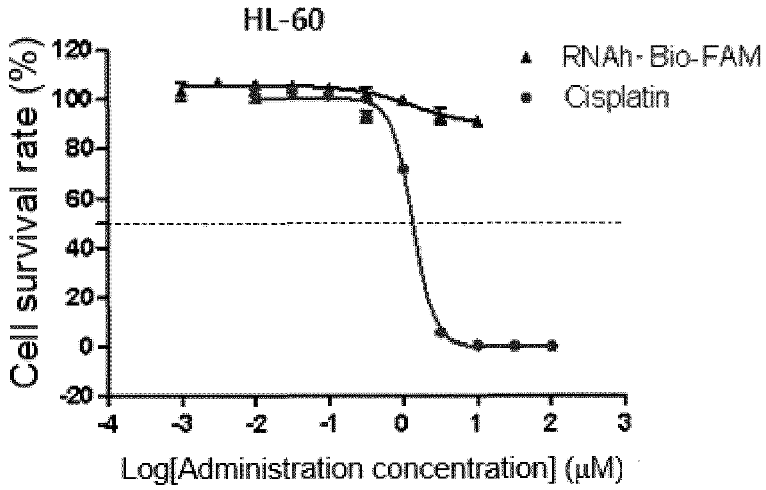
HL-60
RNAh- Bio-FAM
Cisplatin
Cell survival rate (%)
120
100
80
60
40
20
0
-20
-4
-3
-2
-1
0
1
2
3
Log[Administration concentration] (μM)

Fig.83

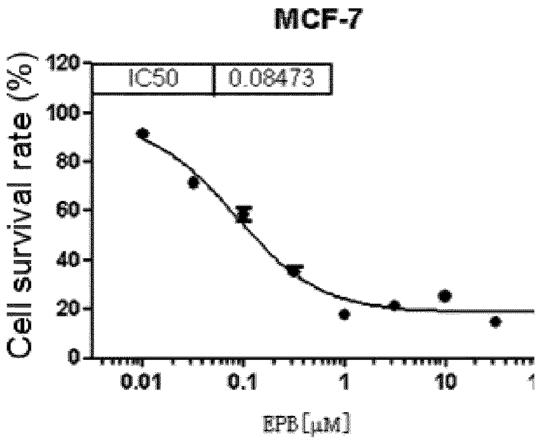


Fig.84a

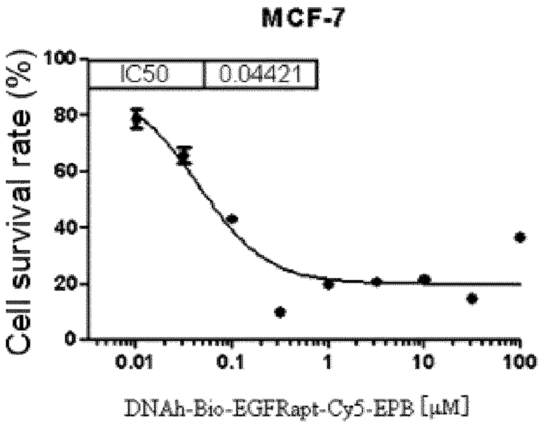


Fig.84b

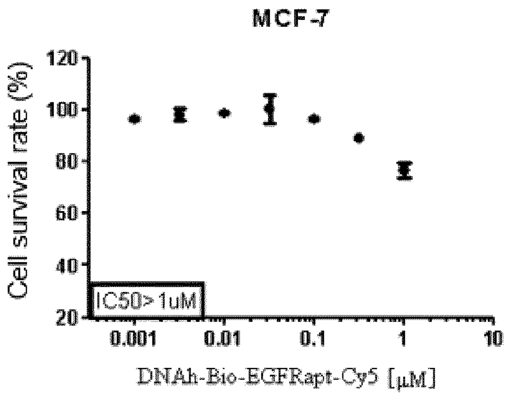


Fig.84c

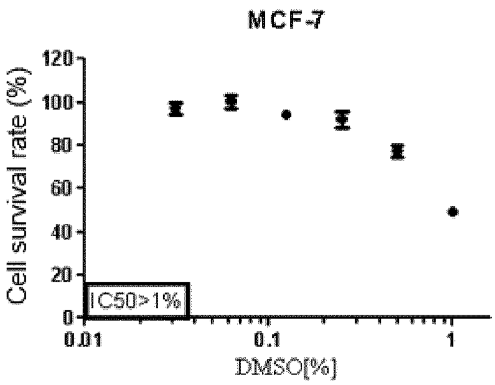


Fig.84d

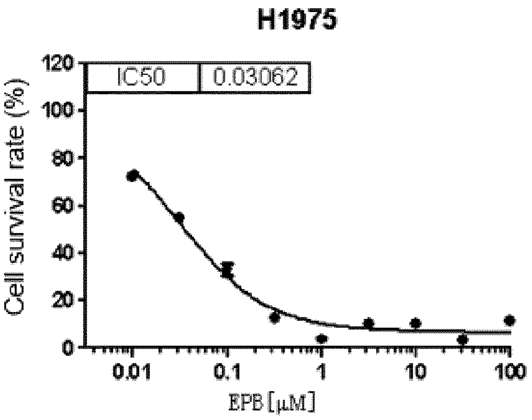


Fig.85a

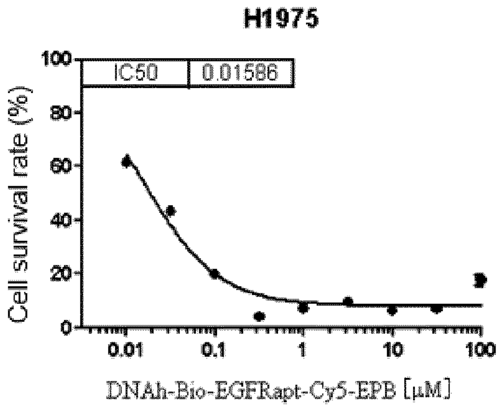


Fig.85b

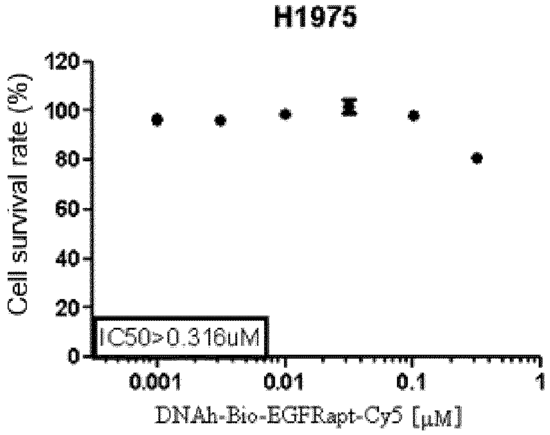


Fig.85c

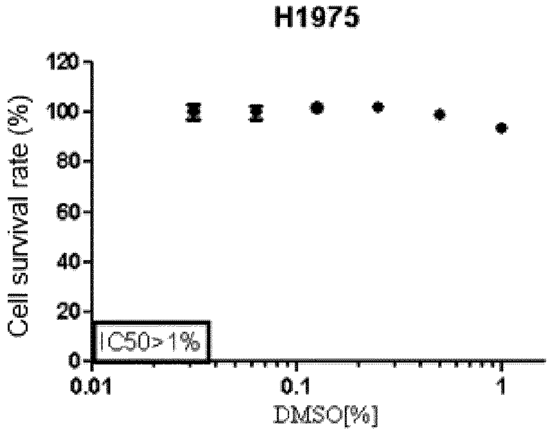


Fig.85d

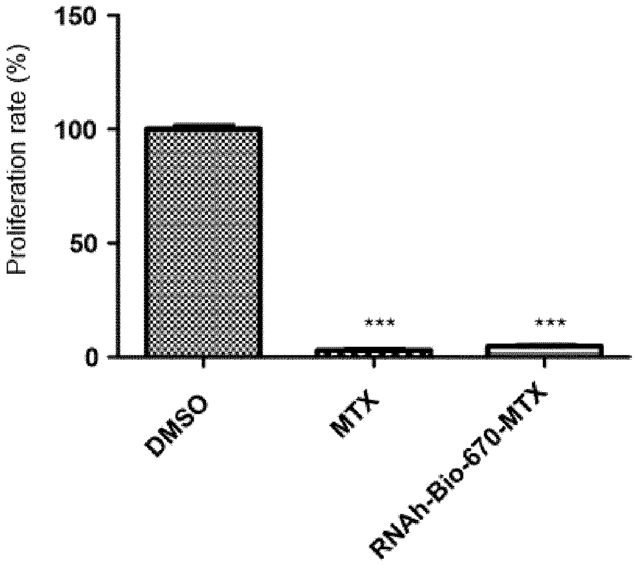


Fig.86

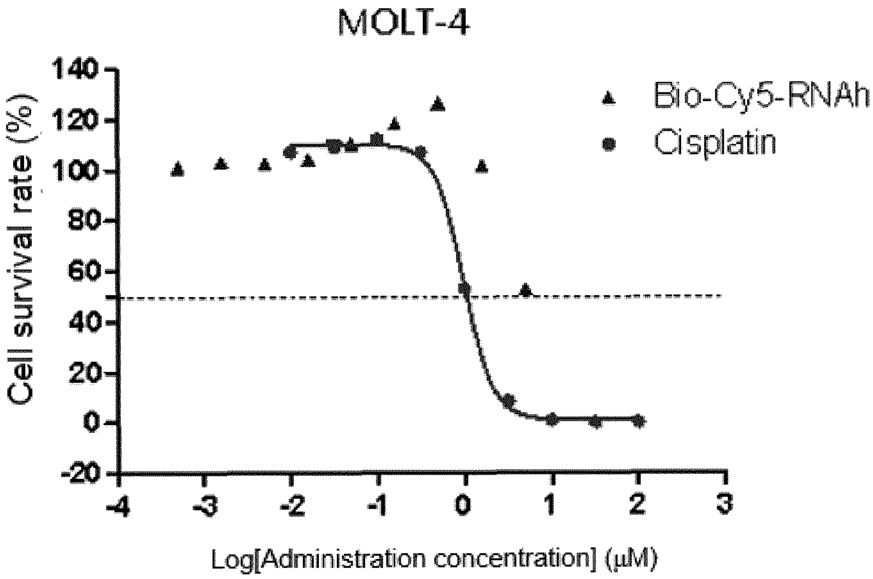
MOLT-4
Bio-Cy5-RNAh
Cisplatin
Cell survival rate (%)
140
120
100
80
60
40
20
0
-20
-4
-3
-2
-1
0
1
2
3
Log[Administration concentration] (μM)

Fig.87

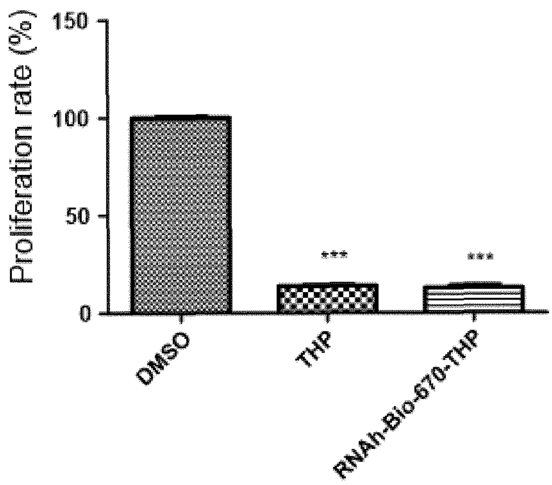
Proliferation rate (%)
150
100
50
0
***
***
DMSO
THP
RNAh-Bio-670-THP

Fig.88

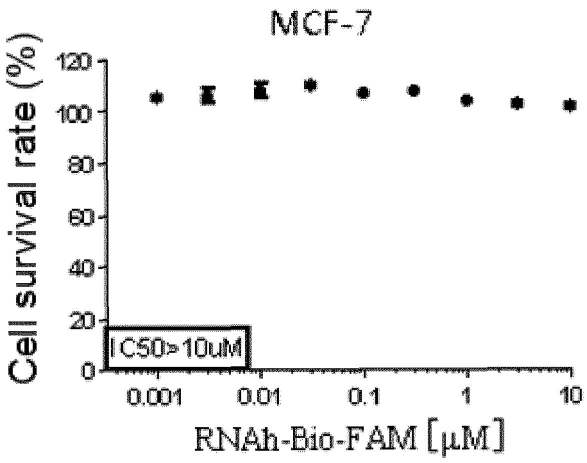


Fig.89

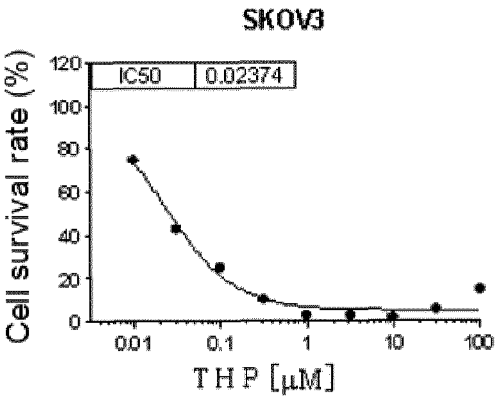


Fig.90a

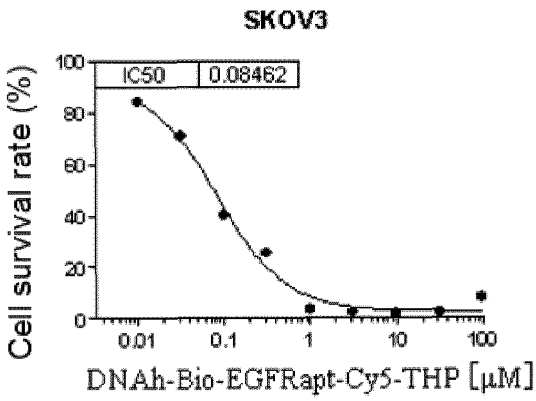


Fig.90b

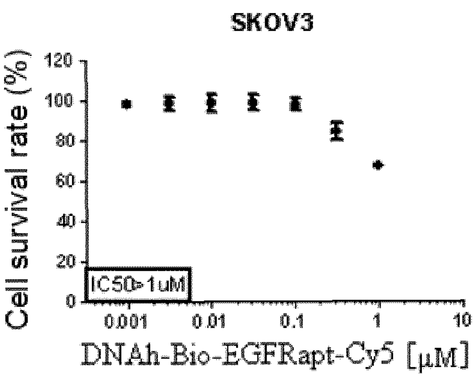


Fig.90c

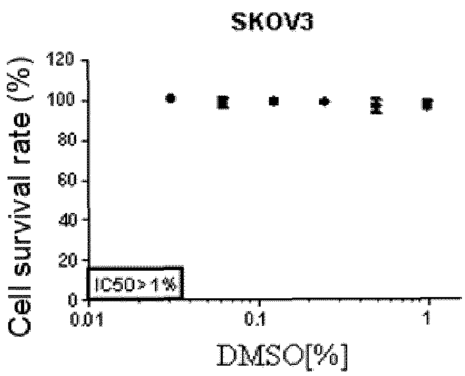


Fig.90d

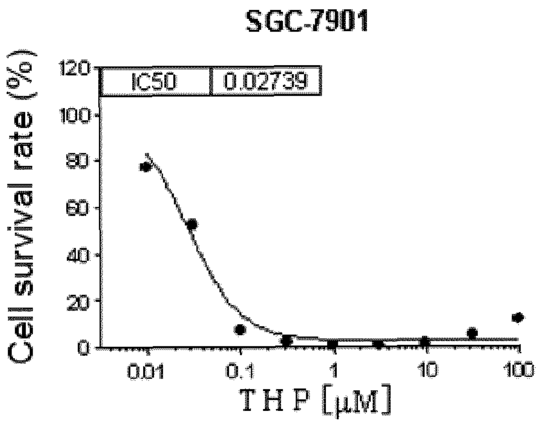


Fig.91a

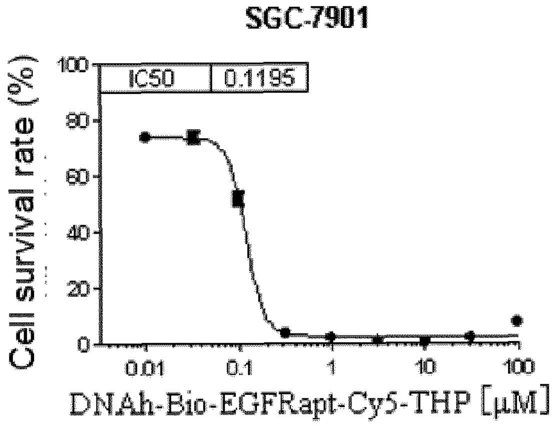


Fig.91b

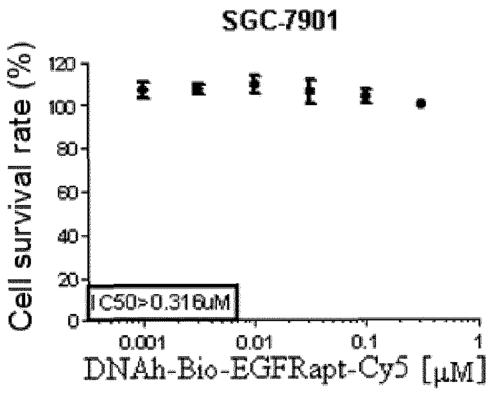


Fig.91c

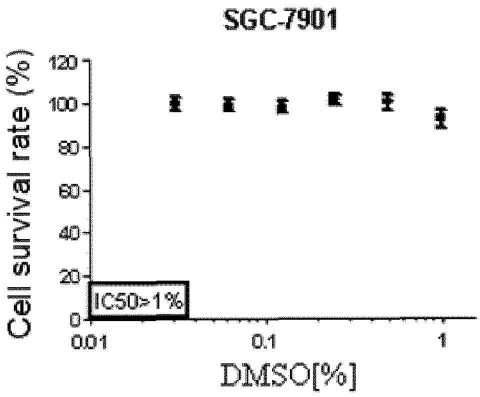


Fig.91d

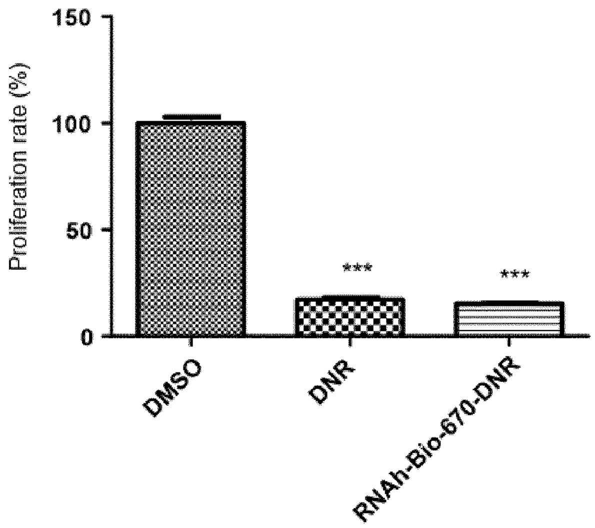
150
100
50
0
Proliferation rate (%)
***
***
DMSO
DNR
RNAh-Bio-670-DNR

Fig.92

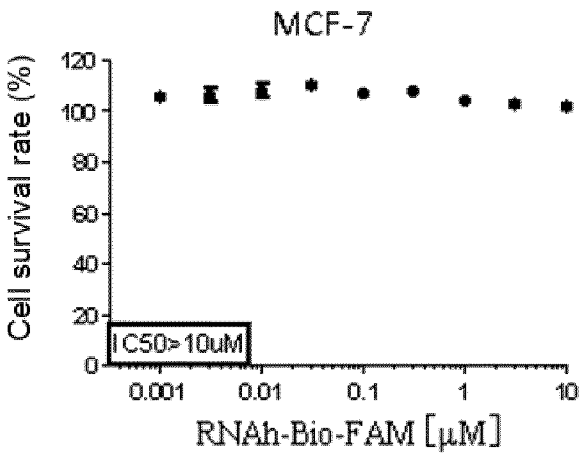
MCF-7
120
100
80
60
40
20
0
Cell survival rate (%)
IC50>10uM
0.001
0.01
0.1
1
10
RNAh-Bio-FAM [μM]

Fig.93

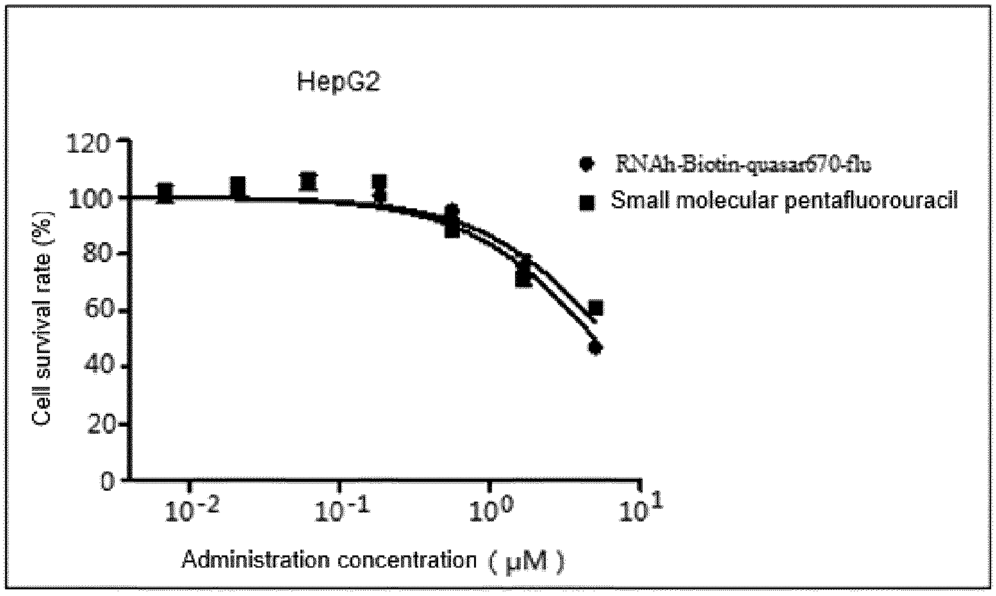
HepG2
RNAh-Biotin-quasar670-flu
Small molecular pentafluorouracil
120
100
80
60
40
20
0
Cell survival rate (%)
$10^{-2}$
$10^{-1}$
$10^{0}$
$10^{1}$
Administration concentration ( μM )

Fig.94

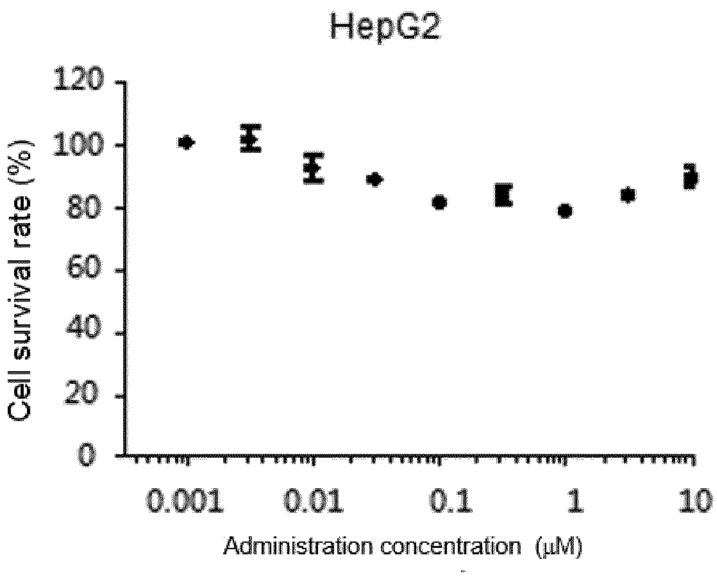
HepG2
120
100
80
60
40
20
0
Cell survival rate (%)
0.001
0.01
0.1
1
10
Administration concentration (μM)

Fig.95

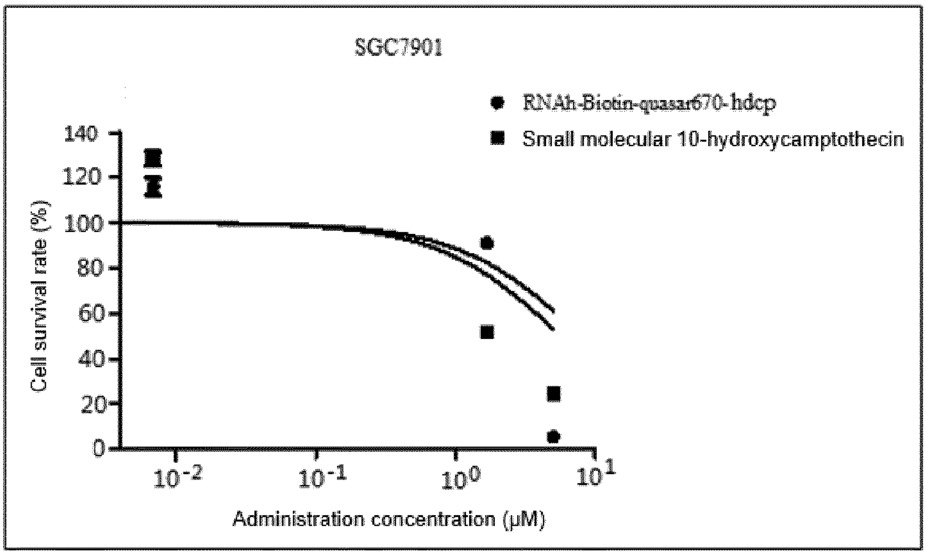
SGC7901
RNAh-Biotin-quasar670-hdcp
Small molecular 10-hydroxycamptothecin
Cell survival rate (%)
140
120
100
80
60
40
20
0
$10^{-2}$
$10^{-1}$
$10^{0}$
$10^{1}$
Administration concentration (μM)

Fig.96

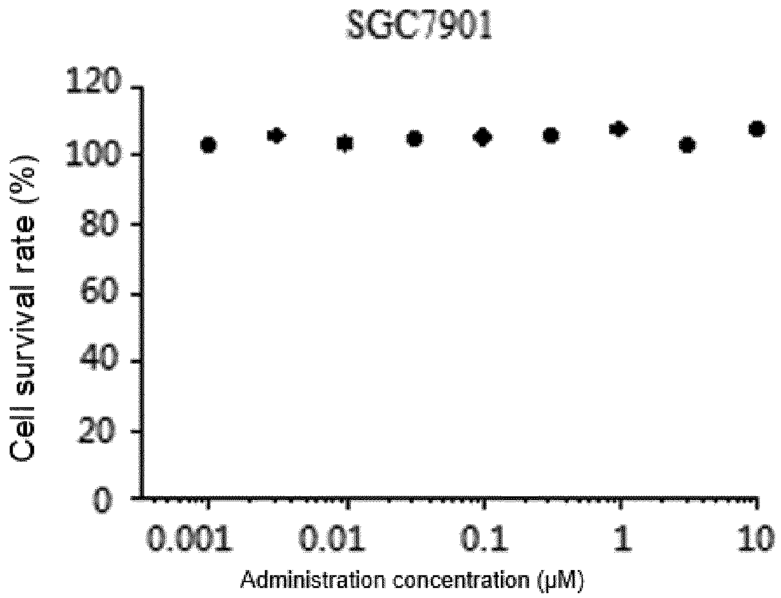
SGC7901
Cell survival rate (%)
120
100
80
60
40
20
0
0.001
0.01
0.1
1
10
Administration concentration (μM)

Fig.97

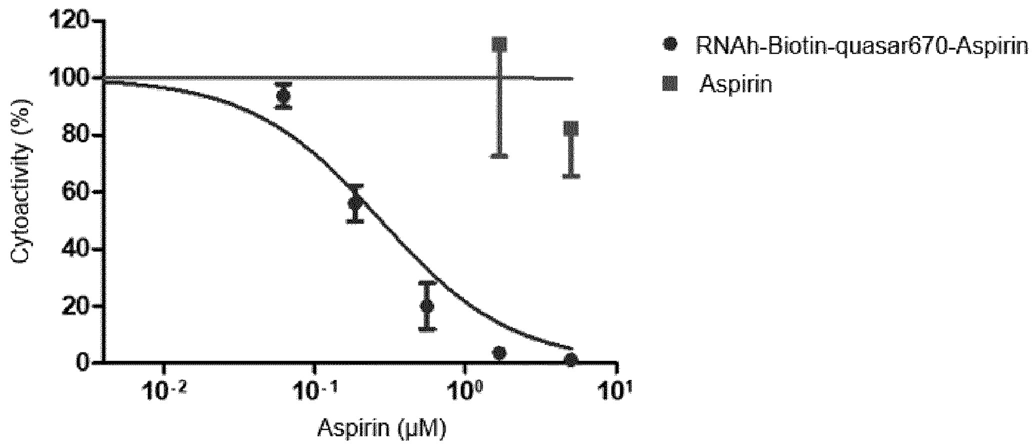
120
100
80
60
40
20
0
Cytoactivity (%)
$10^{-2}$
$10^{-1}$
$10^{0}$
$10^{1}$
Aspirin (μM)
RNAh-Biotin-quasar670-Aspirin
Aspirin

Fig.98

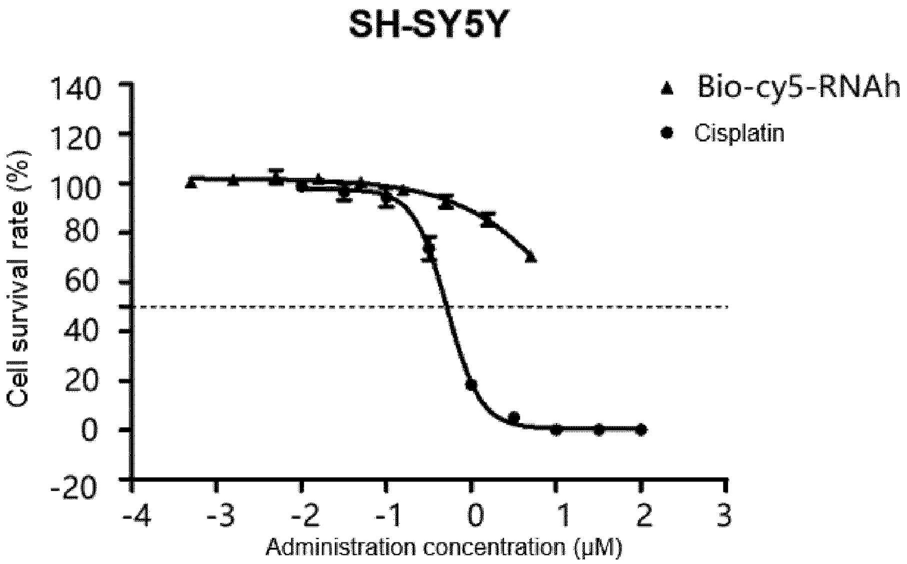
SH-SY5Y
140
120
100
80
60
40
20
0
-20
Cell survival rate (%)
-4
-3
-2
-1
0
1
2
3
Administration concentration (μM)
Bio-cy5-RNAh
Cisplatin

Fig.99

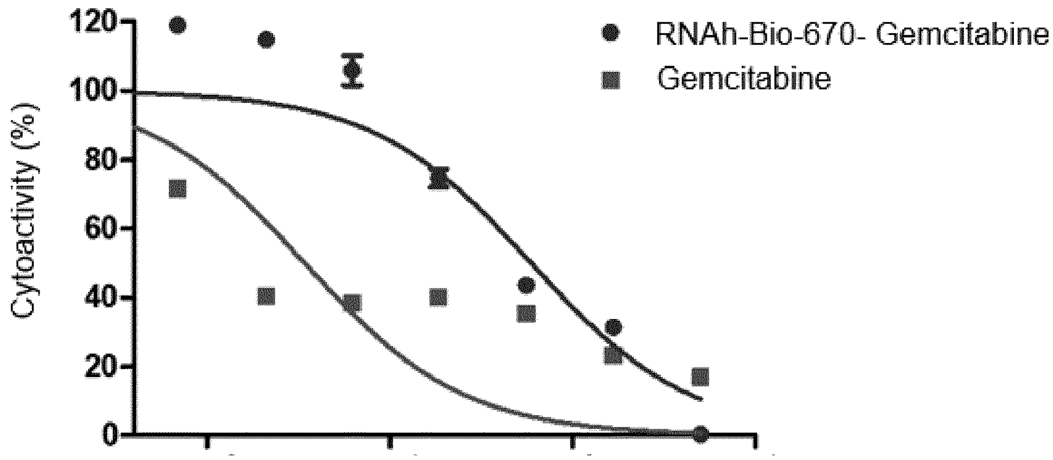
120
100
80
60
40
20
0
Cytoactivity (%)
RNAh-Bio-670- Gemcitabine
Gemcitabine

Fig.100

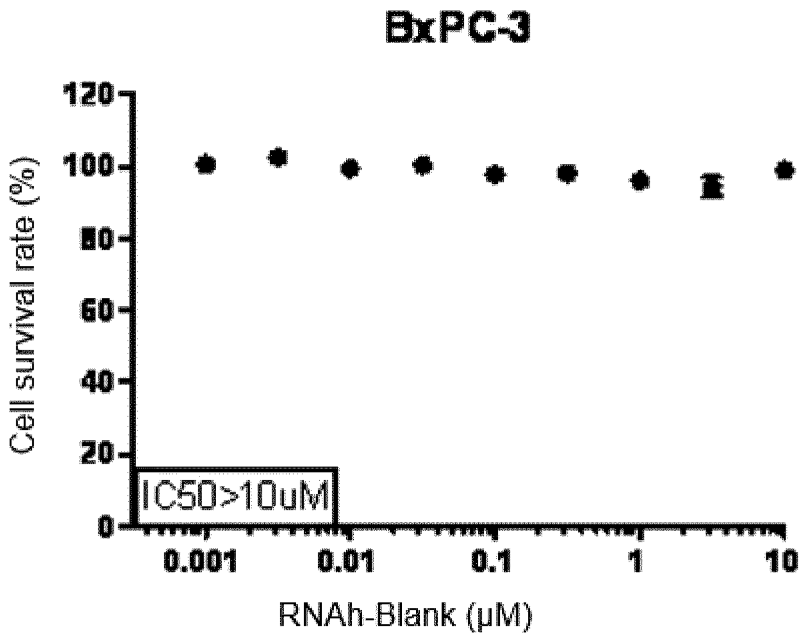


Fig.101

**INTERNATIONAL SEARCH REPORT**

International application No.
**PCT/CN2019/109563**

**A. CLASSIFICATION OF SUBJECT MATTER**

C07H 21/00(2006.01)i; A61K 48/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07H A61K A61P

Documentation searched other than minimum documentation to the extent that such documents are included in the fields searched

Electronic data base consulted during the international search (name of data base and, where practicable, search terms used)

CPEA, PLABS, LEXIS, AUABS, DWPI, FRABS, ILABS, SIPOABS, SGABS, CNABS, CNTXT, USTXT, EPTXT, WOTXT, CNKI, 万方学术数据库, WANFANG, baidu学术, BAIDU, 中国专利生物序列检索系统, China Patents Biological Sequence Search System, NCBI, ScienceDirect, Springer, ELSEVIER, NCBI, EMBL: 王力源, 王萌, 马润林, 郭培宣, 百药智达, PEIXUAN GUO, RUNLIN MA, self-assembly, RNA, rna nano+, rna nanoparticles, drug, siRNA, doxorubicin, t-shaped, 3wj, SEQ ID NOs: 1-6/52-54

**C. DOCUMENTS CONSIDERED TO BE RELEVANT**

| Category* | Citation of document, with indication, where appropriate, of the relevant passages | Relevant to claim No. |
|---|---|---|
| X | CN 103403189 A (UNIVERSITY OF CINCINNATI) 20 November 2013 (2013-11-20) claims 1-13, 15, 17, 18, 27, and 28, and descroption, paragraphs 0148, 0155, 0189-0197, and 0207, and figures 10E and 10G | 1-20 |
| X | CN 107614685 A (UNIVERSITY OF KENTUCKY RESEARCH FOUNDATION) 19 January 2018 (2018-01-19) claims 1-56, 119, and 127, and description, paragraphs 0009-0019, 0021, 0024, 0032, 0038, 0039, 0041, 0068, 0069, and 0075-0089, figures 2, 4, 12, 18, 19, and 21, and embodiments 1-3 | 1-20 |
| X | GUO, S. J. et al. "Methods for construction and characterization of simple or special multifunctional RNA nanoparticles based on the 3WJ of phi29 DNA packaging motor" *Methods,* Vol. 143, 09 March 2018 (2018-03-09), abstract, p. 124, right column 3.1, p. 127, left column 4.1 to p. 131, 4.8, and figures 2 and 4-6 | 1-20 |
| X | CN 104327141 A (SHANGHAI JIAOTONG UNIVERSITY) 04 February 2015 (2015-02-04) claims 1-11, description, paragraphs 0010-0022, embodiments 1 and 2, and figure 1 | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 December 2019** | **27 December 2019** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/109563**

**C. DOCUMENTS CONSIDERED TO BE RELEVANT**

| Category* | Citation of document, with indication, where appropriate, of the relevant passages | Relevant to claim No. |
|---|---|---|
| X | WO 2016/145008 A2 (UNIVERSITY OF KENTUCKY RESEARCH FOUNDATION) 15 September 2016 (2016-09-15)<br>claims 1-51 | 1-20 |
| X | WO 2016/145005 A1 (UNIVERSITY OF KENTUCKY RESEARCH FOUNDATION) 15 September 2016 (2016-09-15)<br>claims 1-51 | 1-20 |
| X | PI, F.M. et al. "RNA Nanoparticles Harboring Annexin A2 Aptamer Can Target Ovarian Cancer for Tumor-Specific Doxorubicin Delivery, Author manuscript" *Nanomedicine,* Vol. 13, No. 3, 01 April 2018 (2018-04-01),<br>abstract, p. 3, part "Methods" to p. 10, paragraph 1, and figures 1 and 2 | 1-20 |

Form PCT/ISA/210 (second sheet) (January 2015)

**INTERNATIONAL SEARCH REPORT**
**Information on patent family members**

International application No.
**PCT/CN2019/109563**

| Patent document cited in search report | | | Publication date (day/month/year) | Patent family member(s) | | | Publication date (day/month/year) |
|---|---|---|---|---|---|---|---|
| CN | 103403189 | A | 20 November 2013 | US | 9297013 | B2 | 29 March 2016 |
| | | | | WO | 2012170372 | A2 | 13 December 2012 |
| | | | | WO | 2012170372 | A3 | 18 April 2013 |
| | | | | CN | 103403189 | B | 25 November 2015 |
| | | | | US | 2014179758 | A1 | 26 June 2014 |
| CN | 107614685 | A | 19 January 2018 | WO | 2016168784 | A2 | 20 October 2016 |
| | | | | WO | 2016168784 | A3 | 24 November 2016 |
| | | | | US | 2018092997 | A1 | 05 April 2018 |
| CN | 104327141 | A | 04 February 2015 | CN | 104327141 | B | 04 August 2017 |
| WO | 2016/145008 | A2 | 15 September 2016 | CN | 107429251 | A | 01 December 2017 |
| | | | | US | 2019119682 | A1 | 25 April 2019 |
| | | | | WO | 2016145008 | A3 | 03 November 2016 |
| WO | 2016/145005 | A1 | 15 September 2016 | US | 2019106452 | A1 | 11 April 2019 |
| | | | | CN | 107531740 | A | 02 January 2018 |

Form PCT/ISA/210 (patent family annex) (January 2015)